# EUROPEAN PATENT APPLICATION

(11) **EP 4 458 830 A1**
(43) Date of publication of application: **06.11.2024**
(21) Application number: 22914375.5
(22) Date of filing: 20.12.2022
(51) Int. Cl.: C07D 495/14, A61K 31/381, A61K 31/4353, A61P 35/00

(54) **TRICYCLIC COMPOUND, METHOD FOR PREPARING SAME, AND USE THEREOF**

(30) Priority: 30.12.2021 CN 202111666009; 29.04.2022 CN 202210467340
(71) Applicant: Sichuan Kelun-Biotech Biopharmaceutical Co., Ltd., Chengdu, Sichuan 611138 (CN)
(72) Inventor: LIU, Jinming, Chengdu, Sichuan 611138 (CN); REN, Yun, Chengdu, Sichuan 611138 (CN); LI, Xiaoyong, Chengdu, Sichuan 611138 (CN); YUAN, Xiaoxi, Chengdu, Sichuan 611138 (CN); ZHANG, Yungang, Chengdu, Sichuan 611138 (CN); TIAN, Qiang, Chengdu, Sichuan 611138 (CN); SONG, Hongmei, Chengdu, Sichuan 611138 (CN); GE, Junyou, Chengdu, Sichuan 611138 (CN)
(74) Representative: Mewburn Ellis LLP
(86) International application number: PCT/CN2022/140196
(87) International publication number: WO 2023/125121

(57) **Abstract**

The present invention belongs to the field of pharmaceuticals, and particularly relates to a tricyclic compound, a method for preparing same, and use thereof. In particular, the present invention relates to a compound represented by formula (I) or a pharmaceutically acceptable salt, ester, stereoisomer, tautomer, polymorph, solvate, *N*-oxide, isotope-labeled compound, metabolite or prodrug thereof, a method for preparing same, a pharmaceutical composition comprising same, and uses thereof.

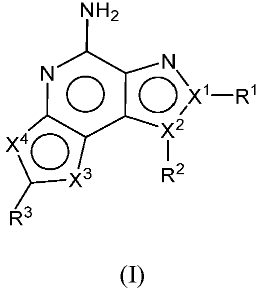

## Description

The present application is based on the application with CN application number 202111666009.2 and the application date of December 30, 2021, and the application with CN application number 202210467340.X and the application date of April 29, 2022, and claims their priorities. The disclosure of these CN applications is hereby incorporated into the present application in their entirety.

### Technical Field

The present invention belongs to the field of medicinal chemistry, and in particular relates to a tricyclic compound or pharmaceutically acceptable salt, ester, stereoisomer, tautomer, polymorph, solvate, *N*-oxide, isotope-labeled compound, metabolite or prodrug thereof, a method for preparing the same, a pharmaceutical composition containing the same, and use thereof.

### Background Art

Toll-like receptors (Toll-like receptors, TLRs) belong to the pattern recognition receptor (PRR) family, which can specifically recognize pathogen-associated molecular patterns (PAMP), is an important class of proteins involved in non-specific immunity, and is a bridge between nonspecific and specific immunity. So far, 13 kinds of Toll-like receptors have been found in mammals, and 10 kinds (TLR1-10) have been found in humans, among which TLR1/2/4/5/6/10 are expressed on the cell surface and can quickly recognize the products of bacterial metabolism, TLR3/7/8/9 are expressed in endosomes, which mainly recognize and monitor exogenous nucleic acid substances in pathogen cells.

TLR7 has a limited expression distribution in humans, and is mainly expressed by B cells and plasmacytoid dendritic cells (pDCs). In response to viral infection, TLR7 induces high levels of interferon-α, and enhances costimulation and antigen presentation ability activation of pDCs, promotes the proliferation of CD4+ T cells and further activates CD8+ T cells to generate adaptive T cell responses. TLR8 expression is relatively more widespread, and mainly expressed by monocytes, NK cells and myeloid dendritic cells (mDC) in humans. Stimulation of TLR8 can induce the release of various proinflammatory cytokines, such as tumor necrosis factor-α (TNF-α), IL-6, IL-12, and interferon-γ. At the same time, the TLR8 signaling pathway has also been proved to be a necessary and sufficient condition for reversing the suppressive function of Tregs cells. Therefore, TLR7 and TLR8 both play an important role in the body's innate immunity and acquired immunity, and are popular targets for anticancer and antiviral immunotherapy. At present, there have been reports involving multiple TLR7 and/or TLR8 agonists from different companies, but there is still a great demand for TLR7 and/or TLR8 agonists with better activity, safety and physicochemical properties.

At present, a new type of immune-stimulating antibody conjugate (ISAC) is emerging, which is formed by coupling an immunomodulator such as TLR7 and/or TLR8 agonist with a tumor-targeting antibody through a cleavable/non-cleavable linker. The immune-stimulating antibody conjugate combines the precise tumor localization of the antibody with the durable anti-tumor activity and immune memory efficacy of the immunomodulator such as TLR7 and/or TLR8 agonist, and safely triggers anti-tumor immune responses while achieving systemic administration.

Therefore, aiming at the above aspects, the present invention provides a class of tricyclic compounds with TLR7 and/or TLR8 agonistic activity. This type of compounds has better activity, safety and physical and chemical properties, and it can be further used in immune-stimulating antibody conjugates, which are of great significance for the development of antitumor and antiviral drugs.

### Contents of the Invention

In one aspect, the present application provides a class of tricyclic compounds, which has strong agonistic effect on TLR7 and/or TLR8, and therefore is better in use for prevention and/or treatment of a disease associated with the mediation of TLR7 and/or TLR8. The compounds also have various excellent properties, such as good physicochemical properties (e.g., solubility, physical and/or chemical stability) and good safety.

The compound is a compound of Formula (I) or a pharmaceutically acceptable salt, ester, stereoisomer, tautomer, polymorph, solvate, *N*-oxide, isotope-labeled compound, metabolite or prodrug thereof: 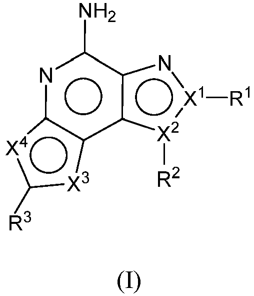 wherein,
X¹ is selected from the group consisting of N and C;
X² is selected from the group consisting of N and C;
and at least one of X¹ and X² is selected from N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is hydrogen, or has the formula of -L¹-L²-L³-L⁴;
L¹ is selected from the group consisting of a covalent bond and C₁₋₆ alkylene;
L² is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L³ is selected from the group consisting of a covalent bond, -O-, -S-, -C(O)-, -O-C(O)-, - C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ₘ-NR⁵-, -NR⁵-S(O)_{M}- and -NR⁵-S(O)ₘ-NR⁵-;
L⁴ is selected from the group consisting of hydrogen, C₁₋₆ alkyl, -(O-CH₂CH₂-)ₙO-C₁₋₆ alkyl and -C₁₋₆ alkylene-(O-CH₂CH₂-)ₙO-C₁₋₆ alkyl, optionally, each alkyl group is independently substituted with one or more groups independently selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R³ is hydrogen, or has the formula of -L⁵-L⁶-L⁷-L⁸;
L⁵ is selected from the group consisting of a covalent bond and C₁₋₆ alkylene;
L⁶ is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L⁷ is selected from the group consisting of a covalent bond, -O-, -S-, -C(O)-, -O-C(O)-, - C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L⁸ is selected from the group consisting of hydrogen, C₁₋₆ alkyl, -(O-CH₂CH₂-)_{q}O-C₁₋₆ alkyl and -C₁₋₆ alkylene-(O-CH₂CH₂-)_{q}O-C₁₋₆ alkyl, optionally, each alkyl group is substituted with one or more groups independently selected from the group consisting of hydrogen, halogen, hydroxyl, -NH₂, -NH-C(O)-O-C₁₋₆ alkyl, C₁₋₆ alkoxy, carboxyl and - C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently selected from the group consisting of hydrogen and C₁₋₆ alkyl;
each R⁶ is independently selected from the group consisting of hydrogen and C₁₋₆ alkyl;
each m is independently 1 or 2;
each n is independently selected from the group consisting of integers 1 to 25;
each p is independently 1 or 2;
each q is independently selected from the group consisting of integers 1 to 25.

The present application further provides the following drug-linker or a pharmaceutically acceptable salt, ester, stereoisomer, tautomer, polymorph, solvate, *N*-oxide, isotope-labeled compound, metabolite or prodrug thereof: D-L'
wherein, D is a fragment of the compounds described in any one of the first aspect;
L' is a linker.

The present application also provides an immune-stimulating antibody conjugate described in Formula (ISAC-I) or a pharmaceutically acceptable salt, ester, stereoisomer, tautomer, polymorph, solvate, *N*-oxides, isotope-labeled compound, metabolite or prodrug thereof: 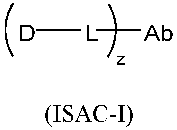 wherein,
D is a fragment of the compound described in any one of the first aspect;
L is a fragment of the linker L', wherein L' is as defined in the second aspect;
Ab is an antibody capable of targeting a target antigen, optionally, the antibody is modified (e.g., modified by a cross-linking agent);
z is selected from the group consisting of 1 to 10.

In another aspect, the present application provides use of the compound of Formula (I) in the manufacture of a drug-linker compound.

In another aspect, the present application provides use of the compound of Formula (I) and/or the drug-linker in the manufacture of an immune-stimulating antibody conjugate.

In another aspect, the present application provides a method for preparing the immune-stimulating antibody conjugate, which comprises a step of using the compound of Formula (I) and/or the drug-linker.

In another aspect, the application provides a pharmaceutical composition, comprising a prophylactically and/or therapeutically effective amount of the compound or pharmaceutically acceptable salt, ester, stereoisomer, tautomer, polymorph, solvate, N-oxide, isotope-labeled compound, metabolite or prodrug thereof as described in the present application, or the drug-linker or pharmaceutically acceptable salt, ester, stereoisomer, tautomer, polymorph, solvate, N-oxides, isotope-labeled compound, metabolite or prodrug thereof, or the immune-stimulating antibody conjugate described in Formula (ISAC-I), and one or more pharmaceutically acceptable carriers.

In another aspect, the present application provides a kit, comprising the compound of Formula (I) or pharmaceutically acceptable salt, ester, stereoisomer, tautomer, polymorph, solvate, *N*-oxide, isotope-labeled compound, metabolite or prodrug thereof, or the drug-linker or pharmaceutically acceptable salt, ester, stereoisomer, tautomer, polymorph, solvate, *N*-oxide, isotope-labeled compound, metabolite or prodrug thereof, or the immune-stimulating antibody conjugate of Formula (ISAC-I), or the pharmaceutical composition.

in another aspect, the application provides use of the compound of Formula (I) or pharmaceutically acceptable salt, ester, stereoisomer, tautomer, polymorph, solvate, *N*-oxide, isotope-labeled compound, metabolite or prodrug thereof, or the immune-stimulating antibody conjugate of Formula (ISAC-I), or the drug-linker or pharmaceutically acceptable salt, ester, stereoisomer isomer, tautomer, polymorph, solvate, N-oxide, isotope-labeled compound, metabolite or prodrug thereof, or the pharmaceutical composition, in the manufacture of a medicament for the prevention and/or treatment of a TLR7 and/or TLR8-mediated disease (e.g., a tumor).

In another aspect, the present application provides a method for preventing and/or treating a disease mediated by TLR7 and/or TLR8 (e.g., a tumor), comprising a step of administering to a subject in need thereof an effective amount of the compound of Formula (I) or pharmaceutically acceptable salt, ester, stereoisomer, tautomer, polymorph, solvate, *N*-oxide, isotope-labeled compound, metabolite or prodrug thereof, or the immune-stimulating antibody conjugate of Formula (ISAC-I), or the drug-linker or pharmaceutically acceptable salt, ester, stereoisomer, tautomer, polymorph, solvate, *N*-oxide, isotope-labeled compound, metabolite or prodrug thereof, or the pharmaceutical composition.

In another aspect, the application provides a method of preparing the compounds described herein.

### Definitions

Unless defined otherwise hereinafter, all technical and scientific terms used herein have the same meaning as commonly understood by one of ordinary skill in the art. References to techniques used herein are intended to refer to techniques commonly understood in the art, including those variations of the techniques or substitutions of equivalent techniques that are obvious to those skilled in the art. While the following terms are believed to be well understood by those skilled in the art, the following definitions are set forth to better explain the present invention.

As used herein, the terms "including", "comprising", "having", "containing" or "involving" and other variations thereof herein are inclusive or open-ended, and do not exclude other unrecited elements or method steps.

As used herein, the term "alkyl" is defined as a straight or branched chain saturated aliphatic hydrocarbon group. For example, as used herein, the term "C₁₋₆ alkyl" refers to a straight or branched chain group having 1 to 6 carbon atoms (e.g., methyl, ethyl, n-propyl, isopropyl, n-butyl, isobutyl, sec-butyl, tert-butyl, n-pentyl or n-hexyl) optionally substituted with one or more (e.g., 1 to 3) suitable substituents such as halogen. The term "C₁₋₃ alkyl" refers to a straight or branched chain group (e.g., methyl, ethyl, n-propyl, isopropyl) having 1 to 3 carbon atoms, which is optionally substituted with one or more (e.g., 1 to 3) suitable substituents such as halogen.

As used herein, the term "alkoxy" refers to "alkyl-O-", in which the "alkyl" is as defined above. For example, the term "C₁₋₆ alkoxy" refers to "C₁₋₆ alkyl-O-", and the "C₁₋₆ alkyl" is as defined above. The "C₁₋₃ alkoxy" in the present invention refers to "C₁₋₃ alkyl-O-", and the "C₁₋₃ alkyl" is as defined above. Exemplary C₁₋₆ alkoxy groups include methoxy, ethoxy, n-propoxy, isopropoxy, n-butoxy, isobutoxy, sec-butoxy, tert-butoxy, n-pentoxy or n-hexyloxy.

The "C₁₋₆ alkylene" used in the present invention refers to a divalent group formed by removing two hydrogens from a linear or branched alkane containing 1 to 6 carbon atoms, including "C₁₋₅ alkylene ", "C₁₋₄ alkylene", "C₁₋₃ alkylene", "C₁₋₂ alkylene", specific examples include, but are not limited to: -CH₂-, -CH₂CH₂-, -CH₂CH₂CH₂-, -CH₂CH₂CH₂CH₂-, - CH₂CH₂CH₂CH₂CH₂-, -CH₂CH₂CH₂CH₂CH₂CH₂-, etc.

As used herein, the term "cycloalkyl" refers to a saturated or partially unsaturated non-aromatic monocyclic or polycyclic (e.g., bicyclic) hydrocarbon ring (e.g., monocyclic ring, such as cyclopropyl, cyclobutyl, cyclopentyl, cyclohexyl, cycloheptyl, cyclooctyl, cyclononyl, or bicyclic ring including spiro, fused or bridged system, such as bicyclo[1.1.1]pentyl, bicyclo[2.2.1]heptyl, bicyclo[3.2.1]octyl or bicyclo[5.2.0]nonyl, decalinyl, etc.), which is optionally substituted with one or more (e.g., 1 to 3) suitable substituents. For example, the term "C₃₋₆ cycloalkyl" refers to a saturated or partially unsaturated non-aromatic monocyclic or polycyclic (e.g., bicyclic) hydrocarbon ring (e.g., cyclopropyl, cyclobutyl, cyclopentyl or cyclohexyl), which is optionally substituted with one or more (e.g., 1 to 3) suitable substituents, such as cyclopropyl substituted with methyl.

As used herein, the term "halogen" group is defined to include fluorine, chlorine, bromine or iodine.

As used herein, the term "halo" refers to substitution with one or more (e.g., 1 to 3) same or different halogen atoms.

As used herein, the term "haloalkyl" refers to an alkyl substituted with one or more (e.g., 1 to 3) same or different halogen atoms. For example, the term "C₁₋₆ haloalkyl" refers to a haloalkyl having 1 to 6 carbon atoms, such as -CF₃, -C₂F₅, -CHF₂, -CH₂F, -CH₂CF₃, -CH₂Cl or -CH₂CH₂CF₃ and the like.

As used herein, the term "heterocycle" or "heterocyclyl" refers to a saturated or partially unsaturated non-aromatic monocyclic or polycyclic ring group, for example, in the ring, there are 2, 3, 4, 5, 6, 7, 8 or 9 carbon atoms and one or more (e.g., 1, 2, 3 or 4) heteroatoms independently selected from the group consisting of N, O or S(O)t (wherein, t is 0, 1 or 2), such as 3- to 12-membered heterocyclyl, 3- to 7-membered heterocyclyl, 3- to 6-membered heterocyclyl, 5- to 6-membered heterocyclyl, etc., such as 5- to 10-membered nitrogen-containing heterocyclyl, 6- to 10-membered oxygen-containing heterocyclyl, 6- to 8-membered sulfur-containing heterocyclyl, 5- to 8-membered oxygen-containing heterocyclyl, etc. Representative examples of heterocyclyl include, but are not limited to, oxiranyl, aziridinyl, azetidinyl, oxetanyl, tetrahydrofuryl, tetrahydropyrrolidinyl, hexahydro-1*H*-pyrrolinyl, pyrrolidonyl, imidazolidinyl, pyrazolidinyl, tetrahydropyranyl, tetrahydropyridyl, piperidinyl, morpholinyl, dithianyl, thiomorpholinyl, piperazinyl, etc.

As used herein, the term "aryl" or "aromatic ring" refers to an all-carbon monocyclic or fused-ring polycyclic aromatic group having a conjugated π-electron system. For example, the term "C₆₋₁₀ aryl" or "C₆₋₁₀ aromatic ring" refers to an aromatic group containing 6 to 10 carbon atoms, such as phenyl (ring) or naphthyl (ring). Aryl is optionally substituted with one or more (e.g., 1 to 3) suitable substituents (e.g., halogen, -OH, -CN, -NO₂, C₁₋₆ alkyl, etc.).

As used herein, the term "heteroaryl" refers to an aromatic cyclic group in which at least one ring atom is heteroatom, such as nitrogen atom, oxygen atom or sulfur atom. Optionally, the ring atoms (e.g., carbon, nitrogen or sulfur atoms) in the ring structure may be substituted with oxygen. Specific examples include, but are not limited to, 5- to 10-membered heteroaryl, 5- to 10-membered nitrogen-containing heteroaryl, 6- to 10-membered oxygen-containing heteroaryl, 6- to 8-membered nitrogen-containing heteroaryl, 5- to 8-membered oxygen-containing heteroaryl, etc., such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, 1,4-dioxinyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azacyclooctatetraenyl, etc.

Optionally, the hydrogen in the groups referred to in the present invention may be substituted with deuterium.

The term "substituted" means that one or more (e.g., 1, 2, 3 or 4) hydrogens on the designated atom are selectively substituted with an indicated group, provided that the designated atom has a valence that does not excess the normal valence under the current circumstances and such substitution results in a stable compound. Combinations of substituents and/or variables are permissible only if such combinations result in stable compounds.

If a substituent is described as being "optionally substituted by", the substituent can be (1) unsubstituted or (2) substituted. If the carbon of a substituent is described as being optionally substituted with one or more of the listed substituents, one or more hydrogens on the carbon (to the extent of any hydrogen present) may be independently and/or together substituted or unsubstituted with selected substituents. If the nitrogen of a substituent is described as being optionally substituted with one or more of the listed substituents, one or more hydrogens on the nitrogen (to the extent of any hydrogen present) may each be independently substituted or unsubstituted with selected substituents.

If substituents are described as being "independently selected from" a set of groups, each substituent is selected independently of the other. Accordingly, each substituent may be the same as or different from another (other) substituent.

As used herein, the term "one or more" means one or more than one under reasonable conditions, such as 2, 3, 4, 5, 6, 7, 8, 9 or 10.

As used herein, unless otherwise indicated, the site of attachment of a substituent may be any suitable position of the substituent.

When a bond for a substituent is shown as being through a bond connecting two atoms in a ring, then such substituent may be bonded to any ring-forming atom in such substitutable ring.

The present invention also comprises all pharmaceutically acceptable isotope-labeled compounds which are identical to the compounds described herein except that one or more atoms are replaced with atoms having the same atomic number but an atomic mass or mass number different from the atoms that predominate in nature. Examples of isotopes suitable for inclusion in the compounds include, but are not limited to, isotopes of hydrogen (e.g., ²H, ³H, deuterium D, tritium T); isotopes of carbon (e.g., ¹¹C, ¹³C, and ¹⁴C); isotopes of chlorine (e.g., ¹⁷Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O and ¹⁸O); isotopes of phosphorus (e.g., ³²P)isotopes of sulfur (e.g., ³⁵S). Certain isotope-labeled compounds of the present invention (e.g., those incorporating radioactive isotopes) are useful in tissue distribution studies (e.g., assays) of drug and/or substrate. The radioisotopes tritium (i.e., ³H) and carbon-14 (i.e., ¹⁴C) are particularly useful for this purpose due to their ease of incorporation and detection. Substitution with positron-emitting isotopes such as ¹¹C, ¹⁸F, ¹⁵O, and ¹³N can be used in positron emission tomography (PET) studies to examine substrate recipient occupancy. The isotope-labeled compounds of the present invention can be prepared by methods similar to those described in the accompanying schemes and/or examples and by using an appropriate isotope-labeled reagent in place of the non-labeled reagent previously used. Pharmaceutically acceptable solvates of the present invention include those wherein the solvent of crystallization may be isotopically substituted, such as D₂O, acetone-*d6* or DMSO-*d₆*.

The term "stereoisomer" refers to an isomer formed as a result of at least one asymmetric center. For compounds with one or more (e.g., 1, 2, 3 or 4) asymmetric centers, they may result in racemic mixtures, single enantiomers, diastereomeric mixtures and single diastereoisomers. Certain individual molecules may also exist as geometric isomers (cis/trans). Similarly, the compounds of the present invention may exist as mixtures of two or more structurally distinct forms (commonly referred to as tautomers) in rapid equilibrium. Representative examples of tautomers include keto-enol tautomers, phenol-keto tautomers, nitroso-oxime tautomers, imine-enamine tautomers, etc. It is to be understood that the scope of the present application encompasses all such isomers or mixtures thereof in any proportion (e.g., 60%, 65%, 70%, 75%, 80%, 85%, 90%, 95%, 96%, 97%, 98%, 99%).

The present invention covers all possible crystalline forms or polymorphs of the compounds of the present invention, which may be a single polymorph or a mixture of two or more polymorphs in any proportion.

It should also be understood that certain compounds of the present invention may exist in free form for use in therapy, or, where appropriate, as pharmaceutically acceptable derivatives thereof. In the present invention, pharmaceutically acceptable derivatives include but are not limited to: pharmaceutically acceptable salts, solvates, metabolites or prodrugs, which can directly or indirectly provide the compounds of the present invention or metabolites or residues thereof. Therefore, when a "compound of the present invention" is referred to herein, it is also intended to cover the above-mentioned various derivative forms of the compound.

The pharmaceutically acceptable salts of the compounds of the present invention include acid addition salts and base addition salts. Suitable acid addition salts are formed from acids which can form pharmaceutically acceptable salts. Suitable base addition salts are formed from bases which can form pharmaceutically acceptable salts. For a review of suitable salts, see "Handbook of Pharmaceutical Salts: Properties, Selection, and Use" by Stahl and Wermuth (Wiley-VCH, 2002). Methods for preparing pharmaceutically acceptable salts of the compounds of the present invention are known to those skilled in the art.

The compound of the present invention may exist in the form of a solvate, preferably a hydrate, wherein the compound of the present invention comprises a polar solvent as a structural element of the crystal lattice of the compound. The amount of polar solvent, especially water, may be present in stoichiometric or non-stoichiometric ratio.

Those skilled in the art will appreciate that not all nitrogen-containing heterocycles are capable of forming *N*-oxides since nitrogen requires available lone pairs of electrons to oxidize to form an oxide. Those skilled in the art will be able to recognize that tertiary amines are capable of forming *N*-oxides. Synthetic methods for the preparation of *N*-oxides of heterocycles and tertiary amines are well known to those skilled in the art, and include the use of peroxyacids such as peracetic acid and m-chloroperbenzoic acid (MCPBA), hydrogen peroxide, alkyl hydroperoxides such as t-butyl hydroperoxide, sodium perborate and dioxirane such as dimethyldioxirane to oxidize heterocycles and tertiary amines. These methods for preparing *N-*oxides have been extensively described and reviewed in the literature, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present invention are metabolites of the compounds of the present invention, i.e., the substances formed in vivo upon administration of the compounds of the present invention. Such products may be generated by, for example, oxidation, reduction, hydrolysis, amidation, deamidation, esterification, enzymatic hydrolysis, etc., of the administered compounds. Accordingly, the present invention comprises metabolites of the compounds of the present invention, including compounds produced by contacting the compounds of the present invention with a mammal for a time sufficient to produce metabolites thereof.

The present invention further comprises within its scope prodrugs of the compounds of the present invention, which are certain derivatives of the compounds of the present invention which themselves may have little or no pharmacological activity and can be converted into the compounds of the present invention with desired activity, for example, by hydrolytic cleavage, when they are administered to or on the body. Typically, such prodrugs are functional group derivatives of the compounds, and are readily converted in vivo into the compounds with the desired therapeutic activity. Additional information on the use of prodrugs can be found in "Prodrugs as Novel Delivery Systems", Vol. 14, ACS Symposium Series (T. Higuchi and V. Stella), and "Bioreversible Carriers in Drug Design," Pergamon Press, 1987 (Edited by E. B. Roche, American Pharmaceutical Association). The prodrugs of the present invention can be obtained, for example, by using certain moieties known to those skilled in the art as "pro-moiety (e.g., as described in "Design of Prodrugs", H. Bundgaard (Elsevier, 1985))" to substitute appropriate functional groups present in the compounds of the present invention.

The present invention also encompasses the compounds of the present invention which contain protecting groups. During any process for the preparations of the compounds of the present invention, it may be necessary and/or desirable to protect sensitive or reactive groups on any of the molecules involved, thereby forming chemically protected forms of the compounds of the present invention. This can be achieved by conventional protecting groups, for example, the protecting groups as described in "Protective Groups in Organic Chemistry", ed. J.F.W. McOmie, Plenum Press, 1973; and T.W. Greene & P.G.M. Wuts, Protective Groups in Organic Synthesis, John Wiley & Sons, 1991, and all these references are incorporated herein by reference. Protecting groups can be removed at an appropriate subsequent stage using methods known in the art.

The term "about" means within the range of ±10%, preferably within the range of ±5%, more preferably within the range of ±2% of the stated value.

The term "conjugate" refers to a substance obtained by linking a small molecule drug with a targeting moiety. In some embodiments of the present invention, the small molecule drug is linked to the targeting moiety through a linker. The linker can be cleaved under specific environment (e.g., a low intracellular pH environment, an acidic tumor microenvironment) or specific action (e.g., action of lysosomal protease), thereby separating the small molecule drug from the targeting moiety.

The term "linker" refers to a fragment that links a small molecule drug to a targeting moiety.

The term "targeting moiety" refers to a portion of a conjugate that is capable of specifically binding to a target (or a portion of target) on the surface of a cell. Through the interaction of the targeting moiety with the target, the conjugate can be delivered to a specific cell population.

The term "fragment" refers to the remaining part of a compound molecule after losing one or more atoms or atomic groups (e.g., hydrogen atoms or hydroxyl groups). For example " fragment of a compound " refers to the remaining part of a compound obtained by losing a hydrogen atom or a hydroxyl group after the compound described herein is linked to the linker in the immune-stimulating antibody conjugate.

The term "cross-linking agent" refers to a reagent with the same or different reactive groups at both molecular ends, which can covalently interact with amino groups, sulfhydryl groups, hydroxyl groups or maleimide groups on other molecules to produce crosslinking effect, for example, the cross-linking agent SATA.

### Compound

An object of the present application is to provide a compound of Formula (I) or a pharmaceutically acceptable salt, ester, stereoisomer, tautomer, polymorph, solvate, *N*-oxide, isotope-labeled compounds, metabolite or prodrug thereof: 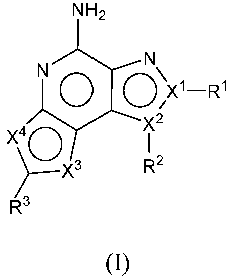 wherein,
X¹ is selected from the group consisting of N and C;
X² is selected from the group consisting of N and C;
and at least one of X¹ and X² is selected from N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is hydrogen, or has the formula of -L¹-L²-L³-L⁴;
L¹ is selected from the group consisting of a covalent bond and C₁₋₆ alkylene;
L² is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L³ is selected from the group consisting of a covalent bond, -O-, -S-, -C(O)-, -O-C(O)-, - C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ₘ-NR⁵-, -NR⁵-S(O)ₘ- and -NR⁵-S(O)ₘ-NR⁵-;
L⁴ is selected from the group consisting of hydrogen, C₁₋₆ alkyl, -(O-CH₂CH₂)ₙ-O-C₁₋₆ alkyl and -C₁₋₆ alkylene-(O-CH₂CH₂)ₙ-O-C₁₋₆ alkyl, optionally, each alkyl is independently substituted with one or more groups independently selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R³ is hydrogen, or has the formula of -L⁵-L⁶-L⁷-L⁸;
L⁵ is selected from the group consisting of a covalent bond and C₁₋₆ alkylene;
L⁶ is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L⁷ is selected from the group consisting of a covalent bond, -O-, -S-, -C(O)-, -O-C(O)-, - C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L⁸ is selected from the group consisting of hydrogen, C₁₋₆ alkyl, -(O-CH₂CH₂)₄-O-C₁₋₆ alkyl and -C₁₋₆ alkylene-(O-CH₂CH₂)₄-O-C₁₋₆ alkyl, optionally, each alkyl is independently substituted with one or more groups independently selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently selected from the group consisting of hydrogen and C₁₋₆ alkyl;
each R⁶ is independently selected from the group consisting of hydrogen and C₁₋₆ alkyl;
each m is independently 1 or 2;
each n is independently selected from the group consisting of integers 1 to 25;
each p is independently 1 or 2;
each q is independently selected from the group consisting of integers 1 to 25.

In some embodiments, X¹ is N. In some embodiments, X¹ is C.

In some embodiments, X² is N. In some embodiments, X² is C.

In some embodiments, only one of X¹ and X² is N.

In some embodiments, X³ is O. In some embodiments, X³ is S. In some embodiments, X³ is N.

In some embodiments, X⁴ is O. In some embodiments, X⁴ is S. In some embodiments, X⁴ is N. In some embodiments, X⁴ is CR⁴, preferably, X⁴ is CH.

In some embodiments, R¹ is selected from the group consisting of C₁₋₄ alkyl and -C₁₋₄ alkylene-O-C₁₋₄ alkyl;

preferably, R¹ is selected from the group consisting of C₁₋₄ alkyl and -C₁₋₂ alkylene-O-C₁₋₂ alkyl, such as methyl, ethyl, n-propyl, isopropyl, n-butyl, isobutyl, sec-butyl, tert-butyl, 2-methoxyethyl, or ethoxymethyl. In some embodiments, R¹ is n-butyl, 2-methoxyethyl, or ethoxymethyl; preferably, R¹ is n-butyl.

In some embodiments, R² is hydrogen, or has the formula of -L¹-L²-L³-L⁴, wherein,
L¹ is selected from the group consisting of a covalent bond and C₁₋₆ alkylene;
L² is selected from the group consisting of a covalent bond, C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl, such as piperidinyl; e.g., 3- to 6-membered saturated or unsaturated oxygen-containing monoheterocyclyl, such as tetrahydropyranyl), C₆₋₁₀ aryl (e.g., phenyl) and 5- to 10-membered heteroaryl (e.g., 5- to 6-membered nitrogen-containing heteroaryl, 5- to 6-membered oxygen-containing heteroaryl, 5- to 6-membered sulfur-containing heteroaryl), optionally, the cycloalkyl, heterocyclyl, aryl and heteroaryl are independently substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L² is selected from the group consisting of a covalent bond, 3- to 12-membered heterocyclyl (e.g., 3- to 6-membered saturated or unsaturated nitrogen-containing monoheterocyclyl, such as piperidinyl; e.g., 3- to 6-membered saturated or unsaturated oxygen-containing monoheterocyclyl, such as tetrahydropyranyl) and C₆₋₁₀ aryl (e.g., phenyl), optionally, the heterocyclyl and aryl are each independently substituted with one or more groups independently selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L² is selected from the group consisting of a covalent bond, piperidinyl and tetrahydropyranyl;
L³ is a covalent bond or -C(O)-;
L⁴ is selected from the group consisting of hydrogen, C₁₋₆ alkyl, -(O-CH₂CH₂)ₙ-O-C₁₋₆ alkyl (e.g., -(O-CH₂CH₂)ₙ-O-methyl, -(O-CH₂CH₂)ₙ-O-ethyl) and -C₁₋₆ alkylene-(O-CH₂CH₂)ₙ-O-C₁₋₆ alkyl (e.g., -CH₂-(O-CH₂CH₂)ₙ-O-methyl, -CH₂-CH₂-(O-CH₂CH₂)ₙ-O-methyl, -CH₂-(O-CH₂CH₂)ₙ-O-ethyl, -CH₂-CH₂-(O-CH₂CH₂)ₙ-O-ethyl), optionally, each alkyl (e.g., methyl or ethyl) is independently substituted with one or more groups independently selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl and -C(O)-O-C₁₋₆ alkyl (e.g., -C(O)-O-methyl), each n is independently selected from the group consisting of 1, 2, 3, 4, 5, 6, 7; preferably, L⁴ is selected from the group consisting of hydrogen, C₁₋₆ alkyl and -C₁₋₆ alkylene-(O-CH₂CH₂)ₙ-O-C₁₋₆ alkyl, optionally, each alkyl is independently substituted with one or more groups independently selected from the group consisting of hydroxyl, -NH₂, C₁₋₆ alkoxy, carboxyl and - C(O)-O-C₁₋₆ alkyl, each n is selected from the group consisting of 1, 2, 3, 4, 5, 6 and 7; preferably, L⁴ is selected from the group consisting of hydrogen, C₁₋₄ alkyl and -C₁₋₄ alkylene-(O-CH₂CH₂)ₙ-O-C₁₋₄ alkyl, optionally, each alkyl is independently substituted with one or more groups independently selected from the group consisting of hydroxyl, -NH₂, C₁₋₄ alkoxy, carboxyl and - C(O)-O-C₁₋₄ alkyl, n is selected from the group consisting of 1, 2, 3, 4, 5, 6 and 7.

In some embodiments, R² is hydrogen.

In some embodiments, R² has the formula of -L¹-L²-L³-L⁴.

In some embodiments, L¹ is a covalent bond. In some embodiments, L¹ is C₁₋₆ alkylene, such as -CH₂-, -CH₂-CH₂-, -CH₂-CH₂-CH₂-, -CH₂-CH₂-CH₂-CH₂-. In some embodiments, L¹ is a covalent bond or -CH₂-.

In some embodiments, L² is a covalent bond.

In some embodiments, L² is selected from the group consisting of C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl, such as piperidinyl; for example, 3- to 6-membered saturated or unsaturated oxygen-containing monoheterocyclyl, such as tetrahydropyranyl), C₆₋₁₀ aryl (e.g., phenyl) and 5- 10-membered heteroaryl (e.g., 5- to 6-membered nitrogen-containing heteroaryl, 5- to 6-membered oxygen-containing heteroaryl, 5- to 6-membered sulfur-containing heteroaryl), optionally, the cycloalkyl, heterocyclyl, aryl and heteroaryl is substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., - CH₂-NH₂).

In some embodiments, L² is selected from the group consisting of 3- to 12-membered heterocyclyl (e.g., 3- to 6-membered saturated or unsaturated nitrogen-containing monoheterocyclyl, such as piperidinyl; for example, 3- to 6-membered saturated or unsaturated nitrogen-containing monoheterocyclyl, such as tetrahydropyranyl) and C₆₋₁₀ aryl (e.g., phenyl), the heterocyclyl and aryl are optionally substituted with -C₁₋₆ alkylene-NH₂ (e.g., -CH₂ -NH₂).

In some embodiments, L³ is a covalent bond. In some embodiments, L³ is -C(O)-.

In some embodiments, L⁴ is selected from the group consisting of hydrogen, C₁₋₆ alkyl, -(O-CH₂CH₂)ₙ-O-C₁₋₆ alkyl (e.g., -(O-CH₂CH₂)ₙ-O-methyl, -(O-CH₂CH₂)ₙ-O-ethyl) and -C₁₋₆ alkylene-(O-CH₂CH₂)ₙ-O-C₁₋₆ alkyl (e.g., -CH₂-(O-CH₂CH₂)ₙ-O-methyl, -CH₂-CH₂-(O-CH₂CH₂)ₙ-O-methyl, -CH₂-(O-CH₂CH₂)ₙ-O-ethyl, -CH₂-CH₂-(O-CH₂CH₂)ₙ-O-ethyl), n is each independently selected from the group consisting of 1, 2, 3, 4, 5, 6, 7; the alkyl (e.g., methyl or ethyl) is optionally substituted with one or more groups selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl, and -C(O)-O-C₁₋₆ alkyl (e.g., -C(O)-O-methyl).

In some embodiments, L⁴ is selected from the group consisting of hydrogen, C₁₋₆ alkyl (e.g., methyl, ethyl), -(O-CH₂CH₂)ₙ-O-C₁₋₆ alkyl (e.g., -(O-CH₂CH₂)ₙ-O-methyl, -(O-CH₂CH₂)ₙ-O-ethyl) and -C₁₋₆ alkylene-(O-CH₂CH₂)ₙ-O-C₁₋₆ alkyl (e.g., -CH₂-(O-CH₂CH₂)ₙ-O-methyl, -CH₂-CH₂-(O-CH₂CH₂)ₙ-O-methyl, -CH₂-(O-CH₂CH₂)ₙ-O-ethyl, -CH₂-CH₂-(O-CH₂CH₂)ₙ-O-ethyl), n is each independently selected from the group consisting of 1, 2, 3, 4, 5, 6, 7; the alkyl (e.g., methyl or ethyl) is optionally substituted with one or more groups selected from the group consisting of hydrogen, hydroxyl, -NH₂, C₁₋₆ alkoxy (e.g., methoxy), carboxyl and -C(O)-O-C₁₋₆ alkyl (e.g., - C(O)-O-methyl).

In some embodiments, L⁴ is selected from the group consisting of -(O-CH₂CH₂)ₙ-O-C₁₋₆ alkyl (e.g., -(O-CH₂CH₂)ₙ-O-methyl, -(O-CH₂CH₂)ₙ-O-ethyl) and -C₁₋₆ alkylene-(O-CH₂CH₂)ₙ-O-C₁₋₆ alkyl (e.g., -CH₂-(O-CH₂CH₂)ₙ-O-methyl, -CH₂-CH₂-(O-CH₂CH₂)ₙ-O-methyl, -CH₂-(O-CH₂CH₂)ₙ-O-ethyl, -CH₂-CH₂-(O-CH₂CH₂)ₙ-O-ethyl), n is each independently selected from the group consisting of 1, 2, 3, 4, 5, 6, 7; the alkyl (e.g., methyl or ethyl) is optionally substituted with one or more groups selected from the group consisting of hydroxyl, -NH₂, carboxyl and -C(O)-O-C₁₋₆ alkyl (e.g., -C(O)-O-methyl).

In some embodiments, R² is selected from the group consisting of hydrogen, C₁₋₆ alkyl, C₁₋₆ alkylene-NH₂, C₁₋₆ alkylene-3- to 12-membered heterocyclyl, C₁₋₆ alkylene-C₆₋₁₀ aryl, C₁₋₆ alkylene-3- to 12-membered heterocyclyl-C₁₋₆ alkylene-(O-CH₂CH₂)ₙ-O-C₁₋₆ alkyl, C₁₋₆ alkylene-3- to 12-membered heterocyclyl-C(O)-C₁₋₆ alkylene-(O-CH₂CH₂)ₙ-O-C₁₋₆ alkyl; n is selected from the group consisting of 1, 2, 3, 4, 5, 6, 7; the 3- 12-membered heterocyclyl or C₆₋₁₀ aryl is optionally substituted with C₁₋₆ alkyl, -C₁₋₆ alkylene-NH₂ (e.g., -CH₂-NH₂) and -C₁₋₆ alkylene-methoxy (e.g., -CH₂CH₂-OCH₃); the alkyl and heterocyclyl are optionally substituted with one or more groups selected from the group consisting of hydrogen, hydroxyl, -NH₂, carboxyl or -C(O)-O-C₁₋₆ alkyl (e.g., -C(O)-O-methyl). In some embodiments, each C₁₋₆ alkylene is independently methylene, ethylene or butylene. In some embodiments, each C₁₋₆ alkyl is independently methyl or ethyl. In some embodiments, the 3- to 12-membered heterocyclyl is a 3- to 6-membered saturated or unsaturated nitrogen-containing monoheterocyclyl, such as piperidinyl. In some embodiments, the 3- to 12-membered heterocyclyl is a 3- to 6-membered saturated or unsaturated oxygen-containing monoheterocyclyl, such as tetrahydropyranyl. In some embodiments, the C₆₋₁₀ aryl is phenyl.

In some embodiments, R² is selected from the group consisting of hydrogen, C₁₋₆ alkylene-NH₂, C₁₋₆ alkylene-3- to 12-membered heterocyclyl, C₁₋₆ alkylene-C₆₋₁₀ aryl, C₁₋₆ alkylene-3- to 12-membered heterocyclyl-C₁₋₆ alkylene-(O-CH₂CH₂)ₙ-O-C₁₋₆ alkyl, C₁₋₆ alkylene-3- to 12-membered heterocyclyl-C(O)-C₁₋₆ alkylene-(O-CH₂CH₂)ₙ-O-C₁₋₆ alkyl; n is selected from the group consisting of 1, 2, 3, 4, 5, 6, 7; the 3- to 12-membered heterocyclyl or C₆₋₁₀ aryl is optionally substituted with -C₁₋₆ alkylene-NH₂ (e.g., -CH₂-NH₂); the alkyl and heterocyclyl are optionally substituted with one or more groups selected from the group consisting of hydrogen, hydroxyl, - NH₂, carboxyl or -C(O)-O-C₁₋₆ alkyl (e.g., -C(O)-O-methyl). In some embodiments, each C₁₋₆ alkylene is independently methylene, ethylene or butylene. In some embodiments, each C₁₋₆ alkyl is independently methyl or ethyl. In some embodiments, the 3- to 12-membered heterocyclyl is a 3- to 6-membered saturated or unsaturated nitrogen-containing monoheterocyclyl, such as piperidinyl. In some embodiments, the 3- to 12-membered heterocyclyl is a 3- to 6-membered saturated or unsaturated oxygen-containing monoheterocyclyl, such as tetrahydropyranyl. In some embodiments, the C₆₋₁₀ aryl is phenyl.

In some embodiments, R² is selected from the group consisting of hydrogen, methyl, -CH₂-CH₂-CH₂-CH₂-NH₂, -CH₂-piperidine, -CH₂-piperidinyl-methyl, -CH₂-tetrahydropyran, -CH₂-phenyl-CH₂-NH₂, -CH₂-piperidinyl-CH₂-CH₂-(O-CH₂CH₂)₂-NH₂, -CH₂-piperidinyl- CH₂-CH₂-(O-CH₂CH₂)₆-O-methyl, -CH₂-piperidinyl-C(O)-CH₂-CH₂-(O-CH₂CH₂)₂-NH₂, -CH₂-piperidinyl-C(O)-CH₂-CH₂-(O-CH₂CH₂)₇-NH₂, -CH₂-piperidinyl-C(O)-CH₂- (O-CH₂CH₂)₂-OH, -CH₂-piperidinyl-C(O)-CH₂-(O-CH₂CH₂)₄-OH, -CH₂-piperidinyl-C(O)-CH₂- O-CH₂CH₂-O-CH₂-COOH, -CH₂-piperidinyl-C(O)-CH₂-(O-CH₂CH₂)₃-O-CH₂-COOH, -CH₂-piperidinyl-C(O)-CH₂-O-CH₂CH₂-O-CH₂-COOCH₃, -CH₂-piperidinyl-C(O)-CH₂-(O-CH₂CH₂)₃-O-CH₂-COOCH₃, - CH₂-piperidinyl-CH₂-CH₂-O-CH₃, -CH₂-piperidinyl-CH₂-CH₂-O-CH₂CH₂-O-CH₃, -CH₂-piperidinyl-CH₂-CH₂-(O- CH₂CH₂)₂-O-CH₃.

In some embodiments, R² is selected from the group consisting of hydrogen, -CH₂-CH₂-CH₂-CH₂-NH₂, -CH₂-piperidine, -CH₂-tetrahydropyran, -CH₂-phenyl-CH₂-NH₂, -CH₂-piperidyl-CH₂-CH₂-(O-CH₂CH₂)₂-NH₂, -CH₂-piperidinyl-CH₂-CH₂-(O-CH₂CH₂)₆-O- methyl, -CH₂-piperidinyl-C(O) -CH₂-CH₂-(O-CH₂CH₂)₂-NH₂, -CH₂-piperidinyl-C(O)-CH₂-CH₂- (O-CH₂CH₂)₇-NH₂, -CH₂-piperidinyl-C(O)-CH₂-(O-CH₂CH₂)₂-OH, -CH₂-piperidinyl-C(O)-CH₂- (O-CH₂CH₂)₄-OH, -CH₂-piperidinyl-C(O)-CH₂-O-CH₂CH₂-O-CH₂-COOH, -CH₂-piperidinyl- C(O)-CH₂-(O-CH₂CH₂)₃-O-CH₂-COOH, -CH₂-piperidinyl-C(O)-CH₂-O-CH₂CH₂-O-CH₂- COOCH₃, -CH₂-piperidinyl-C(O)-CH₂-(O-CH₂CH₂)₃-O-CH₂-COOCH₃.

In some embodiments, R³ is hydrogen, or has the formula of -L⁵-L⁶-L⁷-L⁸, wherein
L⁵ is selected from the group consisting of a covalent bond and C₁₋₆ alkylene, such as -CH₂-, -CH₂-CH₂-, -CH₂-CH₂-CH₂- or -CH₂-CH₂-CH₂-CH₂-; preferably, L⁵ is a covalent bond;
L⁶ is selected from the group consisting of a covalent bond, C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)), C₆₋₁₀ aryl (e.g., phenyl) and 5- to 10-membered heteroaryl (e.g., 5- to 6-membered nitrogen-containing heteroaryl, 5- to 6-membered oxygen-containing heteroaryl, 5- to 6-membered sulfur-containing heteroaryl), optionally, the cycloalkyl, heterocyclyl, aryl and heteroaryl are each independently substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L⁶ is selected from the group consisting of a covalent bond and 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)), the heterocyclyl is optionally substituted with one or more groups independently selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L⁶ is selected from the group consisting of a covalent bond, 1,2,3,6-tetrahydropyridyl, piperazinyl and tetrahydropyranyl;
L⁷ is a covalent bond or -C(O)-; preferably, L⁷ is a covalent bond;
L⁸ is selected from the group consisting of hydrogen, C₁₋₆ alkyl, -(O-CH₂CH₂)₄-O-C₁₋₆ alkyl (e.g., -(O-CH₂CH₂)_{q}-O-methyl, -(O-CH₂CH₂)_{q}-O-ethyl) and -C₁₋₆ alkylene-(O-CH₂CH₂)₄-O-C₁₋₆ alkyl (e.g., -CH₂-(O-CH₂CH₂)_{q}-O-methyl, -CH₂-CH₂-(O-CH₂CH₂)₄-O-methyl, -CH₂-(O-CH₂CH₂)₄-O-ethyl, -CH₂-CH₂-(O-CH₂CH)₄-O-ethyl), optionally, each alkyl (e.g., methyl or ethyl) is independently substituted with one or more groups independently selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl and -C(O)-O-C₁₋₆ alkyl (e.g., -C(O)-O-methyl), each q is independently selected from the group consisting of 1, 2, 3, 4, 5, 6, 7; preferably, L⁸ is selected from the group consisting of hydrogen, C₁₋₆ alkyl and -C₁₋₆ alkylene-(O-CH₂CH₂)_{q}-O-C₁₋₆ alkyl, optionally, each alkyl is substituted with one or more groups independently selected from the group consisting of hydroxyl, -NH₂, C₁₋₆ alkoxy, carboxyl and -C(O)-O-C₁₋₆ alkyl, q is selected from the group consisting of 1, 2, 3, 4, 5, 6 and 7; preferably, L⁸ is selected from the group consisting of hydrogen, C₁₋₄ alkyl and -C₁₋₄ alkylene-(O-CH₂CH₂)₄-O-C₁₋₄ alkyl, optionally, each alkyl is independently substituted with one or more C₁₋₄ alkoxy groups, and q is selected from the group consisting of 1, 2, 3, 4, 5, 6 and 7.

In some embodiments, R³ is hydrogen.

In some embodiments, R³ is of formula -L⁵-L⁶-L⁷-L⁸.

In some embodiments, L⁵ is a covalent bond. In some embodiments, L⁵ is C₁₋₆ alkylene, such as -CH₂-, -CH₂-CH₂-, -CH₂-CH₂-CH₂-, -CH₂-CH₂-CH₂-CH₂-.

In some embodiments, L⁶ is a covalent bond.

In some embodiments, L⁶ is selected from the group consisting of C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)), C₆₋₁₀ aryl (e.g., phenyl) and 5- to 10-membered heteroaryl (e.g., 5- to 6-membered nitrogen-containing heteroaryl, 5- to 6-membered oxygen-containing heteroaryl, 5- to 6-membered sulfur-containing heteroaryl), the cycloalkyl, heterocyclyl, aryl and heteroaryl are optionally substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂).

In some embodiments, L⁶ is selected from the group consisting of C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl (e.g., piperidyl, tetrahydropyridyl (e.g., 1,2,3,6-tetrahydropyridyl), piperazinyl)), C₆₋₁₀ aryl (e.g., phenyl) and 5 -10-membered heteroaryl (e.g., 5- to 6-membered nitrogen-containing heteroaryl, 5- to 6-membered oxygen-containing heteroaryl, 5- to 6-membered sulfur-containing heteroaryl), the cycloalkyl, heterocyclyl, aryl and heteroaryl are optionally substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂).

In some embodiments, L⁶ is selected from the group consisting of 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)). In some embodiments, the heterocyclyl is substituted with C₁₋₆ alkyl (e.g., methyl, ethyl, n-propyl, isopropyl, n-butyl, isobutyl, sec-butyl, tert-butyl, n-pentyl, n-hexyl).

In some embodiments, L⁶ is selected from the group consisting of 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl)). In some embodiments, the heterocyclyl is substituted with C₁₋₆ alkyl (e.g., methyl, ethyl, n-propyl, isopropyl, n-butyl, isobutyl, sec-butyl, tert-butyl, n-pentyl, n-hexyl).

In some embodiments, L⁷ is a covalent bond.

In some embodiments, L⁸ is selected from the group consisting of hydrogen, C₁₋₆ alkyl, -(O-CH₂CH₂)₄-O-C₁₋₆ alkyl (e.g., -(O-CH₂CH₂)_{q}-O-methyl, -(O-CH₂CH₂)_{q}-O-ethyl) and -C₁₋₆ alkylene-(O-CH₂CH₂)₄-O-C₁₋₆ alkyl (e.g., -CH₂-(O-CH₂CH₂)_{q}-O-methyl, -CH₂-CH₂-(O-CH₂CH₂)_{q}-O-methyl, -CH₂-(O-CH₂CH₂)_{q}-O-ethyl, -CH₂-CH₂-(O-CH₂CH₂)₄-O-ethyl), q is each independently selected from the group consisting of 1, 2, 3, 4, 5, 6, 7; the alkyl (e.g., methyl or ethyl) is optionally substituted with one or more groups selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl, and -C(O)-O-C₁₋₆ alkyl (e.g., -C(O)-O-methyl).

In some embodiments, L⁸ is selected from the group consisting of hydrogen, C₁₋₆ alkyl (e.g., methyl, ethyl, n-propyl, isopropyl, n-butyl, isobutyl, sec-butyl, tert-butyl, n-pentyl, n-hexyl), - CH₂-C2₂-O-CH₃, -CH₂-CH₂-O-CH₂CH₂-O-CH₃ and -CH₂-CH₂-(O-CH2CH₂)₂-O-CH₃.

In some embodiments, L⁸ is selected from the group consisting of hydrogen, C₁₋₆ alkyl (e.g., methyl, ethyl, n-propyl, isopropyl, n-butyl, isobutyl, sec-butyl, tert-butyl, n-pentyl, n-hexyl).

In some embodiments, R³ is selected from the group consisting of hydrogen, C₁₋₆ alkyl (e.g., methyl), 3- to 6-membered saturated or unsaturated nitrogen-containing monoheterocyclyl (e.g., piperidyl, tetrahydropyridyl (e.g., 1,2,3,6- Tetrahydropyridyl), piperazinyl) optionally substituted with C₁₋₆ alkyl (e.g., methyl), -C₁₋₆ alkylene-C₁₋₆ alkoxy (e.g., -CH₂-CH₂-O-CH₃), -C₁₋₆ alkylene-(O-CH₂CH₂)_{q}-O-C₁₋₆ alkyl (e.g., -CH₂-CH₂-O-CH₂CH₂-O-CH₃ and -CH₂-CH₂-(O-CH₂CH₂)₂-O-CH₃), 3- to 6-membered saturated or unsaturated oxygen-containing monoheterocyclyl (e.g., tetrahydropyranyl) optionally substituted with C₁₋₆ alkyl (e.g., methyl), q is each independently selected from the group consisting of 1, 2, 3, 4, 5, 6, 7.

In some embodiments, R³ is selected from the group consisting of hydrogen, C₁₋₆ alkyl (e.g., methyl), 3- to 6-membered saturated or unsaturated nitrogen-containing monoheterocyclyl (e.g., piperidyl, tetrahydropyridyl (e.g., 1,2,3,6-tetrahydropyridyl), piperazinyl) optionally substituted with C₁₋₆ alkyl (e.g., methyl).

In some embodiments, R³ is selected from the group consisting of hydrogen, methyl, tetrahydropyranyl, and piperidinyl, 1,2,3,6-tetrahydropyridyl and piperazinyl substituted with methyl, -CH₂-CH₂-O-CH₃, -CH₂-CH₂-O-CH₂CH₂-O-CH₃ and -CH₂-CH₂-(O-CH₂CH₂)₂-O-CH₃.

In some embodiments, R³ is selected from the group consisting of hydrogen, methyl, and piperidinyl, 1,2,3,6-tetrahydropyridyl and piperazinyl substituted with methyl.

In some embodiments, R⁴ is selected from the group consisting of hydrogen, halogen, cyano, C₁₋₆ alkyl, C₁₋₆ alkoxy, and C₃₋₁₀ cycloalkyl. In some embodiments, R⁴ is selected from the group consisting of hydrogen, C₁₋₃ alkyl, C₁₋₃ alkoxy, and C₃₋₆ cycloalkyl. In some embodiments, R⁴ is hydrogen.

In some embodiments, each R⁵ is independently selected from the group consisting of hydrogen and C₁₋₃ alkyl (e.g., methyl, ethyl, n-propyl, isopropyl); preferably, R⁵ is hydrogen.

In some embodiments, each R⁶ is independently selected from the group consisting of hydrogen and C₁₋₃ alkyl (e.g., methyl, ethyl, n-propyl, isopropyl).

In some embodiments, n and q are each independently selected from the group consisting of 1, 2, 3, 4, 5, 6, 7, 8, 9, 10, 11, 12, 13, 14, 15, 16, 17, 18, 19, 20, 21, 22, 23, 24, 25.

The present invention covers the compounds of Formula (I) obtained by any combination of the above-mentioned preferred groups.

In some embodiments, the compound of the present invention has a structure of Formula (II-A), Formula (II-B), Formula (II-C), Formula (II-D), Formula (II-E) or Formula (II-F): 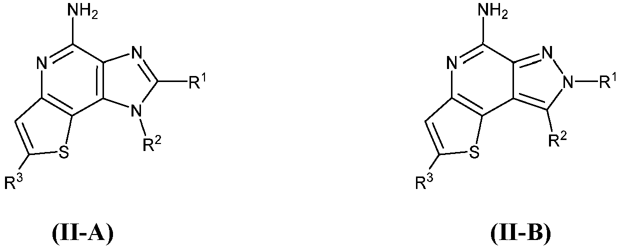 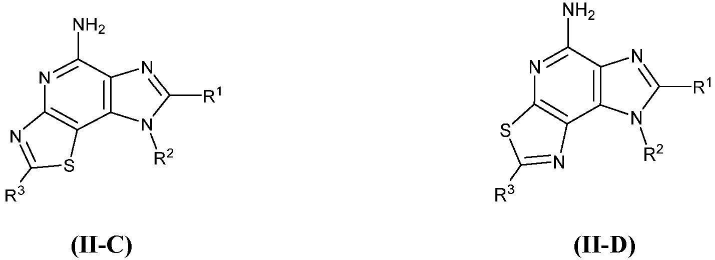 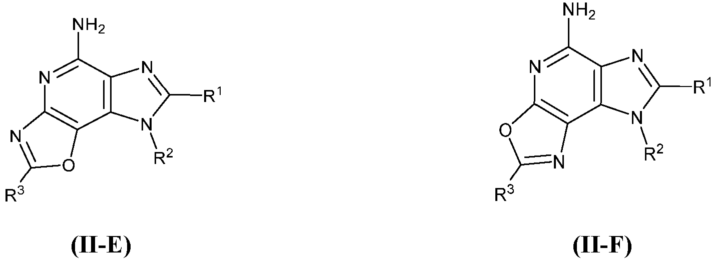 wherein, each of the groups R¹, R² and R³ is as defined above.

In some embodiments, the compound of the present invention has a structure of Formula (III-A) or Formula (III-B): 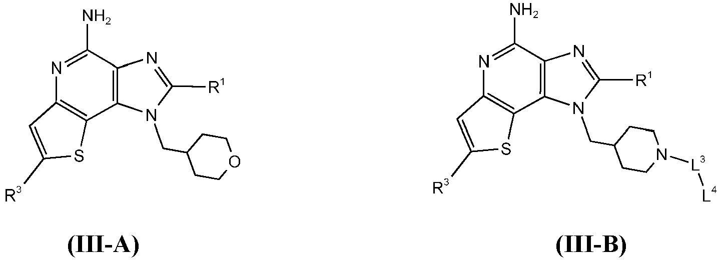 wherein, each of the groups R¹, R³, L³ and L⁴ is as defined above.

In some embodiments, the compound of the present invention has a structure of Formula (IV-A) or Formula (IV-B): 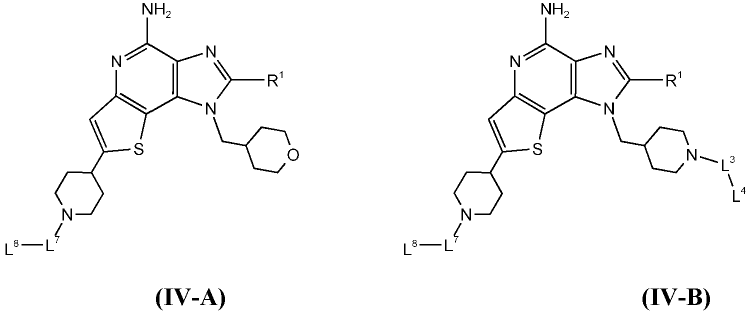 wherein, each of the groups R¹, L³, L⁴, L⁷ and L⁸ is as defined above.

In some embodiments, the compound of the present invention has a structure of Formula (V-A) or Formula (V-B): 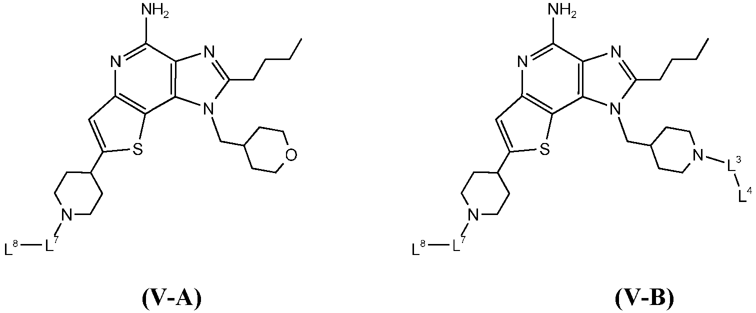 wherein, each of the groups L³, L⁴, L⁷ and L⁸ is as defined above.

In some embodiments, the compound of the present invention is selected from the group consisting of:

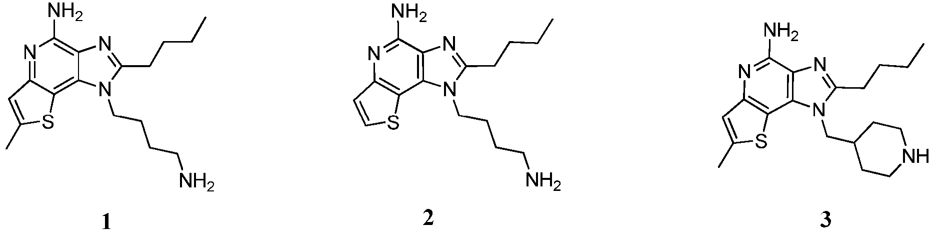

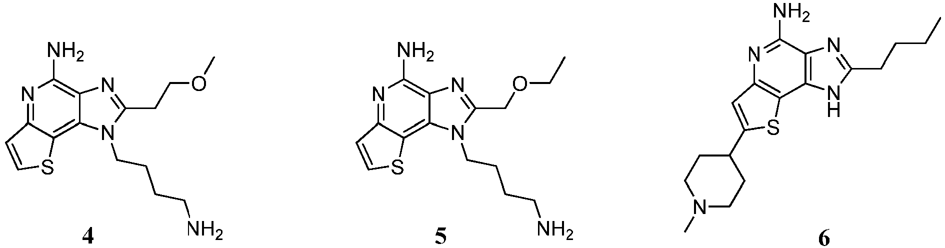

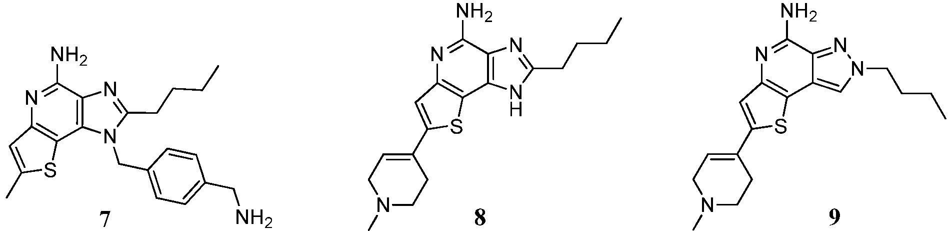

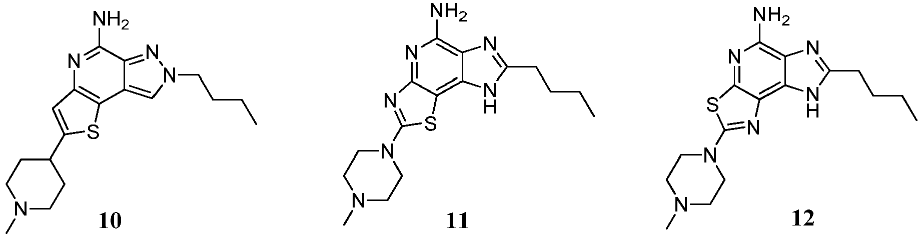

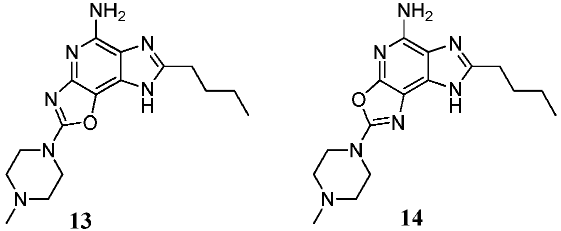

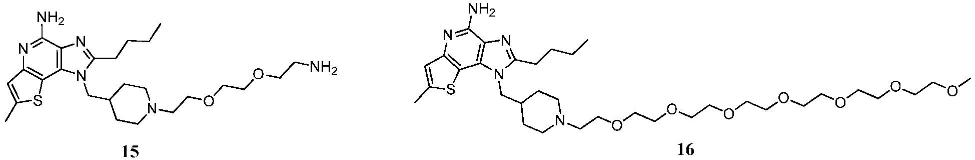

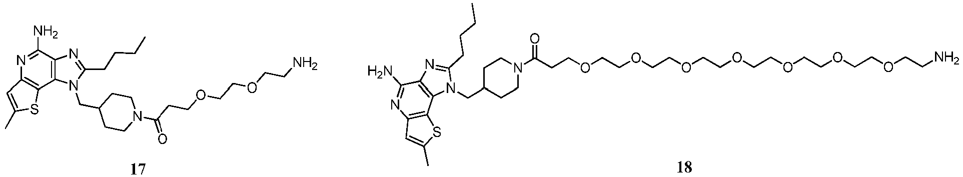

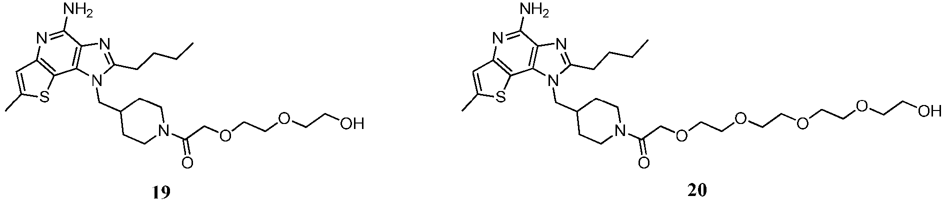

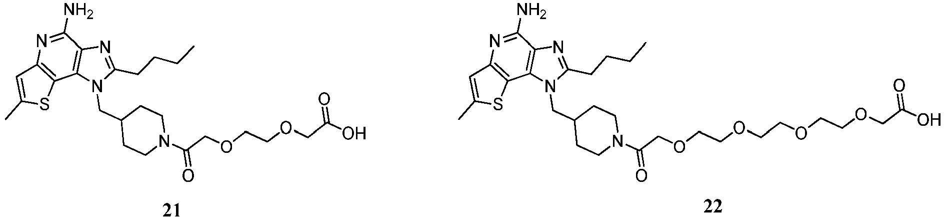

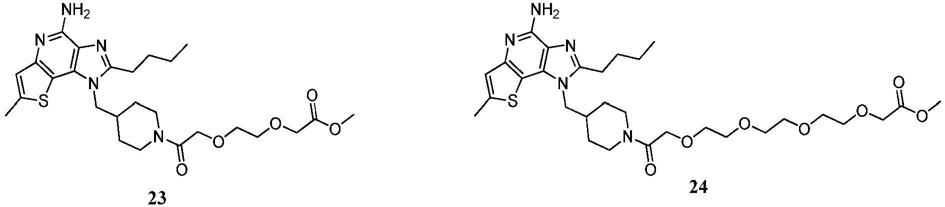

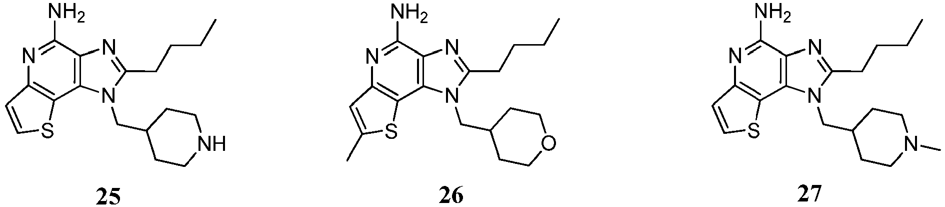

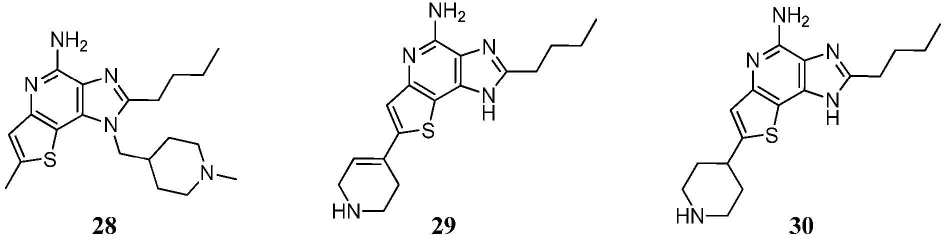

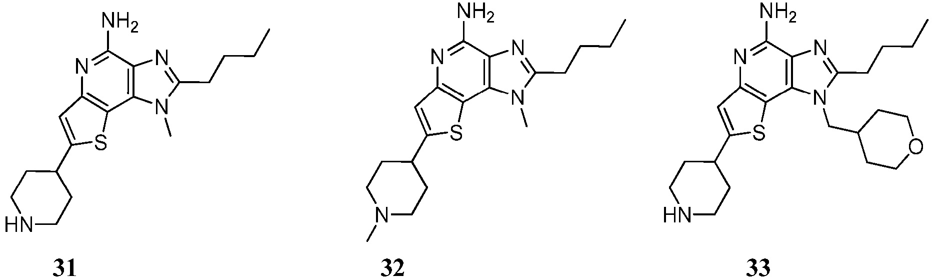

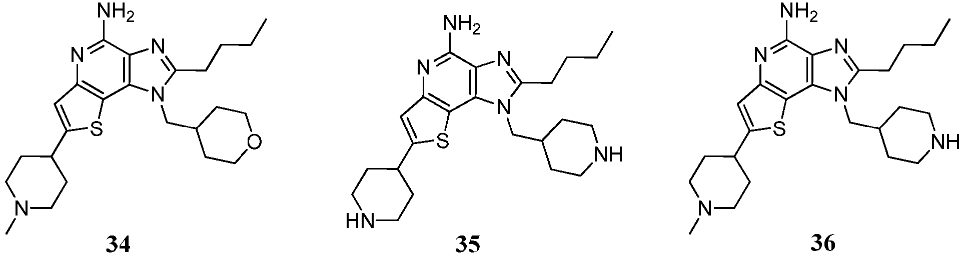

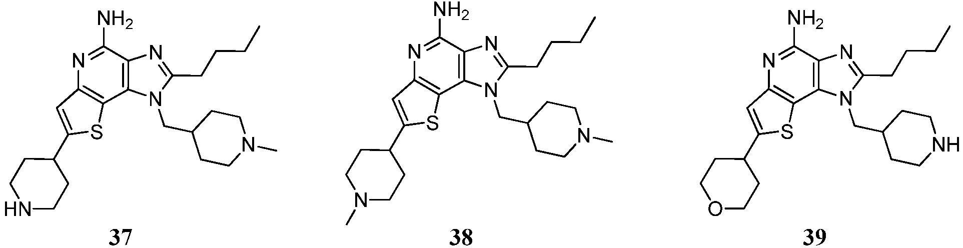

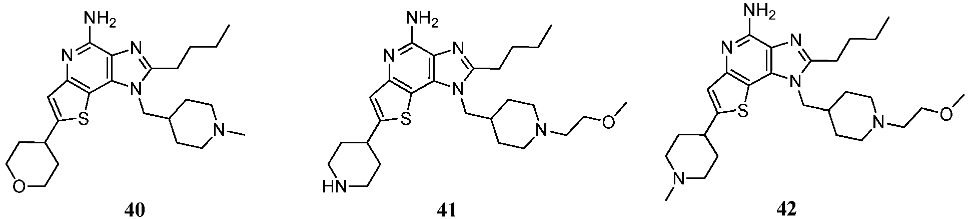

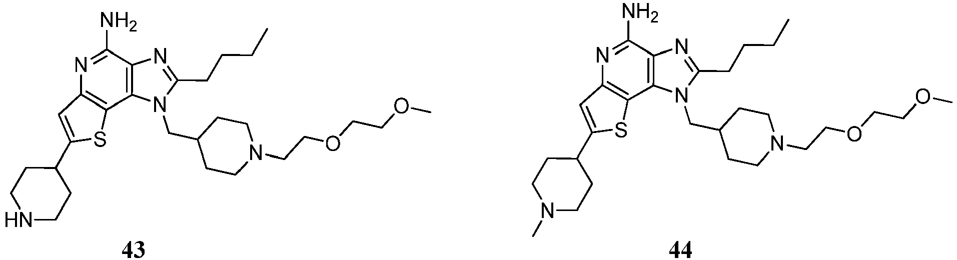

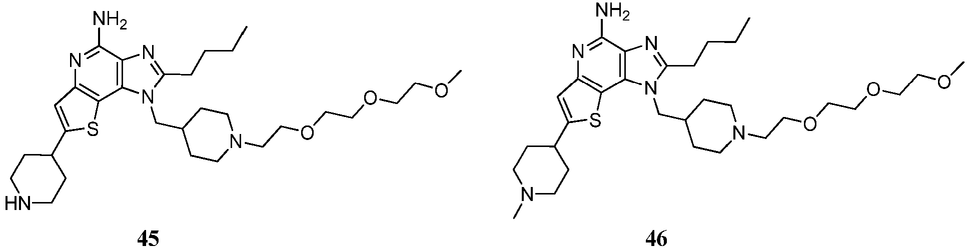

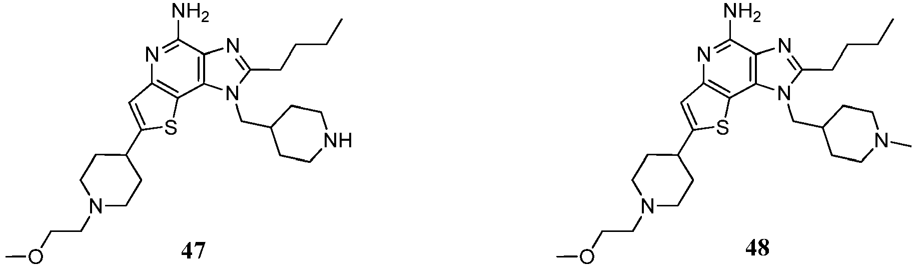

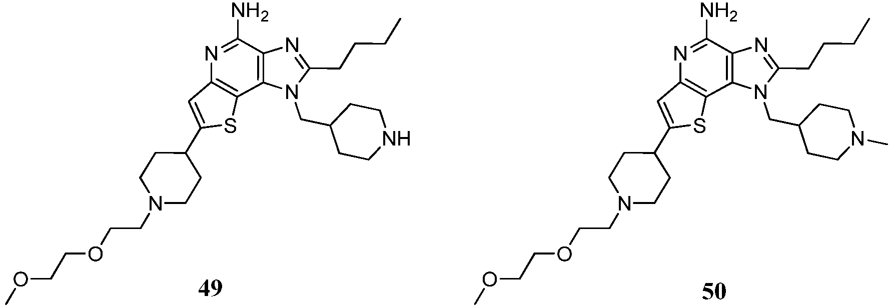

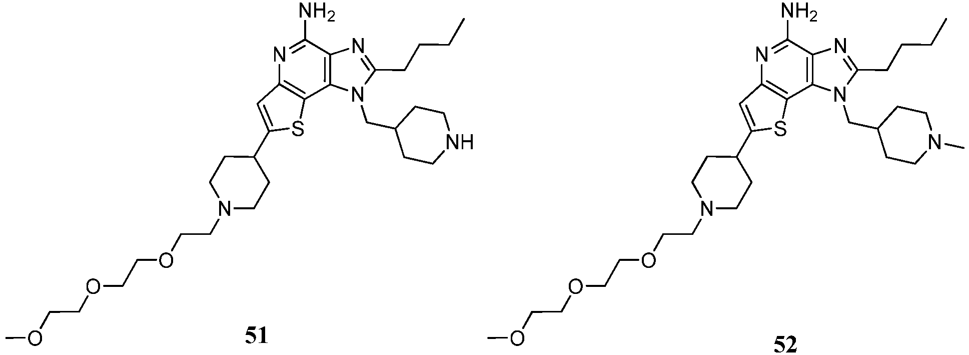

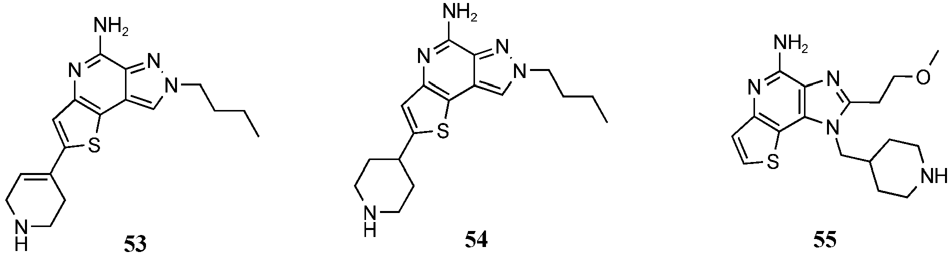

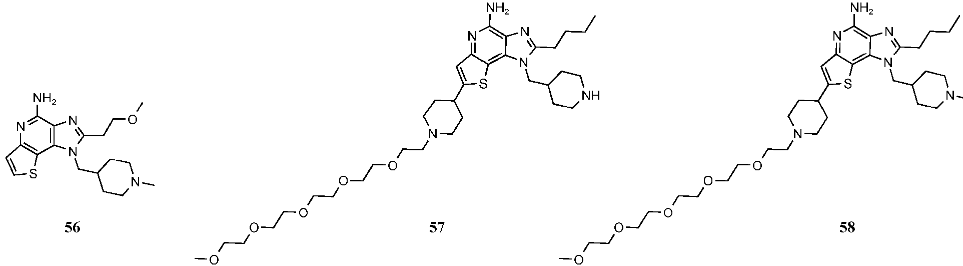

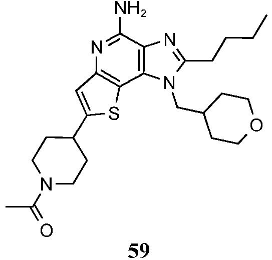

The present invention also provides a drug-linker or a pharmaceutically acceptable salt, ester, stereoisomer, tautomer, polymorph, solvate, *N*-oxide, isotope-labeled compound, metabolite or prodrug thereof: D-L'
wherein, D is a fragment of the aforementioned compound;
L' is a linker.

In some embodiments, L' is selected from the group consisting of -L⁹-L¹⁰-L¹¹, wherein,
L⁹ is absent or selected from 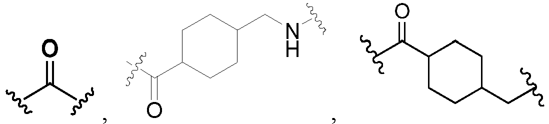 and 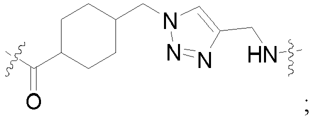
L¹⁰ is absent or selected from the group consisting of -(CH₂CH₂O)ₛCH₂CH₂NH-, - (OCH₂CH₂)ₛNH- and -C(O)-(CH₂CH₂O)ₛCH₂CH₂NH-; s is selected from the group consisting of integers 1 to 10;
L¹¹ is 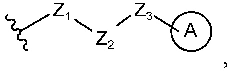 wherein,
Z₁ is selected from the group consisting of a chemical bond and
Z₂ is selected from the group consisting of a chemical bond and C₁₋₂₀ alkylene; 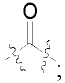
Z₃ is selected from the group consisting of a chemical bond, C₂₋₆ alkenylene and C₂₋₆ alkynylene;
A is selected from the group consisting of 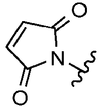 and 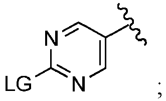
LG is a leaving group of nucleophilic substitution reaction, and selected from the group consisting of halogen, nitro, benzenesulfonate, p-toluenesulfonate, trifluoromethanesulfonate, - S(O)₂-R⁷ and -S(O)-R⁷;
R⁷ is selected from the group consisting of C₁₋₆ alkyl and C₁₋₆ haloalkyl.

In some embodiments, L' is selected from 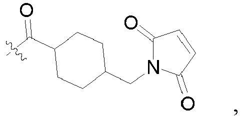 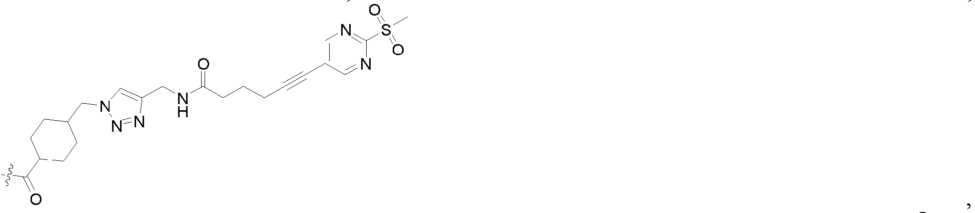 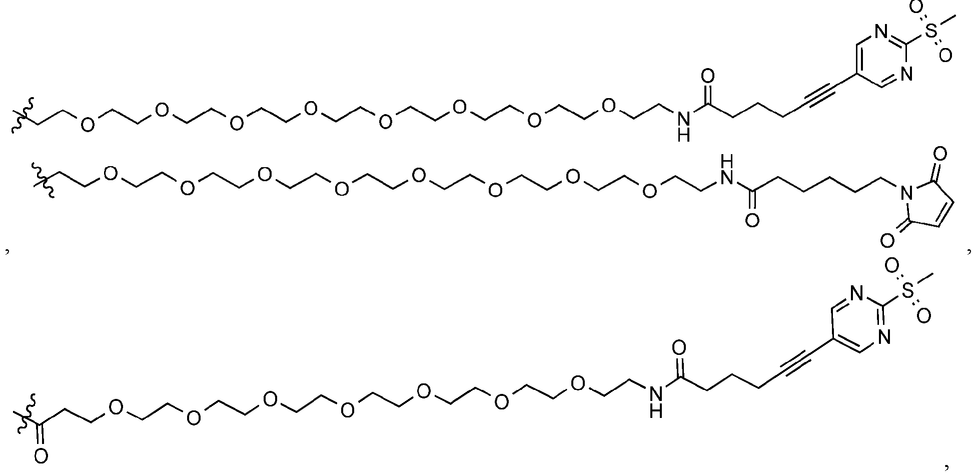 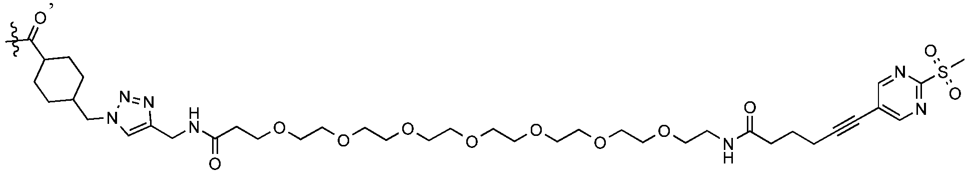 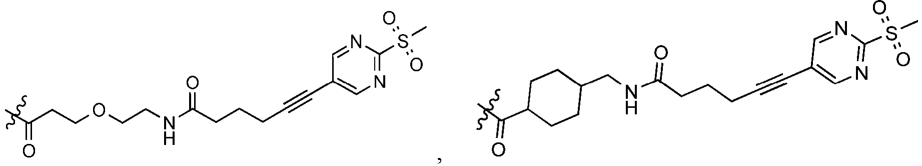 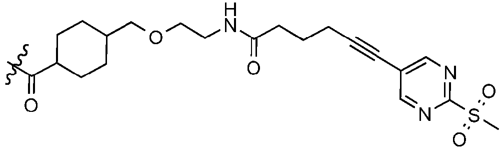 and

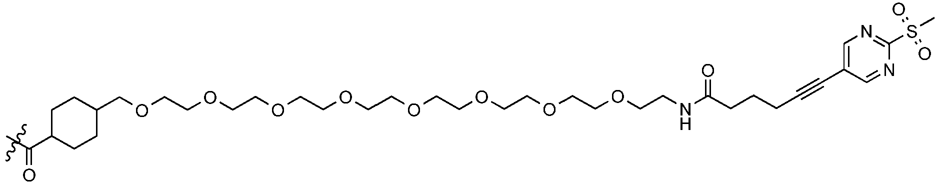

The present invention also provides a drug-linker or a pharmaceutically acceptable salt, ester, stereoisomer, tautomer, polymorph, solvate, *N*-oxide, isotope-labeled compound, metabolite or prodrug thereof, wherein D-L' has a structure as follows:

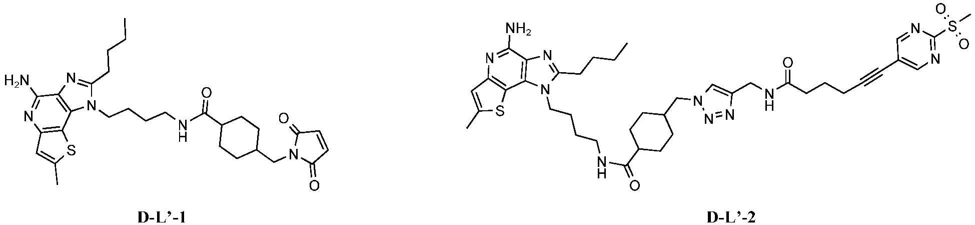

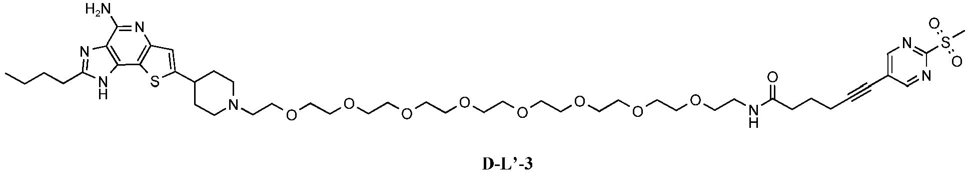

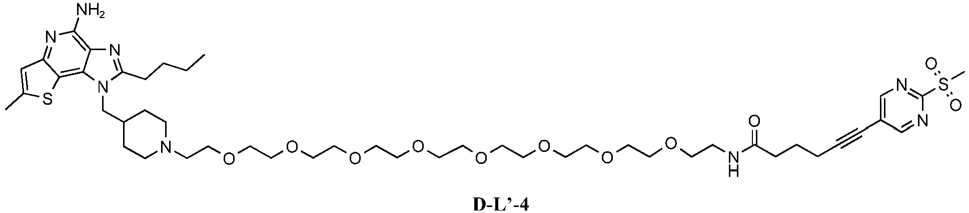

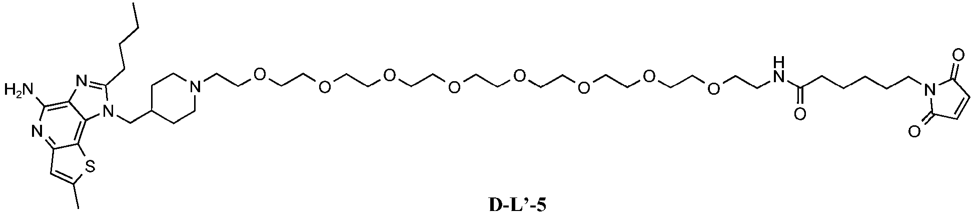

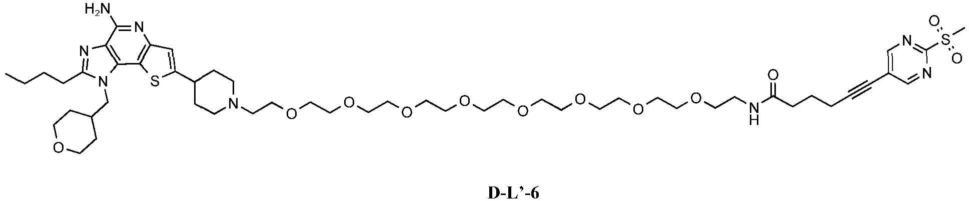

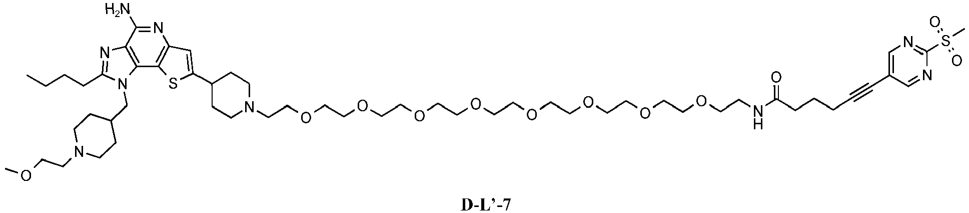

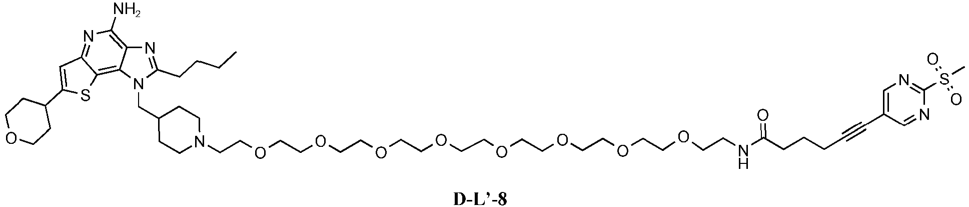

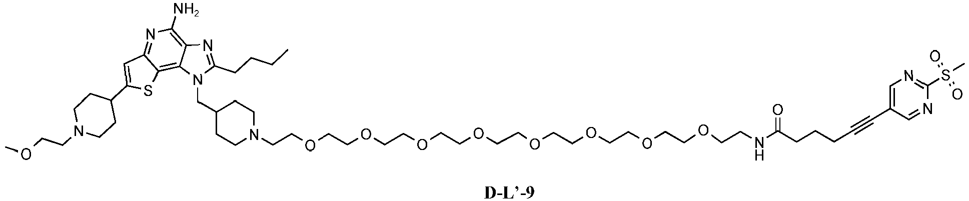

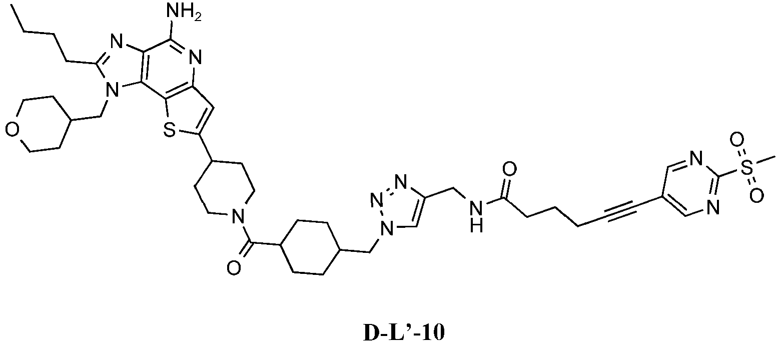

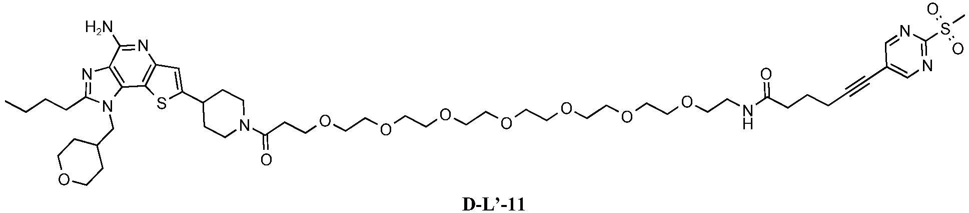

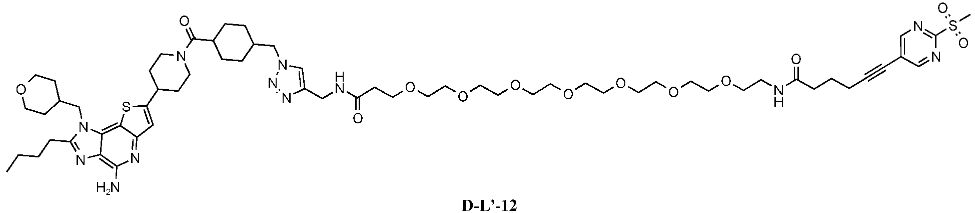

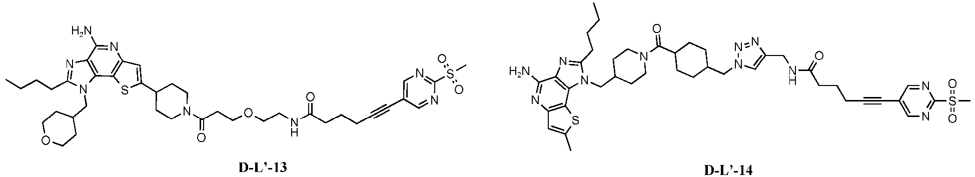

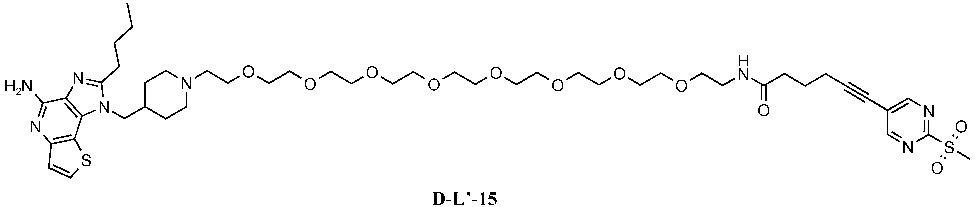

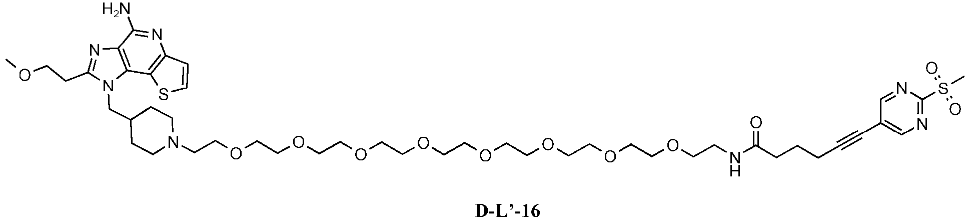

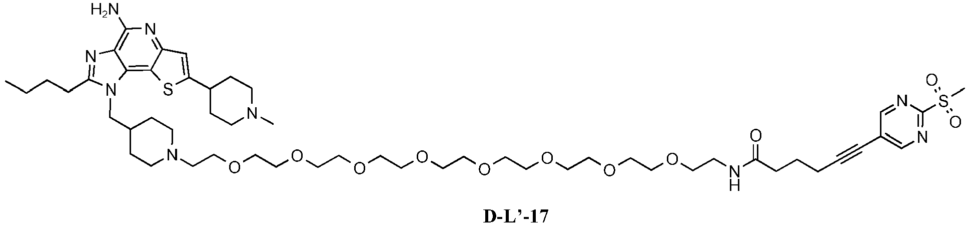

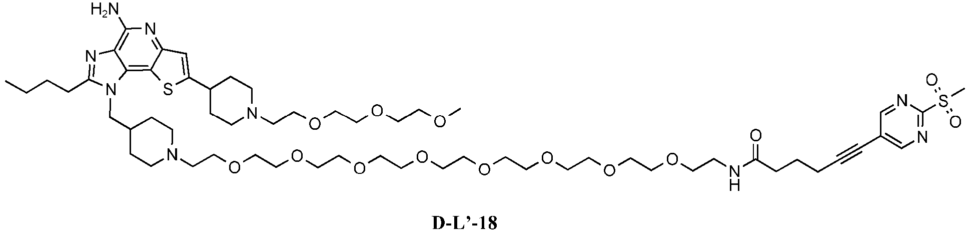

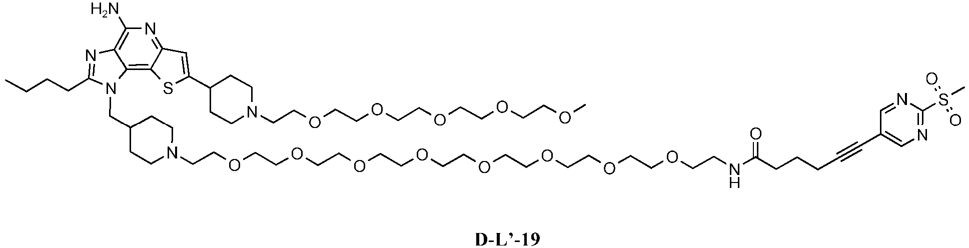

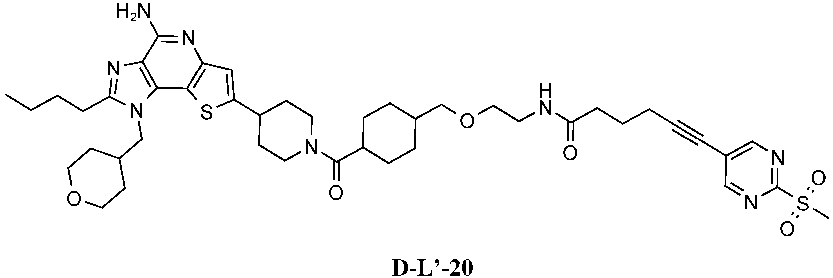

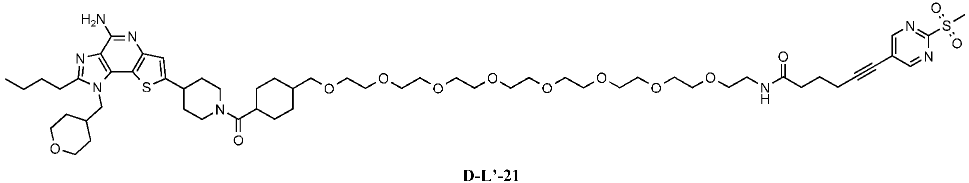

The present invention also provides an immune-stimulating antibody conjugate of Formula (ISAC-I) or a pharmaceutically acceptable salt, ester, stereoisomer, tautomer, polymorph, solvate, N-oxide, isotope-labeled compound, metabolite or prodrug thereof: 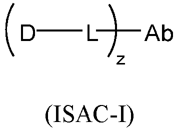 wherein,
D is a fragment of the compound as described in any one of the preceding items;
L is a fragment of the linker L', wherein L' is as defined in any of the preceding items;
Ab is an antibody capable of targeting to a target antigen, optionally, the antibody is modified (e.g., modified by a cross-linking agent);
z is selected from the group consisting of 1 to 10;
preferably, the immune-stimulating antibody conjugate has a DAR value of 1 to 10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 1 to 8, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 1.0 to 5.5, 1.0 to 6.0, 1.0 to 6.5, 1.0 to 7.0, 1.0 to 7.5, 1.0 to 8.0, 1.5 to 2.0, 1.5 to 2.5, 1.5 to 3.0, 1.5 to 3.5, 1.5 to 4.0, 1.5 to 4.5, 1.5 to 5.0, 1.5 to 5.5, 1.5 to 6.0, 1.5 to 6.5, 1.5 to 7.0, 1.5 to 7.5, 1.5 to 8.0, 2.0 to 2.5, 2.0 to 3.0, 2.0 to 3.5, 2.0 to 4.0, 2.0 to 4.5, 2.0 to 5.0, 2.0 to 5.5, 2.0 to 6.0, 2.0 to 6.5, 2.0 to 7.0, 2.0 to 7.5, 2.0 to 8.0, 2.5 to 3.0, 2.5 to 3.5, 2.5 to 4.0, 2.5 to 4.5, 2.5 to 5.0, 2.5 to 5.5, 2.5 to 6.0, 2.5 to 6.5, 2.5 to 7.0, 2.5 to 7.5, 2.5 to 8.0, 3.0 to 3.5, 3.0 to 4.0, 3.0 to 4.5, 3.0 to 5.0, 3.0 to 5.5, 3.0 to 6.0, 3.0 to 6.5, 3.0 to 7.0, 3.0 to 7.5, 3.0 to 8.0, 3.5 to 4.0, 3.5 to 4.5, 3.5 to 5.0, 3.5 to 5.5, 3.5 to 6.0, 3.5 to 6.5, 3.5 to 7.0, 3.5 to 7.5, 3.5 to 8.0, 4.0 to 4.5, 4.0 to 5.0, 4.0 to 5.5, 4.0 to 6.0, 4.0 to 6.5, 4.0 to 7.0, 4.0 to 7.5, 4.0 to 8.0, 4.5 to 5.0, 4.5 to 5.5, 4.5 to 6.0, 4.5 to 6.5, 4.5 to 7.0, 4.5 to 7.5, 4.5 to 8.0, 5.0 to 5.5, 5.0 to 6.0, 5.0 to 6.5, 5.0 to 7.0, 5.0 to 7.5, 5.0 to 8.0, 5.5 to 6.0, 5.5 to 6.5, 5.5 to 7.0, 5.5 to 7.5, 5.5 to 8.0, 6.0 to 6.5, 6.0 to 7.0, 6.0 to 7.5, 6.0 to 8.0, 6.5 to 7.0, 6.5 to 7.5, 6.5 to 8.0, 7.0 to 7.5, 7.0 to 8.0 or 7.5 to 8.0.

In some embodiments, the L is a structure formed by an addition reaction or nucleophilic substitution reaction of the aforementioned L'. In some specific embodiments, the L is the corresponding linker fragment with a succinimide moiety generated by an addition reaction of the maleimide in L', or, L is a linker formed by the removal of a leaving group (e.g., methylsulfonyl) of L' via an nucleophilic substitution reaction.

In some embodiments, the cross-linking agent contains two identical or different reactive groups (e.g., carboxyl, carbonyl, ester, hydroxyl, thiol, amino, etc.). In some embodiments, the reactive groups in the cross-linking agent are free or protected. In some embodiments, the antibody reacts with one reactive group in the cross-linking agent to obtain the cross-linking modified antibody. In some embodiments, in the cross-linking modified antibody, another reactive group of the cross-linking agent moiety that is not reacted with the antibody is in a protected or deprotected form. In some embodiments, one of the two reactive groups of the cross-linking agent is linked to the antibody, and the other is linked to L. In some embodiments, the cross-linking agent is the cross-linking agent SATA. In some embodiments, the Formula (ISAC-I) has a structure as follows:

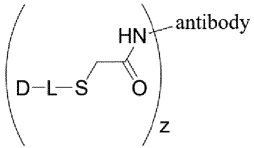

The present invention also provides an immune-stimulating antibody conjugate of the Formula (ISAC-I) or a pharmaceutically acceptable salt, ester, stereoisomer, tautomer, polymorph, solvate, N-oxide, isotope-labeled compound, metabolite or prodrug thereof: 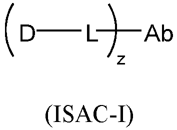 wherein,
D is selected from the group consisting of a fragment of the compound of Formula (I);
L is a linker;
Ab is an antibody capable of targeting to a target antigen, such as an antibody targeting to a tumor cell; optionally, the antibody is modified (e.g., modified by a cross-linking agent); such as Trastuzumab monoclonal antibody or Trastuzumab monoclonal antibody modified by the cross-linking agent SATA;
z is selected from the group consisting of 1 to 10.

### Preparation method

Another object of the present invention is to provide a method for preparing the compound of the present invention. For example, the present invention provides a method for preparing a compound of Formula (II-A), wherein R² is not hydrogen and R³ is not hydrogen, comprising the steps of: 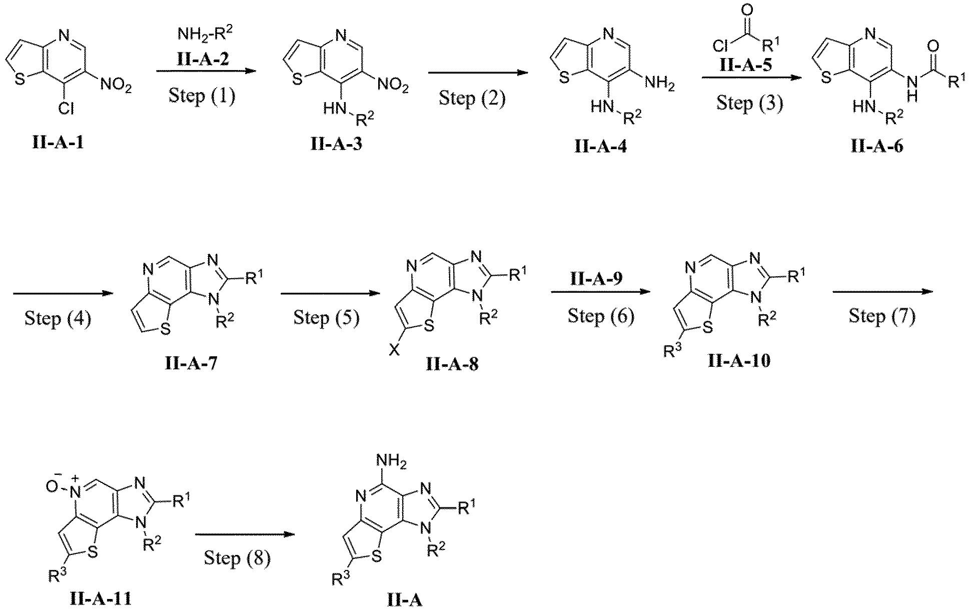 wherein, X represents halogen atom, including chlorine, bromine and iodine.

When R³ is methyl, II-A-9 is trimethylboroxane. When R³ is not methyl, II-A-9 is 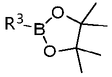 R¹, R² and R³ are as defined above, and neither R² nor R³ is hydrogen;

### (1) Reacting compound II-A-1 with compound II-A-2 to obtain compound II-A-3;

The reaction is preferably carried out in a suitable organic solvent. The organic solvent may be selected from the group consisting of *N,N-*dimethylformamide, N,N-dimethylacetamide, *N-*methylpyrrolidone, toluene, tetrahydrofuran and any combination thereof, preferably *N,N-*dimethylformamide. The reaction is preferably carried out in the presence of a suitable organic or inorganic base, which may be selected from the group consisting of*N,N*-diisopropylethylamine, triethylamine, 4-dimethylaminopyridine, potassium carbonate, sodium carbonate, preferably *N,N-*diisopropylethylamine. The reaction is preferably carried out at a suitable temperature, preferably -10 to 80 °C. The reaction is preferably carried out for a suitable period of time, such as 1 to 12 hours.

### (2) Subjecting compound II-A-3 to a reduction reaction to obtain compound II-A-4;

The reaction is preferably carried out under a reducing system of sodium hydrosulfite, palladium metal catalyst or platinum metal catalyst and hydrogen. The palladium metal catalyst or platinum metal catalyst may be selected from the group consisting of palladium on carbon, palladium hydroxide, platinum dioxide, preferably palladium on carbon. The reaction is preferably carried out in a suitable solvent, which may be selected from the group consisting of methanol, ethanol, isopropanol, ethyl acetate, tetrahydrofuran, water and any combination thereof. The reaction is preferably carried out at a suitable temperature, preferably -10 to 80 °C. The reaction is preferably carried out for a suitable period of time, such as 2 to 16 hours.

### (3) Reacting compound II-A-4 with compound II-A-5 to obtain compound II-A-6;

The reaction is preferably carried out in a suitable organic solvent. The organic solvent may be selected from the group consisting of *N,N-*dimethylformamide, *N,N-*dimethylacetamide, *N-*methylpyrrolidone, dichloromethane, tetrahydrofuran and any combination thereof, preferably tetrahydrofuran. The reaction is preferably carried out in the presence of a suitable organic or inorganic base, which may be selected from the group consisting of *N,N-*diisopropylethylamine, triethylamine, 4-dimethylaminopyridine, potassium carbonate, sodium carbonate, preferably *N,N-*diisopropylethylamine. The reaction is preferably carried out at a suitable temperature, preferably -10 to 60°C. The reaction is preferably carried out for a suitable period of time, such as 2 to 12 hours.

### (4) Subjecting compound II-A-6 to a ring-closing reaction to obtain compound II-A-7;

The reaction is preferably carried out in a suitable organic solvent. The organic solvent may be selected from the group consisting of toluene, xylene, mesitylene, *N,N-*dimethylformamide, *N-*methylpyrrolidone and any combination thereof, preferably toluene. The reaction is preferably carried out with the participation of o-chlorobenzoic acid. The reaction is preferably carried out at a suitable temperature, preferably 50 to 150°C. The reaction is preferably carried out for a suitable period of time, such as 2 to 12 hours.

### (5) Subjecting compound II-A-7 to a halogenation reaction to obtain compound II-A-8;

The reaction is preferably carried out in a suitable organic solvent. The organic solvent may be selected from the group consisting of *N,N-*dimethylformamide, *N,N*-dimethylacetamide, *N-*methylpyrrolidone, formic acid, glacial acetic acid and any combination thereof, preferably a combination of *N,N-*dimethylformamide and glacial acetic acid. The reaction is preferably carried out in the presence of a suitable halogenating reagent which may be selected from the group consisting of *N*-chlorosuccinimide, *N*-bromosuccinimide, dibromohydantoin, bromine, *N-*iodosuccinimide and any combination thereof, preferably *N*-bromosuccinimide. The reaction is preferably carried out at a suitable temperature, preferably -10 to 60°C. The reaction is preferably carried out for a suitable period of time, such as 2 to 36 hours.

### (6) Subjecting compound II-A-8 and compound II-A-9 to a coupling reaction to obtain compound II-A-10;

The coupling reaction is preferably carried out in the presence of a metal catalyst and a base. Preferably, the metal catalyst is a palladium metal catalyst such as tris(dibenzylideneacetone)dipalladium, 1,1'-bisdiphenylphosphinoferrocene palladium dichloride, tetrakis(triphenylphosphine) palladium, palladium acetate, preferably 1,1'-bisdiphenylphosphinoferrocene palladium dichloride. The base is an organic base or an inorganic base, such as *N,N*-diisopropylethylamine, triethylamine, sodium tert-butoxide, potassium carbonate, cesium carbonate, sodium carbonate, preferably potassium carbonate. The reaction is preferably carried out in a suitable solvent, which can be selected from the group consisting of *N,N*-dimethylformamide, *N-*methylpyrrolidone, toluene, ethanol, ethylene glycol dimethyl ether, 1,4-dioxane, water and any combination thereof, preferably a combination of 1,4-dioxane and water. The reaction is preferably carried out at a suitable temperature, preferably 60 to 150°C. The reaction is preferably carried out for a suitable period of time, such as 1 to 12 hours.

### (7) Reacting compound II-A-10 to obtain compound II-A-11;

The reaction is preferably carried out in a suitable organic solvent. The organic solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of a suitable oxidant, which can be selected from the group consisting of m-chloroperoxybenzoic acid, hydrogen peroxide, tert-butyl hydroperoxide and any combination thereof, preferably m-chloroperoxybenzoic acid. The reaction is preferably carried out at a suitable temperature, preferably -10 to 80°C. The reaction is preferably carried out for a suitable period of time, such as 1 to 12 hours.

### (8) Reacting compound II-A-11 to obtain compound II-A;

The reaction is preferably carried out in a suitable solvent. The solvent may be selected from the group consisting of *N,N-*dimethylformamide, dichloromethane, chloroform, tetrahydrofuran, ethyl acetate, ammonia water and any combination thereof, preferably a combination of dichloromethane and ammonia water. The reaction is preferably carried out in the presence of p-toluenesulfonyl chloride. The reaction is preferably carried out at a suitable temperature, preferably -10 to 80°C. The reaction is preferably carried out for a suitable period of time, such as 2 to 24 hours.

In particular, the present invention also provides a method for preparing a compound of Formula (II-A'), wherein R³ is hydrogen and R² is not hydrogen, comprising the following steps: 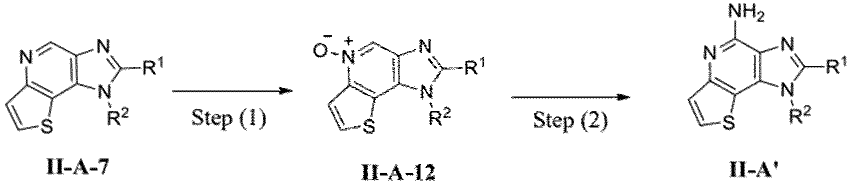 wherein, R¹ and R² are as defined above, and R² is not hydrogen;

### (1) Reacting compound II-A-7 to obtain compound II-A-12;

The reaction is preferably carried out in a suitable organic solvent. The organic solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of a suitable oxidant, which may be selected from the group consisting of m-chloroperoxybenzoic acid, hydrogen peroxide, tert-butyl hydroperoxide and any combination thereof, preferably m-chloroperoxybenzoic acid. The reaction is preferably carried out at a suitable temperature, preferably -10 to 80°C. The reaction is preferably carried out for a suitable period of time, such as 1 to 12 hours.

### (2) Reacting compound II-A-12 to obtain compound II-A';

The reaction is preferably carried out in a suitable solvent. The solvent may be selected from the group consisting of *N,N-*dimethylformamide, dichloromethane, chloroform, tetrahydrofuran, ethyl acetate, ammonia water and any combination thereof, preferably a combination of dichloromethane and ammonia water. The reaction is preferably carried out in the presence of p-toluenesulfonyl chloride. The reaction is preferably carried out at a suitable temperature, preferably -10 to 80°C. The reaction is preferably carried out for a suitable period of time, such as 2 to 24 hours.

Particularly, the present invention also provides a method for preparing a compound of Formula (II-A"), wherein R² is hydrogen and R³ is not hydrogen, comprising the following steps: 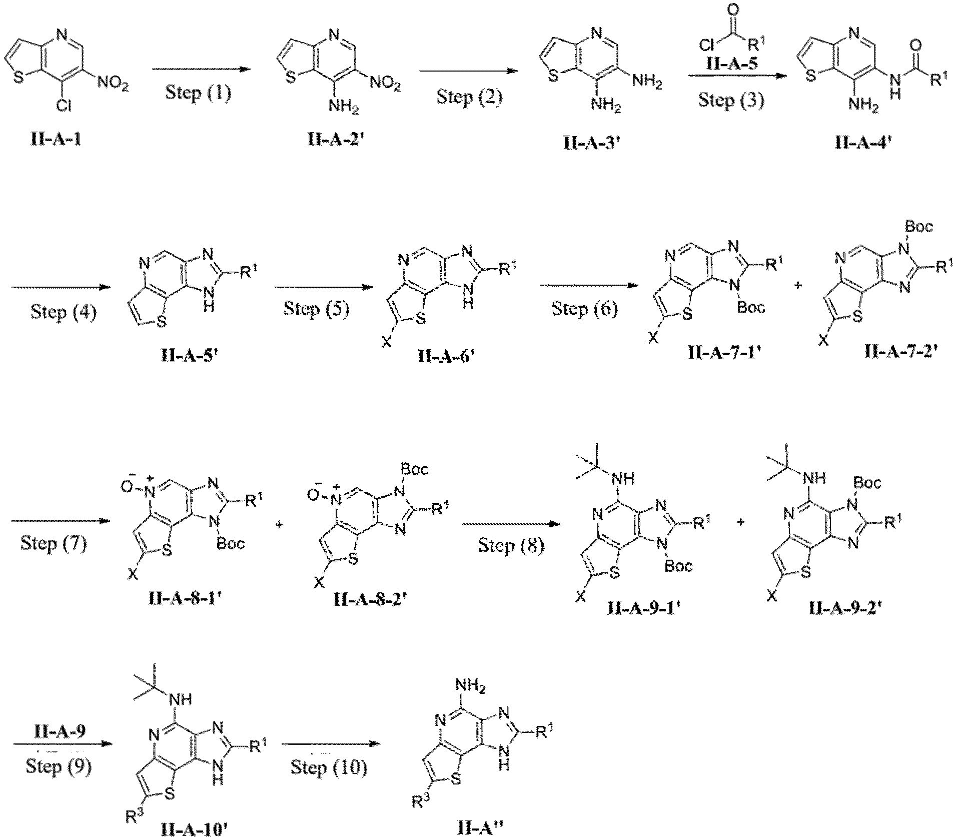 wherein, X represents halogen atom, including chlorine, bromine and iodine.

When R³ is methyl, **II-A-9** is trimethylboroxine. When R³ is not methyl, **I-A-9** is 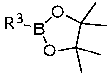 R¹ and R³ are as defined above, and R³ is not hydrogen;

### (1) Subjecting compound II-A-1 to a substitution reaction with ammonia in methanol to obtain compound II-A-2';

The reaction is preferably carried out in a suitable organic solvent. The organic solvent may be selected from the group consisting of methanol, ethanol, isopropanol, 1,4-dioxane, tetrahydrofuran and any combination thereof, preferably methanol. The reaction is preferably carried out at a suitable temperature, preferably 0 to 100°C. The reaction is preferably carried out for a suitable period of time, such as 1 to 12 hours.

### (2) Subjecting compound II-A-2' to a reduction reaction to obtain compound II-A-3';

The reaction is preferably carried out under a reducing system of sodium hydrosulfite, palladium metal catalyst or platinum metal catalyst and hydrogen. The palladium metal catalyst or platinum metal catalyst may be selected from the group consisting of palladium on carbon, palladium hydroxide, platinum dioxide, preferably palladium on carbon. The reaction is preferably carried out in a suitable solvent, which may be selected from the group consisting of methanol, ethanol, isopropanol, ethyl acetate, tetrahydrofuran, water and any combination thereof. The reaction is preferably carried out at a suitable temperature, preferably -10 to 80°C. The reaction is preferably carried out for a suitable period of time, such as 2 to 16 hours.

### (3) Reacting compound II-A-3' with compound II-A-5 to obtain compound II-A-4';

The reaction is preferably carried out in a suitable organic solvent. The organic solvent may be selected from the group consisting of *N,N-*dimethylformamide, *N,N-*dimethylacetamide, *N-*methylpyrrolidone, dichloromethane, tetrahydrofuran and any combination thereof, preferably tetrahydrofuran. The reaction is preferably carried out in the presence of a suitable organic or inorganic base, which may be selected from the group consisting of *N,N-*diisopropylethylamine, triethylamine, 4-dimethylaminopyridine, potassium carbonate, sodium carbonate, preferably *N,N-*diisopropylethylamine. The reaction is preferably carried out at a suitable temperature, preferably -10 to 60°C. The reaction is preferably carried out for a suitable period of time, such as 2 to 12 hours.

### (4) Subjecting compound II-A-4' to a ring-closing reaction to obtain compound II-A-5';

The reaction is preferably carried out in a suitable organic solvent. The organic solvent may be selected from the group consisting of methanol, ethanol, isopropanol, 1,4-dioxane, tetrahydrofuran and any combination thereof, preferably ethanol. The reaction is preferably carried out in the presence of a suitable organic or inorganic base, which may be selected from the group consisting of *N,N-*diisopropylethylamine, triethylamine, sodium hydroxide, potassium hydroxide, potassium carbonate, sodium carbonate, preferably sodium hydroxide. The reaction is preferably carried out at a suitable temperature, preferably 0 to 100°C. The reaction is preferably carried out for a suitable period of time, such as 2 to 12 hours.

### (5) Subjecting compound II-A-5' to a halogenation reaction to obtain compound II-A-6';

The reaction is preferably carried out in a suitable organic solvent. The organic solvent may be selected from the group consisting of *N,N-*dimethylformamide, *N,N*-dimethylacetamide, *N-*methylpyrrolidone, formic acid, glacial acetic acid and any combination thereof, preferably a combination of *N,N-*dimethylformamide and glacial acetic acid. The reaction is preferably carried out in the presence of a suitable halogenating reagent which may be selected from the group consisting of *N*-chlorosuccinimide, *N*-bromosuccinimide, dibromohydantoin, bromine, *N-*iodosuccinimide and any combination thereof, preferably *N*-bromosuccinimide. The reaction is preferably carried out at a suitable temperature, preferably -10 to 60°C. The reaction is preferably carried out for a suitable period of time, such as 2 to 36 hours.

### (6) Reacting compound II-A-6' with di-tert-butyl dicarbonate to obtain a mixture of compound II-A-7-1' and compound II-A-7-2';

The reaction is preferably carried out in a suitable organic solvent. The organic solvent may be selected from the group consisting of *N,N*-dimethylformamide, dichloromethane, tetrahydrofuran and any combination thereof, preferably tetrahydrofuran. The reaction is preferably carried out in the presence of a suitable organic or inorganic base, which may be selected from the group consisting of *N,N*-diisopropylethylamine, triethylamine, 4-dimethylaminopyridine, potassium carbonate, sodium carbonate, preferably a mixture of triethylamine and 4-dimethylaminopyridine. The reaction is preferably carried out at a suitable temperature, preferably -10 to 60°C. The reaction is preferably carried out for a suitable period of time, such as 2 to 24 hours.

### (7) Subjecting the mixture of compound II-A-7-1' and compound II-A-7-2' to a reaction to obtain a mixture of compound II-A-8-1' and compound II-A-8-2';

The reaction is preferably carried out in a suitable organic solvent. The organic solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of a suitable oxidant, which can be selected from the group consisting of m-chloroperoxybenzoic acid, hydrogen peroxide, tert-butyl hydroperoxide and any combination thereof, preferably m-chloroperoxybenzoic acid. The reaction is preferably carried out at a suitable temperature, preferably -10 to 80°C. The reaction is preferably carried out for a suitable period of time, such as 1 to 12 hours.

### (8) Reacting the mixture of compound II-A-8-1' and compound II-A-8-2' with tert-butylamine to obtain a mixture of compound II-A-9-1' and compound II-A-9-2';

The reaction is preferably carried out in a suitable solvent. The solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of p-toluenesulfonyl chloride. The reaction is preferably carried out at a suitable temperature, preferably -10 to 80°C. The reaction is preferably carried out for a suitable period of time, such as 2 to 24 hours.

### (9) Subjecting the mixture of compound II-A-9-1' and compound II-A-9-2' to a coupling reaction with compound II-A-9 to obtain compound II-A-10';

The coupling reaction is preferably carried out in the presence of a metal catalyst and a base. Preferably, the metal catalyst is a palladium metal catalyst such as tris(dibenzylideneacetone)dipalladium, 1,1'-bisdiphenylphosphinoferrocene palladium dichloride, tetrakis(triphenylphosphine)palladium, palladium acetate, preferably 1,1'-bisdiphenylphosphinoferrocene palladium dichloride. The base is an organic base or an inorganic base, such as *N,N-*diisopropylethylamine, triethylamine, sodium tert-butoxide, potassium carbonate, cesium carbonate, sodium carbonate, preferably potassium carbonate. The reaction is preferably carried out in a suitable solvent, which may be selected from the group consisting of *N,N*-dimethylformamide, *N-*methylpyrrolidone, toluene, ethanol, ethylene glycol dimethyl ether, 1,4-dioxane, water and any combination thereof, preferably a combination of 1,4-dioxane and water. The reaction is preferably carried out at a suitable temperature, preferably 60 to 150°C. The reaction is preferably carried out for a suitable period of time, such as 1 to 12 hours.

### (10) Subjecting compound II-A-10' to a reaction to obtain compound II-A";

The organic solvent may be selected from the group consisting of methanol, ethanol, isopropanol, 1,4-dioxane and any combination thereof, preferably methanol. The reaction is preferably carried out in the presence of a suitable inorganic acid selected from the group consisting of sulfuric acid, hydrochloric acid, hydrobromic acid and any combination thereof, preferably hydrochloric acid. The reaction is preferably carried out at a suitable temperature, preferably -10 to 80°C. The reaction is preferably carried out for a suitable period of time, such as 1 to 12 hours.

In particular, the present invention provides a method for preparing a compound of Formula (II-A), wherein R² is not hydrogen and R³ is not hydrogen, comprising the steps of: 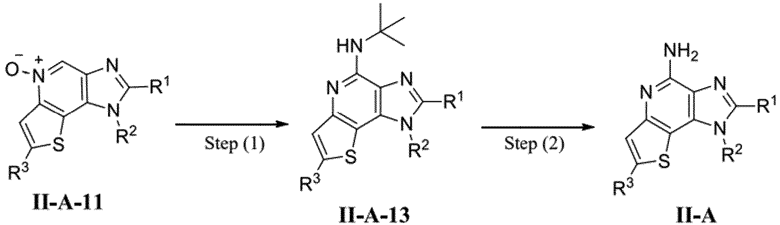 wherein, R¹, R² and R³ are as defined above, and R² and R³ are not hydrogen;

### (1) Reacting compound II-A-11 with tert-butylamine to obtain compound II-A-13;

The reaction is preferably carried out in a suitable solvent. The solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of p-toluenesulfonyl chloride. The reaction is preferably carried out at a suitable temperature, preferably -10 to 80°C. The reaction is preferably carried out for a suitable period of time, such as 2 to 24 hours.

### (2) Subjecting compound II-A-13 to a reaction to obtain compound II-A;

The reaction is preferably carried out in a suitable organic solvent. The organic solvent may be selected from the group consisting of methanol, ethanol, isopropanol, 1,4-dioxane and any combination thereof, preferably methanol. The reaction is preferably carried out in the presence of a suitable inorganic acid selected from the group consisting of sulfuric acid, hydrochloric acid, hydrobromic acid and any combination thereof, preferably hydrochloric acid. The reaction is preferably carried out at a suitable temperature, preferably -10 to 80°C. The reaction is preferably carried out for a suitable period of time, such as 1 to 12 hours.

In particular, the present invention also provides a method for preparing a compound of Formula (II-A) wherein R² is not hydrogen and R³ is not hydrogen, comprising the following steps: 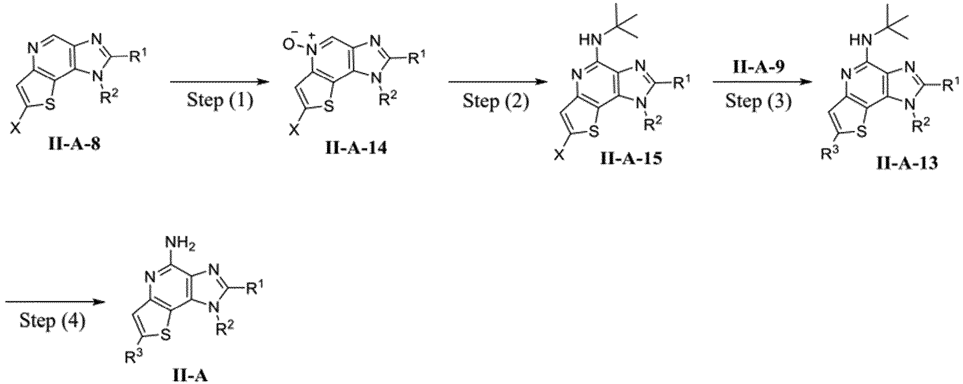 wherein, X represents halogen atom, including chlorine, bromine and iodine.

When R³ is methyl, **II-A-9** is trimethylboroxine. When R³ is not methyl, **II-A-9** is 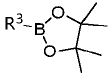 R¹, R² and R³ are as defined above, and neither R² nor R³ is hydrogen;

### (1) Reacting compound II-A-8 to obtain compound II-A-14;

The reaction is preferably carried out in a suitable organic solvent. The organic solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of a suitable oxidant, which may be selected from the group consisting of m-chloroperoxybenzoic acid, hydrogen peroxide, tert-butyl hydroperoxide and any combination thereof, preferably m-chloroperoxybenzoic acid. The reaction is preferably carried out at a suitable temperature, preferably -10 to 80°C. The reaction is preferably carried out for a suitable period of time, such as 1 to 12 hours.

### (2) Reacting compound II-A-14 with tert-butylamine to obtain compound II-A-15;

The reaction is preferably carried out in a suitable solvent. The solvent may be selected from the group consisting of *N,N-*dimethylformamide, dichloromethane, chloroform, tetrahydrofuran, ethyl acetate and any combination thereof, preferably dichloromethane. The reaction is preferably carried out in the presence of p-toluenesulfonyl chloride. The reaction is preferably carried out at a suitable temperature, preferably -10 to 80°C. The reaction is preferably carried out for a suitable period of time, such as 2 to 24 hours.

### (3) Subjecting compound II-A-15 and compound II-A-9 to a coupling reaction to obtain compound II-A-13;

The coupling reaction is preferably carried out in the presence of a metal catalyst and a base. Preferably, the metal catalyst is a palladium metal catalyst such as tris(dibenzylideneacetone)dipalladium, 1,1'-bisdiphenylphosphinoferrocene palladium dichloride, tetrakis(triphenylphosphine)palladium, palladium acetate, preferably 1,1'-bisdiphenylphosphinoferrocene palladium dichloride. The base is an organic base or an inorganic base, such as *N,N*-disopropylethylamine, triethylamine, sodium tert-butoxide, potassium carbonate, cesium carbonate, sodium carbonate, preferably potassium carbonate. The reaction is preferably carried out in a suitable solvent, which may be selected from the group consisting of *N,N*-dimethylformamide, *N*-methylpyrrolidone, toluene, ethanol, ethylene glycol dimethyl ether, 1,4-dioxane, water and any combination thereof, preferably a combination of 1,4-dioxane and water. The reaction is preferably carried out at a suitable temperature, preferably 60 to 150°C. The reaction is preferably carried out for a suitable period of time, such as 1 to 12 hours.

### (4) Subjecting compound II-A-13 to a reaction to obtain compound II-A;

The reaction is preferably carried out in a suitable organic solvent. The organic solvent may be selected from the group consisting of methanol, ethanol, isopropanol, 1,4-dioxane and any combination thereof, preferably methanol. The reaction is preferably carried out in the presence of a suitable inorganic acid selected from the group consisting of sulfuric acid, hydrochloric acid, hydrobromic acid and any combination thereof, preferably hydrochloric acid. The reaction is preferably carried out at a suitable temperature, preferably -10 to 80°C. The reaction is preferably carried out for a suitable period of time, such as 1 to 12 hours.

The specific conditions of the above-mentioned reaction steps are well known in the art, and the present invention is not specifically limited thereto. According to the teaching of the present invention combined with common knowledge in the field, those skilled in the art can select and replace each substituent in the general formula to prepare different compounds, and these selections and replacements are all within the protection scope of the present invention.

### Pharmaceutical composition and kit

Another object of the present invention is to provide a pharmaceutical composition, comprising a prophylactically or therapeutically effective amount of the compound or pharmaceutically acceptable salt, ester, stereoisomer, tautomer, polymorph, solvate, N-oxide, isotope-labeled compound, metabolite or prodrug thereof of the present invention, and one or more pharmaceutically acceptable carriers.

Another object of the present invention is to provide a kit, comprising the compound or pharmaceutically acceptable salt, ester, stereoisomer, tautomer, polymorph, solvate, N-oxide, isotope-labeled compound, metabolite or prodrug thereof of the present invention, or the pharmaceutical composition of the present invention. Optionally, the kit further comprises an instruction for use.

The "pharmaceutically acceptable carrier" in the present invention refers to a diluent, adjuvant, excipient or vehicle administered together with a therapeutic agent, and it is suitable for contacting a tissue from human being and/or other animal without undue toxicity, irritation, allergic response or other problems or complications commensurate with a reasonable benefit/risk ratio.

The pharmaceutically acceptable carrier that can be used in the pharmaceutical composition of the present invention include, but is not limited to, sterile liquid. The pharmaceutical composition may, for example, be in the form of a solid preparation, a semi-solid preparation, a liquid preparation or a gaseous preparation or the like.

The pharmaceutical composition of the present invention may act systemically and/or topically. For this purpose, the pharmaceutical composition may be administered by a suitable route, for example by injection or transdermal administration; or by oral or inhalation administration.

The content or amount of the compound of the present invention in the pharmaceutical composition may be about 0.001 mg to about 1000 mg, suitably 0.01 to 800 mg, preferably 0.05 to 500 mg.

In some embodiments, the present invention provides a method for preparing the pharmaceutical composition of the present invention, which comprises combining the compound or pharmaceutically acceptable salt, ester, stereoisomer, tautomer, polymorph, solvate, *N*-oxide, isotope-labeled compound, metabolite or prodrug thereof of the present invention with one or more pharmaceutically acceptable carriers.

### Therapeutic method and use

The compound or pharmaceutically acceptable salt, ester, stereoisomer, tautomer, polymorph, solvate, *N*-oxide, isotope-labeled compound, metabolite or prodrug thereof of the present invention, or the pharmaceutical composition of the present invention, has a strong agonistic effect on the target TLR7 and/or TLR8.

Another object of the present invention is to provide use of the compound of the present invention in the manufacture of an immune-stimulating antibody conjugate. In some embodiments, the immune-stimulating antibody conjugate comprises a small molecule ligand capable of interacting with TLR7 and/or TLR8.

Another object of the present invention is to provide use of the compound or pharmaceutically acceptable salt, ester, stereoisomer, tautomer, polymorph, solvate, *N*-oxide, isotope-labeled compound, metabolite or prodrug thereof of the present invention, or the immune-stimulating antibody conjugate of the present invention, or the pharmaceutical composition of the present invention, in the manufacture of a medicament for the prevention and/or treatment of a disease mediated by TLR7 and/or TLR8.

Another object of the present invention is to provide a method for preventing and/or treating a disease mediated by TLR7 and/or TLR8, comprising a step of administering to a subject in need thereof a prophylactically or therapeutically effective amount of the compound or pharmaceutically acceptable salt, ester, stereoisomer, tautomer, polymorph, solvate, *N*-oxide, isotope-labeled compound, metabolite or prodrug thereof of the present invention, or the immune-stimulating antibody conjugate of the present invention, or the pharmaceutical composition of the present invention.

In some embodiments, the disease mediated by TLR7 and/or TLR8 is preferably a tumor.

The term "effective amount" as used herein refers to an amount sufficient to achieve a desired prophylactic or therapeutic effect, for example, an amount that achieves alleviation of one or more symptoms associated with the disease being treated.

The dosage regimens may be adjusted to provide the optimum desired response. For example, a single bolus may be administered, several divided doses may be administered over time, or the dose may be proportionally reduced or increased as indicated by the exigencies of the therapeutic situation. It should be noted that dosage values may vary with the type and severity of the condition to be alleviated and may comprise single or multiple doses. It should be further understood that for any given individual, the particular dosage regimen will be adjusted over time according to the needs of the individual and the professional judgment of the person administering or supervising the administration of the compound of the present invention.

The amount of the compound of the present invention administered will depend on the individual being treated, the severity of disease or condition, the rate of administration, the treatment of the compound, and the judgment of the prescribing physician. In some cases, a dosage level up to the lower limit of the foregoing range may be sufficient, while in other cases, a larger dose may still be employed without causing any deleterious side effects, provided that the larger dose is first divided into several smaller doses to be administered throughout the whole day.

As used herein, unless otherwise stated, the term "treatment" refers to reversing, alleviating, ameliorating a disease or condition to which such term applies or a progression of one or more symptoms of such disease or condition.

The term "prevention" refers to inhibiting and delaying the onset of a disease, including not only the prevention before the development of the disease, but also the prevention of the recurrence of the disease after treatment.

The "subject" as used herein includes a human or non-human animal. Exemplary human subjects include a human subject suffering from a disease (e.g., a disease described herein) (referred to as a patient) or a normal subject. The "non-human animal" in the present invention includes all vertebrates, such as non-mammals (e.g., birds, amphibians, reptiles) and mammals, such as non-human primates, livestock and/or domesticated animals (e.g., sheep, dogs,, cats, cows, pigs, etc.).

### Brief Description of the Drawings

The accompanying drawings described herein are used to provide a further understanding of the present invention and constitute a part of the application. The schematic examples of the present invention and their descriptions are used to explain the present invention and do not constitute improper limitations to the present invention. In the drawings:

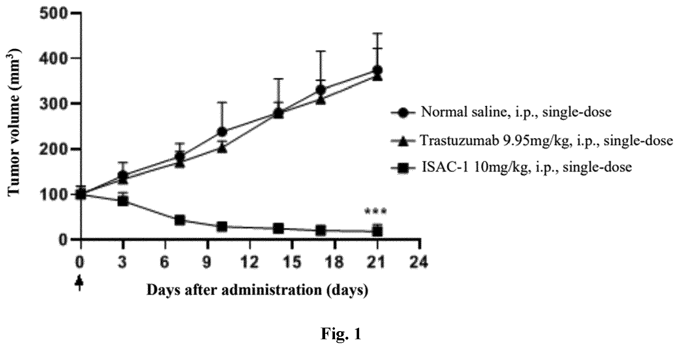

Fig. 1 shows curves of tumor volume changes in relation to time in the groups of Experimental Example 4.

### Specific Models for Carrying Out the present invention

To make the purpose and technical solution of the present invention clearer, the embodiments of the present invention will be described in detail below in conjunction with examples. However, those skilled in the art will understand that the following examples are only used to illustrate the present invention, and should not be deemed as any limitation to the scope of the present invention. Those without specific conditions in the examples were carried out according to conventional conditions or the conditions suggested by the manufacturer. The reagents or instruments used which manufacturers were not given were all commercially available conventional products.

Compound structures were determined by nuclear magnetic resonance (¹H NMR) or mass spectroscopy (MS). ¹H NMR was determined by JEOL Eclipse 400 NMR instrument, the measuring solvent was deuterated methanol (CD₃OD), deuterated chloroform (CDCl₃) or hexadeuteriodimethylsulfoxide (DMSO-*d₆*), and the internal standard was tetramethylsilane (TMS), and the chemical shifts (δ) were given in parts per million (ppm).

MS was determined by Agilent (ESI) mass spectrometer, manufacturer: Agilent, model: Agilent 6120B.

Preparation method with high performance liquid chromatography:
Instrument model: Agilent 1260, chromatographic column: Waters SunFire Prep C18 OBD (19 mm×150 mm×5.0 µm); chromatographic column temperature: 25°C; flow rate: 20.0 mL/min; detection wavelength: 214 nm; elution gradient: (0 min: 10%A, 90%B; 16.0 min: 90%A, 10%B); mobile phase A: acetonitrile; mobile phase B: 0.05% formic acid in water.

Aluminum plate (20×20 cm) produced by Merck was used as thin-layer chromatography silica gel plate (TLC), and GF 254 (1 mm) produced in Yantai was used for separation and purification of TLC.

The reaction was monitored by thin-layer chromatography (TLC) or LC-MS; the solvent system for development includes: dichloromethane/methanol system, n-hexane/ethyl acetate system, and petroleum ether/ethyl acetate system, the volume ratio of solvents was adjusted according to the polarity of the compound or by adding triethylamine or the like.

For the microwave reaction, Biotage Initiator+ (400 W, RT ~300°C) microwave reactor was used.

Column chromatography generally used 200-300 mesh silica gel as carrier. The eluent system includes: dichloromethane/methanol system, and petroleum ether/ethyl acetate system. The volume ratio of solvents was adjusted according to the polarity of the compound, and could also be adjusted by adding a small amount of triethylamine.

Without specified otherwise in the examples, the temperature of reaction was room temperature (20°C to 35°C);

The reagents used in the present invention were purchased from companies such as Acros Organics, Aldrich Chemical Company, Topbiochem Ltd., etc.

In general synthesis methods and examples, and intermediate synthesis examples, the meanings of abbreviations are as follows.

| Abbreviation | Meaning |
|---|---|
| TLC | Thin layer chromatography |
| LC-MS | Liquid chromatography-mass spectrometry |
| DMF | *N,N*-Dimethylformamide Dimethylformamide |
| DMSO | Dimethyl sulfoxide |
| SATA | *N*-Succinimidyl-*S*-acetylthioacetate |
| EDTA | Ethylenediaminetetraacetic acid |
| CD₃OD | Deuterated methanol |
| CDCl₃ | Deuterated chloroform |
| DMSO-*d₆* | Hexadeuteriodimethyl sulfoxide |
| D₂O | Deuterated water |
| TMS | Tetramethylsilane |
| NMR | Nuclear magnetic resonance |
| MS | Mass spectrometry |
| s | Singlet peak (singlet) |
| d | Doublet peak (doublet) |
| t | Triplet peak (triplet) |
| q | Quartet peak (quartet) |
| dd | Double doublet peak (double doublet) |
| m | Multiplet peak (multiplet) |
| br | Broad peak (broad) |
| *J* | Coupling constant |
| Hz | Hertz |

### I. Compound Examples

### Example 1: Preparation of 1-(4-aminobutyl)-2-butyl-7-methyl-1H-imidazo[4,5-d]thieno[3,2-b]pyridine-4-amine (Compound 1)

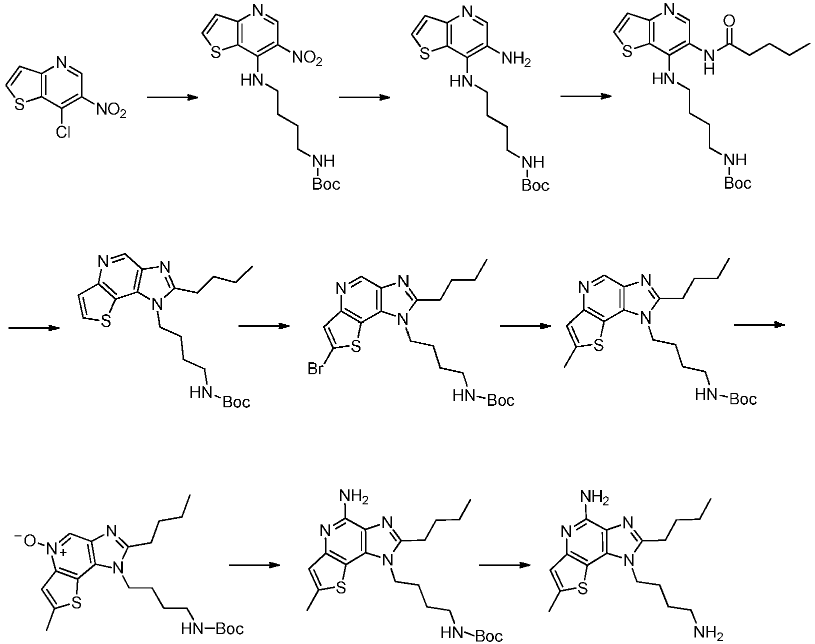

### Step 1: Preparation of tert-butyl (4-((6-nitrothieno[3,2-b]pyridin-7-yl)amino) butyl)carbamate

7-Chloro-6-nitrothieno[3,2-b]pyridine (10.0 g, 46.59 mmol) and N-tert-butoxycarbonyl-1,4-butanediamine (10.53 g, 55.91 mmol) were dissolved in *N,N*-dimethylformamide (150 mL), and cooled in an ice-water bath, *N,N*-diisopropylethylamine (12.04 g, 93.18 mmol) was added to the reaction solution at 0°C, slowly warmed up to room temperature and stirred for 6 hours. The reaction solution was poured into water (1500 mL), extracted three times with ethyl acetate (450 mL), the organic phases were combined, washed three times with saturated brine (150 mL), the organic phase was dried over anhydrous sodium sulfate and concentrated to obtain the title compound (16.61 g, yield: 97.3%).

MS m/z (ESI): 367.1 [M+H]⁺.

### Step 2: Preparation of tert-butyl (4-((6-aminothieno[3,2-b]pyridin-7-yl)amino) butyl)carbamate

Tert-butyl (4-((6-nitrothieno[3,2-*b*]pyridin-7-yl)amino)butyl)carbamate (16.61 g, 49.37 mmol) was dissolved in a mixed solvent of tetrahydrofuran (100 mL) and methanol (100 mL), added with 10% palladium on carbon (2.0 g, 11.97 mmol), replaced with hydrogen three times, the reaction system was stirred at room temperature for 12 hours, subjected to suction filtration, the filter cake was washed with methanol (30 mL), and the filtrate was concentrated to obtain the title compound (14.47 g, yield: 87.1%).

MS m/z (ESI): 337.1 [M+H]⁺.

### Step 3: Preparation of tert-butyl (4-((6-pentanamidothieno[3,2-b]pyridin-7-yl)amino) butyl)carbamate

Tert-butyl (4-((6-aminothieno[3,2-*b*]pyridin-7-yl)amino)butyl)carbamate (14.47 g, 43.01 mmol) and *N,N*-diisopropylethylamine (11.12 g, 86.02 mmol) were dissolved in tetrahydrofuran (250 mL), cooled in an ice-water bath, and slowly added dropwise n-valeryl chloride (7.78 g, 64.52 mmol) at 0°C, then slowly warmed to room temperature and stirred for 4 hours. Water (100 mL) was added to the reaction system to quench the reaction, the system was concentrated to remove THF as much as possible, the obtained residue was added with water (300 mL), and extracted three times with ethyl acetate (300 mL), the organic phases were combined, and washed with saturated brine (150 mL) three times, the organic phase was dried over anhydrous sodium sulfate and concentrated to obtain the title compound (16.72 g, yield: 92.4%).

MS m/z (ESI): 421.0 [M+H]⁺.

### Step 4: Preparation of tert-butyl (4-(2-butyl-1H-imidazo[4,5-d]thieno[3,2-b]pyridin-1-yl) butyl)carbamate

Tert-butyl (4-((6-pentanamidothieno[3,2-*b*]pyridin-7-yl)amino)butyl)carbamate (16.72 g, 39.76 mmol) and o-chlorobenzoic acid (3.11 g, 19.88 mmol) were added into toluene (300 mL), heated to reflux, then water was separated out, and the reaction was carried out under stirring for 4 hours. After being cooled to room temperature, the reaction system was concentrated to remove the solvent, and the obtained residue was purified by silica gel column chromatography (eluent: 100% ethyl acetate) to obtain the title compound (14.76 g, yield: 92.2%).

MS m/z (ESI): 403.2 [M+H]⁺.

### Step 5: Preparation of tert-butyl (4-(7-bromo-2-butyl-1H-imidazo[4,5-d]thieno[3,2-b] pyridin-1-yl)butyl)carbamate

Tert-butyl (4-(2-butyl-1*H*-imidazo[4,5-*d*]thieno[3,2-*b*]pyridin-1-yl)butyl)carbamate (5.41 g, 13.44 mmol) was completely dissolved in a mixed solvent of *N,N-*dimethylformamide (75 mL) and glacial acetic acid (25 mL), and cooled in an ice-water bath, and *N*-bromosuccinimide (4.78 g, 26.88 mmol) was slowly added in batches to the reaction solution at 0°C, warmed slowly to room temperature and stirred for 24 hours. The reaction solution was poured into water (300 mL), extracted three times with ethyl acetate (300 mL), the organic phases were combined, washed three times with saturated aqueous sodium bicarbonate (150 mL), washed three times with saturated brine (150 mL), the organic phase was dried over anhydrous sodium sulfate and concentrated, and the obtained residue was purified by silica gel column chromatography (eluent: ethyl acetate:petroleum ether = 1:1, volume ratio) to obtain the title compound (4.95 g, yield: 76.5%).

MS m/z (ESI): 481.0 [M+H]⁺.

### Step 6: Preparation of tert-butyl (4-(2-butyl-7-methyl-1H-imidazo[4,5-d]thieno[3,2-b] pyridin-1-yl)butyl)carbamate

Tert-butyl (4-(7-bromo-2-butyl-1*H*-imidazo[4,5-*d*]thieno[3,2-b]pyridin-1-yl)butyl) carbamate (1.0 g, 2.08 mmol), trimethylboroxine (50 wt% in THF, 2.62 g, 10.4 mmol), potassium carbonate (575.0 mg, 4.16 mmol) and 1,1'-bisdiphenylphosphinoferrocene palladium dichloride (307.3 mg, 0.42 mmol) were sequentially added to a mixed solvent of 1,4-dioxane (10 mL) and water (1 mL), and the reaction system was bubbled with nitrogen for 5 minutes, then heated to 100°C and stirred for 2 hours under microwave. After being cooled to room temperature, the reaction system was concentrated to remove the solvent, and the obtained residue was purified by silica gel column chromatography (eluent: 100% ethyl acetate) to obtain the title compound (0.65 g, yield: 75.0%).

MS m/z (ESI): 417.1 [M+H]⁺.

### Step 7: Preparation of 1-(4-((tert-butoxycarbonyl)amino)butyl)-2-butyl-7-methyl- 1H-imidazo[4,5-d]thieno[3,2-b]pyridine-5-oxide

Tert-butyl (4-(2-butyl-7-methyl-1*H*-imidazo[4,5-*d*]thieno[3,2-*b*]pyridin-1-yl)butyl) carbamate (0.65 g, 1.56 mmol) was dissolved in dichloromethane (10 mL), cooled in an ice-water bath, and m-chloroperoxybenzoic acid (538.5 mg, 3.12 mmol) was slowly added in batches to the reaction solution at 0°C, and slowly warmed to room temperature and stirred for 4 hours. The reaction solution was poured into water (100 mL), extracted three times with dichloromethane (60 mL), the organic phases were combined, washed twice with saturated aqueous sodium bicarbonate (30 mL), washed twice with saturated aqueous sodium sulfite (30 mL), and washed three times with saturated brine (45 mL), and the organic phase was dried over anhydrous sodium sulfate and concentrated to obtain the title compound (640 mg, yield: 94.8%).

MS m/z (ESI): 433.2 [M+H]⁺.

### Step 8: Preparation of tert-butyl (4-(4-amino-2-butyl-7-methyl-1H-imidazo[4,5-d]thieno[3,2-b]pyridin-1-yl)butyl)carbamate

1-(4-((Tert-butoxycarbonyl)amino)butyl)-2-butyl-7-methyl-1*H*-imidazo[4,5-*d*]thieno[3,2-*b*]pyridine-5-oxide (640 mg, 1.48 mmol) was dissolved in a mixed solvent of dichloromethane (10 mL) and ammonia water (5 mL), cooled in an ice-water bath, and p-toluenesulfonyl chloride (564.1 mg, 2.96 mmol) was slowly added in batches to the reaction solution at 0°C, warmed slowly to room temperature and stirred for 12 hours. The reaction system was added with water (100 mL), and extracted three times with dichloromethane (60 mL), the organic phases were combined, and washed three times with saturated brine (45 mL), the organic phase was dried over anhydrous sodium sulfate and concentrated, and the obtained residue was purified by silica gel column chromatography (eluent: ethyl acetate:methanol=15:1, volume ratio) to obtain the title compound (266 mg, yield: 41.6%).

MS m/z (ESI): 432.2 [M+H]⁺.

### Step 9: Preparation of 1-(4-aminobutyl)-2-butyl-7-methyl-1H-imidazo[4,5-d]thieno[3,2-b] pyridine-4-amine (Compound 1)

Tert-butyl (4-(4-amino-2-butyl-7-methyl-1*H*-imidazo[4,5-*d*]thieno[3,2-*b*]pyridin-1-yl)butyl) carbamate (266 mg, 0.62 mmol) was dissolved in a mixed solvent of methanol (10 mL) and hydrogen chloride in 1,4-dioxane (4N, 5 mL), and stirred at room temperature for 12 hours. The solvent was removed by concentration, then a mixed solvent of dichloromethane and methanol (volume ratio 4:1, 10 mL) and saturated aqueous sodium bicarbonate (2 mL) was added sequentially to the residue, and stirred at room temperature for 5 minutes, then the solvent was removed by concentration. The obtained residue was purified by preparative high performance liquid chromatography to obtain the title compound (130 mg, yield: 63.3%).

MS m/z (ESI): 332.1 [M+H]⁺.

¹H NMR (400 MHz, DMSO-*d₆*): δ 8.34 (s, 2H), 6.98 (d, *J =* 1.2 Hz, 1H), 6.06 (s, 2H), 4.24 (t, *J =* 8.0 Hz, 2H), 2.86 (t, *J =* 8.0 Hz, 2H), 2.78 (t, *J =* 8.0 Hz, 2H), 2.56 (d, *J =* 1.2 Hz, 3H), 1.83-1.75 (m, 4H), 1.62-1.58 (m, 2H), 1.47-1.40 (m, 2H), 0.96 (t, *J =* 8.0 Hz, 3H).

### Example 2: Preparation of 1-(4-aminobutyl)-2-butyl-1H-imidazo[4,5-d]thieno[3,2-b]pyridine-4-amine (Compound 2)

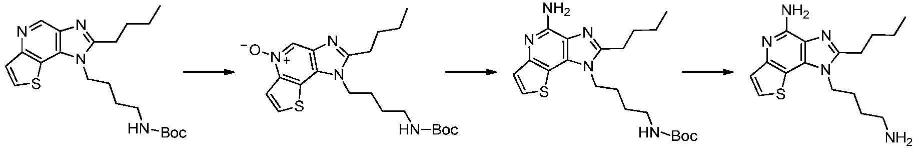

### Step 1: Preparation of 1-(4-((tert-butoxycarbonyl)amino)butyl)-2-butyl-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl (4-(2-butyl-1*H*-imidazo[4,5-*d*]thieno[3,2-*b*]pyridin-1-yl)butyl) carbamate to obtain the title compound (210 mg, yield: 93.2%).

MS m/z (ESI): 419.0 [M+H]⁺.

### Step 2: Preparation of tert-butyl (4-(4-amino-2-butyl-1H-imidazo[4,5-d]thieno[3,2-b] pyridin-1-yl)butyl)carbamate

The synthetic route of Example 1 was applied, the raw material 1-(4-((tert-butoxycarbonyl)amino)butyl)-2-butyl-7-methyl-1*H*-imidazo[4,5-*d*]thieno[3,2-*b*]pyridine-5-oxide of step 8 was replaced with 1-(4-((tert-butoxycarbonyl)amino)butyl)-2-butyl-1*H*- imidazo[4,5-*d*]thieno[3,2-*b*]pyridine-5-oxide to obtain the title compound (152 mg, yield: 45.4%).

MS m/z (ESI): 418.1 [M+H]⁺.

### Step 3: Preparation of 1-(4-aminobutyl)-2-butyl-1H-imidazo[4,5-d]thieno[3,2-b] pyridine-4-amine (Compound 2)

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl (4-(4-amino-2-butyl-1*H*-imidazo[4,5-*d*]thieno[3,2-*b*] pyridin-1-yl)butyl)carbamate to obtain the title compound (62 mg, yield: 52.5%).

MS m/z (ESI): 318.0 [M+H]⁺.

¹H NMR (400 MHz, DMSO-*d₆*): δ 8.32 (s, 2H), 7.74-7.73 (m, 1H), 7.27-7.25 (m, 1H), 6.14 (s, 2H), 4.30 (t, *J =* 8.0 Hz, 2H), 2.91-2.87 (m, 2H), 2.79-2.76 (m, 2H), 1.85-1.76 (m, 4H), 1.63-1.58 (m, 2H), 1.50-1.41 (m, 2H), 0.96 (t, *J =* 8.0 Hz, 3H).

### Example 3: Preparation of 2-butyl-7-methyl-1-(piperidin-4-ylmethyl)-1H-imidazo[4,5-d]thieno[3,2-b]pyridine-4-amine (Compound 3)

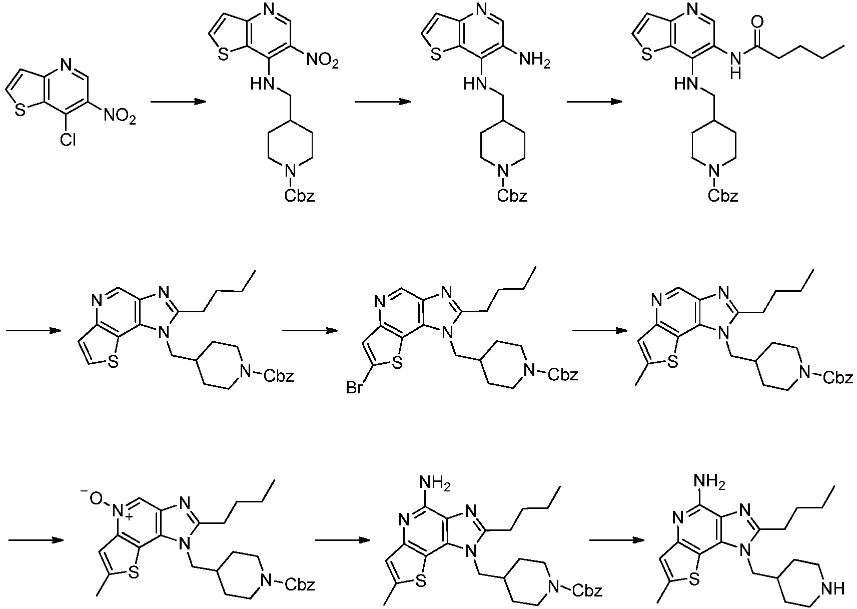

### Step 1: Preparation of benzyl 4-(((6-nitrothieno[3,2-b]pyridin-7-yl)amino)methyl) piperidine-1-carboxylate

The synthetic route of Example 1 was applied, the raw material *N*-tert-butoxycarbonyl-1,4-butanediamine of step 1 was replaced with benzyl 4-(aminomethyl)tetrahydro-1(2*H*)-picolinate to obtain the title compound (1.53 g, yield: 92.5%).

MS m/z (ESI): 427.0 [M+H]⁺.

### Step 2: Preparation of benzyl 4-(((6-aminothieno[3,2-b]pyridin-7-yl)amino)methyl) piperidine-1-carboxylate

Benzyl 4-(((6-Nitrothieno[3,2-*b*]pyridin-7-yl)amino)methyl)piperidine-1-carboxylate (2.0 g, 4.69 mmol) was completely dissolved in anhydrous ethanol (30 mL), added with water (5 mL), cooled in an ice-water bath, and sodium hydrosulfite (8.16 g, 46.9 mmol) was added to the reaction solution at 0°C, and slowly warmed to room temperature and stirred for 4 hours. The reaction solution was poured into water (150 mL), stirred to precipitate a solid, filtered, washed with water, and dried to obtain the title compound (1.65 g, yield: 88.7%).

MS m/z (ESI): 397.1 [M+H]⁺.

### Step 3: Preparation of benzyl 4-(((6-pentanamidothieno[3,2-b]pyridin-7-yl)amino)methyl) piperidine-1-carboxylate

The synthetic route of Example 1 was applied, the raw material tert-butyl (4-((6-aminothieno[3,2-*b*]pyridin-7-yl)amino)butyl)carbamate of step 3 was replaced with benzyl 4-(((6-aminothieno[3,2-*b*]pyridin-7-yl)amino)methyl)piperidine-1-carboxylate to obtain the title compound (1.47 g, yield: 90.2%).

MS m/z (ESI): 481.1 [M+H]⁺.

### Step 4: Preparation of benzyl 4-((2-butyl-1H-imidazo[4,5-d]thieno[3,2-b]pyridin-1-yl) methyl)piperidine-1 -carboxylate

The synthetic route of Example 1 was applied, the raw material tert-butyl (4-((6-pentanamidothieno[3,2-*b*]pyridin-7-yl)amino)butyl)carbamate of step 4 was replaced with benzyl 4-(((6-pentanamidothieno[3,2-*b*]pyridin-7-yl)amino)methyl)piperidine-1- carboxylate to obtain the title compound (1.18 g, yield: 91.8%).

MS m/z (ESI): 463.1 [M+H]⁺.

### Step 5: Preparation of benzyl 4-((7-bromo-2-butyl-1H-imidazo[4,5-d]thieno[3,2-b] pyridin-1-yl)methyl)piperidine-1-carboxylate

The synthetic route of Example 1 was applied, the raw material tert-butyl (4-(2-butyl-1*H-*imidazo[4,5-*d*]thieno[3,2-*d*]pyridin-1-yl)butyl)carbamate of step 5 was replaced with benzyl 4-((2-butyl-1*H*-imidazo[4,5-*d*]thieno[3,2-*b*]pyridin-1-yl)methyl) piperidine-1-carboxylate to obtain the title compound (726 mg, yield: 74.3%).

MS m/z (ESI): 541.1 [M+H]⁺.

### Step 6: Preparation of benzyl 4-((2-butyl-7-methyl-1H-imidazo[4,5-d]thieno[3,2-b] pyridin-1-yl)methyl)piperidine-1-carboxylate

The synthetic route of Example 1 was applied, the raw material tert-butyl (4-(7-bromo-2-butyl-1*H*-imidazo[4,5-*d*]thieno[3,2-*b*]pyridin-1-yl)butyl)carbamate of step 6 was replaced with benzyl 4-((7-bromo-2-butyl-1*H*-imidazo[4,5-*d*]thieno[3,2-*b*]pyridin-1-yl) methyl)piperidine-1-carboxylate to obtain the title compound (623 mg, yield: 76.7%).

MS m/z (ESI): 477.1 [M+H]⁺.

### Step 7: Preparation of 1-((1-((benzyloxy)carbonyl)piperidin)-4-yl)methyl)-2-butyl-7-methyl-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with benzyl 4-((2-butyl-7-methyl-1*H*-imidazo[4,5-*d*]thieno[3,2-*b*]pyirdin-1-yl) methyl)piperidine-1-carboxylate to obtain the title compound (517 mg, yield: 93.2%).

MS m/z (ESI): 493.2 [M+H]⁺.

### Step 8: Preparation of benzyl 4-((4-amino-2-butyl-7-methyl-1H-imidazo[4,5-d]thieno[3,2-b] pyridin-1-yl)methyl)piperidine-1-carboxylate

The synthetic route of Example 1 was applied, the raw material 1-(4-((tert-butoxycarbonyl)amino)butyl)-2-butyl-7-methyl-1*H*-imidazo[4,5-*d*]thieno[3,2-*b*]pyridine-5-oxide of step 8 was replaced with 1-((1-((benzyloxy)carbonyl)piperidin-4-yl)methyl)-2-butyl-7- methyl-1*H-*imidazo[4,5-*d*]thieno[3,2-*b*]pyridine-5-oxide to obtain the title compound (287 mg, yield: 38.2%).

MS m/z (ESI): 492.2 [M+H]⁺.

### Step 9: Preparation of 2-butyl-7-methyl-1-(piperidin-4-ylmethyl)-1H-imidazo[4,5-d]thieno[3,2-b]pyridine-4-amine (Compound 3)

Benzyl 4-((4-amino-2-butyl-7-methyl-1*H*-imidazo[4,5-*d*]thieno[3,2-*b*]pyridin-1-yl)methyl) piperidine-1-carboxylate (150 mg, 0.31 mmol) was added to trifluoroacetic acid (6 mL), heated to 50°C and stirred for 3 hours. The solvent was removed by concentration, then a mixed solvent of dichloromethane and methanol (volume ratio 4:1, 10 mL) and saturated aqueous sodium bicarbonate (10 mL) were added sequentially to the residue, stirred at room temperature for 5 minutes, then the solvent was removed by concentration. The obtained residue was purified by preparative high performance liquid chromatography to obtain the title compound (22 mg, yield: 19.9%).

MS m/z (ESI): 358.1 [M+H]⁺.

¹H NMR (400 MHz, DMSO-*d₆*): δ 8.24 (s, 1H), 6.99-6.98 (m, 1H), 6.11 (br, 2H), 4.14 (d, *J* = 8.0 Hz, 2H), 3.26-3.23 (m, 2H), 2.88-2.76 (m, 4H), 2.57-2.55 (m, 3H), 2.19-2.12(m, 1H), 1.87-1.76 (m, 2H), 1.68-1.62(m, 2H), 1.56-1.39 (m, 4H), 0.96 (t, *J*= 8.0 Hz, 3H).

### Example 4: Preparation of 1-(4-aminobutyl)-2-(2-methoxyethyl)-1H-imidazo[4,5-d]thieno[3,2-b]pyridine-4-amine (Compound 4)

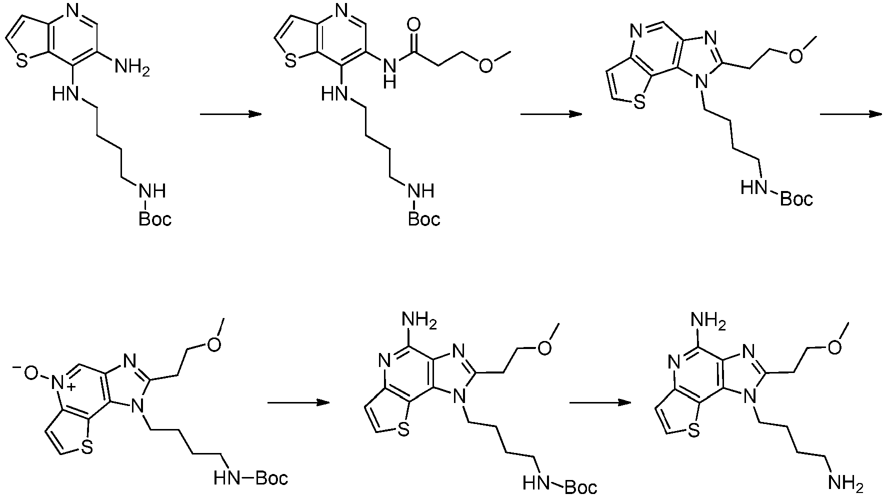

### Step 1: Preparation of tert-butyl (4-((6-(3-methoxypropionamido)thieno[3,2-b]pyridin-7-yl) amino)butyl)carbamate

The synthetic route of Example 1 was applied, the raw material n-pentanoyl chloride of step 3 was replaced with 3-methoxypropionyl chloride to obtain the title compound (847 mg, yield: 93.2%).

MS m/z (ESI): 423.1 [M+H]⁺.

### Step 2: Preparation of tert-butyl (4-(2-(2-methoxyethyl)-1H-imidazo[4,5-d]thieno[3,2-b] pyridin-1-yl)butyl)carbamate

The synthetic route of Example 1 was applied, the raw material tert-butyl (4-((6-pentanamidothieno[3,2-*b*]pyridin-7-yl)amino)butyl)carbamate of step 4 was replaced with tert-butyl (4-((6-(3-methoxypropionamido)thieno[3,2-*b*]pyridin-7-yl)amino)butyl) carbamate to obtain the title compound (480 mg, yield rate: 46.3%).

MS m/z (ESI): 405.1 [M+H]⁺.

### Step 3: Preparation of 1-(4-((tert-butoxycarbonyl)amino)butyl)-2-(2-methoxyethyl)-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl (4-(2-(2-methoxyethyl)-1*H*-imidazo[4,5-*d*]thieno[3,2-*b*]pyridin-1-yl) carbamate to obtain the title compound (200 mg, yield: 90.7%).

MS m/z (ESI): 421.1 [M+H]⁺.

### Step 4: Preparation of tert-butyl (4-(4-amino-2-(2-methoxyethyl)-1H-imidazo[4,5-d]thieno[3,2-b]pyridin-1-yl)butyl)carbamate

The synthetic route of Example 1 was applied, the raw material 1-(4-((tert-butoxy carbonyl)amino)butyl)-2-butyl-7-methyl-1*H*-imidazo[4,5-*d*]thieno[3,2-*b*]pyridine-5-oxide of step 8 was replaced with 1-(4-((tert-butoxycarbonyl)amino)butyl)-2-(2-methoxyethyl)-1*H-*imidazo[4,5-*d*]thieno[3,2-b]pyridine-5-oxide to obtain the title compound (84 mg, yield: 42.7%).

MS m/z (ESI): 420.2 [M+H]⁺.

### Step 5: Preparation of 1-(4-aminobutyl)-2-(2-methoxyethyl)-1H-imidazo[4,5-d]thieno[3,2-b] pyridine-4-amine (Compound 4)

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl (4-(4-amino-2-(2-methoxyethyl)-1*H*-imidazo[4,5-*d*]thieno[3,2-*b*]pyridin-1-yl)butyl)carbamate to obtain the title compound (22 mg, yield: 74.7%).

MS m/z (ESI): 320.1 [M+H]⁺.

¹H NMR (400 MHz, DMSO-*d₆*): δ 14.49 (br, 1H), 8.55 (br, 2H), 8.17-8.12 (m, 3H), 7.55-7.53 (m, 1H), 4.41 (t, *J =* 8.0 Hz, 2H), 3.85 (t, *J =* 8.0 Hz, 2H), 3.31 (s, 3H), 3.26 (t, *J =* 8.0 Hz, 2H), 2.84-2.79 (m, 2H), 1.93-1.85 (m, 2H), 1.73-1.66 (m, 2H).

### Example 5: Preparation of 1-(4-aminobutyl)-2-(2-ethoxymethyl)-1H-imidazo[4,5-d] thieno[3,2-b]pyridine-4-amine (Compound 5)

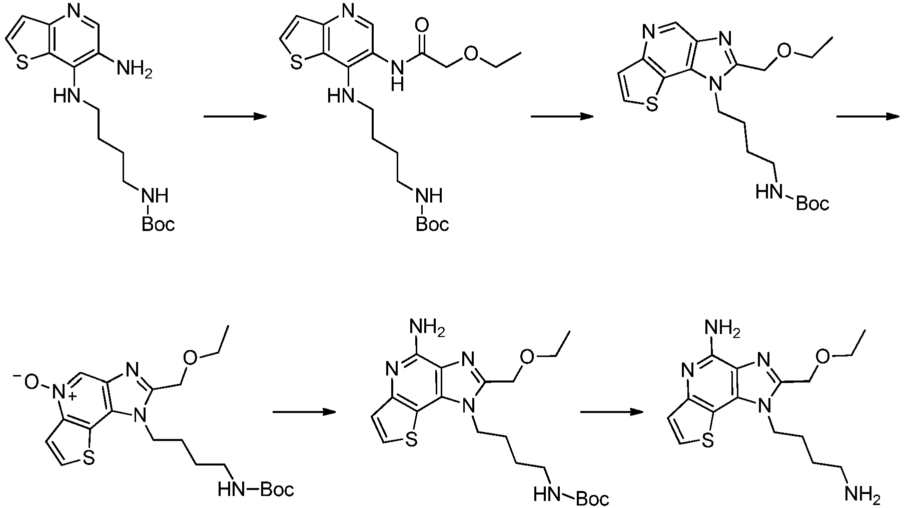

### Step 1: Preparation of tert-butyl (4-((6-(2-ethoxyacetamido)thieno[3,2-b]pyridin-7-yl) amino)butyl)carbamate

The synthetic route of Example 1 was applied, the raw material n-pentanoyl chloride of step 3 was replaced with 2-ethoxyacetyl chloride to obtain the title compound (863 mg, yield: 92.7%).

MS m/z (ESI): 423.1 [M+H]⁺.

### Step 2: Preparation of tert-butyl (4-(2-(ethoxymethyl)-1H-imidazo[4,5-d]thieno[3,2-b] pyridin-1-yl)butyl)carbamate

The synthetic route of Example 1 was applied, the raw material tert-butyl (4-((6-pentanamidothieno[3,2-*b*]pyridin-7-yl)amino)butyl)carbamate of step 4 was replaced with tert-butyl (4-((6-(2-ethoxyacetamido)thieno[3,2-*b*]pyridin-7-yl)amino)butyl)carbamate to obtain the title compound (517 mg, yield rate: 50.4%).

MS m/z (ESI): 405.2 [M+H]⁺.

### Step 3: Preparation of 1-(4-((tert-butoxycarbonyl)amino)butyl)-2-(ethoxymethyl)-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl (4-(2-(ethoxymethyl)-1*H*-imidazo[4,5-*d*]thieno[3,2-*b*]pyridin-1-yl) butyl)carbamate to obtain the title compound (187 mg, yield: 89.3%).

MS m/z (ESI): 421.1 [M+H]⁺.

### Step 4: Preparation of tert-butyl (4-(4-amino-2-(ethoxymethyl)-1H-imidazo[4,5-d]thieno[3,2-b]pyridin-1-yl)butyl)carbamate

The synthetic route of Example 1 was applied, the raw material 1-(4-((tert-butoxycarbonyl)amino)butyl)-2-butyl-7-methyl-1*H*-imidazo[4,5-*d*]thieno[3,2-*b*]pyridine-5-oxide of step 8 was replaced with 1-(4-((tert-butoxycarbonyl)amino)butyl)-2- (ethoxymethyl)-1*H-*imidazo[4,5-*d*]thieno[3,2-*b*]pyridine-5-oxide to obtain the title compound (76 mg, yield: 38.5%).

MS m/z (ESI): 420.2 [M+H]⁺.

### Step 5: Preparation of 1-(4-aminobutyl)-2-(2-ethoxymethyl)-1H-imidazo[4,5-d] thieno[3,2-b]pyridine-4-amine (Compound 5)

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl (4-(4-amino-2-(ethoxymethyl)-1*H*-imidazo[4,5-*d*]thieno[3,2-*b*]pyridin-1-yl)butyl)carbamate to obtain the title compound (33 mg, yield: 81.4%).

MS m/z (ESI): 320.1 [M+H]⁺.

¹H NMR (400 MHz, DMSO-*d₆*): δ 14.49 (br, 1H), 8.70 (br, 2H), 8.20 (d, *J =* 4.0 Hz, 1H), 8.11 (br, 2H), 7.56 (d, *J =* 4.0 Hz, 1H), 4.84 (s, 2H), 4.46-4.42 (m, 2H), 3.62-3.56 (m, 2H), 2.84-2.79 (m, 2H), 1.98-1.90 (m, 2H), 1.76-1.68 (m, 2H), 1.18 (t, *J =* 8.0 Hz, 3H).

### Example 6: Preparation of 2-butyl-7-(1-methylpiperidin-4-yl)-1H-imidazo[4,5-d] thieno[3,2-b]pyridine-4-amine (Compound 6)

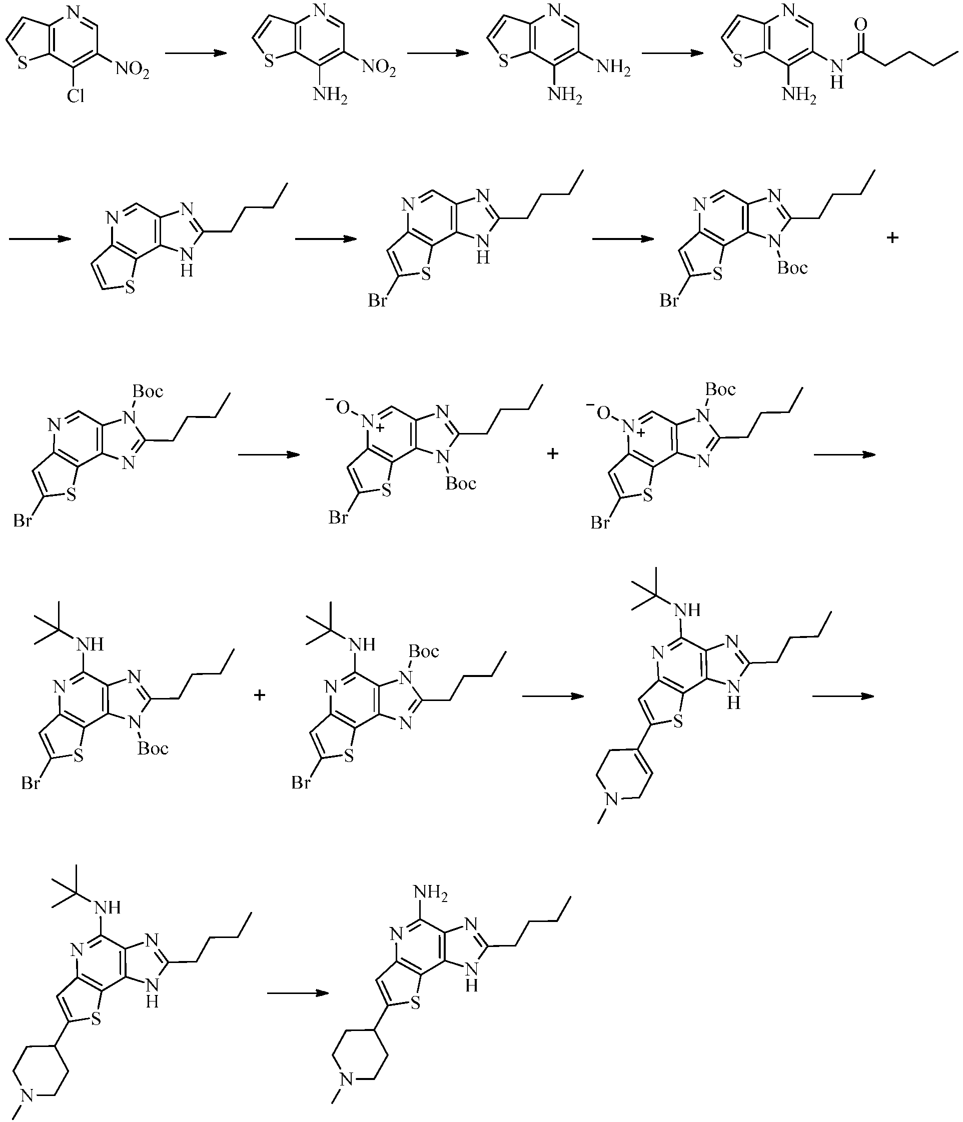

### Step 1: Preparation of 6-nitrothieno[3,2-b]pyridine-7-amine

7-Chloro-6-nitrothieno[3,2-*b*]pyridine (2.0 g, 9.23 mmol) was dissolved in methanol (20 mL), added with ammonia in methanol (7N, 26.4 mL, 184.8 mmol), heated to 50°C and stirred for 4 hours. After being cooled to room temperature, the reaction system was concentrated to remove the solvent so as to obtain the title compound of this step (1.73 g, yield: 96.1%).

MS m/z (ESI): 196.1 [M+H]⁺.

### Step 2: Preparation of thieno[3,2-b]pyridine-6,7-diamine

The synthetic route of Example 1 was applied, the raw material tert-butyl (4-((6-nitrothieno[3,2-*b*]pyridin-7-yl)amino)butyl)carbamate of step 2 was replaced with 6-nitrothieno[3,2-*b*]pyridine-7-amine to obtain the title compound (1.4 g, yield: 98.4%).

MS m/z (ESI): 166.1 [M+H]⁺.

### Step 3: Preparation of N-(7-aminothieno[3,2-b]pyridin-6-yl)pentanamide

The synthetic route of Example 1 was applied, the raw material tert-butyl (4-((6-aminothieno[3,2-*b*]pyridin-7-yl)amino)butyl)carbamate of step 3 was replaced with thieno[3,2-*b*]pyridine-6,7-diamine to obtain the title compound (2.5 g, yield: 97.6%).

MS m/z (ESI): 250.1 [M+H]⁺.

### Step 4: Preparation of 2-butyl-1H-imidazo[4,5-d]thieno[3,2-b]pyridine

*N*-(7-aminothieno[3,2-*b*]pyridin-6-yl)pentanamide (2.5 g, 8.48 mmol) was added into ethanol (30 mL), added with sodium hydroxide (1.7 g, 42.4 mmol) at room temperature, heated to 50°C and stirred for 6 hours. The reaction system was added with water (150 mL) to quench the reaction, adjusted with dilute hydrochloric acid to pH = 6, and extracted three times with ethyl acetate (150 mL), the organic phases were combined, washed twice with saturated sodium bicarbonate solution (60 mL), washed twice with saturated brine (60 mL), the organic phase was dried over anhydrous sodium sulfate and concentrated to obtain the title compound (1.5 g, yield: 76.5%).

MS m/z (ESI): 232.0 [M+H]⁺.

### Step 5: Preparation of 7-bromo-2-butyl-1H-imidazo[4,5-d]thieno[3,2-b]pyridine

The synthetic route of Example 1 was applied, the raw material tert-butyl (4-(2-butyl-1*H-*imidazo[4,5-*d*]thieno[3,2-*b*]pyridin-1-yl)butyl)carbamate of step 5 was replaced with 2-butyl-1*H-*imidazo[4,5-*d*]thieno[3,2-*b*]pyridine to obtain the title compound (1.4 g, yield: 65.7%).

MS m/z (ESI): 310.0 [M+H]⁺.

### Step 6: Preparation of a mixture of tert-butyl 7-bromo-2-butyl-1H-imidazo[4,5-d]thieno [3,2-b]pyridine-1-carboxylate and tert-butyl 7-bromo-2-butyl-3H-imidazo[4,5-d]thieno[3,2-b] pyridine-3-carboxylate

7-Bromo-2-butyl-1*H*-imidazo[4,5-*d*]thieno[3,2-*b*]pyridine (1 g, 3.20 mmol) and 4-dimethylaminopyridine (40 mg, 0.32 mmol) were dissolved in tetrahydrofuran (10 mL), added with triethylamine (0.65 g, 6.40 mmol) and di-tert-butyl dicarbonate (1.1 g, 4.8 mmol) successively at room temperature, heated to 40°C and stirred for 12 hours. The reaction solution was poured into water (100 mL), extracted three times with dichloromethane (60 mL), the organic phases were combined, washed three times with saturated brine (30 mL), and the organic phase was dried over anhydrous sodium sulfate and concentrated, the obtained residue was purified by silica gel column chromatography (eluent: ethyl acetate:petroleum ether=3:1, volume ratio) to obtain a mixture of the title compounds of this step (1.0 g, yield: 76.7%).

MS m/z (ESI): 410.0 [M+H]⁺.

### Step 7: Preparation of a mixture of 7-bromo-1-(tert-butoxycarbonyl)-2-butyl-1H-imidazo [4,5-d]thieno[3,2-b]pyridine-5-oxide and 7-bromo-3-(tert-butoxycarbonyl)-2-butyl-3H-imidazo [4,5-d]thieno[3,2-b]pyridine-5-oxide

A mixture of tert-butyl 7-bromo-2-butyl-1*H*-imidazo[4,5-*d*]thieno[3,2-*b*]pyridine-1-carboxylate and tert-butyl 7-bromo-2-butyl-3*H*-imidazo[4,5-*d*]thieno[3,2-*b*]pyridine-3-carboxylate (1.0 g, 2.43 mmol) was dissolved in dichloromethane (10 mL), cooled in an ice-water bath, slowly added with m-chloroperoxybenzoic acid (0.98 g, 4.86 mmol) in batches to the reaction solution at 0°C, slowly warmed to room temperature and stirred for 4 hours. The reaction solution was poured into water (100 mL), extracted three times with dichloromethane (60 mL), the organic phases were combined, washed twice with saturated aqueous sodium bicarbonate (30 mL), washed twice with saturated aqueous sodium sulfite (30 mL), and washed three times with saturated brine (45 mL), and the organic phase was dried over anhydrous sodium sulfate and concentrated to obtain a mixture of the title compounds of this step (1.0 g, yield: 90.9%).

MS m/z (ESI): 426.0 [M+H]⁺.

### Step 8: Preparation of a mixture of tert-butyl 7-bromo-2-butyl-4-(tert-butylamino)-1H-imidazo[4,5-d]thieno[3,2-b]pyridine-1-carboxylate and tert-butyl 7-bromo-2-butyl-4- (tert-butylamino)-3H-imidazo[4,5-d]thieno[3,2-b]pyridine-3-carboxylate

A mixture of 7-bromo-1-(tert-butoxycarbonyl)-2-butyl-1*H*-imidazo[4,5-*d*]thieno[3,2-*b*] pyridine-5-oxide and 7-bromo-3-(tert-butoxycarbonyl)-2-butyl-3*H*-imidazo[4,5-*d*]thieno[3,2-*b*] pyridine-5-oxide (1.0 g, 2.34 mmol) and tert-butylamine (0.85 g, 11.7 mmol) were dissolved in dichloromethane (20 mL), cooled in an ice-water bath, and p-toluenesulfonyl chloride (0.89 g, 4.68 mmol) was slowly added in batches to the reaction solution at 0°C, and slowly heated to room temperature and stirred for 12 hours. The reaction system was added with water (100 mL), extracted three times with dichloromethane (60 mL), the organic phases were combined, washed with saturated brine (45 mL) three times, the organic phase was dried over anhydrous sodium sulfate and concentrated, and the obtained residue was purified by silica gel column chromatography (eluent: ethyl acetate:petroleum ether=5:1, volume ratio) to obtain a mixture of the title compounds of this step (0.6 g, yield: 53.1%).

MS m/z (ESI): 481.2 [M+H]⁺.

### Step 9: Preparation of N-(tert-butyl)-2-butyl-7-(1-methyl-1,2,3,6-tetrahydropyridin-4-yl)-1H-imidazo[4,5-d]thieno[3,2-b]pyridine-4-amine

A mixture of tert-butyl 7-bromo-2-butyl-4-(tert-butylamino)-1*H*-imidazo[4,5-*d*]thieno[3,2-*b*] pyridine-1-carboxylate and tert-butyl 7-bromo-2-butyl-4-(tert-butylamino)-3*H*-imidazo[4,5-*d*] thieno[3,2-*b*]pyridine-3-carboxylate (0.1 g, 0.21 mmol), 1-methyl-4-(4,4,5,5-tetramethyl- 1,3,2-dioxaborocyclopentan-2-yl)-1,2,3,6-tetrahydropyridine (95.0 mg, 0.42 mmol), potassium carbonate (90.0 mg, 0.63 mmol) and 1,1'-bisdiphenylphosphinoferrocene palladium dichloride (15 mg, 0.02 mmol) were added to a mixed solvent of 1,4-dioxane (4 mL) and water (0.5 mL). After the reaction system was bubbled with nitrogen for 5 minutes, the temperature was raised to 90°C under microwave and stirred for 2 hours. After being cooled to room temperature, the reaction system was concentrated to remove the solvent, and the obtained residue was purified by silica gel column chromatography (eluent: dichloromethane:methanol=10:1, volume ratio) to obtain the title compound (40 mg, yield: 50.0%).

MS m/z (ESI): 398.3 [M+H]⁺.

### Step 10: Preparation ofN-(tert-butyl)-2-butyl-7-(1-methylpiperidin-4-yl)-1H-imidazo[4,5-d] thieno[3,2-b]pyridine-4-amine

*N*-(Tert-butyl)-2-butyl-7-(1-methyl-1,2,3,6-tetrahydropyridin-4-yl)-1*H*-imidazo[4,5-*d*]thieno[3,2-*b*]pyridine-4-amine (40 mg, 0.1 mmol) was dissolved in methanol (5 mL), 10% palladium on carbon (20 mg, 0.12 mmol) was added, hydrogen replacement was conducted three times, and the reaction system was heated to room temperature and stirred for 12 hours, and subjected to suction filtration, the filter cake was washed with methanol (5 mL), and the filtrate was concentrated to obtain the title compound (30 mg, yield: 75.0%).

MS m/z (ESI): 400.1 [M+H]⁺.

### Step 11: Preparation of 2-butyl-7-(1-methylpiperidin-4-yl)-1H-imidazo[4,5-d]thieno[3,2-b] pyridine-4-amine (Compound 6)

*N*-(Tert-butyl)-2-butyl-7-(1-methylpiperidin-4-yl)-1*H*-imidazo[4,5-*d*]thieno[3,2-*b*]pyridine-4-amine (20 mg, 0.05 mmol) was dissolved in methanol (3 mL), and concentrated hydrochloric acid (0.5 mL) was added at room temperature, warmed to 50°C and stirred for 4 hours. After being cooled to room temperature, the reaction system was concentrated to remove the solvent, and the obtained residue was purified by preparative high performance liquid chromatography to obtain the title compound (18 mg, yield: 94.7%).

MS m/z (ESI): 344.1 [M+H]⁺.

¹H NMR (400 MHz, CD₃OD): δ 7.31 (s, 1H), 3.70-3.62 (m, 2H), 3.56-3.45 (m, 1H), 3.26-3.19 (m, 2H), 3.00-2.96 (m, 2H), 2.94 (s, 3H), 2.42-2.38 (m, 2H), 2.12-2.08 (m, 2H), 1.91-1.84 (m, 2H), 1.51-1.42 (m, 2H), 1.00 (t, *J =* 8.0 Hz, 3H).

### Example 7: Preparation of 2-butyl-7-(1-methyl-1,2,3,6-tetrahydropyridin-4-yl)-1H-imidazo [4,5-d]thieno[3,2-b]pyridine-4-amine (Compound 8)

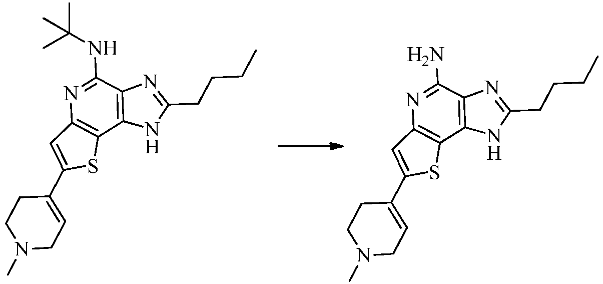

The synthetic route of Example 6 was applied, the raw material N-(tert-butyl)- 2-butyl-7-(1-methylpiperidin-4-yl)-1*H*-imidazo[4,5-*d*]thieno[3,2-*b*]pyridine-4-amine of step 11 was replaced with *N*-(tert-butyl)-2-butyl-7-(1-methyl-1,2,3,6-tetrahydropyridin-4-yl)-1*H*-imidazo[4,5-*d*]thieno[3,2-*b*]pyridine-4-amine to obtain the title compound (25 mg, yield: 78.2%).

MS m/z (ESI): 342.1 [M+H]⁺.

¹H NMR (400 MHz, DMSO-*d₆*): δ 11.15 (s, 1H), 8.54 (s, 2H), 7.43 (s, 1H), 6.37 (s, 1H), 4.01-3.93 (m, 2H), 3.82 -3.79 (m, 1H), 3.62-3.57 (m, 1H), 3.29-3.27 (m, 1H), 2.96-2.81 (m, 7H), 1.82-1.71 (m, 2H), 1.41-1.35 (m, 2H), 0.93 (t, *J* = 8.0 Hz, 3H).

### Example 8: Preparation of 2-butyl-1-(piperidin-4-ylmethyl)-1H-imidazo[4,5-d]thieno[3,2-b] pyridine-4-amine (Compound 25)

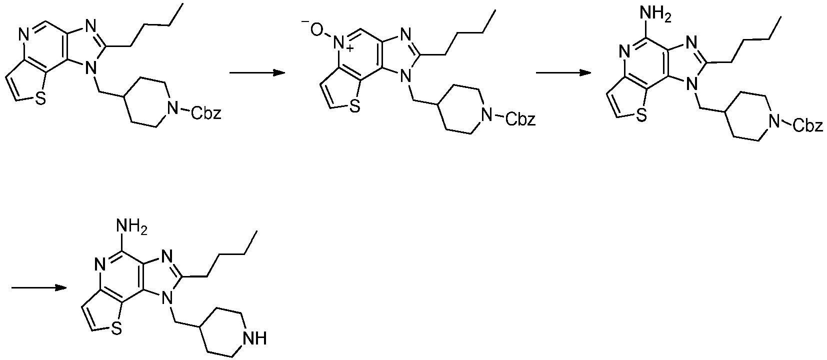

### Step 1: Preparation of 1-((1-((benzyloxy)carbonyl)piperidin-4-yl)methyl)-2-butyl-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridine-1-yl)butyl)carbamate of step 7 was replaced with benzyl 4-((2-butyl-1*H*-imidazo[4,5-*d*]thieno[3,2-*b*]pyridin-1-yl)methyl)piperidine-1-carboxylate (250 mg, yield: 85.6%).

MS m/z (ESI): 479.2 [M+H]⁺.

### Step 2: Preparation of benzyl 4-((4-amino-2-butyl-1H-imidazo[4,5-d]thieno[3,2-b] pyridin-1 -yl)methyl)piperidine-1 -carboxylate

The synthetic route of Example 1 was applied, the raw material 1-(4-((tert-butoxycarbonyl)amino)butyl)-2-butyl-7-methyl-1*H*-imidazo[4,5-*d*]thieno[3,2-*b*]pyridine-5-oxide of step 8 was replaced with 1-((1-((benzyloxy)carbonyl)piperidin-4-yl)methyl)-2-butyl-1*H-*imidazo[4,5-d]thieno[3,2-*b*]pyridine-5-oxide to obtain the title compound (155 mg, yield: 56.7%).

MS m/z (ESI): 478.2 [M+H]⁺.

### Step 3: Preparation of 2-butyl-1-(piperidin-4-ylmethyl)-1H-imidazo[4,5-d]thieno[3,2-b] pyridine-4-amine (Compound 25)

The synthetic route of Example 3 was applied, the raw material benzyl 4-((4-amino-2- butyl-7-methyl-1*H*-imidazo[4,5-*d*]thieno[3,2-*b*]pyridin-1-yl)methyl)piperidine-1-carboxylate of step 9 was replaced with benzyl 4-((4-amino-2-butyl-1*H*-imidazo[4,5-*d*]thieno[3,2-*b*] pyridin-1-yl)methyl)piperidine-1-carboxylate to obtain the title compound (20 mg, yield: 53.8%).

MS m/z (ESI): 344.2 [M+H]⁺.

¹H NMR (400 MHz, CDCl3): δ 7.40 (d, *J =* 8.0 Hz, 1H), 7.31 (d, *J =* 8.0 Hz, 1H), 5.19 (s, 2H), 4.09 (d, *J =* 8.0 Hz, 2H), 3.28-3.25 (m, 2H), 2.80-2.76 (m, 2H), 2.63-2.57 (m, 4H), 2.11-2.06 (m, 1H), 1.83-1.75 (m, 2H), 1.76- 1.66 (m, 3H), 1.46-1.36 (m, 2H), 0.99 (t, *J =* 8.0 Hz, 3H).

### Example 9: Preparation of 2-butyl-7-methyl-1-((tetrahydro-2H-pyran-4-yl)methyl)-1H-imidazo[4,5-d]thieno[3,2-b]pyridine-4-amine (Compound 26)

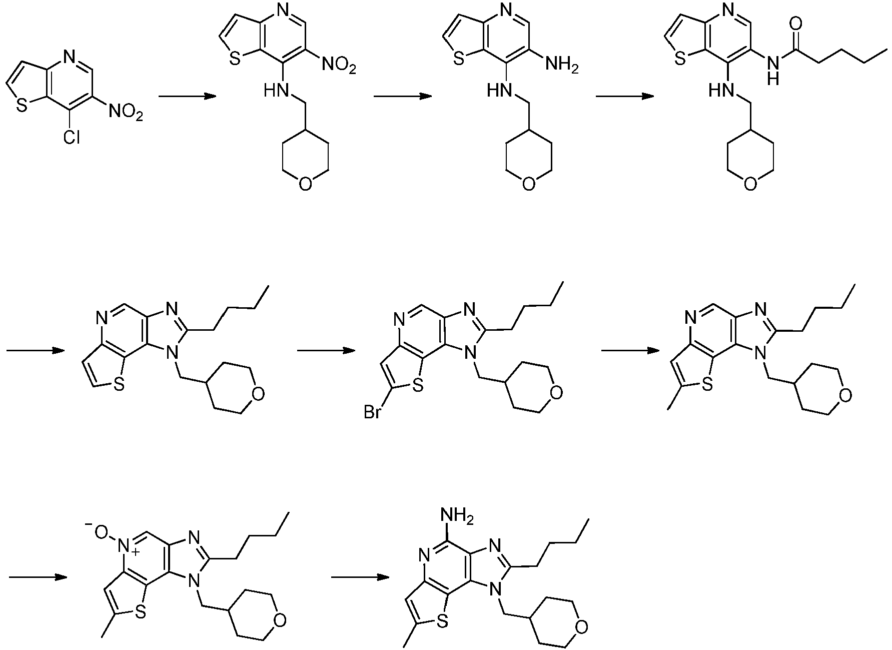

### Step 1: Preparation of 6-nitro-N-((tetrahydro-2H-pyran-4-yl)methyl)thieno[3,2-b] pyridine-7-amine

The synthetic route of Example 1 was applied, the raw material *N-*tert-butoxycarbonyl-1,4-butanediamine of step 1 was replaced with 4-aminomethyltetrahydropyran to obtain the title compound (0.8 g, yield: 93.6%).

MS m/z (ESI): 294.1 [M+H]⁺.

### Step 2: Preparation of N⁷-((tetrahydro-2H-pyran-4-yl)methyl)thieno[3,2-b] pyridine-6,7-diamine

The synthetic route of Example 1 was applied, the raw material tert-butyl (4-((6-nitrothieno[3,2-*b*]pyridin-7-yl)amino)butyl)carbamate of step 2 was replaced with 6-nitro-*N-*((tetrahydro-2*H*-pyran-4-yl)methyl)thieno[3,2-*b*]pyridine-7-amine to obtain the title compound (0.55 g, yield: 76.6%).

MS m/z (ESI): 264.1 [M+H]⁺.

### Step 3: Preparation of N-(7-(((tetrahydro-2H-pyran-4-yl)methyl)amino)thieno[3,2-b] pyridin-6-yl)pentanamide

The synthetic route of Example 1 was applied, the raw material tert-butyl (4-((6-aminothieno[3,2-*b*]pyridin-7-yl)amino)butyl)carbamate of step 3 was replaced with *N*⁷-((tetrahydro-2*H*-pyran-4-yl)methyl)thieno[3,2-*b*]pyridine-6,7-diamine to obtain the title compound (0.7 g, yield: 85.3%).

MS m/z (ESI): 348.1 [M+H]⁺.

### Step 4: Preparation of 2-butyl-1-((tetrahydro-2H-pyran-4-yl)methyl)-1H-imidazo[4,5-d] thieno[3,2-b]pyridine

The synthetic route of Example 1 was applied, the raw material tert-butyl (4-((6-pentanamidothieno[3,2-*b*]pyridin-7-yl)amino)butyl)carbamate of step 4 was replaced with *N*-(7-(((tetrahydro-2*H*-pyran-4-yl)methyl)amino)thieno[3,2-*b*]pyridin-6-yl)pentanamide to obtain the title compound (0.6 g, yield rate: 82.2%).

MS m/z (ESI): 330.1 [M+H]⁺.

### Step 5: Preparation of 7-bromo-2-butyl-1-((tetrahydro-2H-pyran-4-yl)methyl)- 1H-imidazo[4,5-d]thieno[3,2-b]pyridine

The synthetic route of Example 1 was applied, the raw material tert-butyl (4-(2-butyl-1*H-*imidazo[4,5-*d*]thieno[3,2-*b*]pyridin-1-yl)butyl)carbamate of step 5 was replaced with 2-butyl-1-((tetrahydro-2*H*-pyran-4-yl)methyl)-1*H*-imidazo[4,5-*d*]thieno[3,2-*b*]pyridine to obtain the title compound (0.7 g, yield: 76.1%).

MS m/z (ESI): 408.0 [M+H]⁺.

### Step 6: Preparation of 2-butyl-7-methyl-1-((tetrahydro-2H-pyran-4-yl)methyl)-1H-imidazo[4,5-d]thieno[3,2-b]pyridine

The synthetic route of Example 1 was applied, the raw material tert-butyl (4-(7-bromo-2-butyl-1*H*-imidazo[4,5-*d*]thieno[3,2-*b*]pyridin-1-yl)butyl)carbamate of step 6 was replaced with 7-bromo-2-butyl-1-((tetrahydro-2*H*-pyran-4-yl)methyl)-1*H*-imidazo[4,5-*d*]thieno[3,2-*b*]pyridine to obtain the title compound (0.15 g, yield: 80.3%).

MS m/z (ESI): 344.2 [M+H]⁺.

### Step 7: Preparation of 2-butyl-7-methyl-1-((tetrahydro-2H-pyran-4-yl)methyl)-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with 2-butyl-7-methyl-1-((tetrahydro-2*H*-pyran-4-yl)methyl)-1*H*-imidazo[4,5-*d*] thieno[3,2-*b*]pyridine (0.15 g, yield: 76.4%).

MS m/z (ESI): 360.1 [M+H]⁺.

### Step 8: Preparation of 2-butyl-7-methyl-1-((tetrahydro-2H-pyran-4-yl)methyl)-1H-imidazo[4,5-d]thieno[3,2-b]pyridine-4-amine (Compound 26)

The synthetic route of Example 1 was applied, the raw material 1-(4-((tert-butoxy carbonyl)amino)butyl)-2-butyl-7-methyl-1*H*-imidazo[4,5-*d*]thieno[3,2-*b*]pyridine-5-oxide of step 8 was replaced with 2-butyl-7-methyl-1-((tetrahydro-2*H*-pyran-4-yl)methyl)-1*H*- imidazo[4,5-*d*]thieno[3,2-*b*]pyridine-5-oxide to obtain the title compound (12 mg, yield: 12.0%).

MS m/z (ESI): 359.1 [M+H]⁺.

¹H NMR (400 MHz, DMSO-*d₆*): δ 6.97 (s, 1H), 6.05 (s, 2H), 4.12 (d, *J* = 8.0 Hz, 2H), 3.85-3.82 (m, 2H), 3.23-3.16 (m, 2H), 2.89-2.81 (m, 2H), 2.56 (s, 3H), 2.08-2.05 (m, 1H), 1.83-1.75 (m, 2H), 1.52-1.37 (m, 6H), 0.95 (t, *J* = 8.0 Hz, 3H).

### Example 10: Preparation of 1-((1-(2-(2-(2-aminoethoxy)ethoxy)ethyl)piperidin-4-yl) methyl)-2-butyl-7-methyl-1H-imidazo[4,5-d]thieno[3,2-b]pyridine-4-amine (Compound 15)

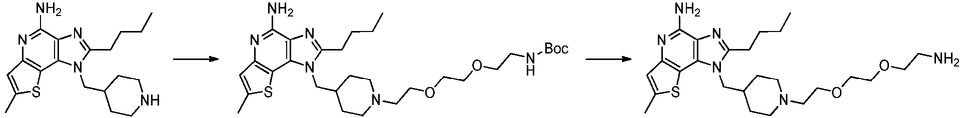

### Step 1: Preparation of tert-butyl (2-(2-(2-(4-((4-amino-2-butyl-7-methyl-1H-imidazo[4,5-d] thieno[3,2-b]pyridin-1-yl)methyl)piperidin-1-yl)ethoxy)ethoxy)ethyl)carbamate

2-Butyl-7-methyl-1-(piperidin-4-ylmethyl)-1*H*-imidazo[4,5-*d*]thieno[3,2-*b*]pyridine-4-amine (60.0 mg, 0.17 mmol), 2,2-dimethyl-4-oxo-3,8,11-trioxa-5-azatridecan-13-yl 4-methylbenzenesulfonate (77.0 mg, 0.19 mmol) and *N*,*N*-diisopropylethylamine (40.0 mg, 0.31 mmol) were dissolved in *N*,*N-*dimethylformamide (2 mL), heated to 70°C and stirred for 12 hours. The reaction solution was poured into water (30 mL), extracted three times with ethyl acetate (30 mL), the organic phases were combined, washed three times with saturated brine (30 mL), the organic phase was dried over anhydrous sodium sulfate, concentrated, and the obtained residue was purified by preparative high performance liquid chromatography to obtain the title compound (30.1 mg, yield: 30.0%).

MS m/z (ESI): 589.4 [M+H]⁺.

### Step 2: Preparation of 1-((1-(2-(2-(2-aminoethoxy)ethoxy)ethyl)piperidin-4-yl)methyl)-2-butyl-7-methyl-1H-imidazo[4,5-d]thieno[3,2-b]pyridine-4-amine (Compound 15)

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl (2-(2-(2-(4-((4-amino-2-butyl-7-methyl-1*H*-imidazo[4,5-*d*] thieno[3,2-*b*]pyridin-1-yl)methyl)piperidin-1-yl)ethoxy)ethoxy)ethyl)carbamate to obtain the title compound (10.7 mg, yield: 53.1%).

MS m/z (ESI): 489.4 [M+H]⁺.

¹H NMR (400 MHz, DMSO-*d₆*): δ 10.94 (s, 1H), 8.42-8.24 (m, 4H), 7.30 (s, 1H), 4.25-4.23 (m, 2H), 3.86-3.83 (m, 2H), 3.65-3.60 (m, 2H), 3.50-3.48 (m, 2H), 3.21-3.18 (m, 2H), 2.95-2.90 (m, 4H), 2.66 (s, 3H), 2.51-2.48 (m, 2H), 1.88-1.73 (m, 5H), 1.54-1.42 (m, 2H), 1.33-1.27 (m, 6H), 0.96 (t, *J* = 8.0 Hz, 3H).

### Example 11: Preparation of 1-((1-(2,5,8,11,14,17,20-heptaoxadocosan-22-yl)piperidin-4-yl) methyl)-2-butyl-7-methyl-1H-imidazo[4, 5-d]thieno[3,2-b]pyridine-4-amine (Compound 16)

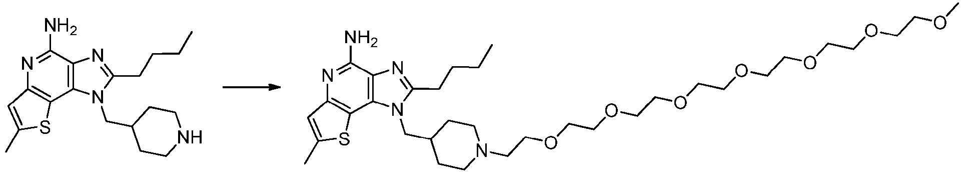

The synthetic route of Example 10 was applied, the raw material 2,2-dimethyl-4-oxo-3,8,11-trioxa-5-azatridecan-13-yl 4-methylbenzenesulfonate of step 1 was replaced with 2,5,8,11,14,17,20-heptoxan-22-yl 4-methylbenzenesulfonate to obtain the title compound (25.3 mg, yield: 37.6%).

MS m/z (ESI): 680.3 [M+H]⁺.

¹H NMR (400 MHz, DMSO-*d₆*): δ 6.97-6.95 (m, 1H), 6.05 (s, 2H), 4.10-4.09 (m, 2H), 3.50-3.30 (m, 26*H*), 3.20 (s, 3H), 2.87-2.82 (m, 4H), 2.57-2.55 (m, 3H), 2.42-2.39 (m, 2H), 1.85-1.74 (m, 5H), 1.48-1.36 (m, 6H), 0.94 (t, *J =* 8.0 Hz, 3H).

### Example 12: Preparation of 1-(4-((4-amino-2-butyl-7-methyl-1H-imidazo[4,5-d] thieno[3,2-b]pyridin-1-yl)methyl)piperidin-1-yl)-3-(2-(2-aminoethoxy)ethoxy)propan-1-one (Compound 17)

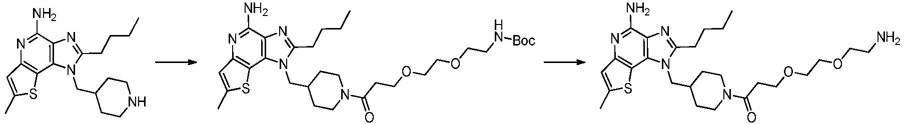

### Step 1: Preparation of tert-butyl (2-(2-(3-(4-((4-amino-2-butyl-7-methyl-1H-imidazo[4,5-d] thieno[3,2-b]pyridin-1-yl)methyl)piperidin-1-yl)-3-oxopropoxy)ethoxy)ethyl)carbamate

2-Butyl-7-methyl-1-(piperidin-4-ylmethyl)-1*H*-imidazo[4,5-*d*]thieno[3,2-*b*]pyridine-4-amine (40.0 mg, 0.11 mmol), *N*-tert-butoxycarbonyl-3-[2-(2-aminoethoxy)ethoxy]propionic acid (33.6 mg, 0.12 mmol), triethylamine (48.6 mg, 0.48 mmol) and 2-(7-azobenzotriazole)-*N*,*N*,*N*',*N*'-tetramethyluronium hexafluorophosphate (45.6 mg, 0.12 mmol) were sequentially added into *N,N-*dimethylformamide (2 mL), the reaction system was stirred at room temperature for 1 hour. The reaction solution was poured into water (30 mL), extracted three times with ethyl acetate (30 mL), the organic phases were combined, washed three times with saturated brine (30 mL), the organic phase was dried over anhydrous sodium sulfate and concentrated, and the residue was purified by preparative high performance liquid chromatography to obtain the title compound (20.7 mg, yield: 30.5%).

MS m/z (ESI): 617.4 [M+H]⁺.

### Step 2: Preparation of 1-(4-((4-amino-2-butyl-7-methyl-1H-imidazo[4,5-d]thieno[3,2-b] pyridin-1-yl)methyl)piperidin-1-yl)-3-(2-(2-aminoethoxy)ethoxy)propan-1-one (Compound 17)

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl (2-(2-(3-(4-((4-amino-2-butyl-7-methyl-1*H*-imidazo[4,5-*d*] thieno[3,2-*b*]pyridin-1-yl)methyl)piperidin-1-yl)-3-oxopropoxy)ethoxy)ethyl)carbamate to obtain the title compound (4.2 mg, yield: 42.8%).

MS m/z (ESI): 517.3 [M+H]⁺.

¹H NMR (400 MHz, DMSO-*d₆*): δ 8.33 (s, 2H), 6.98-6.97 (m, 1H), 6.09 (s, 2H), 4.43-4.40 (m, 2H), 4.12-4.11 (m, 2H), 3.92-3.88 (m, 2H), 3.64-3.60 (m, 2H), 3.56-3.54 (m, 2H), 2.92-2.83 (m, 5H), 2.58-2.54 (m, 4H), 2.44- 2.37 (m, 2H), 2.08-1.99 (m, 1H), 1.83-1.75 (m, 2H), 1.54-1.17 (m, 8H), 0.95 (t, *J =* 8.0 Hz, 3H).

### Example 13: Preparation of 2-butyl-7-methyl-1-((1-methylpiperidin-4-yl)methyl)-1H-imidazo[4,5-d]thieno[3,2-b]pyridine-4-amine (Compound 28)

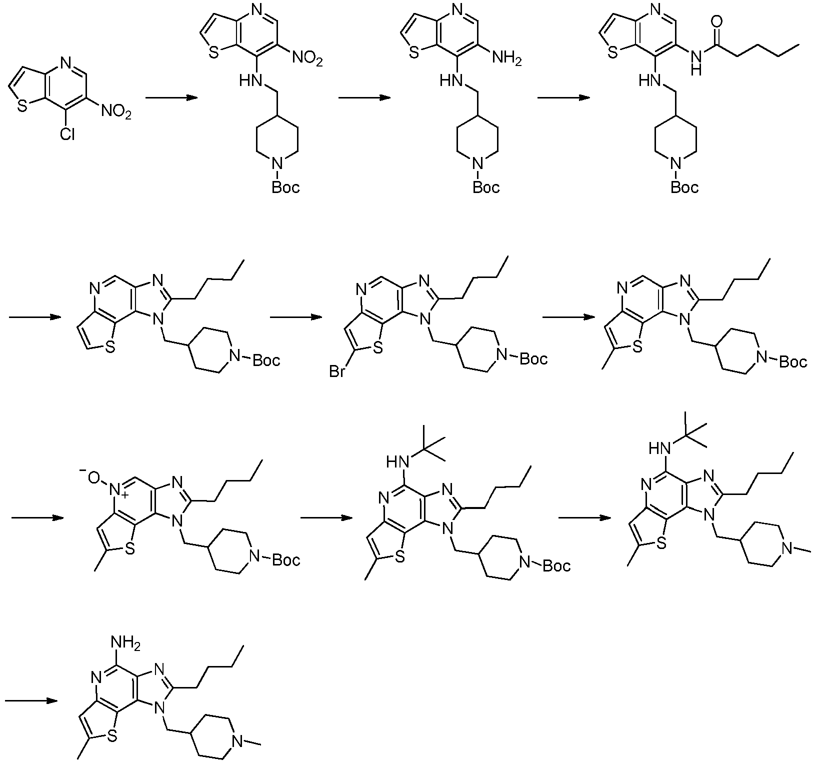

### Step 1: Preparation of tert-butyl 4-(((6-nitrothieno[3,2-b]pyridin-7-yl)amino)methyl) piperidine-1-carboxylate

The synthetic route of Example 1 was applied, the raw material *N-*tert-butoxycarbonyl-1,4-butanediamine of step 1 was replaced with 1-tert-butoxycarbonyl- 4-aminomethylpiperidine to obtain the title compound (29.0 g, yield: 93.9%).

MS m/z (ESI): 393.2 [M+H]⁺.

### Step 2: Preparation of tert-butyl 4-(((6-aminothieno[3,2-b]pyridin-7-yl)amino)methyl) piperidine-1-carboxylate

The synthetic route of Example 1 was applied, the raw material tert-butyl (4-((6-nitrothieno[3,2-*b*]pyridin-7-yl)amino)butyl)carbamate of step 2 was replaced with tert-butyl 4-(((6-nitrothieno[3,2-*b*]pyridin-7-yl)amino)methyl)piperidine-1-carboxylate to obtain the title compound (22.0 g, yield: 92.4%).

MS m/z (ESI): 363.2 [M+H]⁺.

### Step 3: Preparation of tert-butyl 4-(((6-pentanamidothieno[3,2-b]pyridin-7-yl)amino)methyl) piperidine-1-carboxylate

The synthetic route of Example 1 was applied, the raw material tert-butyl (4-((6-aminothieno[3,2-*b*]pyridin-7-yl)amino)butyl)carbamate of step 3 was replaced with tert-butyl 4-(((6-aminothieno[3,2-*b*]pyridin-7-yl)amino)methyl)piperidine-1-carboxylate to obtain the title compound (30.0 g, yield: 98.4%).

MS m/z (ESI): 447.3 [M+H]⁺.

### Step 4: Preparation of tert-butyl 4-((2-butyl-1H-imidazo[4,5-d]thieno[3,2-b]pyridin-1-yl) methyl)piperidine-1 -carboxylate

The synthetic route of Example 1 was applied, the raw material tert-butyl (4-((6-pentanamidothieno[3,2-*b*]pyridin-7-yl)amino)butyl)carbamate of step 4 was replaced with tert-butyl 4-(((6-pentanamidothieno[3,2-*b*]pyridin-7-yl)amino)methyl) piperidine-1-carboxylate to obtain the title compound (19.0 g, yield: 79.5%).

MS m/z (ESI): 429.2 [M+H]⁺.

### Step 5: Preparation of tert-butyl 4-((7-bromo-2-butyl-1H-imidazo[4,5-d]thieno[3,2-b] pyridin-1 -yl)methyl)piperidine-1 -carboxylate

The synthetic route of Example 1 was applied, the raw material tert-butyl (4-(2-butyl-1*H-*imidazo[4,5-*d*]thieno[3,2-*b*]pyridin-1-yl)butyl)carbamate of step 5 was replaced with tert-butyl 4-((2-butyl-1*H*-imidazo[4,5-*d*]thieno[3,2-*b*]pyridin-1-yl)methyl) piperidine-1-carboxylate to obtain the title compound (18.0 g, yield: 72.9%).

MS m/z (ESI): 507.2 [M+H]⁺.

### Step 6: Preparation of tert-butyl 4-((2-butyl-7-methyl-1H-imidazo[4,5-d]thieno[3,2-b] pyridin-1 -yl)methyl)piperidine-1 -carboxylate

The synthetic route of Example 1 was applied, the raw material tert-butyl (4-(7-bromo-2-butyl-1*H*-imidazo[4,5-*d*]thieno[3,2-*b*]pyridin-1-yl)butyl)carbamate of step 6 was replaced with tert-butyl 4-((7-bromo-2-butyl-1*H*-imidazo[4,5-*d*]thieno[3,2-*b*]pyridin-1-yl) methyl)piperidine-1-carboxylate to obtain the title compound (4.0 g, yield: 82.5%).

MS m/z (ESI): 443.2 [M+H]⁺.

### Step 7: Preparation of 1-((1-(tert-butoxycarbonyl)piperidin-4-yl)methyl)-2-butyl-7-methyl-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl 4-((2-butyl-7-methyl-1*H-*imidazo[4,5-*d*]thieno[3,2-*b*]pyridin-1-yl) methyl)piperidine-1-carboxylate to obtain the title compound (5.02 g, yield: 89.3%).

MS m/z (ESI): 459.3 [M+H]⁺.

### Step 8: Preparation of tert-butyl 4-((2-butyl-4-(tert-butylamino)-7-methyl-1H-imidazo[4,5-d] thieno[3,2-b]pyridin-1-yl)methyl)piperidine-1-carboxylate

The synthetic route of Example 1 was applied, the raw material 1-(4-((tert-butoxy carbonyl)amino)butyl)-2-butyl-7-methyl-1*H*-imidazo[4,5-*d*]thieno[3,2-*b*]pyridine-5-oxide of step 8 was replaced with 1-((1-(tert-butoxycarbonyl)piperidin-4-yl)methyl)-2-butyl-7-methyl- 1*H-*imidazo[4,5-*d*]thieno[3,2-*b*]pyridine-5-oxide to obtain the title compound (4.05 g, yield: 75.7%).

MS m/z (ESI): 514.3 [M+H]⁺.

### Step 9: Preparation of N-(tert-butyl)-2-butyl-7-methyl-1-((1-methylpiperidin-4-yl) methyl)-1H-imidazo[4,5-d]thieno[3,2-b]pyridine-4-amine

Tert-butyl 4-((2-butyl-4-(tert-butylamino)-7-methyl-1*H*-imidazo[4,5-*d*]thieno[3,2-*b*] pyridin-1-yl)methyl)piperidine-1-carboxylate (100 mg, 0.19 mmol) was dissolved in tetrahydrofuran (3 mL), lithium aluminum hydride (37.7 mg, 0.99 mmol) was added at room temperature, heated to 70°C and stirred for 4 hours. Sodium sulfate decahydrate (300.0 mg) was added into the reaction solution at room temperature and stirred for 30 minutes, filtered, the filter cake was washed with tetrahydrofuran (10 mL), the filtrate was concentrated, and the residue was purified by silica gel column chromatography (eluent: dichloro Methane:methanol=10:1, volume ratio) to obtain the title compound (40.3 mg, yield: 49.6%).

MS m/z (ESI): 428.3 [M+H]⁺.

### Step 10: Preparation of 2-butyl-7-methyl-1-((1-methylpiperidin-4-yl)methyl)-1H-imidazo[4,5-d]thieno[3,2-b]pyridine-4-amine (Compound 28)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with *N*-(tert-butyl)-2-butyl-7-methyl-1-((1-methylpiperidin-4-yl)methyl)-1*H*- imidazo[4,5-*d*]thieno[3,2-*b*]pyridine-4-amine to obtain the title compound (16.7 mg, yield: 54.7%).

MS m/z (ESI): 372.2 [M+H]⁺.

¹H NMR (400 MHz, DMSO-*d₆*): δ 6.97-6.96 (m, 1H), 6.05 (s, 2H), 4.11-4.09 (m, 2H), 3.34 (s, 3H), 2.86-2.82 (m, 2H), 2.75-2.72 (m, 2H), 2.57-2.55 (m, 3H), 1.87-1.71 (m, 5H), 1.45-1.40 (m, 6H), 0.96 (t, *J* = 8.0 Hz, 3H).

### Example 14: Preparation of 2-butyl-1-methyl-7-(1-methylpiperidin-4-yl)-1H-imidazo[4,5-d] thieno[3,2-b]pyridine-4-amine (Compound 32)

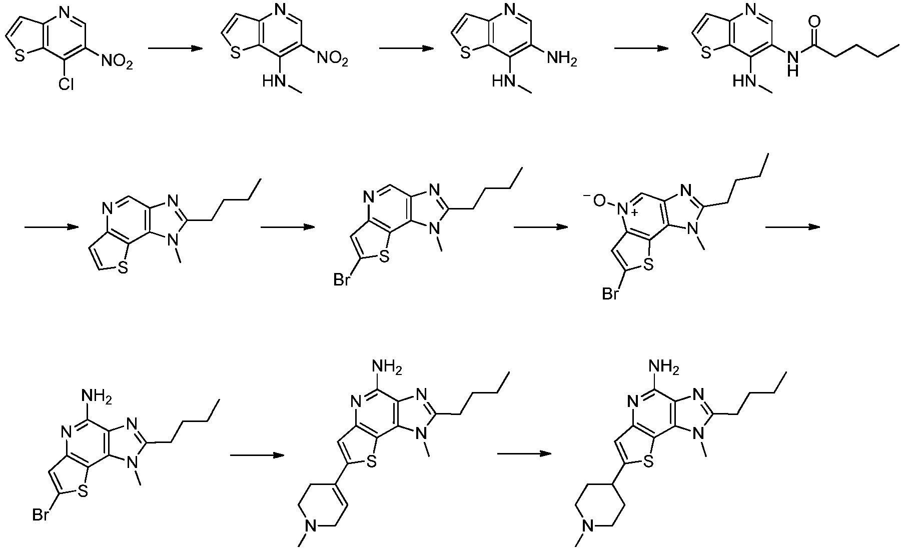

### Step 1: Preparation ofN-methyl-6-nitrothieno[3,2-b]pyridine-7-amine

The synthetic route of Example 1 was applied, the raw material *N-*tert-butoxycarbonyl-1,4-butanediamine of step 1 was replaced with methylamine in tetrahydrofuran solution (2.0 M) to obtain the title compound (760 mg, yield: 77.9%).

MS m/z (ESI): 210.0 [M+H]⁺.

### Step 2: Preparation of N⁷-methylthieno[3,2-b]pyridine-6,7-diamine

The synthetic route of Example 1 was applied, the raw material tert-butyl (4-((6-nitrothieno[3,2-*b*]pyridin-7-yl)amino)butyl)carbamate of step 2 was replaced with *N*-methyl-6-nitrothieno[3,2-*b*]pyridine-7-amine to obtain the title compound (620 mg, yield: 97.3%).

MS m/z (ESI): 180.1 [M+H]⁺.

### Step 3: Preparation ofN-(7-(methylamino)thieno[3,2-b]pyridin-6-yl)pentanamide

The synthetic route of Example 1 was applied, the raw material tert-butyl (4-((6-aminothieno[3,2-*b*]pyridin-7-yl)amino)butyl)carbamate of step 3 was replaced with *N*⁷-methylthieno[3,2-*b*]pyridine-6,7-diamine to obtain the title compound (1.05 g, yield: 93.7%).

MS m/z (ESI): 264.1 [M+H]⁺.

### Step 4: Preparation of 2-butyl-1-methyl-1H-imidazo[4,5-d]thieno[3,2-b]pyridine

The synthetic route of Example 1 was applied, the raw material tert-butyl (4-((6-pentanamidothieno[3,2-*b*]pyridin-7-yl)amino)butyl)carbamate of step 4 was replaced with *N*-(7-(methylamino)thieno[3,2-*b*]pyridin-6-yl)pentanamide to obtain the title compound (605 mg, yield: 67.9%).

MS m/z (ESI): 246.1 [M+H]⁺.

### Step 5: Preparation of 7-bromo-2-butyl-1-methyl-1H-imidazo[4,5-d]thieno[3,2-b]pyridine

The synthetic route of Example 1 was applied, the raw material tert-butyl (4-(2-butyl-1*H-*imidazo[4,5-*d*]thieno[3,2-*b*]pyridin-1-yl)butyl)carbamate of step 5 was replaced with 2-butyl-1-methyl-1*H*-imidazo[4,5-*d*]thieno[3,2-*b*]pyridine to obtain the title compound (576 mg, yield: 79.6%).

MS m/z (ESI): 324.0 [M+H]⁺.

### Step 6: Preparation of 7-bromo-2-butyl-1-methyl-1H-imidazo[4,5-d]thieno[3,2-b] pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with 7-bromo-2-butyl-1-methyl-1*H*-imidazo[4,5-*d*]thieno[3,2-*b*]pyridine to obtain the title compound (622 mg, yield: 95.3%).

MS m/z (ESI): 340.0 [M+H]⁺.

### Step 7: Preparation of 7-bromo-2-butyl-1-methyl-1H-imidazo[4,5-d]thieno[3,2-b] pyridine-4-amine

The synthetic route of Example 1 was applied, the raw material 1-(4-((tert-butoxy carbonyl)amino)butyl)-2-butyl-7-methyl-1*H*-imidazo[4,5-*d*]thieno[3,2-*b*]pyridine-5-oxide of step 8 was replaced with 7-bromo-2-butyl-1-methyl-1*H*-imidazo[4,5-*d*]thieno[3,2-*b*] pyridine-5-oxide to obtain the title compound (534 mg, yield: 56.9%).

MS m/z (ESI): 339.0 [M+H]⁺.

### Step 8: Preparation of 2-butyl-1-methyl-7-(1-methyl-1,2,3,6-tetrahydropyridin-4-yl)-1H-imidazo[4,5-d]thieno[3,2-b]pyridine-4-amine

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine- 3-carboxylate as the raw material of step 9 was replaced with 7-bromo-2-butyl-1-methyl-1*H*-imidazo[4,5-*d*]thieno[3,2-*b*]pyridine-4-amine to obtain the title compound (52 mg, yield: 43.5%).

MS m/z (ESI): 356.3 [M+H]⁺.

### Step 9: Preparation of 2-butyl-1-methyl-7-(1-methylpiperidin-4-yl)-1H-imidazo[4,5-d] thieno[3,2-b]pyridine-4-amine (Compound 32)

the synthetic route of Example 6 was applied, the raw material *N*-(tert-butyl)-2-butyl-7- (1-methyl-1,2,3,6-tetrahydropyridin-4-yl)-1*H*-imidazo[4,5-*d*]thieno[3,2-*b*]pyridine-4-amine of step 10 was replaced with 2-butyl-1-methyl-7-(1-methyl-1,2,3,6-tetrahydropyridin-4-yl)- 1*H-*imidazo[4,5-*d*]thieno[3,2-*b*]pyridine-4-amine to obtain the title compound (12.5 mg, yield: 89.8%).

MS m/z (ESI): 358.3 [M+H]⁺.

¹H NMR (400 MHz, DMSO-*d₆*): δ 8.18 (s, 2H), 7.04 (s, 1H), 3.87 (s, 3H), 3.12-2.84 (m, 5H), 2.48-2.35 (m, 5H), 2.07-2.04 (m, 2H), 1.82-1.70 (m, 4H), 1.44-1.37 (m, 2H), 0.93 (t, *J =* 8.0 Hz, 3H).

### Example 15: Preparation of 2-butyl-7-(1-methylpiperidin-4-yl)-1-((tetrahydro-2H- pyran-4-yl)methyl)-1H-imidazo[4,5-d]thieno[3,2-b]pyridine-4-amine (Compound 34)

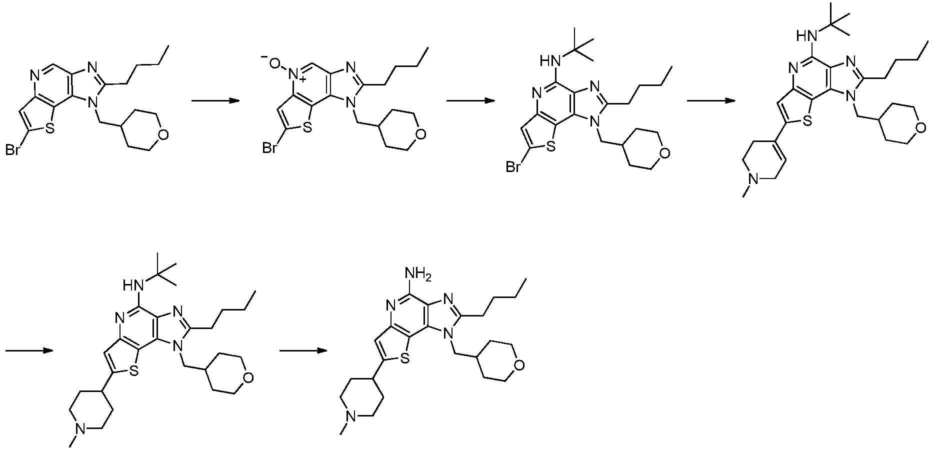

### Step 1: Preparation of 7-bromo-2-butyl-1-((tetrahydro-2H-pyran-4-yl)methyl)-1H-imidazo[4,5-d]thieno[3,2-b]pyridine-5-oxide

The synthetic route of Example 6 was applied, the mixture of tert-butyl 7-bromo-2-butyl-1*H-*imidazo[4,5-*d*]thieno[3,2-*b*]pyridine-1-carboxylate and tert-butyl 7-bromo-2-butyl-3*H-*imidazo[4,5-*d*]thieno[3,2-*b*]pyridine-3-carboxylate as the raw material of step 7 was replaced with 7-bromo-2-butyl-1-((tetrahydro-2*H*-pyran-4-yl)methyl)- 1*H*-imidazo[4,5-*d*]thieno[3,2-*b*]pyridine to obtain the title compound (0.65 g, yield: 93.2%).

MS m/z (ESI): 424.0 [M+H]⁺.

### Step 2: Preparation of 7-bromo-N-(tert-butyl)-2-butyl-1-((tetrahydro-2H-pyran-4-yl) methyl)-1H-imidazo[4,5-d]thieno[3,2-b]pyridine-4-amine

The synthetic route of Example 6 was applied, the mixture of 7-bromo-1-(tert-butoxycarbonyl)-2- butyl-1*H*-imidazo[4,5-*d*]thieno[3,2-*b*]pyridine-5-oxide and 7-bromo-3-(tert-butoxycarbonyl)-2- butyl-3*H*-imidazo[4,5-*d*]thieno[3,2-*b*]pyridine-5-oxide as the raw material of step 8 was replaced with 7-bromo-2-butyl-1-((tetrahydro-2*H*-pyran-4-yl)methyl)-1*H*-imidazo[4,5-*d*] thieno[3,2-*b*]pyridine-5-oxide to obtain the title compound (0.42 g, yield: 56.5%).

MS m/z (ESI): 479.1 [M+H]⁺.

### Step 3: Preparation of N-(tert-butyl)-2-butyl-7-(1-methyl-1,2,3,6-tetrahydropyridin-4-yl)- 1-((tetrahydro-2H-pyran-4-yl)methyl)-1H-imidazo[4,5-d]thieno[3,2-b]pyridine-4-amine

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine-3-carboxylate as the raw material of step 9 was replaced with 7-bromo-*N*-(tert-butyl)-2-butyl-1- ((tetrahydro-2*H*-pyran-4-yl)methyl)-1*H*-imidazo[4,5-*d*]thieno[3,2-*b*]pyridine-4-amine to obtain the title compound (93.8 mg, yield: 67.7%).

MS m/z (ESI): 496.2 [M+H]⁺.

### Step 4: Preparation of N-(tert-butyl)-2-butyl-7-(1-methylpiperidin-4-yl)-1- ((tetrahydro-2H-pyran-4-yl)methyl)-1H-imidazo[4,5-d]thieno[3,2-b]pyridine-4-amine

The synthetic route of Example 6 was applied, the raw material *N*-(tert-butyl)-2-butyl-7- (1-methyl-1,2,3,6-tetrahydropyridin-4-yl)-1*H*-imidazo[4,5-*d*]thieno[3,2-*b*]pyridine-4-amine of step 10 was replaced with *N*-(tert-butyl)-2-butyl-7-(1-methyl-1,2,3,6-tetrahydropyridin-4-yl)- 1-((tetrahydro-2*H*-pyran-4-yl)methyl)-1*H*-imidazo[4,5-*d*]thieno[3,2-*b*]pyridine-4-amine to obtain the title compound (90.7 mg, yield: 90.0%).

MS m/z (ESI): 498.2 [M+H]⁺.

### Step 5: Preparation of 2-butyl-7-(1-methylpiperidin-4-yl)-1-((tetrahydro-2H-pyran-4-yl) methyl)-1H-imidazo[4,5-d]thieno[3,2-b]pyridine-4-amine (Compound 34)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with *N-*(tert-butyl)-2-butyl-7-(1-methylpiperidin-4-yl)-1-((tetrahydro-2*H*-pyran-4-yl) methyl)-1*H*-imidazo[4,5-*d*]thieno[3,2-*b*]pyridine-4-amine to obtain the title compound (20.6 mg, yield: 29.7%).

MS m/z (ESI): 442.2 [M+H]⁺.

¹H NMR (400 MHz, CD₃OD): δ 7.17 (s, 1H), 4.25-4.23 (m, 2H), 3.95-3.92 (m, 2H), 3.34-3.29 (m, 4H), 3.18-3.12 (m, 2H), 2.98-2.94 (m, 3H), 2.83 (s, 3H), 2.39-2.35 (m, 2H), 2.27-2.01 (m, 3H), 1.94-1.86 (m, 2H), 1.59-1.47 (m, 6H), 1.03 (t, *J=* 8.0 Hz, 3H).

### Example 16: Preparation of 2-butyl-7-(1-methylpiperidin-4-yl)-1-((1-methylpiperidin-4-yl) methyl)-1H-imidazo[4,5-d]thieno[3,2-b]pyridine-4-amine (Compound 38)

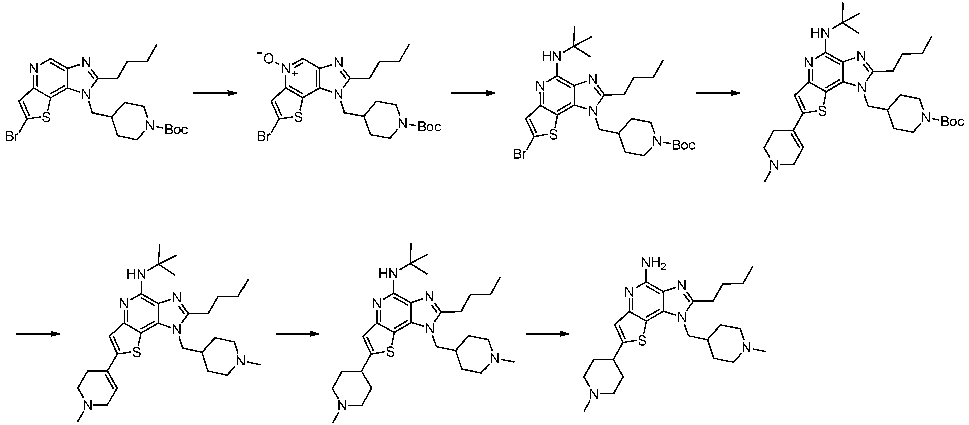

### Step 1: Preparation of 7-bromo-1-((1-(tert-butoxycarbonyl)piperidin-4-yl)methyl)-2-butyl-1H-imidazo[4,5-d]thieno[3,2-b]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl 4-((7-bromo-2-butyl-1*H*-imidazo[4,5-*d*]thieno[3,2-*b*]pyridin-1-yl) methyl)piperidine-1-carboxylate to obtain the title compound (4.1 g, yield: 89.3%).

MS m/z (ESI): 523.1 [M+H]⁺.

### Step 2: Preparation of tert-butyl 4-((7-bromo-2-butyl-4-(tert-butylamino)-1H-imidazo[4,5-d] thieno[3,2-b]pyridin-1-yl)methyl)piperidine-1-carboxylate

The synthetic route of Example 6 was applied, the mixture of 7-bromo-1-(tert-butoxycarbonyl)- 2-butyl-1*H*-imidazo[4,5-*d*]thieno[3,2-*b*]pyridine-5-oxide and 7-bromo-3-(tert-butoxycarbonyl)- 2-butyl-3*H*-imidazo[4,5-*d*]thieno[3,2-*b*]pyridine-5-oxide as the raw material of step 8 was replaced with 7-bromo-1-((1-(tert-butoxycarbonyl)piperidin-4-yl)methyl)-2-butyl- 1*H-*imidazo[4,5-*d*]thieno[3,2-*b*]pyridine-5-oxide to obtain the title compound (2.8 g, yield: 64.7%).

MS m/z (ESI): 578.2 [M+H]⁺.

### Step 3: Preparation of tert-butyl 4-((2-butyl-4-(tert-butylamino)-7-(1-methyl-1,2,3,6-tetrahydropyridin-4-yl)-1H-imidazo[4,5-d]thieno[3,2-b]pyridin-1-yl)methyl)piperidine-1-carboxylate

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine-3-carboxylate as the raw material of 9 was replaced with tert-butyl 4-((7-bromo-2-butyl-4- (tert-butylamino)-1*H-*imidazo[4,5-*d*]thieno[3,2-*b*]pyridin-1-yl)methyl)piperidine-1-carboxylate to obtain the title compound (143 mg, yield: 49.2%).

MS m/z (ESI): 595.3 [M+H]⁺.

### Step 4: Preparation ofN-(tert-butyl)-2-butyl-7-(1-methyl-1,2,3,6-tetrahydropyridin-4-yl)-1-((1-methylpiperidin-4-yl)methyl)-1H-imidazo[4,5-d]thieno[3,2-b]pyridine-4-amine

The synthetic route of Example 13 was applied, the raw material tert-butyl 4-((2-butyl-4-(tert-butylamino)-7-methyl-1*H*-imidazo[4,5-*d*]thieno[3,2-*b*]pyridin-1-yl)methyl)piperidine-1-carboxylate of step 9 was replaced with tert-butyl 4-((2-butyl-4-(tert-butylamino)-7-(1-methyl-1,2,3,6-tetrahydropyridin-4-yl)-1*H*-imidazo[4,5-*d*]thieno[3,2-*b*]pyridin-1-yl)methyl)piperidine-1-carboxylate to obtain the title compound (110 mg, yield: 64.2%).

MS m/z (ESI): 509.3 [M+H]⁺.

### Step 5: Preparation of N-(tert-butyl)-2-butyl-7-(1-methylpiperidin-4-yl)-1-((1-methyl piperidin-4-yl)methyl)-1H-imidazo[4,5-d]thieno[3,2-b]pyridine-4-amine

The synthetic route of Example 6 was applied, the raw material *N*-(tert-butyl)-2-butyl-7- (1-methyl-1,2,3,6-tetrahydropyridin-4-yl)-1*H*-imidazo[4,5-*d*]thieno[3,2-*b*]pyridine-4-amine of step 10 was replaced with *N*-(tert-butyl)-2-butyl-7-(1-methyl-1,2,3,6-tetrahydropyridin-4-yl)- 1-((1-methylpiperidin-4-yl)methyl)-1*H*-imidazo[4,5-*d*]thieno[3,2-*b*]pyridine-4-amine to obtain the title compound (60 mg, yield: 62.4%).

MS m/z (ESI): 511.4 [M+H]⁺.

### Step 6: Preparation of 2-butyl-7-(1-methylpiperidin-4-yl)-1-((1-methylpiperidin-4-yl) methyl)-1H-imidazo[4,5-d]thieno[3,2-b]pyridine-4-amine (Compound 38)

The synthetic route of Example 6 was applied, the raw material *N*-(tert-butyl)-2-butyl- 7-(1-methylpiperidin-4-yl)-1*H*-imidazo[4,5-*d*]thieno[3,2-*b*]pyridine-4-amine of step 11 was replaced with *N*-(tert-butyl)-2-butyl-7-(1-methylpiperidin-4-yl)-1-((1-methylpiperidin-4-yl) methyl)-1*H-*imidazo[4,5-*d*]thieno[3,2-*b*]pyridine-4-amine to obtain the title compound (15 mg, yield: 37.6%)

MS m/z (ESI): 455.3 [M+H]⁺.

¹H NMR (400 MHz, DMSO-*d*₆) δ 7.01 (s, 1H), 6.05 (s, 2H), 4.13-4.11 (m, 2H), 2.86-2.72 (m, 7H), 2.19 (s, 3H), 2.10 (s, 3H), 2.02-1.96 (m, 4H), 1.82-1.65 (m, 7H), 1.48-1.37 (m, 6H), 0.94 (t, *J* = 8.0 Hz, 3H).

### Example 17: Preparation of 2-butyl-1-((1-methylpiperidin-4-yl)methyl)-7-(tetrahydro- 2H-pyran-4-yl)-1H-imidazo[4,5-d]thieno[3,2-b]pyridine-4-amine (Compound 40)

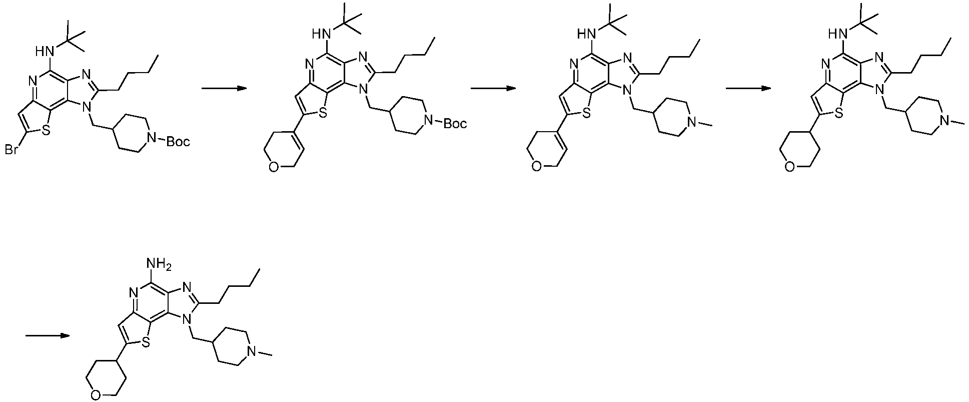

### Step 1: Preparation of tert-butyl 4-((2-butyl-4-(tert-butylamino)-7-(3,6-dihydro-2H-pyran- 4-yl)-1H-imidazo[4,5-d]thieno[3,2-b]pyridin-1-yl)methyl)piperidine-1-carboxylate

Tert-butyl 4-((7-bromo-2-butyl-4-(tert-butylamino)-1*H*-imidazo[4,5-*d*]thieno[3,2-*b*]pyridin-1-yl)methyl)piperidine-1-carboxylate (407 mg, 0.70 mmol), 3,6-dihydro-2*H*-pyran-4-boronic acid pinacol ester (168 mg, 0.80 mmol), potassium carbonate (180 mg, 1.3 mmol) and 1,1'-bisdiphenylphosphinoferrocene palladium dichloride (44 mg, 0.06 mmol) were sequentially added to a mixed solvent of 1,4-dioxane (10 mL) and water (1 mL). After the reaction system was bubbled with nitrogen for 5 minutes, it was heated to 90°C under microwave and stirred for 2 hours. After being cooled to room temperature, the reaction system was concentrated to remove the solvent, and the obtained residue was purified by silica gel column chromatography (eluent: dichloromethane:methanol=10:1, volume ratio) to obtain the title compound (320 mg, yield: 78.6%).

MS m/z (ESI): 582.3 [M+H]⁺.

### Step 2: Preparation of N-(tert-butyl)-2-butyl-7-(3,6-dihydro-2H-pyran-4-yl)-1- ((1-methylpiperidin-4-yl)methyl)-1H-imidazo[4,5-d]thieno[3,2-b]pyridine-4-amine

The synthetic route of Example 13 was applied, the raw material tert-butyl 4-((2-butyl-4-(tert-butylamino)-7-methyl-1*H*-imidazo[4,5-*d*]thieno[3,2-*b*]pyridin-1-yl)methyl)piperidine-1-carboxylate of step 9 was replaced with tert-butyl 4-((2-butyl-4-(tert-butylamino)-7-(3,6-dihydro-2*H*-pyran-4-yl)-1*H*-imidazo[4,5-*d*]thieno[3,2-*b*]pyridin-1-yl)methyl)piperidine-1-carboxylate to obtain the title compound (280 mg, yield: 69.4%).

MS m/z (ESI): 496.3 [M+H]⁺.

### Step 3: Preparation of N-(tert-butyl)-2-butyl-1-((1-methylpiperidin-4-yl)methyl)-7-(tetrahydro-2H-pyran-4-yl)-1H-imidazo[4,5-d]thieno[3,2-b]pyridine-4-amine

The synthetic route of Example 6 was applied, the raw material *N*-(tert-butyl)-2-butyl-7- (1-methyl-1,2,3,6-tetrahydropyridin-4-yl)-1*H*-imidazo[4,5-*d*]thieno[3,2-*b*]pyridine-4-amine of step 10 was replaced with *N*-(tert-butyl)-2-butyl-7-(3,6-dihydro-2*H*-pyran-4-yl)-1- (1-methylpiperidin-4-yl)methyl)-1*H*-imidazo[4,5-*d*]thieno[3*,*2-*b*]pyridine-4-amine to obtain the title compound (98 mg, yield: 78.1%).

MS m/z (ESI): 498.3 [M+H]⁺.

### Step 4: Preparation of 2-butyl-1-((1-methylpiperidin-4-yl)methyl)-7-(tetrahydro-2H- pyran-4-yl)-1H-imidazo[4,5-d]thieno[3,2-b]pyridine-4-amine (Compound 40)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with *N*-(tert-butyl)-2-butyl-1-((1-methylpiperidin-4-yl)methyl)-7-(tetrahydro-2*H*- pyran-4-yl)-1*H*-imidazo[4,5-*d*]thieno[3,2-*b*]pyridine-4-amine to obtain the title compound (27 mg, yield: 82.3%)

MS m/z (ESI): 442.3 [M+H]⁺.

¹H NMR (400 MHz, DMSO-*d₆*): δ 7.03 (s, 1H), 6.09 (s, 2H), 4.14-4.12 (m, 2H), 3.97-3.93 (m, 2H), 3.49-3.43 (m, 2H), 3.18-3.12 (m, 2H), 2.86-2.79 (m, 4H), 2.16 (s, 3H), 1.96-1.93 (m, 2H), 1.83-1.70 (m, 6H), 1.46-1.40 (m, 6H), 0.94 (t, *J* = 8.0 Hz, 3H).

### Example 18: Preparation of 2-butyl-1-[(1-methylhexahydropyridin-4-yl)methyl] thieno[3,2-b]imidazo[4,5-d]pyridine-4-amine (Compound 27)

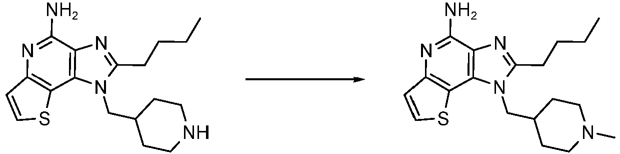

2-Butyl-1-(piperidin-4-ylmethyl)-1*H*-imidazo[4,5-*d*]thieno[3,2-*b*]pyridine-4-amine (50.0 mg, 0.15 mmol) was dissolved in methanol (2 mL), glacial acetic acid (9.6 mg, 0.16 mmol) and aqueous formaldehyde (37 wt.% in H₂O, 121.6 mg, 1.5 mmol) was added to the reaction solution at 0°C. After being stirred at 0°C for 30 minutes, the reaction solution was added with sodium cyanoborohydride (28.3 mg, 0.45 mmol), slowly warmed to room temperature and stirred for 2 hours. The reaction solution was concentrated, and the obtained residue was purified by preparative high performance liquid chromatography to obtain the title compound (15.1 mg, yield: 28.0%).

MS m/z (ESI): 358.2 [M+H]⁺.

¹H NMR (400 MHz, CD₃OD): δ 7.66 (d, *J* = 5.6 Hz, 1H), 7.29 (d, *J =* 5.6 Hz, 1H), 4.26 (d, *J* = 7.6 Hz, 2H),3.01-2.84 (m, 4H), 2.25 (s, 3H), 2.02-1.84 (m, 5H), 1.70-1.46 (m, 6H), 1.02 (t, *J =* 7.6 Hz, 3H).

### Example 19: Preparation of 1-(hexahydropyridin-4-ylmethyl)-2-(2-methoxyethyl) thieno[3,2-b]imidazo[4,5-d]pyridine-4-amine (Compound 55)

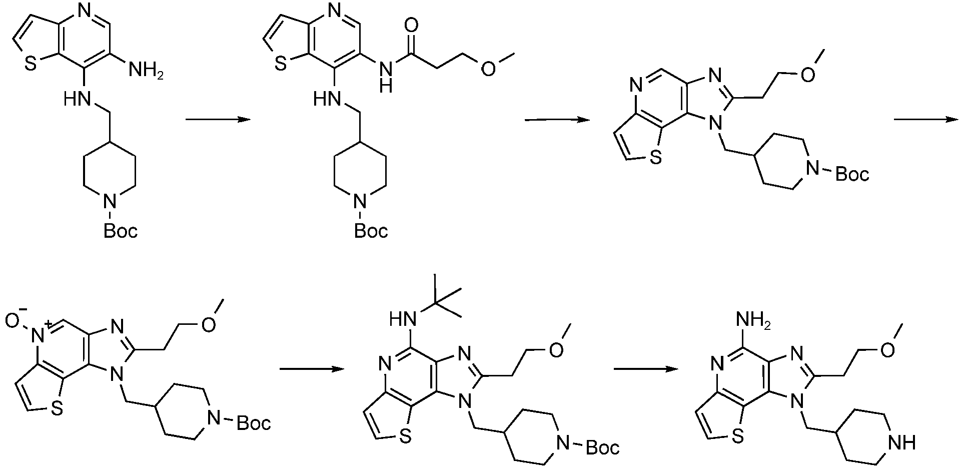

### Step 1: Preparation of tert-butyl 4-[({6-[(3-methoxypropionyl)amino]thieno[3,2-b] pyridin-7-yl} amino)methyl]hexahydropyridine-1 -carboxylate

The synthetic route of Example 1 was applied, the raw material tert-butyl (4-((6-aminothieno[3,2-*b*]pyridin-7-yl)amino)butyl)carbamate of step 3 was replaced with tert-butyl 4-(((6-aminothieno[3,2-*b*]pyridin-7-yl)amino)methyl)piperidine-1-carboxylate, and the raw material n-pentanoyl chloride was replaced with 3-methoxypropionyl chloride to obtain the title compound (1.5 g, yield: 95.5%).

MS m/z (ESI): 449.2 [M+H]⁺.

### Step 2: Preparation of tert-butyl 4- { [2-(2-methoxyethyl)thieno[3,2-b]imidazo[4,5-d] pyridin-1 -yl]methyl }hexahydropyridine-1 -carboxylate

The synthetic route of Example 1 was applied, the raw material tert-butyl (4-((6-pentanamidothieno[3,2-*b*] pyridin-7-yl)amino)butyl)carbamate of step 4 was replaced with tert-butyl 4-[({6-[(3-methoxypropionyl)amino]thieno[3,2-*b*]pyridin-7-yl}amino)methyl] hexahydropyridine-1-carboxylate to obtain the title compound (1.1 g, yield: 74.8%).

MS m/z (ESI): 431.2 [M+H]⁺.

### Step 3: Preparation of 2-(2-methoxyethyl)-1-[(1-(tert-butoxycarbonyl)hexahydropyridin- 4-yl)methyl]thieno[3,2-b]imidazo[4,5-d]pyridine-5-oxide

The synthetic route of Example 1 was applied, the raw material tert-butyl (4-(2-butyl-7-methyl-1*H*-imidazo[4,5-*d*]thieno[3,2-*b*]pyridin-1-yl)butyl)carbamate of step 7 was replaced with tert-butyl 4-{[2-(2-methoxyethyl)thieno[3,2-*b*]imidazo[4,5-*d*]pyridin-1-yl] methyl}hexahydropyridine-1-carboxylate to obtain the title compound (1.1 g, yield: 96.5%).

MS m/z (ESI): 447.2 [M+H]⁺.

### Step 4: Preparation of tert-butyl 4-{[2-(2-methoxyethyl)-4-(tert-butylamino)thieno[3,2-b] imidazo[4,5-d]pyridin-1-yl]methyl}hexahydropyridine-1-carboxylate

The synthetic route of Example 6 was applied, the mixture of 7-bromo-1-(tert-butoxycarbonyl)- 2-butyl-1*H*-imidazo[4,5-*d*]thieno[3,2-*b*]pyridine-5-oxide and 7-bromo-3-(tert-butoxycarbonyl)- 2-butyl-3*H*-imidazo[4,5-*d*]thieno[3,2-*b*]pyridine-5-oxide as the raw material of step 8 was replaced with 2-(2-methoxyethyl)-1-[(1-(tert-butoxycarbonyl)hexahydropyridin-4-yl) methyl]thieno[3,2-*b*]imidazo[4,5-*d*]pyridine-5-oxide to obtain the title compound (0.52 g, yield: 42.2%).

MS m/z (ESI): 502.3 [M+H]⁺.

### Step 5: Preparation of 1-(hexahydropyridin-4-ylmethyl)-2-(2-methoxyethyl)thieno[3,2-b] imidazo[4,5-d]pyridine-4-amine (Compound 55)

The synthetic route of Example 6 was applied, the raw material *N*-(tert-butyl)-2-butyl- 7-(1-methylpiperidin-4-yl)-1*H*-imidazo[4,5-*d*]thieno[3,2-*b*]pyridine-4-amine of step 11 was replaced with tert-butyl 4-{[2-(2-methoxyethyl)-4-(tert-butylamino)thieno[3,2-*b*] imidazo[4,5-*d*]pyridin-1-yl]methyl}hexahydropyridine-1-carboxylate to obtain the title compound (30 mg, yield: 74.3%).

MS m/z (ESI): 346.2 [M+H]⁺.

¹H NMR (400 MHz, CD₃OD): δ 8.03 (d, *J* = 5.6 Hz, 1H), 7.49 (d, *J =* 5.6 Hz, 1H), 4.42 (d, *J* = 7.6 Hz, 2H), 3.97 (t, *J =* 6.0 Hz, 2H), 3.44-3.42 (m, 2H), 3.38 (s, 3H), 3.26 (t, *J =* 6.0 Hz, 2H), 3.01-2.89 (m, 2H), 2.42-2.31 (m, 1H), 1.92-1.89 (m, 2H), 1.78-1.65 (m, 2H).

### Example 20: Preparation of 2-(2-methoxyethyl)-1-[(1-methylhexahydropyridin-4-yl)methyl] thieno[3,2-b]imidazo[4,5-d]pyridine-4-amine (Compound 56)

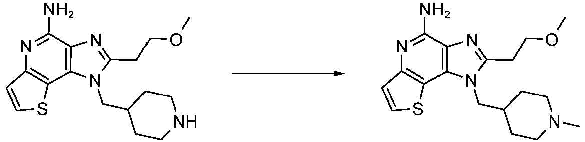

The synthetic route of Example 18 was applied, the raw material 2-butyl-1-(piperidin-4-ylmethyl)-1*H*-imidazo[4,5-*d*]thieno[3,2-*b*]pyridine-4-amine was replaced with 1-(hexahydropyridin-4-ylmethyl)-2-(2-methoxyethyl)thieno[3,2-*b*] imidazo[4,5-*d*]pyridine-4-amine to obtain the title compound (18.3 mg, yield: 32.3%)

MS m/z (ESI): 360.2 [M+H]⁺.

¹H NMR (400 MHz, CD₃OD) δ: 7.92 (d, *J* = 5.6 Hz, 1H), 7.38 (d, *J* = 5.6 Hz, 1H), 4.40 (d, *J* = 7.6 Hz, 2H), 3.95 (t, *J =* 6.0 Hz, 2H), 3.50-3.48 (m, 2H), 3.38 (s, 3H), 3.24 (t, *J=* 6.0 Hz, 2H), 3.02-2.95 (m, 2H), 2.84 (s, 3H), 2.37-2.31 (m, 1H), 1.96-1.91 (m, 2H), 1.79-1.71 (m, 2H).

### Example 21: Preparation of 2-butyl-1-(hexahydropyridin-4-ylmethyl)-7-(1-methyl hexahydropyridin-4-yl)thieno[3,2-b]imidazo[4,5-d]pyridine-4-amine (Compound 36)

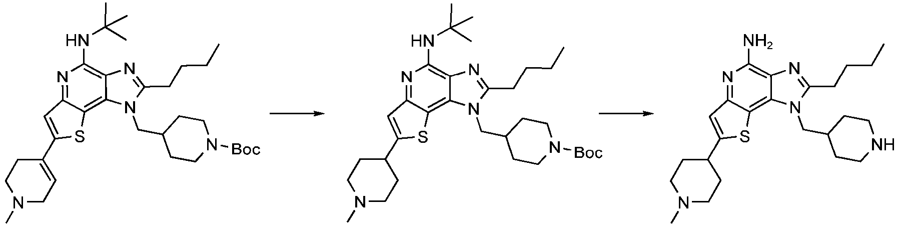

### Step 1: Preparation of tert-butyl 4-{[2-butyl-7-(1-methylhexahydropyridin-4-yl)-4-(tert-butylamino)thieno[3,2-b]imidazo[4,5-d]pyridin-1-yl]methyl}hexahydropyridine-1-carboxylate

The synthetic route of Example 6 was applied, the raw material *N*-(tert-butyl)-2-butyl- 7-(1-methyl-1,2,3,6-tetrahydropyridin-4-yl)-1*H*-imidazo[4,5-*d*]thieno[3,2-*b*]pyridine-4-amine of step 10 was replaced with tert-butyl 4-((2-butyl-4-(tert-butylamino)-7-(1-methyl- 1,2,3,6-tetrahydropyridin-4-yl)-1*H*-imidazo[4,5-*d*]thieno[3,2-*b*]pyridin-1-yl)methyl)piperidine-1-carboxylate to obtain the title compound (50 mg, yield: 66.2%).

MS m/z (ESI): 597.4 [M+H]⁺.

### Step 2: Preparation of 2-butyl-1-(hexahydropyridin-4-ylmethyl)-7-(1-methyl hexahydropyridin-4-yl)thieno[3,2-b]imidazo[4,5-d]pyridine-4-amine (Compound 36)

The synthetic route of Example 6 was applied, the raw material *N*-(tert-butyl)-2-butyl- 7-(1-methylpiperidin-4-yl)-1*H*-imidazo[4,5-*d*]thieno[3,2-*b*]pyridine-4-amine of step 11 was replaced with tert-butyl 4-{[2-butyl-7-(1-methylhexahydropyridin-4-yl)-4-(tert-butylamino) thieno[3,2-*b*]imidazo[4,5-*d*]pyridin-1-yl]methyl}hexahydropyridine-1-carboxylate to obtain the title compound (20 mg, yield: 64.3%).

MS m/z (ESI): 441.3 [M+H]⁺.

¹H NMR (400 MHz, CD₃OD) δ: 7.12 (s, 1H), 4.30 (d, *J =* 7.6 Hz, 2H), 3.54-3.39 (m, 4H), 3.26-3.20 (m, 1H), 3.08-2.89 (m, 6H), 2.81 (s, 3H), 2.32-2.28 (m, 3H), 2.15-2.02 (m, 2H), 1.96-1.68 (m, 6H), 1.56-1.50 (m, 2H), 1.03 (t, *J =* 7.6 Hz, 3H).

### Example 22: Preparation of 2-butyl-1-{[1-(2-methoxyethyl)hexahydropyridin-4-yl]methyl}-7-(1-methylhexahydropyridin-4-yl)thieno[3,2-b]imidazo[4,5-d]pyridine-4-amine (Compound 42)

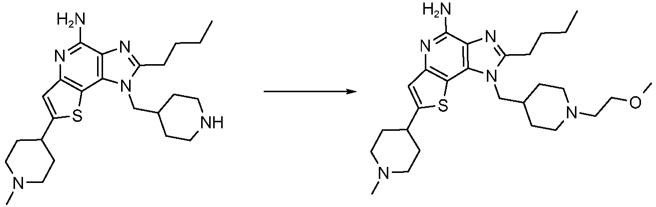

2-Butyl-1-(hexahydropyridin-4-ylmethyl)-7-(1-methylhexahydropyridin-4-yl)thieno[3,2-*b*]imidazo[4,5-*d*]pyridine-4-amine (20.0 mg, 0.05 mmol), 2-bromoethyl methyl ether (13.9 mg, 0.1 mmol) and triethylamine (15.2 mg, 0.15 mmol) were sequentially added to *N,N-*dimethylformamide (2 mL), heated to 70°C and stirred for 12 hours. The reaction solution was poured into water (20 mL), extracted three times with ethyl acetate (30 mL), the organic phases were combined, washed twice with saturated brine (10 mL), and the organic phase was dried over anhydrous sodium sulfate and concentrated, the obtained residue was purified by preparative high performance liquid chromatography to obtain the title compound (4.2 mg, yield: 16.8%).

MS m/z (ESI): 499.4 [M+H]⁺.

¹H NMR (400 MHz, CD₃OD) δ: 7.14 (s, 1H), 4.29 (d, *J =* 7.6 Hz, 2H), 3.65 (t, *J =* 5.2 Hz, 2H), 3.53-3.43 (m, 4H), 3.37 (s, 3H), 3.27-3.25 (m, 1H), 3.15-3.00 (m, 4H), 2.99-2.91 (m, 2H), 2.83 (s, 3H), 2.75-2.72 (m, 2H), 2.39-2.35 (m, 2H), 2.21-1.99 (m, 3H), 1.97-1.86 (m, 2H), 1.81-1.72 (m, 4H), 1.53-1.49 (m, 2H), 1.03 (t, *J* = 7.6 Hz, 3H).

### Example 23: Preparation of 2-butyl-7-(hexahydropyridin-4-yl)-1-{[1-(2-methoxyethyl) hexahydropyridin-4-yl]methyl}thieno[3,2-b]imidazo[4,5-d]pyridine-4-amine (Compound 41)

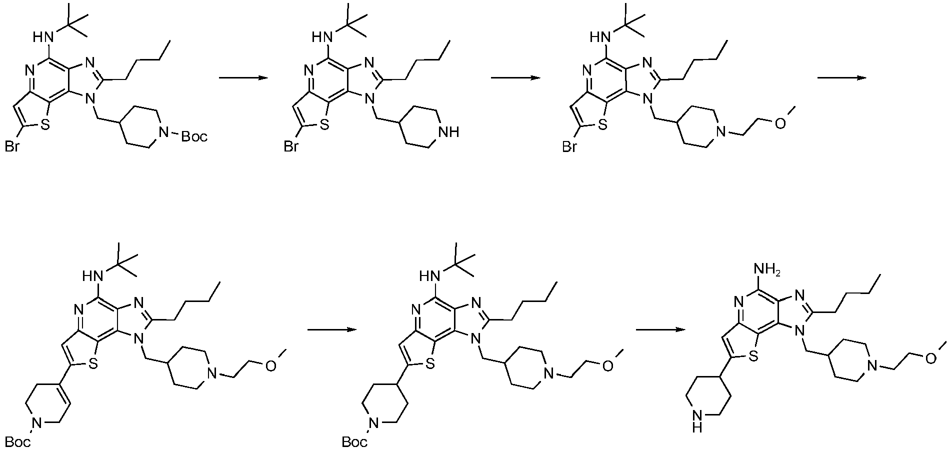

### Step 1: Preparation of 7-bromo-2-butyl-1-(hexahydropyridin-4-ylmethyl)-4- (tert-butylamino)thieno[3,2-b]imidazo[4,5-d]pyridine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl 4-((7-bromo-2-butyl-4-(tert-butylamino)-1*H*-imidazo[4,5-*d*] thieno[3,2-b]pyridin-1-yl)methyl)piperidine-1-carboxylate to obtain the title compound (0.5 g, yield: 95.5%).

MS m/z (ESI): 478.1 [M+H]⁺.

### Step 2: Preparation of 7-bromo-2-butyl-1-{[1-(2-methoxyethyl)hexahydropyridin-4-yl] methyl 1-4-(tert-butylamino)thieno[3,2-b]imidazo[4,5-d]pyridine

The synthetic route of Example 22 was applied, the raw material 2-butyl- 1-(hexahydropyridin-4-ylmethyl)-7-(1-methylhexahydropyridin-4-yl)thieno[3,2-*b*]imidazo[4, 5-d]pyridine-4-amine was replaced with 7-bromo-2-butyl-1-(hexahydropyridin-4-ylmethyl)-4- (tert-butylamino)thieno[3,2-*b*]imidazo[4,5-*d*]pyridine to obtain the title compound (0.42 g, yield: 75.1%).

MS m/z (ESI): 536.2 [M+H]⁺.

### Step 3: Preparation of tert-butyl 4-(2-butyl-1-{[1-(2-methoxyethyl)hexahydropyridin-4-yl] methyl}-4-(tert-butylamino)thieno[3,2-b]imidazo[4,5-d]pyridin-7-yl)-1,2,3,6-tetrahydropyridine-1-carboxylate

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine-3-carboxylate as the raw material of step 9 was replaced with 7-bromo-2-butyl-1-{[1-(2-methoxyethyl) hexahydropyridin-4-yl]methyl}-4-(tert-butylamino)thieno[3,2-*b*]imidazo[4,5-*d*]pyridine, the raw material 1-methyl-4-(4,4,5,5-tetramethyl-1,3,2-dioxaborocyclopentan-2-yl)-1,2,3,6-tetrahydro pyridine was replaced with tert-butyl 4-(4,4,5,5-tetramethyl-1,3,2-dioxaboro cyclopentan-2-yl)-1,2,3,6-tetrahydropyridine-1-carboxylate to obtain the title compound (0.35 g, yield: 70.0%).

MS m/z (ESI): 639.5 [M+H]⁺.

### Step 4: Preparation of tert-butyl 4-(2-butyl-1-{[1-(2-methoxyethyl)hexahydropyridin-4-yl] methyl}-4-(tert-butylamino)thieno[3,2-b]imidazo[4,5-d]pyridin-7-yl)hexahydropyridine-1-carboxylate

The synthetic route of Example 6 was applied, the raw material *N*-(tert-butyl)-2-butyl- 7-(1-methyl-1,2,3,6-tetrahydropyridin-4-yl)-1*H*-imidazo[4,5-*d*]thieno[3,2-*b*]pyridine-4-amine of step 10 was replaced with tert-butyl 4-(2-butyl-1-{[1-(2-methoxyethyl)hexahydropyridin-4-yl] methyl}-4-(tert-butylamino)thieno[3,2-*b*]imidazo[4,5-*d*]pyridin-7-yl)-1,2,3,6-tetrahydropyridine-1-carboxylate to obtain the title compound (0.32 g, yield: 91.5%).

MS m/z (ESI): 641.5 [M+H]⁺.

### Step 5: Preparation of 2-butyl-7-(hexahydropyridin-4-yl)-1- { [1-(2-methoxyethyl)hexahydro pyridin-4-yl]methyl}thieno[3,2-b]imidazo[4,5-d]pyridine-4-amine (Compound 41)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl 4-(2-butyl-1-{[1-(2-methoxyethyl)hexahydropyridin-4-yl]methyl}-4- (tert-butylamino)thieno[3,2-*b*]imidazo[4,5-*d*]pyridin-7-yl)hexahydropyridine-1-carboxylate to obtain the title compound (0.22 g, yield: 91.6%).

MS m/z (ESI): 485.3 [M+H]⁺.

¹H NMR (400 MHz, CD₃OD) δ: 7.35 (s, 1H), 4.36 (d, *J =* 7.6 Hz, 2H), 3.75-3.73 (m, 2H), 3.68-3.61 (m, 3H), 3.60-3.53 (m, 3H), 3.48-3.46 (m, 1H), 3.39 (s, 3H), 3.25-3.19 (m, 2H), 3.06-2.95 (m, 4H), 2.37-2.35 (m, 3H), 2.13 -2.09 (m, 2H), 2.01-1.81 (m, 6H), 1.55-1.52 (m, 2H), 1.04 (t, *J* = 7.6 Hz, 3H).

### Example 24: Preparation of 2-butyl-1-(hexahydropyridin-4-ylmethyl)-7-[1-(2,5,8-trioxa decan-10-yl)hexahydropyridin-4-yl]thieno[3,2-b]imidazo[4,5-d]pyridine-4-amine (Compound 51)

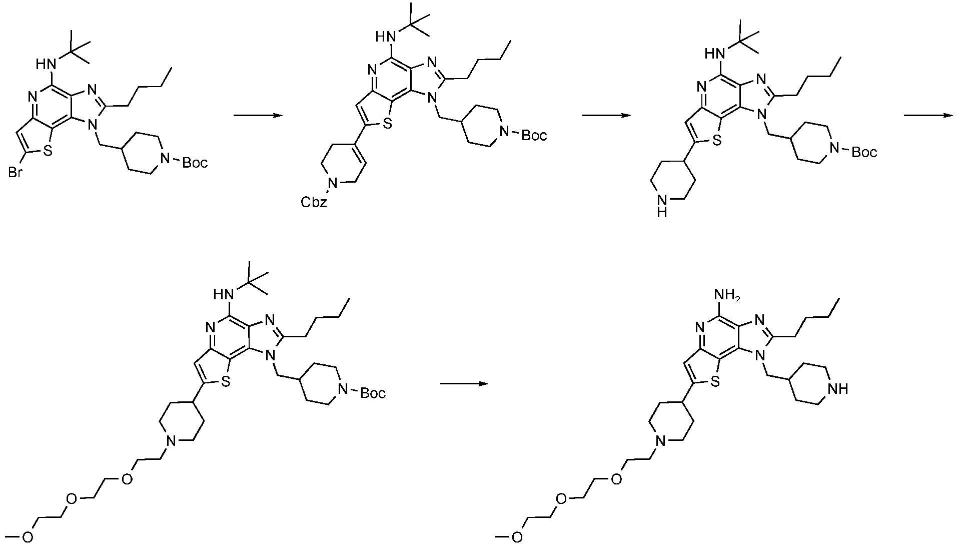

### Step 1: Preparation of benzyl 4-{2-butyl-4-(tert-butylamino)-1-[(1-((tert-butyloxy)carbonyl) hexahydropyridin-4-yl)methyl]thieno[3,2-b]imidazo[4,5-d]pyridin-7-yl}-1,2,3,6-tetrahydropyridine-1-carboxylate

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine-3-carboxylate as the raw material of step 9 was replaced with tert-butyl 4-((7-bromo-2-butyl-4- (tert-butylamino)-1*H-*imidazo[4,5-*d*]thieno[3,2-*b*]pyridin-1-yl)methyl)piperidine-1-carboxylate, and 1-methyl-4-(4,4,5,5-tetramethyl-1,3,2-dioxaborocyclopentan-2-yl)-1,2,3,6-tetrahydro- pyridine was replaced with benzyl 4-(4,4,5,5-tetramethyl-1,3,2-dioxaborocyclopentan-2-yl)- 1,2,3,6-tetrahydropyridine-1-carboxylate to obtain the title compound (0.81 g, yield: 69.5%).

MS m/z (ESI): 715.4 [M+H]⁺.

### Step 2: Preparation of tert-butyl 4-[(2-butyl-7-(hexahydropyridin-4-yl)-4-(tert-butylamino) thieno[3,2-b]imidazo[4,5-d]pyridin-1-yl)methyl]hexahydropyridine-1-carboxylate

Benzyl 4-{2-butyl-4-(tert-butylamino)-1 -[(1 -((tert-butyloxy)carbonyl)hexahydropyridin- 4-yl)methyl]thieno[3,2-*b*]imidazo[4,5-*d*]pyridin-7-yl}-1,2,3,6-tetrahydropyridine-1-carboxylate (0.8 g, 1.12 mmol), ammonium formate (2.12 g, 33.6 mmol) and 10% palladium on carbon (100 mg, 0.094 mmol) were sequentially added to absolute ethanol (20 mL), heated to 80°C and stirred for 12 hours. After being cooled to room temperature, the reaction system was subjected to suction filtration, the filter cake was washed with ethanol (15 mL), the filtrate was concentrated, the residue was added with water (100 mL), extracted three times with ethyl acetate (60 mL), the organic phases were combined, and washed three times with saturated brine (30 mL), the organic phase was dried over anhydrous sodium sulfate and concentrated, and the obtained residue was purified by silica gel column chromatography (eluent: dichloromethane:methanol=10:1, volume ratio) to obtain the title compound (0.55g, yield: 84.6%).

MS m/z (ESI): 583.4 [M+H]⁺.

### Step 3: Preparation of tert-butyl 4-({2-butyl-4-(tert-butylamino)-7-[1-(2,5,8-trioxadecan- 10-yl)hexahydropyridin-4-yl]thieno[3,2-b]imidazo[4,5-d]pyridin-1-yl}methyl)hexahydropyridine-1-carboxylate

Triethylene glycol monomethyl ether (0.5 g, 3.05 mmol) and Dess-Martin oxidant (1.94 g, 4.57 mmol) were sequentially added to anhydrous dichloromethane (10 mL), and the reaction system was stirred at room temperature for 12 hours. The reaction solution was slowly added into a mixed solution of saturated ammonium thiosulfate solution and saturated sodium bicarbonate solution (20 mL, volume ratio 1:1), the mixed system was stirred at room temperature for 0.5 hours, and extracted three times with dichloromethane (15 mL), the organic phases were combined, washed three times with saturated brine (15 mL), the organic phase was dried over anhydrous sodium sulfate, and concentrated to obtain crude 2-(2,5,8-trioxaoctyl)acetaldehyde (0.45 g, harvested rate: 90%).

In another reaction flask, tert-butyl 4-[(2-butyl-7-(hexahydropyridin-4-yl)-4- (tert-butylamino)thieno[3,2-*b*]imidazo[4,5-*d*]pyridin-1-yl)methyl]hexahydropyridine-1-carboxylate (0.3 g, 0.51 mmol), sodium cyanoborohydride (161.8 mmol, 2.6 mmol) and glacial acetic acid (6 mg, 0.1 mmol) were sequentially added into anhydrous methanol (4 mL). The above-mentioned 2-(2,5,8-trioxahoctyl)acetaldehyde (130 mg, 0.78 mmol) in anhydrous methanol solution (1 mL) was slowly added dropwise at room temperature, and the reaction system was stirred at room temperature for 2 hours. The reaction solution was poured into water (50 mL), extracted three times with dichloromethane (30 mL), the organic phases were combined, washed three times with saturated brine (15 mL), the organic phase was dried over anhydrous sodium sulfate, concentrated, and the obtained residue was purified by silica gel column chromatography (eluent: dichloromethane:methanol=10:1, volume ratio) to obtain the title compound (0.22 g, yield: 58.6%).

MS m/z (ESI): 729.5 [M+H]⁺.

### Step 4: Preparation of 2-butyl-1-(hexahydropyridin-4-ylmethyl)-7-[1-(2,5,8-trioxadecan- 10-yl)hexahydropyridin-4-yl]thieno[3,2-b]imidazo[4,5-d]pyridine-4-amine (Compound 51)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl 4-({2-butyl-4-(tert-butylamino)-7-[1-(2,5,8-trioxadecan-10-yl) hexahydropyridin-4-yl]thieno[3,2-*b*]imidazo[4,5-*d*]pyridin-1-yl}methyl)hexahydropyridine-1-carboxyl ate to obtain the title compound (132 mg, yield: 75.8%).

MS m/z (ESI): 573.4 [M+H]⁺.

¹H NMR (400 MHz, CD₃OD) δ: 7.13 (s, 1H), 4.30 (d, *J* = 7.6 Hz, 2H), 3.82 (t, *J* = 5.2 Hz, 2H), 3.71-3.62 (m, 6H), 3.60-3.52 (m, 4H), 3.43-3.38 (m, 2H), 3.36 (s, 3H), 3.20-3.17 (m, 3H), 3.01-2.88 (m, 6H), 2.31-2.28 (m, 3H), 2.08-2.05 (m, 2H), 1.97-1.81 (m, 4H), 1.68-1.66 (m, 2H), 1.56-1.50 (m, 2H), 1.03 (t, *J=* 7.6 Hz, 3H).

### Example 25: Preparation of 2-butyl-1-[(1-methylhexahydropyridin-4-yl)methyl]-7-[1-(2,5,8-trioxadecan-10-yl)hexahydropyridin-4-yl]thieno[3,2-b]imidazo[4,5-d]pyridine-4-amine (Compound 52)

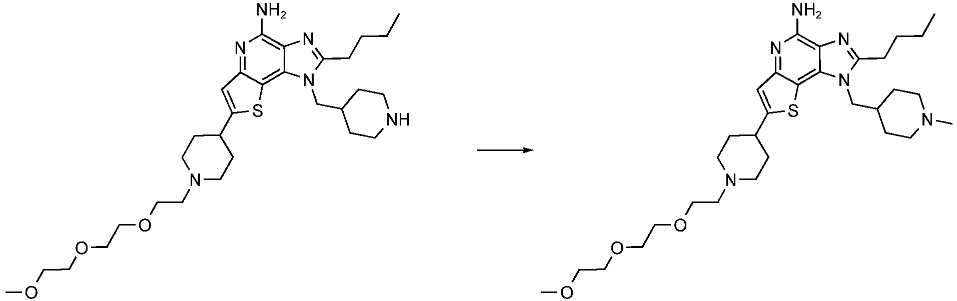

The synthetic route of Example 18 was applied, 2-butyl-1-(piperidin-4-ylmethyl)-1*H-*imidazo[4,5-*d*]thieno[3,2-*b*]pyridine-4-amine was replaced with 2-butyl-1-(hexahydropyridin- 4-ylmethyl)-7-[1-(2,5,8-trioxadecan-10-yl)hexahydropyridin-4-yl]thieno[3,2-*b*]imidazo[4,5-d]pyridine-4-amine to obtain the title compound (7.2 mg, yield: 22.5%).

MS m/z (ESI): 587.4 [M+H]⁺.

¹H NMR (400 MHz, CD₃OD) δ: 7.08 (s, 1H), 4.24 (d, *J =* 7.6 Hz, 2H), 3.82-3.53 (m, 10H), 3.38-3.35 (m, 4H), 3.15-2.85 (m, 8H), 2.63-2.59 (m, 2H), 2.39 (s, 3H), 2.27-1.84 (m, 9H), 1.74-1.45 (m, 6H), 1.02 (t, *J =* 7.6 Hz, 3H).

### Example 26: Preparation of 2-butyl-1-(hexahydropyridin-4-ylmethyl)-7-[1-(2,5,8,11,14-pentaoxahexadecan-16-yl)hexahydropyridin-4-yl]thieno[3,2-b]imidazo[4,5-d]pyridine-4-amine (Compound 57)

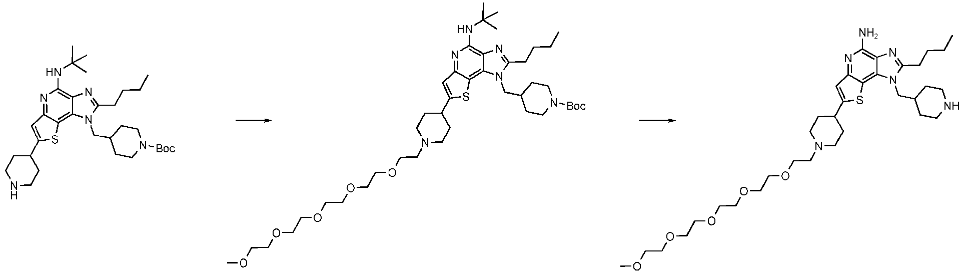

### Step 1: Preparation of tert-butyl 4-({2-butyl-4-(tert-butylamino)-7-[1-(2,5,8,11,14-pentaoxahexadecan-16-yl)hexahydropyridin-4-yl]thieno[3,2-b]imidazo[4,5-d]pyridin-1-yl}methyl)hexahydropyridine-1-carboxylate

The synthetic route of Example 24 was applied, the raw material triethylene glycol monomethyl ether of step 3 was replaced with 2,5,8,11,14-pentoxa-16-hexadecanol to obtain the title compound (0.35 g, Yield: 54.5%).

MS m/z (ESI): 817.5 [M+H]⁺.

### Step 2: Preparation of 2-butyl-1-(hexahydropyridin-4-ylmethyl)-7-[1-(2,5,8,11,14-pentaoxahexadecan-16-yl)hexahydropyridin-4-yl]thieno[3,2-b]imidazo[4,5-d]pyridine-4-amine (Compound 57)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl 4-({2-butyl-4-(tert-butylamino)-7-[1-(2,5,8,11,14-pentaoxahexadecan- 16-yl)hexahydropyridin-4-yl]thieno[3,2-*b*]imidazo[4,5-*d*]pyridin-1-yl}methyl)hexahydropyridine-1-carboxylate to obtain the title compound (0.16 g, yield: 82.4%).

MS m/z (ESI): 661.5 [M+H]⁺.

¹H NMR (400 MHz, CD₃OD) δ: 7.11 (s, 1H), 4.30 (d, *J* = 7.6 Hz, 2H), 3.78 (t, *J* = 5.2 Hz, 2H), 3.70-3.54 (m, 16*H*), 3.51-3.36 (m, 7H), 3.16-3.05 (m, 3H), 2.99-2.86 (m, 4H), 2.78-2.70 (m, 2H), 2.35-2.20 (m, 3H), 2.09-1.79 (m, 6H), 1.74-1.62 (m, 2H), 1.55-1.50 (m, 2H), 1.03 (t, *J* = 7.6 Hz, 3H).

### Example 27: Preparaton of 2-butyl-1-[(1-methylhexahydropyridin-4-yl)methyl]-7-[1-(2,5,8,11,14-pentoxahexadecan-16-yl)hexahydropyridin-4-yl]thieno[3,2-b]imidazo[4,5-d]pyridine-4-amine (Compound 58)

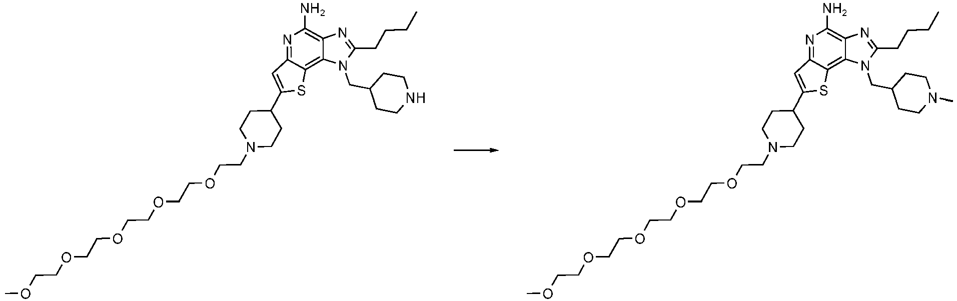

The synthetic route of Example 18 was applied, 2-butyl-1-(piperidin-4-ylmethyl)-1*H-*imidazo[4,5-*d*]thieno[3,2-*b*]pyridine-4-amine was replaced with 2-butyl-1-(hexahydropyridin- 4-ylmethyl)-7-[1-(2,5,8,11,14-pentaoxahexadecan-16-yl)hexahydropyridin-4-yl]thieno[3,2-*b*]imidazo[4,5-*d*]pyridine-4-amine to obtain the title compound (9.2 mg, yield: 31.3%).

MS m/z (ESI): 675.5 [M+H]⁺.

¹H NMR (400 MHz, CD₃OD) δ: 7.07 (s, 1H), 4.23 (d, *J =* 7.6 Hz, 2*H*), 3.75-3.47 (m, 22H), 3.38-3.32 (m, 2H), 3.25-3.22 (m, 2H), 3.05-2.90 (m, 3H), 2.81-2.77 (m, 2H), 2.48-2.44 (m, 2H), 2.31 (s, 3H), 2.20-1.84 (m, 8H), 1.66 -1.48 (m, 6H), 1.02 (t, *J =* 7.6 Hz, 3H).

### Example 28: Preparation of 2-butyl-7-(hexahydropyridin-4-yl)-1-(3,4,5,6-tetrahydro- 2H-pyran-4-ylmethyl)thieno[3,2-b]imidazo[4,5-d]pyridine-4-amine (Compound 33)

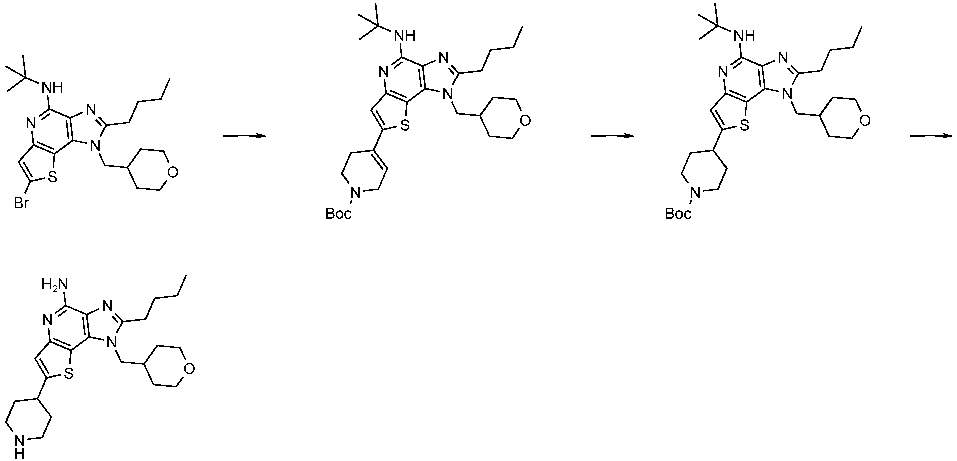

### Step 1: Preparation of tert-butyl 4-{2-butyl-4-(tert-butylamino)-1-(3,4,5,6-tetrahydro-2H-pyran-4-ylmethyl)thieno[3,2-b]imidazo[4,5-d]pyridin-7-yl}-1,2,3,6-tetrahydropyridine-1-carboxylate

7-Bromo-*N-*(tert-butyl)-2-butyl-1-((tetrahydro-2*H-*pyran-4-yl)methyl)-1*H*-imidazo[4,5-*d*]thieno[3,2-*b*]pyridine-4-amine (8.2 g, 17.10 mmol), tert-butyl 4-(4,4,5,5-tetramethyl-1,3,2-dioxaborocyclopentan-2-yl)-1,2,3,6-tetrahydropyridine-1-carboxylate (7.93 g, 25.7 mmol), potassium carbonate (4.73 g, 34.2 mmol) and 1,1'-bisdiphenylphosphinoferrocene palladium dichloride (620.4 mg, 0.86 mmol) were sequentially added to a mixed solvent of 1,4-dioxane (120 mL) and water (10 mL). After the reaction system was bubbled with nitrogen for 5 minutes, it was heated to 100°C and stirred for 2 hours. After being cooled to room temperature, the reaction system was concentrated to remove the solvent, and the obtained residue was purified by silica gel column chromatography (eluent: petroleum ether: ethyl acetate = 1:3, volume ratio) to obtain the title compound (4.01 g, yield : 40.3%).

MS m/z (ESI): 582.3 [M+H]⁺.

### Step 2: Preparation of tert-butyl 4-{2-butyl-4-(tert-butylamino)-1-(3,4,5,6-tetrahydro-2H-pyran-4-ylmethyl)thieno[3,2-b]imidazo[4,5-d]pyridin-7-yl}hexahydropyridine-1-carboxylate

Tert-butyl 4-{2-butyl-4-(tert-butylamino)-1-(3,4,5,6-tetrahydro-2*H*-pyran-4-ylmethyl)thieno [3,2-*b*]imidazo[4,5-*d*]pyridin-7-yl}-1,2,3,6-tetrahydropyridine-1-carboxylate (0.5 g, 0.86 mmol), ammonium formate (1.08 g, 17.2 mmol) and 10% palladium on carbon (20 mg, 0.019 mmol) were sequentially added to absolute ethanol (10 mL), slowly heated to 80°C and stirred for 12 hours. After being cooled to room temperature, the reaction system was subjected to suction filtration, the filter cake was washed with ethanol (15 mL), the filtrate was concentrated, and the residue was purified by silica gel column chromatography (eluent: dichloromethane:methanol=10:1, volume ratio), to obtain the title compound (0.40 g, yield: 79.7%).

MS m/z (ESI): 584.3 [M+H]⁺.

### Step 3: Preparation of 2-butyl-7-(hexahydropyridin-4-yl)-1-(3,4,5,6-tetrahydro-2H-pyran- 4-ylmethyl)thieno[3,2-b]imidazo[4,5-d]pyridine-4-amine (Compound 33)

Tert-butyl 4-{2-butyl-4-(tert-butylamino)-1-(3,4,5,6-tetrahydro-2*H*-pyran-4-ylmethyl)thieno [3,2-*b*]imidazo[4,5-*d*]pyridin-7-yl}hexahydropyridine-1-carboxylate (0.3 g, 0.51 mmol) was dissolved in methanol (3 mL), and added with concentrated hydrochloric acid (0.5 mL) at room temperature, warmed to 50°C and stirred for 4 hours. After being cooled to room temperature, the reaction system was concentrated to remove the solvent, and the obtained residue was purified by preparative high performance liquid chromatography to obtain the title compound (0.19 g, yield: 87.1%).

MS m/z (ESI): 428.3 [M+H]⁺.

¹H NMR (400 MHz, CD₃OD) δ: 7.11 (s, 1H), 4.23 (d, *J =* 7.6 Hz, 2H), 3.95-3.92 (m, 2H), 3.47-3.44 (m, 2H), 3.15-3.08 (m, 2H), 2.98-2.94 (m, 2H), 2.31-2.15 (m, 3H), 2.02-1.86 (m, 4H), 1.57-1.49 (m, 5H), 1.37-1.26 (m, 4H), 1.03 (t, *J* = 7.6 Hz, 3H).

### Example 29: Preparation of 1-{4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}ethyl-1-one (Compound 59)

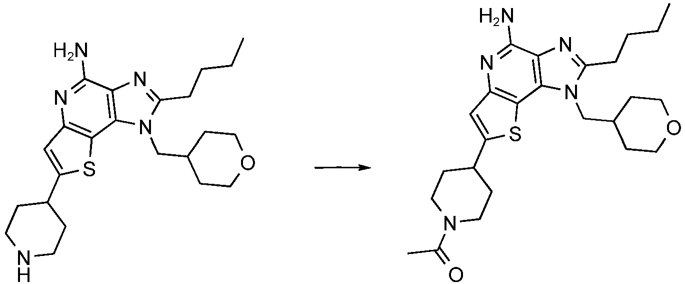

2-Butyl-7-(hexahydropyridin-4-yl)-1-(3,4,5,6-tetrahydro-2*H*-pyran-4-ylmethyl)thieno[3,2-*b*]imidazo[4,5-*d*]pyridine-4-amine (50 mg, 0.12 mmol) and triethylamine (18 mg, 0.18 mmol) were dissolved in anhydrous dichloromethane (2 mL), added with acetic anhydride (13 mg, 0.13 mmol) at 0°C, and stirred at 0°C for 2 hours. The reaction system was concentrated to remove the solvent, and the obtained residue was purified by preparative high performance liquid chromatography to obtain the title compound (37 mg, yield: 65.7%).

MS m/z (ESI): 470.2 [M+H]⁺.

¹H NMR (400 MHz, CD₃OD) δ: 7.11 (s, 1H), 4.67-4.63 (m, 1H), 4.23-4.22 (m, 2H), 4.07-4.03 (m, 1H), 3.95-3.92 (m, 2H), 3.36-3.22 (m, 4H), 2.95 (t, *J* = 8.0 Hz, 2H), 2.82-2.76 (m, 1H), 2.22-2.11 (m, 6H), 1.92-1.86 (m, 2H), 1.82-1.61 (m, 2H), 1.57-1.47 (m, 6H), 1.02 (t, *J* = 8.0 Hz, 3H).

### Example 30: Preparation of 2-butyl-1-(hexahydropyridin-4-ylmethyl)-7-(3,4,5,6-tetrahydro-2H-pyran-4-yl)thieno[3,2-b]imidazo[4,5-d]pyridine-4-amine (Compound 39)

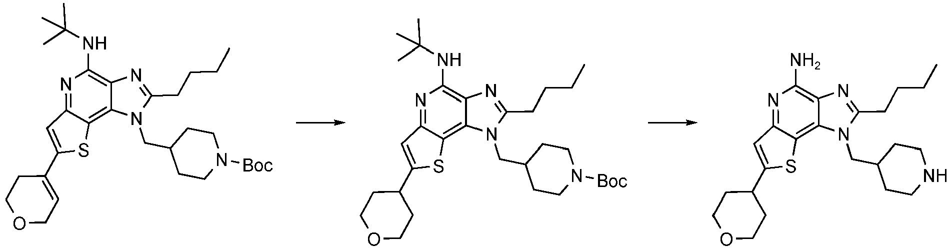

### Step 1: Preparation of tert-butyl 4-({2-butyl-4-(tert-butylamino)-7-(3,4,5,6-tetrahydro-2H-pyran-4-yl)thieno[3,2-b]imidazo[4,5-d]pyridin-1-yl}methyl)hexahydropyridine-1-carboxylate

The synthetic route of Example 6 was applied, the raw material *N*-(tert-butyl)-2-butyl- 7-(1-methyl-1,2,3,6-tetrahydropyridin-4-yl)-1*H*-imidazo[4,5-*d*]thieno[3,2-*b*]pyridine-4-amine of step 10 was replaced with tert-butyl 4-((2-butyl-4-(tert-butylamino)-7-(3,6-dihydro-2*H*- pyran-4-yl)-1*H*-imidazo[4,5-*d*]thieno[3,2-*b*]pyridin-1-yl)methyl)piperidine-1-carboxylate to obtain the title compound (150 mg, yield: 93.2%).

MS m/z (ESI): 584.4 [M+H]⁺.

### Step 2: Preparation of 2-butyl-1-(hexahydropyridin-4-ylmethyl)-7-(3,4,5,6-tetrahydro-2H-pyran-4-yl)thieno[3,2-b]imidazo[4,5-d]pyridine-4-amine (Compound 39)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl 4-({2-butyl-4-(tert-butylamino)-7-(3,4,5,6-tetrahydro-2*H*-pyran-4-yl) thieno[3,2-*b*]imidazo[4,5-*d*]pyridin-1-yl}methyl)hexahydropyridine-1-carboxylate to obtain the title compound (25 mg, yield: 38.1%).

MS m/z (ESI): 428.3 [M+H]⁺.

### Example 31: Preparation of 2-butyl-1-(hexahydropyridin-4-ylmethyl)-7-[1-(2-methoxyethyl) hexahydropyridin-4-yl]thieno[3,2-b]imidazo[4,5-d]pyridine-4-amine (Compound 47)

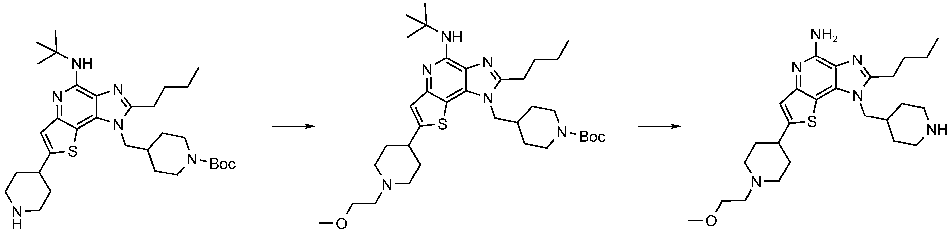

### Step 1: Preparation of tert-butyl 4-({2-butyl-7-[1-(2-methoxyethyl)hexahydropyridin-4-yl]-4-(tert-butylamino)thieno[3,2-b]imidazo[4,5-d]pyridin-1-yl}methyl)hexahydropyridine-1-carboxylate

The synthetic route of Example 24 was applied, the raw material triethylene glycol monomethyl ether of step 3 was replaced with 2-methoxyethanol to obtain the title compound (260 mg, yield: 76.3%).

MS m/z (ESI): 641.4 [M+H]⁺.

### Step 2: Preparation of 2-butyl-1-(hexahydropyridin-4-ylmethyl)-7-[1-(2-methoxyethyl) hexahydropyridin-4-yl]thieno[3,2-b]imidazo[4,5-d]pyridine-4-amine (Compound 47)

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl 4-({2-butyl-7-[1-(2-methoxyethyl)hexahydropyridin-4-yl]-4- (tert-butylamino)thieno[3,2-*b*]imidazo[4,5-*d*]pyridin-1-yl}methyl)hexahydropyridine-1-carboxylate to obtain the title compound (34 mg, yield: 42.3%).

MS m/z (ESI): 485.3 [M+H]⁺.

### II. Preparation of intermediates of the conjugates

### Example 2-1: Preparation of N-(4-(4-amino-2-butyl-7-methyl-1H-imidazo[4,5-d] thieno[3,2-b]pyridin-1-yl)butyl)-4-((2,5-dioxy-2,5-dihydro-1H-pyrrol-1-yl)methyl)cyclohexylcarboxamide (Compound D-L'-1)

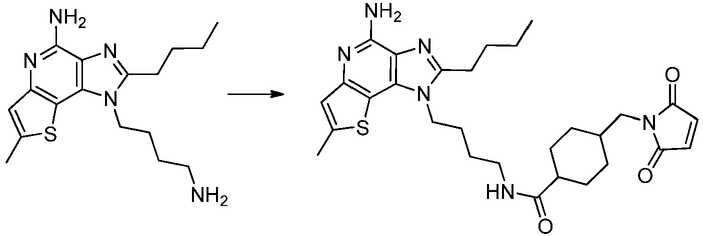

1-(4-aminobutyl)-2-butyl-7-methyl-1*H*-imidazo[4,5-*d*]thieno[3,2-*b*]pyridine-4-amine (Compound **1**) (1.50 g, 4.53 mmol) was dissolved in a mixed solvent of dichloromethane (10 mL) and *N*,*N*-dimethylformamide (10 mL), cooled in an ice-water bath, and 4-(*N-*maleimidomethyl)cyclohexyl-1-carboxylic acid succinimidyl ester (1.82 g, 5.43 mmol) and *N,N-*diisopropylethylamine (1.17 g, 9.06 mmol) were added to the reaction solution at 0°C, slowly warmed to room temperature and stirred for 12 hours. The reaction solution was poured into water (150 mL), extracted three times with dichloromethane (100 mL), the organic phases were combined, washed three times with saturated brine (100 mL), the organic phase was dried over anhydrous sodium sulfate, concentrated, and the obtained residue was purified by silica gel column chromatography (eluent: dichloromethane: methanol = 10:1, volume ratio) to obtain the title compound (1.55 g, yield: 62.1%).

MS m/z (ESI): 551.3 [M+H]⁺.

¹H NMR (400 MHz, DMSO-*d₆*): δ 8.15 (s, 1H), 7.02 (s, 2H), 6.96-6.94 (m, 1H), 6.08 (s, 2H), 4.19 (t, *J =* 8.0 Hz, 2H), 3.23-3.21 (m, 3H), 3.08-3.03 (m, 2H), 2.86-2.82 (m, 2H), 2.55-2.52 (m, 3H), 1.95-1.91 (m, 1H), 1.80 -1.70 (m, 4H), 1.63-1.40 (m, 8H), 1.23-1.16 (m, 2H), 0.95 (t, *J* = 8.0 Hz, 3H), 0.90-0.79 (m, 2H).

### Example 2-2: Preparation of N-[4-(4-amino-2-butyl-7-methylthieno[3,2-b]imidazo[4,5-d] pyridin-1-yl)butyl]-4-({4-[({6-[2-(methyldioxo-λ6-thio)pyrimidin-5-yl]-1-oxohex-5-ynyl}amino)methyl]-1,2,3-triazol-1-yl}methyl)cyclohexylcarboxamide (Compound D-L'-2)

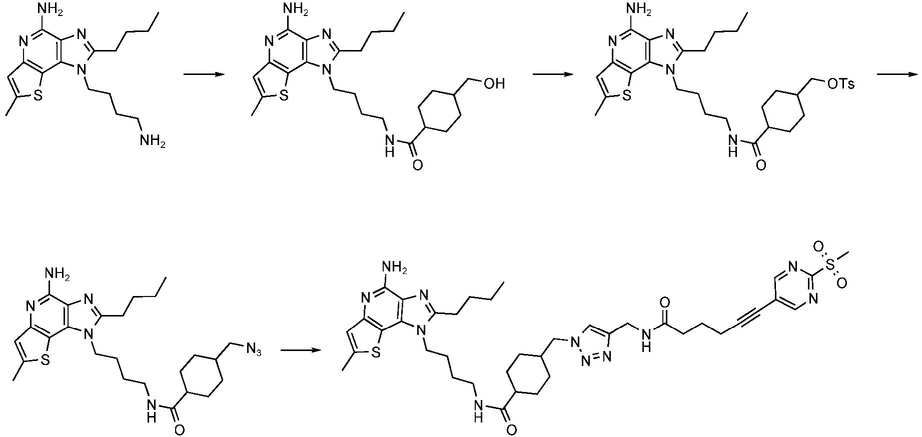

### Step 1: Preparation of N-[4-(4-amino-2-butyl-7-methylthieno[3,2-b]imidazo[4,5-d] pyridin-1-yl)butyl]-4-(hydroxymethyl)cyclohexylcarboxamide

1-(4-aminobutyl)-2-butyl-7-methyl-1*H*-imidazo[4,5-*d*]thieno[3,2-*b*]pyridine-4-amine (0.5 g, 1.51 mmol), 2-(7-azobenzotriazole)-*N*,*N*,*N'*,*N*'-tetramethyluronium hexafluorophosphate (0.69 g, 1.81 mmol), 4-(hydroxymethyl)cyclohexyl-1-carboxylic acid (0.24 g, 1.51 mmol) and *N,N-*diisopropylethylamine (0.39 g,3.02 mmol) were sequentially added to anhydrous *N,N-*dimethylformamide (10 mL), and stirred at room temperature for 12 hours. The reaction solution was poured into water (30 mL), extracted three times with dichloromethane (30 mL), the organic phases were combined, washed three times with saturated brine (15 mL), the organic phase was dried over anhydrous sodium sulfate, concentrated, and the obtained residue was purified by silica gel column chromatography (eluent: dichloromethane:methanol=10:1, volume ratio) to obtain the title compound (0.61 g, yield: 85.6%).

MS m/z (ESI): 472.3 [M+H]⁺.

### Step 2: Preparation of [4-({[4-(4-amino-2-butyl-7-methylthieno[3,2-b]imidazo[4,5-d] pyridin-1-yl)butyl]amino}carbonyl)cyclohexyl]methyl 4-methylbenzenesulfonate

*N*-[4-(4-amino-2-butyl-7-methylthieno[3,2-*b*]imidazo[4,5-*d*]pyridin-1-yl)butyl]-4-(hydroxymethyl)cyclohexylcarboxamide (0.5 g, 1.06 mmol), 4-dimethylaminopyridine (13 mg, 0.11 mmol), *N*,*N*-diisopropylethylamine (0.27 g, 2.12 mmol) and p-toluenesulfonyl chloride (0.3 g, 1.59 mmol) were successively added to anhydrous *N*,*N-*dimethylformamide (10 mL), and stirred at room temperature for 12 hours. The reaction solution was poured into water (30 mL), extracted three times with dichloromethane (30 mL), the organic phases were combined, washed three times with saturated brine (15 mL), the organic phase was dried over anhydrous sodium sulfate, and concentrated, and the obtained residue was purified by silica gel column chromatography (eluent: dichloromethane:methanol=10:1, volume ratio) to obtain the title compound (0.31 g, yield: 46.7%).

MS m/z (ESI): 626.3 [M+H]⁺.

### Step 3: Preparation of N-[4-(4-amino-2-butyl-7-methylthieno[3,2-b]imidazo[4,5-d] pyridin-1-yl)butyl]-4-(azidomethyl)cyclohexylcarboxamide

[4-({[4-(4-amino-2-butyl-7-methylthieno[3,2-*b*]imidazo[4,5-*d*]pyridin-1-yl)butyl]amino}carbonyl)cyclohexyl]methyl 4-methylbenzenesulfonate (0.15 g, 0.24 mmol) and sodium azide (31.2 mg, 0.48 mmol) were sequentially added to anhydrous *N,N-*dimethylformamide (3 mL), warmed to 70°C and stirred for 12 hours. The reaction solution was poured into water (30 mL), extracted three times with dichloromethane (30 mL), the organic phases were combined, washed three times with saturated brine (15 mL), the organic phase was dried over anhydrous sodium sulfate and concentrated to obtain the title compound (110 mg, yield: 92.3%).

MS m/z (ESI): 497.3 [M+H]⁺.

### Step 4: Preparation of N-[4-(4-amino-2-butyl-7-methylthieno[3,2-b]imidazo[4,5-d] pyridin-1-yl)butyl]-4-({4-[({6-[2-(methyldioxo-λ6-thio)pyrimidin-5-yl]-1-oxohex-5-ynyl}amino)methyl]-1,2,3-triazol-1-yl}methyl)cyclohexylcarboxamide

*N*-[4-(4-amino-2-butyl-7-methylthieno[3,2-*b*]imidazo[4,5-*d*]pyridin-1-yl)butyl]-4-(azidomethyl)cyclohexylcarboxamide (100 mg, 0.20 mmol), 6-[2-(methyldioxo-*λ*6-thio)pyrimidin-5-yl]- *N*-(prop-1-ynyl-3-yl)hex-5-ynylamide (68 mg, 0.22 mmol), sodium ascorbate (59 mg, 0.30 mmol) and anhydrous copper sulfate (64 mg, 0.40 mmol) were sequentially added to a mixed solvent of dimethyl sulfoxide (2 mL) and water (0.5 mL), stirred at room temperature for 1 hour. The reaction solution was poured into water (30 mL), extracted three times with dichloromethane (30 mL), the organic phases were combined, washed three times with saturated brine (15 mL), the organic phase was dried over anhydrous sodium sulfate, concentrated, and the obtained residue was purified by preparative high performance liquid chromatography to obtain the title compound (30 mg, yield: 18.7%).

MS m/z (ESI): 802.4 [M+H]⁺.

¹H NMR (400 MHz, DMSO-*d₆*): δ 9.10 (s, 2H), 8.39 (s, 1H), 7.88 (s, 1H), 7.70 (s, 1H), 6.94 (s, 1H), 6.03 (s, 2H), 4.29-4.17 (m, 6H), 3.41-3.35 (m, 6H), 3.05-2.84 (m, 4H), 2.30 (s, 2H), 1.91-1.23 (m, 20H), 1.22-0.93 (m, 5H).

### Example 2-3: Preparation of N-{26-[4-(4-amino-2-butyl-1H-thieno[3,2-b]imidazo[4,5-d] pyridin-7-yl)hexahydropyridin-1-yl]-3,6,9,12,15,18,21,24-octaoxahexacosan-1-yl}-6-[2-(methyldioxo-λ6-thio)pyrimidin-5-yl]hex-5-ynamide (Compound D-L'-3)

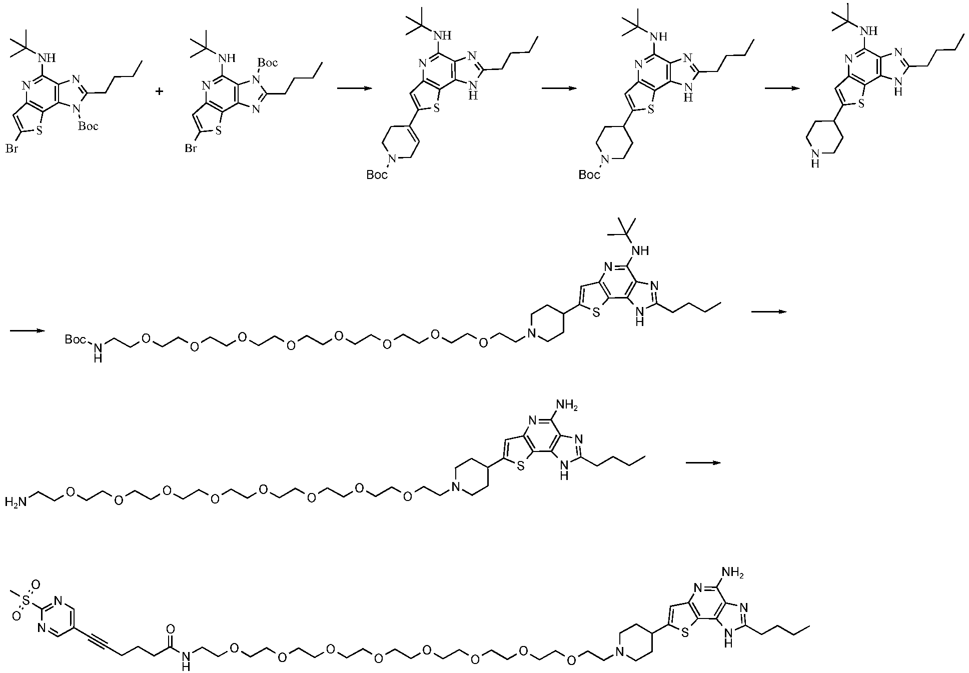

### Step 1: Preparation of tert-butyl 4-{2-butyl-4-(tert-butylamino)-1H-thieno[3,2-b] imidazo[4,5-d]pyridin-7-yl}-1,2,3,6-tetrahydropyridine-1-carboxylate

The synthetic route of Example 6 was applied, the raw material 1-methyl-4-(4,4,5,5-tetramethyl-1,3,2-dioxaborocyclopentan-2-yl)-1,2,3,6-tetrahydropyridine of step 9 was replaced with tert-butyl 4-(4,4,5,5-tetramethyl-1,3,2-dioxaborocyclopentan-2-yl)- 1,2,3,6-tetrahydropyridine-1-carboxylate to obtain the title compound (0.32 g, yield: 56.8%).

MS m/z (ESI): 484.3 [M+H]⁺.

### Step 2: Preparation of tert-butyl 4-{2-butyl-4-(tert-butylamino)-1H-thieno[3,2-b] imidazo[4,5-d]pyridin-7-yl}hexahydropyridine-1-carboxylate

The synthetic route of Example 6 was applied, the raw material *N*-(tert-butyl)-2-butyl- 7-(1-methyl-1,2,3,6-tetrahydropyridin-4-yl)-1*H*-imidazo[4,5-*d*]thieno[3,2-*b*]pyridine-4-amine of step 10 was replaced with tert-butyl 4-{2-butyl-4-(tert-butylamino)-1*H*-thieno[3,2-*b*] imidazo[4,5-d]pyridin-7-yl}-1,2,3,6-tetrahydropyridine-1-carboxylate to obtain the title compound (0.25 g, yield: 83.3%).

MS m/z (ESI): 486.3 [M+H]⁺.

### Step 3: Preparation of 2-butyl-7-(hexahydropyridin-4-yl)-4-(tert-butylamino)-1H-thieno[3,2-b]imidazo[4,5-d]pyridine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl 4-{2-butyl-4-(tert-butylamino)-1*H*-thieno[3,2-*b*] imidazo[4,5-*d* ]pyridin-7-yl}hexahydropyridine-1-carboxylate to obtain the title compound (0.16 g, yield: 80.8%).

MS m/z (ESI): 386.3 [M+H]⁺.

### Step 4: Preparation of tert-butyl {[26-(4-{2-butyl-4-(tert-butylamino)-1H-thieno[3,2-b] imidazo[4,5-d]pyridin-7-yl}hexahydropyridin-1-yl)-3,6,9,12,15,18,21,24-octaoxahexacosan-1-yl]amino}carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydropyridin-4-yl)-4-(tert-butylamino)thieno[3,2-*b*]imidazo[4,5-*d*] pyridin-1-yl)methyl]hexahydropyridine-1-carboxylate was replaced with 2-butyl-7-(hexahydropyridin-4-yl)-4-(tert-butylamino)-1*H*-thieno[3,2-*b*]imidazo[4,5-*d*]pyridine to obtain the title compound (80 mg, yield: 45.2%).

MS m/z (ESI): 881.6 [M+H]⁺.

### Step 5: Preparation of 2-{[23-(4-{2-butyl-4-(tert-butylamino)-1H-thieno[3,2-b] imidazo[4,5-d]pyridin-7-yl}hexahydropyridin-1-yl)-3,6,9,12,15,18,21-heptaoxatricosan-1-yl]oxy}ethyl-1-amine

The synthetic route of Example 6 was applied, the raw material *N*-(tert-butyl)-2-butyl-7- (1-methylpiperidin-4-yl)-1*H*-imidazo[4,5-*d*]thieno[3,2-*b*]pyridine-4-amine of step 11 was replaced with tert-butyl {[26-(4-{2-butyl-4-(tert-butylamino)-1*H*-thieno[3,2-*b*]imidazo[4,5-*d*] pyridin-7-yl}hexahydropyridin-1-yl)-3,6,9,12,15,18,21,24-octaoxahexacosan-1-yl]amino}carboxylate to obtain the title compound (62 mg, yield: 75.3%).

MS m/z (ESI): 725.4 [M+H]⁺.

### Step 6: Preparation of N-{26-[4-(4-amino-2-butyl-1H-thieno[3,2-b]imidazo[4,5-d] pyridin-7-yl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide

2-{[23-(4-{2-Butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine (50 mg, 64.0 µmol), *N,N*-diisopropylethylamine (16.5 mg, 128.0 µmol) and 1-({1-[2-(methyldioxo-*λ*6-thio) pyrimidin-5-yl]-6-oxohex-1-ynyl-6-yl}oxy)tetrahydropyrrol-2,5-dione (28.1 mg, 77 µmol) were sequentially added to *N*,*N-*dimethylformamide (2 mL), and stirred at room temperature for 1 hour. The reaction solution was purified by preparative high performance liquid chromatography to obtain the title compound (20 mg, yield: 32.0%).

MS m/z (ESI): 975.5 [M+H]⁺.

¹H NMR (400 MHz, D₂O): δ 8.75 (s, 2H), 8.50 (s, 1H), 7.07 (s, 1H), 3.96-3.93 (m, 2H), 3.86-3.46 (m, 35H), 3.43 (s, 3H), 3.42-3.25 (m, 4H), 2.91 (t, *J* = 7.6 Hz, 2H), 2.57-2.41 (m, 6H), 2.11 (s, 2H), 1.96-1.75 (m, 4H), 1.44-1.37 (m, 2H), 0.97 (t, *J* = 7.6 Hz, 3H).

### Example 2-4: Preparation of N-(26-{4-[(4-amino-2-butyl-7-methylthieno[3,2-b] imidazo[4,5-d]pyridin-1-yl)methyl]hexahydropyridin-1-yl}-3,6,9,12,15,18,21,24-octaoxahexacosan-1-yl)-6-[2-(methyldioxo-λ6-thio)pyrimidin-5-yl]hex-5-ynylamide (Compound D-L'-4)

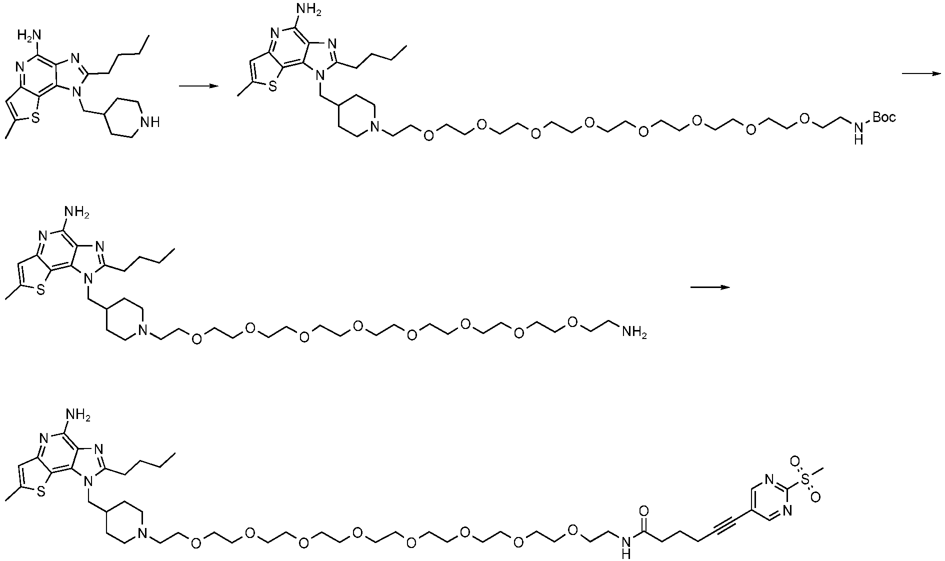

### Step 1: Preparation of tert-butyl [(26-{4-[(4-amino-2-butyl-7-methylthieno[3,2-b] imidazo[4,5-d]pyridin-1-yl)methyl]hexahydropyridin-1-yl}-3,6,9,12,15,18,21,24-octaoxahexacosan-1-yl)amino]carboxylate

the synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydropyridin-4-yl)-4-(tert-butylamino)thieno[3,2-*b*]imidazo[4,5-*d*] pyridin-1-yl)methyl]hexahydropyridine-1-carboxylate was replaced with 2-butyl-7-methyl-1- (piperidin-4-ylmethyl)-1*H*-imidazo[4,5-*d*]thieno[3,2-*b*]pyridine-4-amine to obtain the title compound (70 mg, yield: 52.3%).

MS m/z (ESI): 853.5 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-methylthieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl [(26-{4-[(4-amino-2-butyl-7-methylthieno[3,2-*b*]imidazo[4,5-*d*] pyridin-1-yl)methyl]hexahydropyridin-1-yl}-3,6,9,12,15,18,21,24-octaoxahexacosan-1-yl)amino]carboxylate to obtain the title compound (55 mg, yield: 85.6%).

MS m/z (ESI): 753.5 [M+H]⁺.

### Step 3: Preparation of N-(26-{4-[(4-amino-2-butyl-7-methylthieno[3,2-b]imidazo[4,5-d] pyridin-1 -yl)methyl]hexahydropyridin-1 -yl} -3,6,9,12,15,18,21,24-octoxahexacosan-1 -yl)-6-[2-(methyldioxo-λ6-thio)pyrimidin-5-yl]hex-5-ynylamide

The synthetic route of Example 2-3 was applied, the reaction material 2-{ [23-(4-{2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-butyl-7-methylthieno[3,2-*b*]imidazo[4,5-*d*]pyridine-4-amine to obtain the title compound (19 mg, yield rate: 47.2%).

MS m/z (ESI): 1003.6 [M+H]⁺.

¹H NMR (400 MHz, DMSO-*d₆*): δ 9.12 (s, 2H), 8.00-7.97 (m, 1H), 7.05 (s, 1H), 6.69-6.52 (br, 2H), 4.17 (d, *J* = 7.6 Hz, 2H), 3.61-3.47 (m, 36H), 2.92-2.81 (m, 4H), 2.62-2.54 (m, 8H), 2.27 (t, *J* = 7.6 Hz, 2H), 2.17-2.04 (m, 1H), 1.87-1.64 (m, 8H), 1.45-1.42 (m, 2H), 1.27-1.22 (m, 2H), 0.96 (t, *J* = 7.6 Hz, 3H).

### Example 2-5: Preparation of N-(26-{4-[(4-amino-2-butyl-7-methylthieno[3,2-b]imidazo[4, 5-d]pyridin-1-yl)methyl]hexahydropyridin-1-yl}-3,6,9,12,15,18,21,24-octaoxahexacosan-1-yl)-6-(2,5-dioxo-2,5-dihydro-1H-pyrrol-1-yl)hexanamide (Compound D-L'-5)

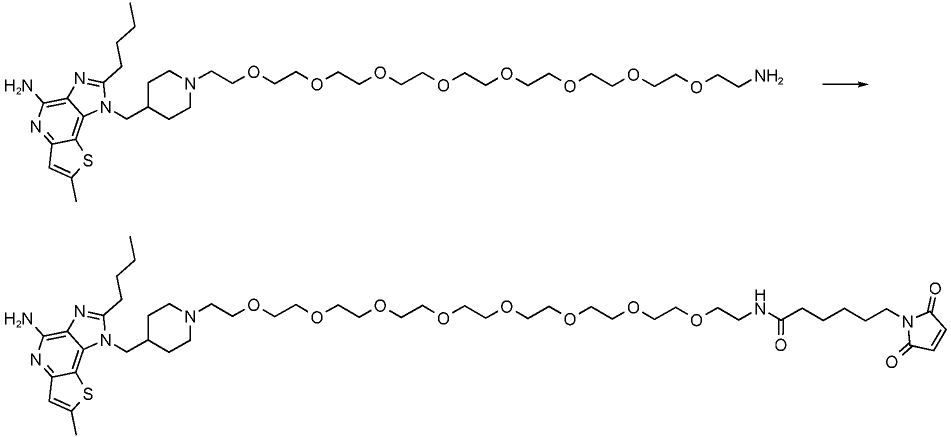

1-{[1-(26-amino-3,6,9,12,15,18,21,24-octaoxahexacosan-1-yl)hexahydropyridin-4-yl]methyl}-2-butyl-7-methylthieno[3,2-*b*]imidazo[4,5-*d*]pyridine-4-amine (20 mg, 26.6 µmol), *N*,*N*-diisopropylethylamine (7 mg, 53.2 µmol) and 1-{6-[(2,5-dioxotetrahydro-1*H*-pyrrol-1-yl) oxy]-6-oxohexyl}pyrrole-2,5-dione (10 mg, 31.9 µmol) were sequentially added to *N,N-*dimethylformamide (1 mL), and stirred at room temperature for 1 hour. The reaction solution was purified by preparative high performance liquid chromatography to obtain the title compound (11 mg, yield: 43.7%).

MS m/z (ESI): 946.5 [M+H]⁺.

¹H NMR (400 MHz, CD₃OD): δ 6.99 (s, 1H), 4.24-4.21 (m, 2H), 3.69 (s, 2H), 3.65-3.53 (m, 31H), 3.53-3.44 (m, 4H), 3.34-3.32 (m, 2H), 2.98-2.84 (m, 4H), 2.61 (s, 3H), 2.39-2.32 (m, 2H), 2.17-2.08 (m, 4H), 1.91-1.85 (m, 2H), 1.76-1.48 (m, 10H), 1.33-1.23 (m, 2H), 1.03 (t, *J* = 7.6 Hz, 3H).

### Example 2-6: Preparation of N-(26-{4-[4-amino-2-butyl-1-(3,4,5,6-tetrahydro-2H-pyran- 4-ylmethyl)thieno[3,2-b]imidazo[4,5-d]pyridin-7-yl]hexahydropyridin-1-yl}-3,6,9,12,15,18,21,24-octaoxahexacosan-1-yl)-6-[2-(methyldioxo-λ6-thio)pyrimidin-5-yl]hex-5-ynylamide (Compound D-L'-6)

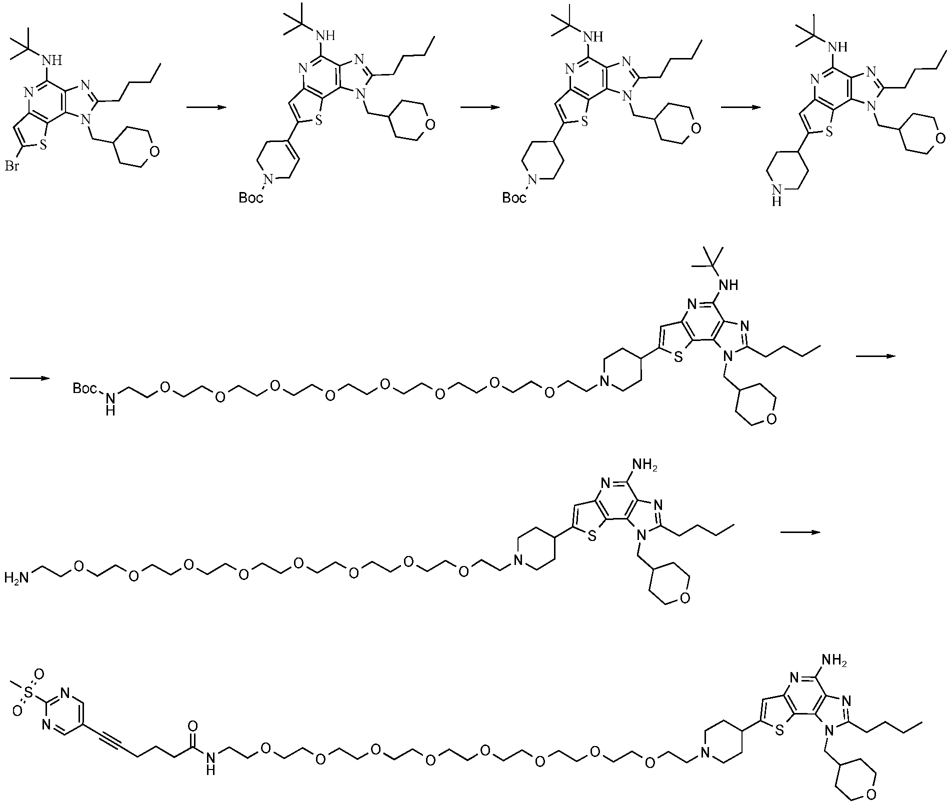

### Step 1: Preparation of tert-butyl 4-{2-butyl-4-(tert-butylamino)-1-(3,4,5,6-tetrahydro-2H-pyran-4-ylmethyl)thieno[3,2-b]imidazo[4,5-d]pyridin-7-yl}-1,2,3,6-tetrahydropyridine-1-carboxylate

The synthetic route of Example 6 was applied, the mixture of tert-butyl 7-bromo-2-butyl-4-(tert-butylamino)-1*H*-imidazo[4,5-*d*]thieno[3,2-*b*]pyridine-1-carboxylate and tert-butyl 7-bromo-2-butyl-4-(tert-butylamino)-3*H*-imidazo[4,5-*d*]thieno[3,2-*b*]pyridine-3-carboxylate as the raw material of step 9 was replaced with 7-bromo-*N*-(tert-butyl)-2-butyl-1- ((tetrahydro-2*H*-pyran-4-yl)methyl)-1*H*-imidazo[4,5-*d*]thieno[3,2-*b*]pyridine-4-amine, the raw material 1-methyl-4-(4,4,5,5-tetramethyl-1,3,2-dioxaborocyclopentan-2-yl)-1,2,3,6- tetrahydropyridine was replaced with tert-butyl 4-(4,4,5,5-tetramethyl-1,3,2- dioxaborocyclopent-2-yl)-1,2,3,6-tetrahydropyridine-1-carboxylate to obtain the title compound (0.25 g, yield: 72.8%).

MS m/z (ESI): 582.3 [M+H]⁺.

### Step 2: Preparation of tert-butyl 4-{2-butyl-4-(tert-butylamino)-1-(3,4,5,6-tetrahydro-2H-pyran-4-ylmethyl)thieno[3,2-b]imidazo[4,5-d]pyridin-7-yl}hexahydropyridine-1-carboxylate

The synthetic route of Example 6 was applied, the raw material *N*-(tert-butyl)-2-butyl-7- (1-methyl-1,2,3,6-tetrahydropyridin-4-yl)-1*H*-imidazo[4,5-*d*]thieno[3,2-*b*]pyridine-4-amine of step 10 was replaced with tert-butyl 4-{2-butyl-4-(tert-butylamino)-1-(3,4,5,6-tetrahydro-2*H*- pyran-4-ylmethyl)thieno[3,2-*b*]imidazo[4,5-*d*]pyridin-7-yl}-1,2,3,6-tetrahydropyridine-1-carboxylate to obtain the title compound (0.22 g, yield: 88.3%).

MS m/z (ESI): 584.4 [M+H]⁺.

### Step 3: Preparation of 2-butyl-7-(hexahydropyridin-4-yl)-4-(tert-butylamino)-1-(3,4,5,6-tetrahydro-2H-pyran-4-ylmethyl)thieno[3,2-b]imidazo[4,5-d]pyridine

The synthetic route of Example 1 was applied, the raw material tert-butyl (4-(4-amino- 2-butyl-7-methyl-1*H*-imidazo[4,5-*d*]thieno[3,2-*b*]pyridin-1-yl)butyl)carbamate of step 9 was replaced with tert-butyl 4-{2-butyl-4-(tert-butylamino)-1-(3,4,5,6-tetrahydro-2*H*-pyran-4-ylmethyl)thieno[3,2-*b*]imidazo[4,5-*d*]pyridin-7-yl}hexahydropyridine-1-carboxylate to obtain the title compound (0.13 g, yield : 81.2%).

MS m/z (ESI): 484.3 [M+H]⁺.

### Step 4: Preparation of tert-butyl {[26-(4-{2-butyl-4-(tert-butylamino)-1-(3,4,5,6-tetrahydro-2H-pyran-4-ylmethyl)thieno[3,2-b]imidazo[4,5-d]pyridin-7-yl}hexahydropyridin-1-yl)-3,6,9,12,15,18,21,24-octaoxohexacosan-1-yl]amino}carboxylate

the synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydro pyridin-4-yl)-4-(tert-butylamino)thieno[3,2-*b*]imidazo[4,5-*d*]pyridin-1-yl)methyl]hexahydropyridine-1-carboxylate was replaced with 2-butyl-7-(hexahydropyridin-4-yl)-4-(tert-butylamino)- 1-(3,4,5,6-tetrahydro-2*H*-pyran-4-ylmethyl)thieno[3,2-*b*]imidazo[4,5-d]pyridine to obtain the title compound (52 mg, yield: 46.3%).

MS m/z (ESI): 979.6 [M+H]⁺.

### Step 5: Preparation of 7-[1-(26-amino-3,6,9,12,15,18,21,24-octaoxahexacosan-1-yl) hexahydropyridin-4-yl]-2-butyl-1-(3,4,5,6-tetrahydro-2H-pyran-4-ylmethyl)thieno[3,2-b]imidazo[4,5-d]pyridine-4-amine

The synthetic route of Example 6 was applied, the raw material *N*-(tert-butyl)-2-butyl- 7-(1-methylpiperidin-4-yl)-1*H*-imidazo[4,5-*d*]thieno[3,2-*b*]pyridine-4-amine of step 11 was replaced with tert-butyl {[26-(4-{2-butyl-4-(tert-butylamino)-1-(3,4,5,6-tetrahydro-2*H*-pyran- 4-ylmethyl)thieno[3,2-*b*]imidazo[4,5-*d*]pyridin-7-yl}hexahydropyridin-1-yl)-3,6,9,12,15,18,21,24-octoxahexacosan-1-yl]amino}carboxylate to obtain the title compound (40 mg, yield: 90.9%).

MS m/z (ESI): 823.5 [M+H]⁺.

### Step 6: Preparation of N-(26-{4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}-3,6,9,12,15,18,21,24-octaoxahexacosan-1-yl)-6-[2-(methyldioxo-λ6-thio)pyrimidin-5-yl]hex-5-ynylamide

The synthetic route of Example 2-3 was applied, the reaction material 2-{[23-(4-{2-butyl- 4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 7-[1-(26-amino-3,6,9,12,15,18,21,24-octaoxahexacosan-1-yl)hexahydropyridin-4-yl]-2-butyl-1-(3,4,5,6-tetrahydro-2*H*-pyran-4-ylmethyl)thieno[3,2-*b*]imidazo[4,5-*d*]pyridine-4-amine to obtain the title compound (19 mg, yield: 37.1%).

MS m/z (ESI): 1073.5 [M+H]⁺.

¹H NMR (400 MHz, DMSO-*d₆*): δ 9.12 (s, 2H), 8.17 (s, 1H), 7.96 (s, 1H), 7.02 (s, 1H), 6.08 (s, 2H), 4.13 (d, *J =* 7.6 Hz, 2H), 3.85-3.82 (m, 2H), 3.57-3.30 (m, 31H), 3.26-3.15 (m, 6H), 3.03-3.00 (m, 2H), 2.92-2.82 (m, 3H), 2.60-2.55 (m, 5H), 2.31-2.17 (m, 8H), 1.84-1.65 (m, 6H), 1.48-1.38 (m, 6H), 0.94 (t, *J =* 7.2 Hz, 3H).

### Example 2-7: Preparation ofN-{26-[4-(4-amino-2-butyl-1-{[1-(2-methoxyethyl)hexahydro pyridin-4-yl]methyl}thieno[3,2-b]imidazo[4,5-d]pyridin-7-yl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide (Compound D-L'-7)

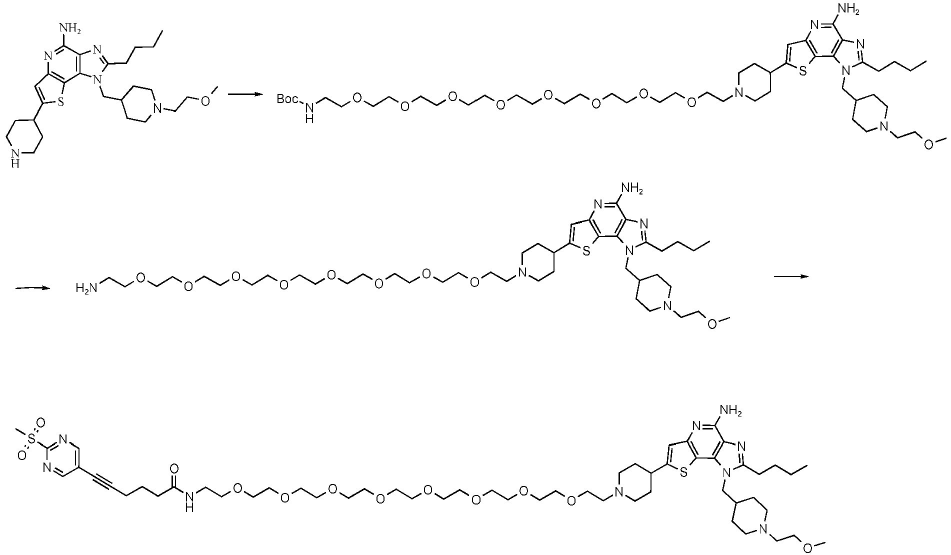

### Step 1: Preparation of tert-butyl ({26-[4-(4-amino-2-butyl-1-{[1-(2-methoxyethyl) hexahydropyridin-4-yl]methyl}thieno[3,2-b]imidazo[4,5-d]pyridin-7-yl)hexahydropyridin-1-yl]-3,6,9,12,15,18,21,24-octaoxahexacosan-1-yl}amino)carboxylate

Tthe synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydropyridin-4-yl)-4-(tert-butylamino)thieno[3,2-*b*]imidazo[4,5-*d*] pyridin-1-yl)methyl]hexahydropyridine-1-carboxylate was replaced with 2-butyl-7-(hexahydro pyridin-4-yl)-1-{[1-(2-methoxyethyl)hexahydropyridin-4-yl]methyl}thieno[3,2-*b*]imidazo[4,5-*d*]pyridine-4-amine to obtain the title compound (45 mg, yield: 43.3%).

MS m/z (ESI): 980.6 [M+H]⁺.

### Step 2: Preparation of 7-[1-(26-amino-3,6,9,12,15,18,21,24-octaoxahexacosan-1-yl) hexahydropyridin-4-yl]-2-butyl-1-{ [1-(2-methoxyethyl)hexahydropyridin-4-yl]methyl}thieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl ({26-[4-(4-amino-2-butyl-1- { [1-(2-methoxyethyl)hexahydropyridin- 4-yl]methyl}thieno[3,2-*b*]imidazo[4,5-*d*]pyridin-7-yl)hexahydropyridin-1-yl]-3,6,9,12,15,18,21,24-octaoxahexacosan-1-yl}amino)carboxylate to obtain the title compound (33 mg, yield: 82.5%).

MS m/z (ESI): 880.5 [M+H]⁺.

### Step 3: Preparation of N-{26-[4-(4-amino-2-butyl-1-{[1-(2-methoxyethyl)hexahydro pyridin-4-yl]methyl}thieno[3,2-b]imidazo[4,5-d]pyridin-7-yl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide

The synthetic route of Example 2-3 was applied, the reaction material 2-{ [23-(4-{2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 7-[1-(26-amino-3,6,9,12,15,18,21,24-octaoxahexadecan-1-yl)hexahydropyridin-4-yl]-2-butyl-1-{[1-(2-methoxyethyl)hexahydropyridin-4-yl]methyl}thieno[3,2-*b*]imidazo[4,5*-d*]pyridine-4-amine to obtain the title compound (10 mg, yield: 23.8%).

MS m/z (ESI): 1130.6 [M+H]⁺.

¹H NMR (400 MHz, DMSO-*d₆*): δ 9.12 (s, 2H), 7.95 (s, 1H), 7.01 (s, 1H), 6.05 (s, 2H), 4.14-4.08 (m, 2H), 3.55 -3.45 (m, 22H), 3.43-3.37 (m, 14H), 3.23-3.15 (m, 7H), 2.99-2.97 (m, 2H), 2.89-2.82 (m, 4H), 2.45-2.32 (m, 3H), 2.26 (t, *J* = 7.6 Hz, 2H), 2.16-1.94 (m, 8H), 1.85-1.65 (m, 8H), 1.49-1.36 (m, 6H), 0.94 (t, *J* = 7.6 Hz, 3H).

### Example 2-8: Preparation of N-[26-(4-{[4-amino-2-butyl-7-(3,4,5,6-tetrahydro-2H-pyran-4-yl)thieno[3,2-b]imidazo[4,5-d]pyridin-1-yl]methyl}hexahydropyridin-1-yl)-3,6,9,12,15,18,21,24-octaoxohexacosan-1-yl]-6-[2-(methyldioxo-λ6-thio)pyrimidin-5-yl]hex-5-ynylamide (Compound D-L'-8)

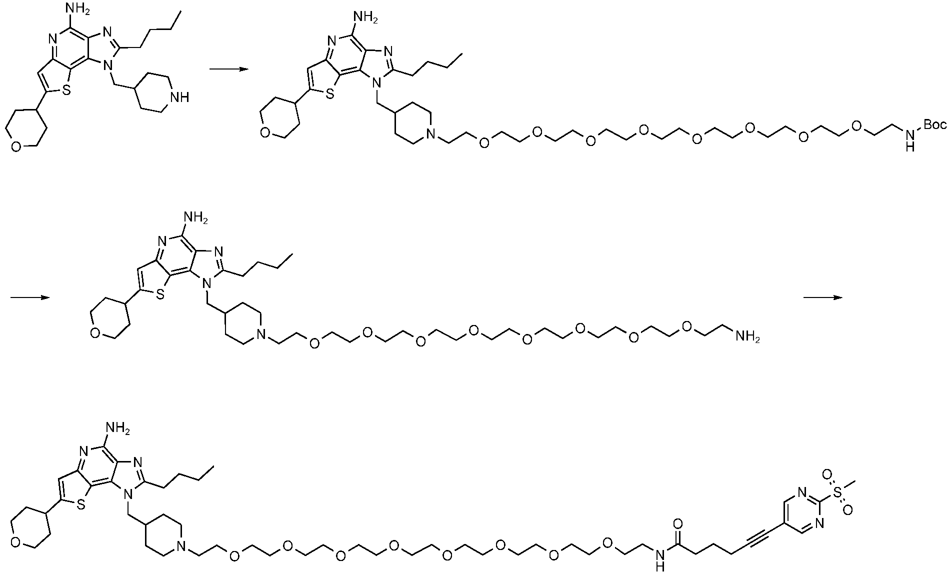

### Step 1: Preparation of tert-butyl { [26-(4- { [4-amino-2-butyl-7-(3,4,5,6-tetrahydro-2H-pyran-4-yl)thieno[3,2-b]imidazo[4,5-d]pyridin-1-yl]methyl}hexahydropyridin-1-yl)-3,6,9,12,15,18,21,24-octaoxahexacosan-1-yl]amino}carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydropyridin-4-yl)-4-(tert-butylamino)thieno[3,2-*b*]imidazo[4,5-*d*] pyridin-1-yl)methyl]hexahydropyridine-1-carboxylate was replaced with 2-butyl-1-(hexahydro pyridin-4-ylmethyl)-7-(3,4,5,6-tetrahydro-2*H*-pyran-4-yl)thieno[3,2-*b*]imidazo[4,5-*d*]pyridine-4-amine to obtain the title compound (115 mg, yield: 54.3%).

MS m/z (ESI): 923.6 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-(3,4,5,6-tetrahydro-2H-pyran-4-yl)thieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl {[26-(4-{[4-amino-2-butyl-7-(3,4,5,6-tetrahydro-2*H*-pyran-4-yl) thieno[3,2-*b*]imidazo[4,5-*d*]pyridin-1-yl]methyl}hexahydropyridin-1-yl)-3,6,9,12,15,18,21,24-octoxahexacosan-1-yl]amino}carboxylate to obtain the title compound (90 mg, yield: 88.2%).

MS m/z (ESI): 823.5 [M+H]⁺.

### Step 3: Preparation of N-[26-(4-{[4-amino-2-butyl-7-(3,4,5,6-tetrahydro-2H-pyran-4-yl) thieno[3,2-b]imidazo[4,5-d]pyridin-1-yl]methyl}hexahydropyridin-1-yl)-3,6,9,12,15,18,21,24-octaoxahexacosan-1-yl]-6-[2-(methyldioxo-λ6-thio)pyrimidin-5-yl]hex-5-ynylamide

The synthetic route of Example 2-3 was applied, the raw material 2-{[23-(4-{2-butyl-4- (tert-butylamino)-1*H*-thieno[3,2-*b*]imidazo[4,5*-d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-butyl-7-(3,4,5,6-tetrahydro-2*H*-pyran-4-yl)thieno[3,2-*b*]imidazo[4,5-*d*]pyridine-4-amine to obtain the title compound (20 mg, yield: 32.6%).

MS m/z (ESI): 1073.5 [M+H]⁺.

¹H NMR (400 MHz, DMSO-*d₆*): δ 9.11 (s, 2H), 7.95 (s, 1H), 7.03 (s, 1H), 6.07 (s, 2H), 4.11 (d, *J* = 7.6 Hz, 2H), 3.98-3.92 (m, 2H), 3.55-3.35 (m, 36H), 3.25-3.11 (m, 3H), 2.92-2.82 (m, 4H), 2.70-2.65 (m, 2H), 2.41 (s, 2H), 2.36-2.31 (m, 2H), 2.26 (t, *J =* 7.2 Hz, 2H), 1.97-1.93 (m, 2H), 1.86-1.65 (m, 8H), 1.44-1.34 (m, 6H), 0.94 (t, *J* = 7.6 Hz, 3H).

### Example 2-9: Preparation ofN-{26-[4-({4-amino-2-butyl-7-[1-(2-methoxyethyl)hexahydro pyridin-4-yl]thieno[3,2-b]imidazo[4,5-d]pyridin-1-yl}methyl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide (Compound D-L'-9)

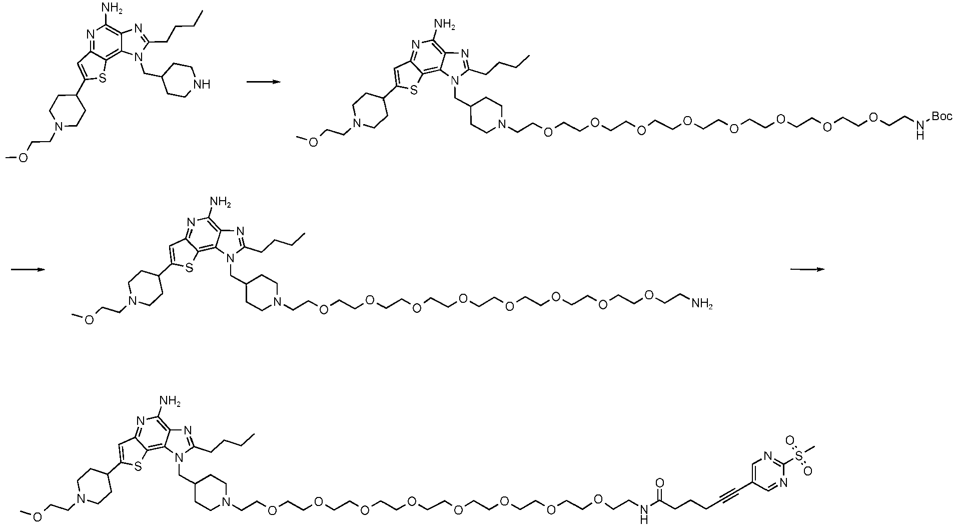

### Step 1: Preparation of tert-butyl ({26-[4-({4-amino-2-butyl-7-[1-(2-methoxyethyl) hexahydropyridin-4-yl]thieno[3,2-b]imidazo[4,5-d]pyridin-1-yl}methyl)hexahydropyridin-1-yl]-3,6,9,12,15,18,21,24-octaoxahexacosan-1-yl}amino)carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydropyridin-4-yl)-4-(tert-butylamino)thieno[3,2-*b*]imidazo[4,5-*d*]pyridin-1-yl)methyl]hexahydropyridine-1-carboxylate was replaced with 2-butyl-1-(hexahydropyridin-4-ylmethyl)- 7-[1-(2-methoxyethyl)hexahydropyridin-4-yl]thieno[3,2-*b*]imidazo[4,5-*d*]pyridine-4-amine to obtain the title compound (90 mg, yield: 24.7%).

MS m/z (ESI): 980.6 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-[1-(2-methoxyethyl)hexahydropyridin-4-yl]thieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl ({26-[4-({4-amino-2-butyl-7-[1-(2-methoxyethyl) hexahydropyridin-4-yl]thieno[3,2-*b*]imidazo[4,5-*d*]pyridin-1-yl}methyl)hexahydropyridin-1-yl]-3,6,9,12,15,18,21,24-octaoxahexacosan-1-yl}amino)carboxylate to obtain the title compound (75 mg, yield: 93.7%).

MS m/z (ESI): 880.5 [M+H]⁺.

### Step 3: Preparation of N-{26-[4-({4-amino-2-butyl-7-[1-(2-methoxyethyl)hexahydro pyridin-4-yl]thieno[3,2-b]imidazo[4,5-d]pyridin-1-yl}methyl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide

The synthetic route of Example 2-3 was applied, the reaction material 2- { [23-(4- { 2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-butyl-7-[1-(2-methoxyethyl)hexahydropyridin-4-yl]thieno[3,2-*b*]imidazo[4,5-*d*]pyridine-4-amine to obtain the title compound (25 mg, yield: 28.1%).

MS m/z (ESI): 1130.6 [M+H]⁺.

¹H NMR (400 MHz, DMSO-*d₆*): δ 9.12 (s, 2H), 8.19 (s, 1H), 7.95 (s, 1H), 7.02 (s, 1H), 6.09 (s, 2H), 4.12 (d, *J* = 7.2 Hz, 2H), 3.56-3.35 (m, 36H), 3.29-3.17 (m, 5H), 3.05-2.80 (m, 7H), 2.60-2.50 (m, 6H), 2.30-2.12 (m, 5H), 2.02-1.92 (m, 4H), 1.86-1.65 (m, 7H), 1.51-1.36 (m, 6H), 0.94 (t, *J* = 7.6 Hz, 3H).

### Example 2-10: Preparation of N-[(1-{[4-({4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}carbonyl)cyclohexyl]methyl}-1,2,3-triazol-4-yl)methyl]-6-[2-(methyldioxo-λ6-thio)pyrimidin-5-yl]hex-5-ynylamide (Compound D-L'-10)

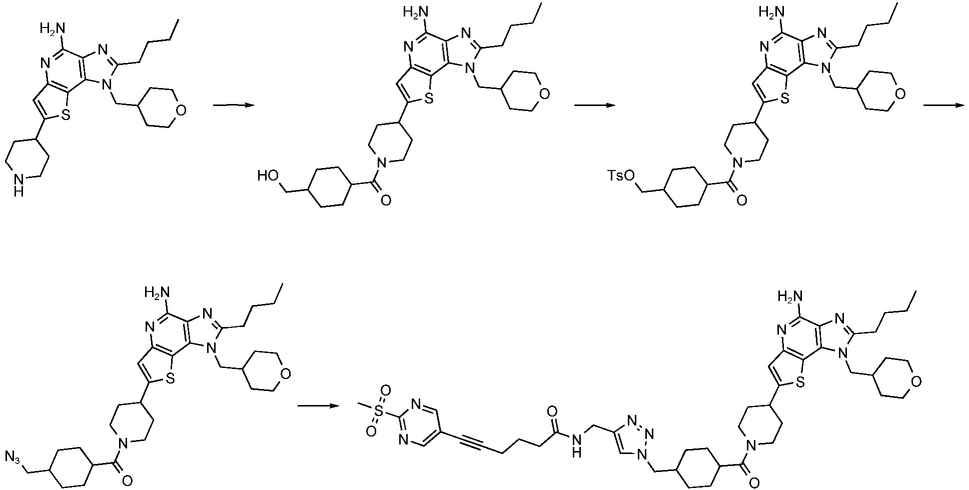

### Step 1: Preparation of {4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}[4-(hydroxymethyl)cyclohexyl]methanone

The synthetic route of step 1 of Example 2-2 was applied, 1-(4-aminobutyl)-2-butyl-7-methyl- 1*H*-imidazo[4,5-*d*]thieno[3,2-*b*]pyridine-4-amine was replaced with 2-butyl-7-(hexahydro pyridin-4-yl)-1-(3,4,5,6-tetrahydro-2*H*-pyran-4-ylmethyl)thieno[3,2-*b*]imidazo[4,5-d]pyridine-4-amine to obtain the title compound (0.27 g, yield: 86.8%).

MS m/z (ESI): 568.4 [M+H]⁺.

### Step 2: Preparation of [4-({4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}carbonyl)cyclohexyl]methyl 4-methylbenzenesulfonate

The synthetic route of step 2 of Example 2-2 was applied, *N*-[4-(4-amino-2-butyl-7-methyl thieno[3,2-*b*]imidazo[4,5-*d*]pyridin-1-yl)butyl]-4-(hydroxymethyl)cyclohexylcarboxamide was replaced with {4-[4-amino-2-butyl-1-(3,4,5,6-tetrahydro-2*H*-pyran-4-ylmethyl)thieno[3,2-*b*] imidazo[4,5-*d*]pyridin-7-yl]hexahydropyridin-1-yl}[4-(hydroxymethyl)cyclohexyl]methanone to obtain the title compound (0.20 g, yield: 51.2%).

MS m/z (ESI): 722.3 [M+H]⁺.

### Step 3: Preparation of {4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}[4-(azidomethyl)cyclohexyl]methanone

The synthetic route of step 3 of Example 2-2 was applied, [4-({ [4-(4-amino-2-butyl-7-methylthieno[3,2-*b*]imidazo[4,5-*d*]pyridin-1-yl)butyl]amino}carbonyl)cyclohexyl]methyl 4-methylbenzenesulfonate was replaced with [4-({4-[4-amino-2-butyl-1-(3,4,5,6-tetrahydro- 2*H-*pyran-4-ylmethyl)thieno[3,2-*b*]imidazo[4,5-*d*]pyridin-7-yl]hexahydropyridin-1-yl}carbonyl)cyclohexyl]methyl 4-methylbenzenesulfonate to obtain the title compound (0.15 g, yield: 87.3%).

MS m/z (ESI): 593.3 [M+H]⁺.

### Step 4: Preparation of N-[(1-{[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}carbonyl)cyclohexyl]methyl}-1,2,3-triazol-4-yl)methyl]-6-[2-(methyldioxo-λ6-thio)pyrimidin-5-yl]hex-5-ynylamide

The synthetic route of step 4 of Example 2-2 was applied, *N*-[4-(4-amino-2-butyl-7-methyl thieno[3,2-*b*]imidazo[4,5-*d*]pyridin-1-yl)butyl]-4-(azidomethyl)cyclohexylcarboxamide was replaced with {4-[4-amino-2-butyl-1-(3,4,5,6-tetrahydro-2*H*-pyran-4-ylmethyl)thieno[3,2-*b*] imidazo[4,5-*d*]pyridin-7-yl]hexahydropyridin-1-yl}[4-(azidomethyl)cyclohexyl]methanone to obtain the title compound (56 mg, yield: 34.5%).

MS m/z (ESI): 898.4 [M+H]⁺.

¹H NMR (400 MHz, DMSO-*d₆*): δ 9.11 (s, 2H), 8.37 (d, *J* = 5.6 Hz, 1H), 7.92 (s, 1H), 7.05 (s, 1H), 6.08 (s, 2H), 4.56-4.53 (m, 1H), 4.35-4.26 (m, 3H), 4.13 (d, *J* = 7.6 Hz, 2H), 4.02 (s, 1H), 3.85-3.82 (m, 2H), 3.41 (s, 3H), 3.26-3.09 (m, 4H), 2.88-2.62 (m, 5H), 2.36-2.25 (m, 3H), 2.15-1.96 (m, 5H), 1.84-1.77 (m, 4H), 1.74-1.28 (m, 16H), 0.94 (t, *J* = 7.2 Hz, 3H).

### Example 2-11: Preparation of N-(24-{4-[4-amino-2-butyl-1-(3,4,5,6-tetrahydro-2H-pyran- 4-ylmethyl)thieno[3,2-b]imidazo[4,5-d]pyridin-7-yl]hexahydropyridin-1-yl}-24-oxo-3,6,9,12,15,18,21-heptaoxotetracosan-1-yl)-6-[2-(methyldioxo-λ6-thio)pyrimidin-5-yl]hex-5-ynylamide (Compound D-L'-11)

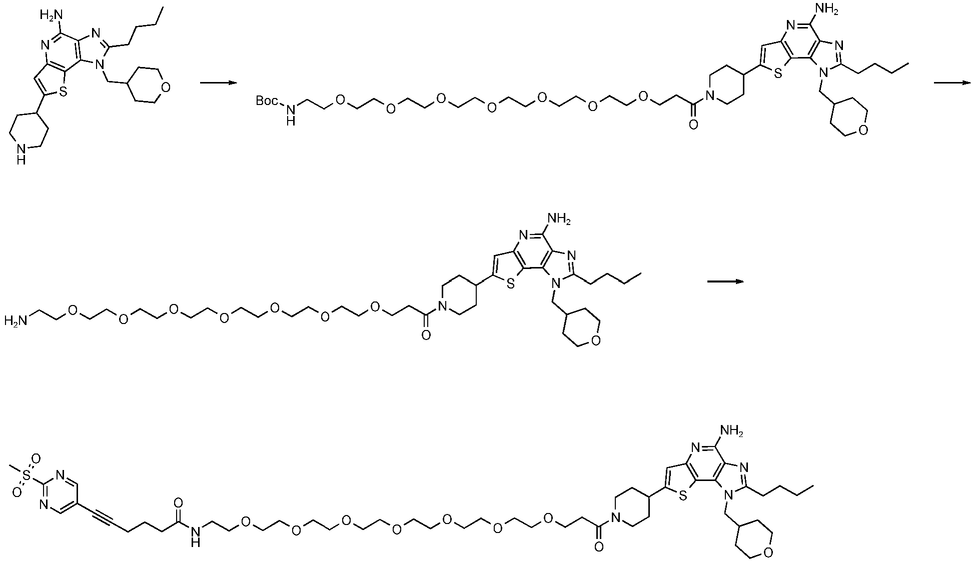

### Step 1: Preparation of tert-butyl [(24-{4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl }-24-oxo-3,6,9,12,15,18,21-heptaoxatetracosan-1-yl)amino]carboxylate

The synthetic route of step 1 of Example 2-2 was applied, 1-(4-aminobutyl)-2-butyl-7-methyl-1*H*-imidazo[4,5-*d*]thieno[3,2-*b*]pyridine-4-amine was replaced with 2-butyl-7-(hexahydro pyridin-4-yl)-1-(3,4,5,6-tetrahydro-2*H*-pyran-4-ylmethyl)thieno[3,2-*b*]imidazo[4,5-*d*]pyridine-4-amine, 4-(hydroxymethyl)cyclohexyl-1-carboxylic acid was replaced with 3-[(2,2-dimethyl-4-oxo-5-aza-3,8,11,14,17,20,23-heptaoxapentacosan-25-yl)oxy]propanoic acid to obtain the title compound (0.15 g, yield: 72.9%).

MS m/z (ESI): 907.5 [M+H]⁺.

### Step 2: Preparation of 1-{4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}-3-[(20-amino-3,6,9,12,15,18-hexaoxaeicosan-1 -yl)oxyl]propan-1-one

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl [(24-{4-[4-amino-2-butyl-1-(3,4,5,6-tetrahydro-2*H*-pyran-4-ylmethyl) thieno[3,2-*b*]imidazo[4,5-*d*]pyridin-7-yl]hexahydropyridin-1-yl}-24-oxo-3,6,9,12,15,18,21-heptaoxatetracosan-1-yl)amino]carboxylate to obtain the title compound (0.12 g, yield: 86.5%).

MS m/z (ESI): 807.5 [M+H]⁺.

### Step 3: Preparation of N-(24-{4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}-24-oxo-3,6,9,12,15,18,21-heptaoxatetracosan-1-yl)-6-[2-(methyldioxyylidene-λ6-thio)pyrimidin-5-yl]hex-5-ynylamide

The synthetic route of Example 2-3 was applied, the raw material 2-{[23-(4-{2-butyl-4- (tert-butylamino)-1*H*-thieno[3,2-*b*]imidazo[4, 5-*d*]pyridin-7-yl }hexahydropyridin-1-yl)-3,6,9,12,15,18,21-heptaoxatricosan-1-yl]oxy}ethyl-1-amine of step 6 was replaced with 1-{4-[4-amino- 2-butyl-1-(3,4,5,6-tetrahydro-2*H*-pyran-4-ylmethyl)thieno[3,2-*b*]imidazo[4,5-*d*]pyridin-7-yl]hexahydropyridin-1 -yl} -3 -[(20-amino-3,6,9,12,15,18-hexaoxaeicosan-1 -yl)oxy]propan-1 - one to obtain the title compound (44 mg, yield: 27.1%).

MS m/z (ESI): 1057.5 [M+H]⁺.

¹H NMR (400 MHz, CD₃OD): δ 8.95 (s, 2H), 7.10 (s, 1H), 4.69-4.67 (m, 1H), 4.24-4.14 (m, 3H), 3.95-3.91 (m, 2H), 3.80-3.73 (m, 2H), 3.65-3.48 (m, 27H), 3.38-3.32 (m, 6H), 3.29-3.21 (m, 2H), 2.97-2.92 (m, 2H), 2.81-2.77 (m, 2H), 2.66-2.55 (m, 3H), 2.39 (t, *J =* 7.6 Hz, 2H), 2.20-2.10 (m, 3H), 1.97-1.85 (m, 4H), 1.82-1.60 (m, 2H), 1.59-1.46 (m, 6H), 1.02 (t, *J* = 7.6 Hz, 3H).

### Example 2-12: Preparation of N-[(1-{[4-({4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}carbonyl)cyclohexyl]methyl}-1,2,3-triazacyclopentan-4-yl)methyl]-3-({27-[2-(methyldioxo-λ6-thio)pyrimidin-5-yl]-22-oxo-21-aza-3,6,9,12,15,18-hexaoxaheptacosan-26-yn-1-yl}oxy)propionamide (Compound D-L'-12)

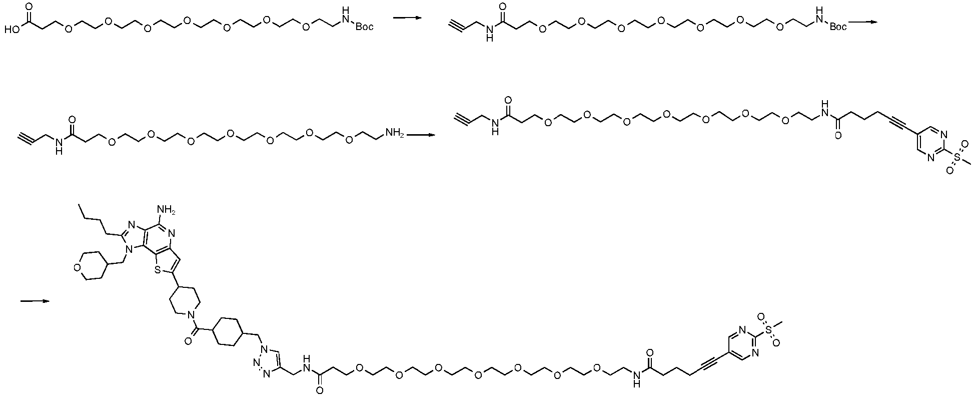

### Step 1: Preparation of tert-butyl [(24-oxo-25-aza-3,6,9,12,15,18,21-heptaoxaoctacosan-27-yn-1-yl)amino]carboxylate

The synthetic route of step 1 of Example 2-2 was applied, 1-(4-aminobutyl)-2-butyl-7-methyl-1*H*-imidazo[4,5-*d*]thieno[3,2-*b*]pyridine-4-amine was replaced with propargylamine, and 4-(hydroxymethyl)cyclohexyl-1-carboxylic acid was replaced with 3-[(2,2-dimethyl-4-oxo- 5-aza-3,8,11,14,17,20,23-heptaoxapentacosan-25-yl)oxy]propionic acid to obtain the title compound (0.16 g, yield: 72.7%).

MS m/z (ESI): 535.3 [M+H]⁺.

### Step 2: Preparation of 3-[(20-amino-3,6,9,12,15,18-hexaoxaeicosan-1-yl)oxy]- N-(prop-2-ynyl)propionamide

The synthetic route of Example 1 was applied, the raw material tert-butyl (4-(4-amino- 2-butyl-7-methyl-1*H-*imidazo[4,5-*d*]thieno[3,2-*b*]pyridin-1-yl)butyl)carbamate of step 9 was replaced with tert-butyl [(24-oxo-25-aza-3,6,9,12,15,18,21-heptaoxaoctacosan- 27-yn-1-yl)amino]carboxylate to obtain the title compound (0.11 g, yield: 84.6%).

MS m/z (ESI): 435.3 [M+H]⁺.

### Step 3: Preparation of 3-({27-[2-(methyldioxo-λ6-thio)pyrimidin-5-yl]-22-oxo-21-aza-3,6,9,12,15,18-hexaoxaheptacosan-26-yn-1-yl}oxy)-N-(prop-2-ynyl)propionamide

The synthetic route of Example 2-3 was applied, the raw material 2-{[23-(4-{2-butyl-4- (tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 3-[(20-amino-3,6,9,12,15,18-hexaoxaeicosan-1-yl)oxy]-*N*-(prop-2-ynyl)propanamide to obtain the title compound (82 mg, yield: 46.2%).

MS m/z (ESI): 685.3 [M+H]⁺.

### Step 4: Preparation of N-[(1-{[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}carbonyl)cyclohexyl]methyl}-1,2,3-triazacyclopentan-4-yl)methyl]-3-({27-[2-(methyldioxo-λ6-thio)pyrimidin-5-yl]-22-oxo-21-aza-3,6,9,12,15,18-hexaoxaheptacosan-26-yn-1-yl}oxy)propionamide

The synthetic route of step 4 of Example 2-2 was applied, *N*-[4-(4-amino-2-butyl-7-methyl thieno[3,2-*b*]imidazo[4,5-*d*]pyridin-1-yl)butyl]-4-(azidomethyl)cyclohexylcarboxamide was replaced with {4-[4-amino-2-butyl-1-(3,4,5,6-tetrahydro-2*H*-pyran-4-ylmethyl)thieno[3,2-*b*] imidazo[4,5-*d*]pyridin-7-yl]hexahydropyridin-1-yl}[4-(azidomethyl)cyclohexyl]methanone, 6-[2-(methyldioxo-λ6-thio)pyrimidin-5-yl]-*N*-(prop-1-ynyl-3-yl)hex-5-ynylamide was replaced with 3-({27-[2-(methyldioxo-λ6-thio)pyrimidin-5-yl]-22-oxo-21-aza-3,6,9,12,15,18-hexaoxaheptacosan-26-yn-1-yl}oxy)-*N*-(prop-2-ynyl)propionamide to obtain the title compound (28 mg, yield: 45.1%).

MS m/z (ESI): 1277.6 [M+H]⁺.

¹H NMR (400 MHz, DMSO-*d₆*): δ 9.12 (s, 2H), 8.35-8.32 (m, 1H), 7.97-7.94 (m, 1H), 7.90 (s, 1H), 7.04 (s, 1H), 6.08 (s, 2H), 4.56-4.53 (m, 1H), 4.37-4.26 (m, 4H), 4.15-4.11 (m, 2H), 4.03-4.01 (m, 1H), 3.85-3.81 (m, 2H), 3.66-3.39 (m, 38H), 3.26-3.15 (m, 4H), 2.92-2.83 (m, 2H), 2.80-2.56 (m, 4H), 2.41-2.22 (m, 4H), 2.10-2.06 (m, 4H), 1.82-1.72 (m, 4H), 1.53- 1.37 (m, 11H), 0.94 (t, *J* = 7.6 Hz, 3H).

### Example 2-13: Preparation of N-{2-[(3-{4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}-3-oxopropyl)oxy]ethyl}-6-[2-(methyldioxo-λ6-thio)pyrimidin-5-yl]hex-5-ynylamide (Compound D-L'-13)

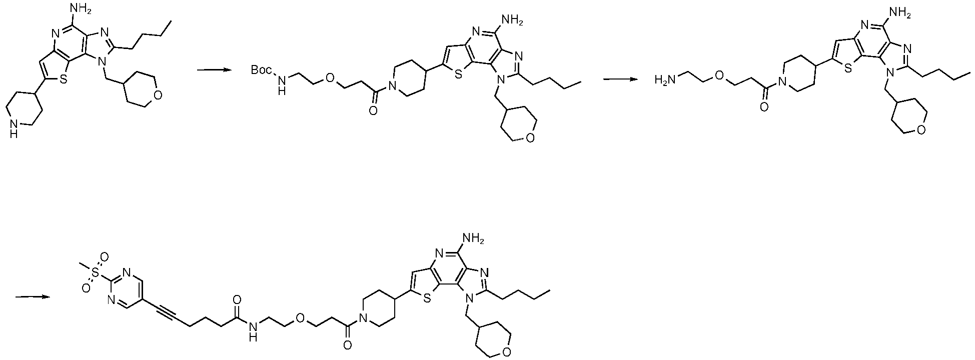

### Step 1: Preparation of tert-butyl ({2-[(3-{4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}-3-oxopropyl)oxy] ethyl } amino)carboxylate

The synthetic route of step 1 of Example 2-2 was applied, 1-(4-aminobutyl)-2-butyl-7-methyl-1*H*- imidazo[4,5-*d*]thieno[3,2-*b*]pyridine-4-amine was replaced with 2-butyl-7-(hexahydropyridin- 4-yl)-1-(3,4,5,6-tetrahydro-2*H*-pyran-4-ylmethyl)thieno[3,2-*b*]imidazo[4,5-*d*]pyridine-4-amine, 4-(hydroxymethyl)cyclohexyl-1-formic acid was replaced with 3-[(2,2-dimethyl-4-oxo-5- aza-3-oxaheptan-7-yl)oxy]propanoic acid to obtain the title compound (0.13 g, yield: 89.1%).

MS m/z (ESI): 643.4 [M+H]⁺.

### Step 2: Preparation of 1-{4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}-3-[(2-aminoethyl)oxy]propan-1-one

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl ({2-[(3-{4-[4-amino-2-butyl-1-(3,4,5,6-tetrahydro-2*H*-pyran-4-yl methyl)thieno[3,2-*b*]imidazo[4,5-*d*]pyridin-7-yl]hexahydropyridin-1-yl}-3-oxopropyl)oxy]ethyl}amino)carboxylate to obtain the title compound (0.12 g, yield: 93.7%).

MS m/z (ESI): 543.3 [M+H]⁺.

### Step 3: Preparation of N-{2-[(3-{4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}-3-oxopropyl)oxy]ethyl}-6-[2-(methyldioxo-λ6-thio)pyrimidin-5-yl]hex-5-ynylamide

The synthetic route of Example 2-3 was applied, the raw material 2-{ [23-(4-{2-butyl-4- (tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{4-[4-amino- 2-butyl-1-(3,4,5,6-tetrahydro-2*H*-pyran-4-ylmethyl)thieno[3,2-*b*]imidazo[4,5-*d*]pyridin-7-yl]hexahydropyridin-1-yl}-3-[(2-aminoethyl)oxy]propan-1-one to obtain the title compound (43 mg, yield: 12.7%).

MS m/z (ESI): 793.3 [M+H]⁺.

¹H NMR (400 MHz, DMSO-*d₆*): δ 9.11 (s, 2H), 8.47 (s, 2H), 7.95-7.91 (m, 1H), 7.26 (s, 1H), 4.56-4.52 (m, 1H), 4.24-4.19 (m, 2H), 4.03-3.99 (m, 1H), 3.87-3.82 (m, 2H), 3.64 (t, *J =* 6.4 Hz, 2H), 3.43-3.39 (m, 6H), 3.34 -3.30 (m, 1H), 3.23-3.17 (m, 4H), 3.16-3.12 (m, 1H), 2.96-2.90 (m, 2H), 2.71-2.59 (m, 3H), 2.54 (t, *J* = 7.2 Hz, 2H), 2.30-2.26 (m, 2H), 2.06 (d, *J* = 7.6 Hz, 3H), 1.86-1.75 (m, 4H), 1.69-1.58 (m, 1H), 1.50-1.40 (m, 6H), 0.95 (t, *J* = 7.2 Hz, 3H).

### Example 2-14: Preparation of N-[(1-{[4-({4-[(4-amino-2-butyl-7-methylthieno[3,2-b] imidazo[4,5-d]pyridin-1 -yl)methyl]hexahydropyridin-1-yl}carbonyl)cyclohexyl]methyl}-1,2,3-triazacyclopent-4-yl)methyl]-6-[2-(methyldioxo-λ6-thio)pyrimidin-5-yl]hex-5-ynylamide (Compound D-L'-14)

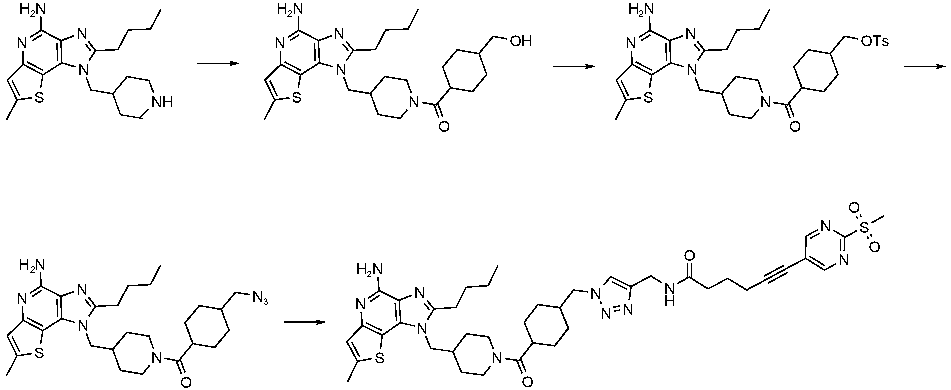

### Step 1: Preparation of {4-[(4-amino-2-butyl-7-methylthieno[3,2-b]imidazo[4,5-d]pyridin- 1-yl)methyl]hexahydropyridin-1-yl} [4-(hydroxymethyl)cyclohexyl]methanone

The synthetic route of step 1 of Example 2-2 was applied, 1-(4-aminobutyl)-2-butyl-7-methyl-1*H*-imidazo[4,5-*d*]thieno[3,2-*b*]pyridine-4-amine was replaced with 2-butyl-7-methyl-1-(piperidin-4-ylmethyl)-1*H*-imidazo[4,5-*d*]thieno[3,2-*b*]pyridine-4-amine to obtain the title compound (0.15 g, yield: 81.3%).

MS m/z (ESI): 498.3 [M+H]⁺.

### Step 2: Preparation of [4-({4-[(4-amino-2-butyl-7-methylthieno[3,2-b]imidazo[4,5-d] pyridin-1-yl)methyl]hexahydropyridin-1-yl} carbonyl)cyclohexyl]methyl 4-methylbenzenesulfonate

The synthetic route of step 2 of Example 2-2 was applied, *N*-[4-(4-amino-2-butyl-7-methyl thieno[3,2-*b*]imidazo[4,5-*d*]pyridin-1-yl)butyl]-4-(hydroxymethyl)cyclohexylcarboxamide was replaced with { 4-[(4-amino-2-butyl-7-methylthieno[3,2-*b*]imidazo[4,5-*d*]pyridin-1-yl)methyl] hexahydropyridin-1-yl}[4-(hydroxymethyl)cyclohexyl]methanone to obtain the title compound (0.1 g, yield: 68.3%).

MS m/z (ESI): 652.3 [M+H]⁺.

### Step 3: Preparation of {4-[(4-amino-2-butyl-7-methylthieno[3,2-b]imidazo[4,5-d]pyridin- 1-yl)methyl]hexahydropyridin-1-yl} [4-(azidomethyl)cyclohexyl]methanone

The synthetic route of step 3 of Example 2-2 was applied, [4-({[4-(4-amino-2-butyl-7-methyl thieno[3,2-*b*]imidazo[4,5-*d*]pyridin-1-yl)butyl]amino}carbonyl)cyclohexyl]methyl 4-methylbenzenesulfonate was replaced with [4-({4-[(4-amino-2-butyl-7-methylthieno[3,2-*b*] imidazo[4,5-*d*]pyridin-1-yl)methyl]hexahydropyridin-1-yl} carbonyl)cyclohexyl]methyl 4-methylbenzenesulfonate to obtain the title compound (94 mg, yield: 91.4%).

MS m/z (ESI): 523.3 [M+H]⁺.

### Step 4: Preparation of N-[(1-{ [4-({4-[(4-amino-2-butyl-7-methylthieno[3,2-b]imidazo[4,5-d] pyridin-1 -yl)methyl]hexahydropyridin-1 -yl} carbonyl)cyclohexyl]methyl} -1,2,3 -triazolane-4-yl)methyl]-6-[2-(methyldioxo-λ6-thio)pyrimidin-5-yl]hex-5-ynylamide

The synthetic route of step 4 of Example 2-2 was applied, *N*-[4-(4-amino-2-butyl-7-methyl thieno[3,2-*b*]imidazo[4,5-*d*]pyridin-1-yl)butyl]-4-(azidomethyl)cyclohexylcarboxamide was replaced with { 4-[(4-amino-2-butyl-7-methylthieno[3,2-*b*]imidazo[4,5-*d*]pyridin-1-yl)methyl] hexahydropyridin-1-yl}[4-(azidomethyl)cyclohexyl]methanone to obtain the title compound (12 mg, yield : 21.4%).

MS m/z (ESI): 828.4 [M+H]⁺.

¹H NMR (400 MHz, DMSO-*d₆*): δ 9.11 (s, 2H), 8.44-8.40 (m, 3H), 7.90 (s, 1H), 7.24 (s, 1H), 4.52-4.16 (m, 7H), 3.94-3.91 (m, 1H), 3.00-2.83 (m, 3H), 2.68-2.50 (m, 6H), 2.41-2.26 (m, 5H), 2.10-2.06 (m, 2H), 1.85-1.23(m, 19H), 0.95 (t, *J* = 7.6 Hz, 3H).

### Example 2-15: Preparation of N-(26-{4-[(4-amino-2-butylthieno[3,2-b]imidazo[4,5-d] pyridin-1-yl)methyl]hexahydropyridin-1-yl}-3,6,9,12,15,18,21,24-octaoxahexacosan-1-yl)-6-[2-(methyldioxo-λ6-thio)pyrimidin-5-yl]hex-5-ynylamide (Compound D-L'-15)

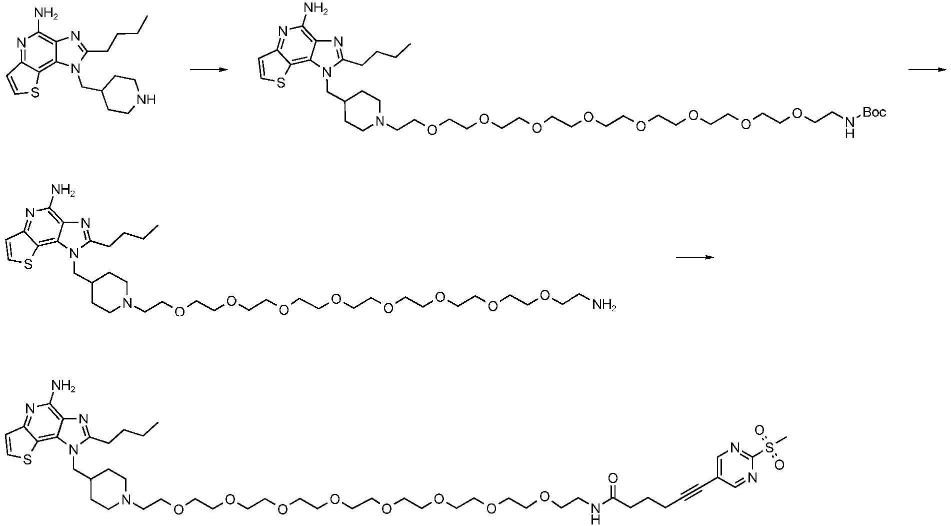

### Step 1: Preparation of tert-butyl [(26-{4-[(4-amino-2-butylthieno[3,2-b]imidazo[4,5-d] pyridin-1-yl)methyl]hexahydropyridin-1-yl}-3,6,9,12,15,18,21,24-octaoxahexacosan-1-yl)amino]carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material 4-[(2-butyl-7-(hexahydropyridin- 4-yl)-4-(tert-butylamino)thieno[3,2-*b*]imidazo[4,5-*d*]pyridin-1-yl)methyl]hexahydropyridine-1-carboxylate was replaced with 2-butyl-1-(piperidin-4-ylmethyl)-1H-imidazo[4,5-*d*]thieno[3,2-*b*] pyridine-4-amine to obtain the title compound (45 mg, yield: 39.6%).

MS m/z (ESI): 839.5 [M+H]⁺.

### Step 2: Preparation of 1-{[1-(26-amino-3,6,9,12,15,18,21,24-octaoxahexacosan-1-yl) hexahydropyridin-4-yl]methyl}-2-butylthieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-d]thieno[3,2-b]pyridin-1-yl)butyl)carbamate of step 9 was replaced with tert-butyl [(26-{4-[(4-amino-2-butylthieno[3,2-*b*]imidazo[4,5-*d*] pyridin-1-yl)methyl]hexahydropyridin-1 -yl} -3,6,9,12,15,18,21,24-octaoxah exacosan-1 - yl)amino]carboxylate to obtain the title compound (33 mg, yield: 84.5%).

MS m/z (ESI): 739.5 [M+H]⁺.

### Step 3: Preparation ofN-(26-{4-[(4-amino-2-butylthieno[3,2-b]imidazo[4,5-d]pyridin-1-yl) methyl]hexahydropyridin-1 -yl} -3,6,9,12,15,18,21,24-octaoxah exacosan-1 -yl)-6-[2-(methyldioxo-λ6-thio)pyrimidin-5-yl]hex-5-ynylamide

The synthetic route of Example 2-3 was applied, the reaction material 2-{[23-(4-{2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-butylthieno[3,2-*b*]imidazo[4,5-*d*]pyridine-4-amine to obtain the title compound (12 mg, yield: 27.3%).

MS m/z (ESI): 989.5 [M+H]⁺.

¹H NMR (400 MHz, CD₃OD) δ: 8.96 (s, 2H), 8.02 (d, *J=* 5.6 Hz, 1H), 7.49 (d, *J =* 5.6 Hz, 1H), 4.39 (d, *J=* 7.6 Hz, 2H), 3.83-3.80 (m, 2H), 3.73-3.46 (m,30H), 3.38-3.32 (m, 8H), 2.99 (t, *J* = 7.6 Hz, 4H), 2.59 (t, *J=* 7.2 Hz, 2H), 2.40 (t, *J=* 7.2 Hz, 3H), 2.02- 1.74 (m, 8H), 1.61-1.48 (m, 3H), 1.04 (t, *J* = 7.6 Hz, 3H).

### Example 2-16: Preparation of N-[26-(4-{[4-amino-2-(2-methoxyethyl)thieno[3,2-b] imidazo[4,5-d]pyridine-1-yl]methyl}hexahydropyridin-1-yl)-3,6,9,12,15,18,21,24-octaoxahexacosan-1-yl]-6-[2-(methyldioxo-λ6-thio)pyrimidin-5-yl]hex-5-ynylamide (Compound D-L'-16)

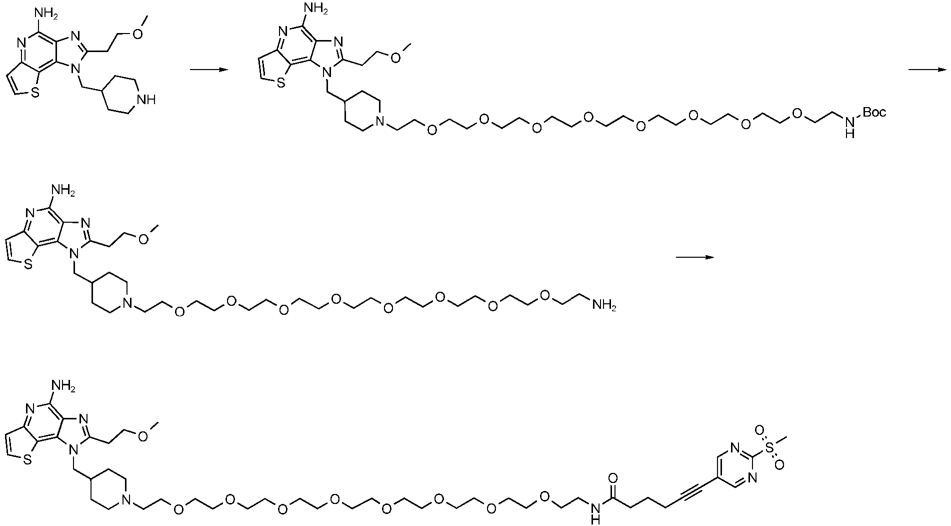

### Step 1: Preparation of tert-butyl {[26-(4-{[4-amino-2-(2-methoxyethyl)thieno[3,2-b] imidazo[4,5-d]pyridin-1-yl]methyl}hexahydropyridin-1-yl)-3,6,9,12,15,18,21,24-octaoxahexacosan-1-yl]amino } carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material 4-[(2-butyl-7-(hexahydropyridin- 4-yl)-4-(tert-butylamino)thieno[3,2-*b*]imidazo[4,5-*d*]pyridin-1-yl)methyl]hexahydropyridine-1-carboxylate was replaced with 1-(hexahydropyridin-4-ylmethyl)-2-(2-methoxyethyl)thieno[3, 2-*b*]imidazo[4,5-*d*]pyridine-4-amine to obtain the title compound (52 mg, yield: 45.2%).

MS m/z (ESI): 841.5 [M+H]⁺.

### Step 2: Preparation of 1-{[1-(26-amino-3,6,9,12,15,18,21,24-octaoxahexacosan-1-yl) hexahydropyridin-4-yl]methyl }-2-(2-methoxyethyl)thieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl {[26-(4-{[4-amino-2-(2-methoxyethyl)thieno[3,2-*b*] imidazo[4,5-*d*]pyridin-1-yl]methyl}hexahydropyridin-1-yl)-3,6,9,12,15,18,21,24-octaoxahexacosan-1-yl]amino}carboxylate to obtain the title compound (39 mg, yield: 86.5%).

MS m/z (ESI): 741.5 [M+H]⁺.

### Step 3: Preparation of N-[26-(4-{[4-amino-2-(2-methoxyethyl)thieno[3,2-b]imidazo[4,5-d] pyridin-1-yl]methyl}hexahydropyridin-1-yl)-3,6,9,12,15,18,21,24-octaoxahexacosan-1-yl]-6-[2-(methyldioxo-λ6-thio)pyrimidin-5-yl]hex-5-ynylamide

The synthetic route of Example 2-3 was applied, the reaction material 2-{[23-(4-{2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-(2-methoxyethyl)thieno[3,2-*b*]imidazo[4,5-*d*]pyridine-4-amine to obtain the title compound (20 mg, yield: 38.5%).

MS m/z (ESI): 991.5 [M+H]⁺.

¹H NMR (400 MHz, CD₃OD): δ 8.95 (s, 2H), 7.68 (d, *J =* 5.6 Hz, 1H), 7.30 (d, *J =* 5.6 Hz, 1H), 4.36-4.32 (m, 2H), 3.94-3.89 (m, 2H), 3.73-3.68 (m, 2H), 3.65-3.48 (m, 32*H*), 3.42-3.35 (m, 8H), 3.23 (t, *J=* 6.4 Hz, 2H), 2.94 (s, 2H), 2.62-2.35 (m, 6H), 2.24-2.10 (m, 1H), 1.95-1.90 (m, 2H), 1.81-1.57 (m, 4H).

### Example 2-17: Preparation of N-[26-(4-{[4-amino-2-butyl-7-(1-methylhexahydropyridin-4-yl)thieno[3,2-b]imidazo[4,5-d]pyridin-1-yl]methyl}hexahydropyridin-1-yl)-3,6,9,12,15,18,21,24-octaoxahexacosan-1-yl]-6-[2-(methyldioxo-λ6-thio)pyrimidin-5-yl]hex-5-ynylamide (Compound D-L'-17)

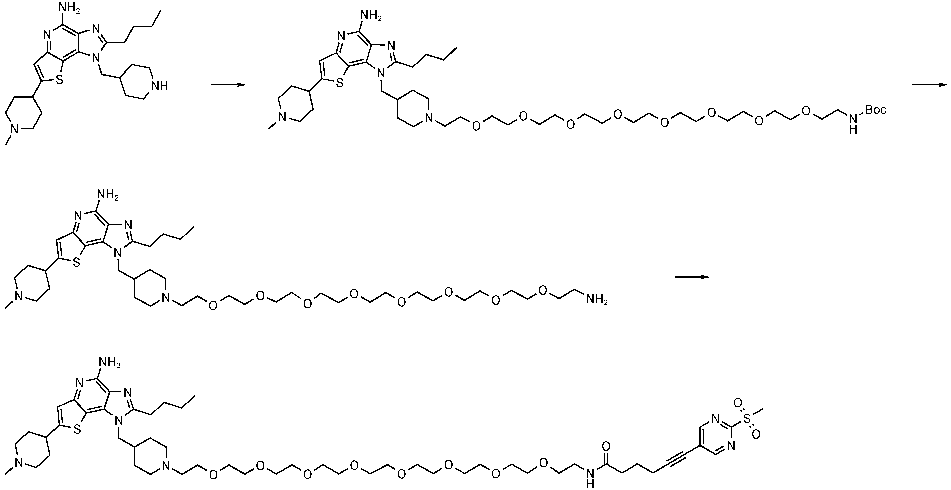

### Step 1: Preparation of tert-butyl {[26-(4-{[4-amino-2-butyl-7-(1-methylhexahydro pyridin-4-yl)thieno[3,2-b]imidazo[4,5-d]pyridin-1-yl]methyl}hexahydropyridin-1-yl)-3,6,9,12,15,18,21,24-octaoxahexacosan-1-yl]amino}carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material 4-[(2-butyl-7-(hexahydropyridin-4-yl)-4-(tert-butylamino)thieno[3,2-*b*]imidazo[4,5-*d*]pyridin-1-yl)methyl]hexahydropyridine-1-carboxylate was replaced with 2-butyl-1-(hexahydropyridin-4-ylmethyl)-7-(1-methylhexahydro pyridin-4-yl)thieno[3,2-*b*]imidazo[4,5-*d*]pyridine-4-amine to obtain the title compound (52 mg, yield: 54.3%).

MS m/z (ESI): 936.6 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-(1-methylhexahydropyridin-4-yl)thieno[3,2-b]imidazo[4,5-d]pyridine-4-amine

The synthetic route of Example 1 was applied, the raw material tert-butyl (4-(4-amino- 2-butyl-7-methyl-1*H*-imidazo[4,5-*d*]thieno[3,2-*b*]pyridin-1-yl)butyl)carbamate of step 9 was replaced with tert-butyl {[26-(4-{[4-amino-2-butyl-7-(1-methylhexahydropyridin-4-yl) thieno[3,2-*b*]imidazo[4,5-*d*]pyridin-1-yl]methyl}hexahydropyridin-1-yl)-3,6,9,12,15,18,21,24-octaoxahexacosan-1-yl]amino}carboxylate to obtain the title compound (39 mg, yield: 84.5%).

MS m/z (ESI): 836.5 [M+H]⁺.

### Step 3: Preparation of N-[26-(4-{[4-amino-2-butyl-7-(1-methylhexahydropyridin-4-yl) thieno[3,2-b]imidazo[4,5-d]pyridin-1-yl]methyl}hexahydropyridin-1-yl)-3,6,9,12,15,18,21,24-octaoxahexacosan-1-yl]-6-[2-(methyldioxo-λ6-thio)pyrimidin-5-yl]hex-5-ynylamide

The synthetic route of Example 2-3 was applied, the raw material 2-{[23-(4-{2-butyl-4- (tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26-amino-3,6,9,12,15,18,21,24-octaoxahexacosan-1-yl)hexahydropyridin-4-yl]methyl}-2-butyl-7-(1-methylhexahydropyridin-4-yl)thieno[3,2-*b*]imidazo[4,5-*d*]pyridine-4-amine to obtain the title compound (23 mg, yield: 46.1%).

MS m/z (ESI): 1086.6 [M+H]⁺.

¹H NMR (400 MHz, CD₃OD): δ 8.95 (s, 2H), 7.11 (s, 1H), 4.28 (d, *J* = 7.6 Hz, 2H), 3.75-3.71 (m, 2H), 3.65-3.49 (m, 30H), 3.43-3.33 (m, 8H), 3.18-3.15 (m, 1H), 3.05-2.91 (m, 4H), 2.80-2.75 (m, 2H), 2.68 (s, 3H), 2.62-2.52 (m, 4H), 2.39 (t, *J* = 7.6 Hz, 2H), 2.31-2.11 (m, 4H), 2.05-1.86 (m, 6H), 1.79-1.65 (m 4*H*), 1.55-1.48 (m, 2H), 1.03 (t, *J*= 7.6 Hz, 3H).

### Example 2-18: Preparation of N-{26-[4-({4-amino-2-butyl-7-[1-(2,5,8-trioxadecan-10-yl) hexahydropyridin-4-yl]thieno[3,2-b]imidazo[4,5-d]pyridin-1-yl}methyl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide (Compound D-L'-18)

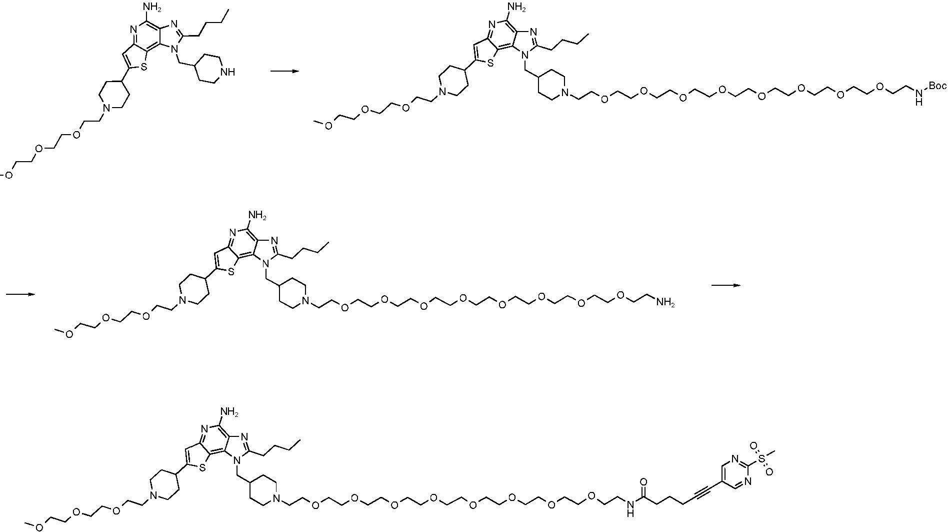

### Step 1: Preparation of tert-butyl ({26-[4-({4-amino-2-butyl-7-[1-(2,5,8-trioxadecan-10-yl) hexahydropyridin-4-yl]thieno[3,2-b]imidazo[4,5-d]pyridin-1-yl}methyl)hexahydropyridin-1-yl]-3,6,9,12,15,18,21,24-octaoxahexacosan-1-yl}amino)carboxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material tert-butyl 4-[(2-butyl-7-(hexahydro pyridin-4-yl)-4-(tert-butylamino)thieno[3,2-*b*]imidazo[4,5-*d*]pyridin-1-yl)methyl]hexahydropyridine-1-carboxylate was replaced with 2-butyl-1-(hexahydropyridin-4-ylmethyl)-7-[1-(2,5,8- trioxadecan-10-yl)hexahydropyridin-4-yl]thieno[3,2-*b*]imidazo[4,5-*d*]pyridine-4-amine to obtain the title compound (28 mg, yield: 61.3%).

MS m/z (ESI): 1068.7 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-[1-(2,5,8-trioxadecan-10-yl)hexahydropyridin-4-yl]thieno[3,2-b]imidazo[4,5-d]pyridine-4-amine

The synthetic route of Example 1 was applied, the raw material tert-butyl (4-(4-amino- 2-butyl-7-methyl-1*H*-imidazo[4,5-*d*]thieno[3,2-*b*]pyridin-1-yl)butyl)carbamate of step 9 was replaced with tert-butyl ({26-[4-({4-amino-2-butyl-7-[1-(2,5,8-trioxadecan-10-yl)hexahydro pyridin-4-yl]thieno[3,2-*b*]imidazo[4,5-*d*]pyridin-1-yl}methyl)hexahydropyridin-1-yl]-3,6,9,12, 15,18,21,24-octoxahexacosan-1-yl}amino)carboxylate to obtain the title compound (22 mg, yield: 86.5%).

MS m/z (ESI): 968.7 [M+H]⁺.

### Step 3: Preparation of N-{26-[4-({4-amino-2-butyl-7-[1-(2,5,8-trioxadecan-10-yl) hexahydropyridine-4-yl]thieno[3,2-b]imidazo[4,5-d]pyridin-1-yl}methyl)hexahydropyridin-1-yl]-3,6,9,12,15,18,21,24-octaoxahexacosan-1-yl}-6-[2-(methyldioxo-λ6-thio)pyrimidin-5-yl]hex-5-ynylamide

The synthetic route of Example 2-3 was applied, the raw material 2-{[23-(4-{2-butyl-4- (tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26-amino-3,6,9,12,15,18,21,24-octaoxahexacosan-1-yl)hexahydropyridin-4-yl]methyl}-2-butyl-7-[1-(2,5,8-trioxadecan-10-yl)hexahydropyridin-4-yl]thieno[3,2-*b*]imidazo[4,5-*d*]pyridine-4-amine to obtain the title compound (15 mg, yield: 53.5%).

MS m/z (ESI): 1218.7 [M+H]⁺.

¹H NMR (400 MHz, CD₃OD): δ 8.96 (s, 2H), 7.06 (s,1H), 4.21 (d, *J* = 7.6 Hz, 2H), 3.73-3.50 (m, 43H), 3.40-3.33 (m, 8H), 3.20-3.16 (m, 2H), 3.10-2.90 (m, 5H), 2.75-2.70 (m, 2H), 2.65-2.55 (m, 4H), 2.42-2.32 (m, 4H), 2.15-1.85 (m, 10H), 1.65-1.45 (m, 6H), 1.02 (t, *J* = 7.6 Hz, 3H).

### Example 2-19: Preparation of N-{26-[4-({4-amino-2-butyl-7-[1-(2,5,8,11,14-pentaoxahexadecan-16-yl)hexahydropyridin-4-yl]thieno[3,2-b]imidazo[4,5-d]pyridin-1-yl }methyl)hexahydropyridin-1-yl]-3,6,9, 12, 15, 18,21,24-octoxahexacosan-1-yl }-6-[2-(methyldioxo-λ6-thio)pyrimidin-5-yl]hex-5-ynylamide (Compound D-L'-19)

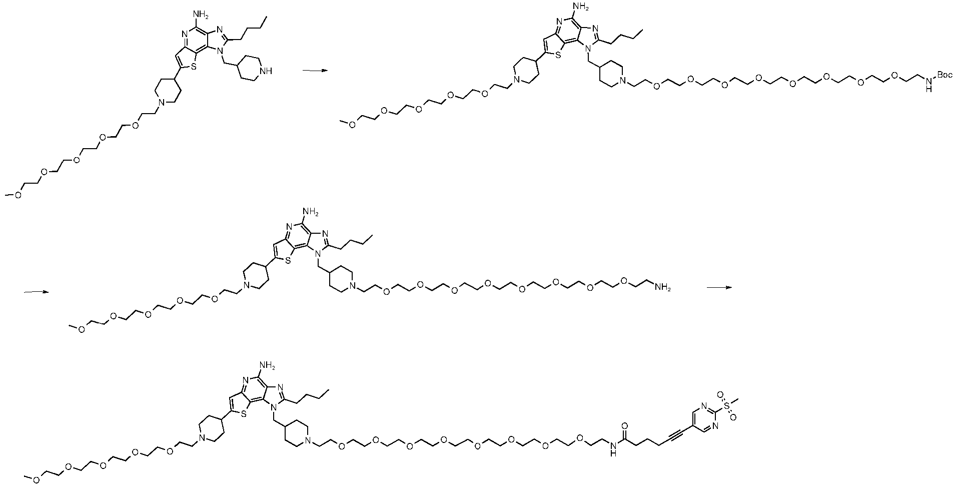

### Step 1: Preparation of tert-butyl ({26-[4-({4-amino-2-butyl-7-[1-(2,5,8,11,14-pentaoxa hexadecan-16-yl)hexahydropyridin-4-yl]thieno[3,2-b]imidazo[4,5-d]pyridin-1-yl}methyl)hexahydropyridin-1-yl]-3,6,9,12,15,18,21,24-octaoxahexacosan-1-yl } amino)carb oxylate

The synthetic route of Example 24 was applied, the raw material triethylene glycol monomethyl ether of step 3 was replaced with tert-butyl [(26-hydroxy-3,6,9,12,15,18,21,24-octaoxahexacosan-1-yl)amino]carboxylate, the raw material 4-[(2-butyl-7-(hexahydro pyridin-4-yl)-4-(tert-butylamino)thieno[3,2-*b*]imidazo[4,5-*d*]pyridin-1-yl)methyl]hexahydropyridine-1-carboxylate was replaced with 2-butyl-1-(hexahydropyridin-4-ylmethyl)-7-[1-(2,5,8, 11,14-pentaoxahexadecan-16-yl)hexahydropyridin-4-yl]thieno[3,2-*b*]imidazo[4,5-*d*]pyridine-4- amine to obtain the title compound (45 mg, yield: 56.3%).

MS m/z (ESI): 1156.8 [M+H]⁺.

### Step 2: Preparation of 1-{[1-(26-amino-3,6,9,12,15,18,21,24-octaoxahexacosan-1-yl) hexahydropyridin-4-yl]methyl}-2-butyl-7-[1-(2,5,8,11,14-pentaoxahexadecan-16-yl)hexahydro pyridin-4-yl]thieno[3,2-b]imidazo[4,5-d]pyridine-4-amine

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl ({26-[4-({4-amino-2-butyl-7-[1-(2,5,8,11,14-pentaoxa hexadecan-16-yl)hexahydropyridin-4-yl]thieno[3,2-*b*]imidazo[4,5-*d*]pyridin-1-yl}methyl)hexahydropyridin-1-yl]-3,6,9,12,15,18,21,24-octaoxahexacosan-1-yl}amino)carboxylate to obtain the title compound (35 mg, yield: 85.3%).

MS m/z (ESI): 1056.7 [M+H]⁺.

### Step 3: Preparation of N-{26-[4-({4-amino-2-butyl-7-[1-(2,5,8,11,14-pentaoxa hexadecan-16-yl)hexahydropyridin-4-yl]thieno[3,2-b]imidazo[4,5-d]pyridin-1-yl}methyl)hexa hydropyridin-1-yl]-3,6,9,12,15,18,21,24-octaoxahexacosan-1-yl}-6-[2-(methyldioxo-λ6-thio) pyrimidin-5-yl]hex-5-ynylamide

The synthetic route of Example 2-3 was applied, the raw material 2-{[23-(4-{2-butyl-4- (tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with 1-{[1-(26- amino-3,6,9,12,15,18,21,24-octaoxahexacosan-1-yl)hexahydropyridin-4-yl]methyl}-2-butyl-7-[1-(2,5,8,11,14-pentaoxahexadecan-16-yl)hexahydropyridin-4-yl]thieno[3,2-*b*]imidazo[4,5-*d*]pyridine-4-amine to obtain the title compound (22 mg, yield: 51.2%).

MS m/z (ESI): 1306.7 [M+H]⁺.

¹H NMR (400 MHz, CD₃OD): δ 8.96 (s, 2H), 7.07 (s, 1H), 4.23 (d, *J* = 7.6 Hz, 2H), 3.73-3.69 (m, 2H), 3.67-3.48 (m, 40H), 3.38-3.32 (m, 10H), 3.23-3.20 (m, 2H), 3.15-2.99 (m, 4H), 2.98-2.92 (m, 2H), 2.72-2.62 (m, 4H), 2.58 (t, *J* = 7.2 Hz, 2H), 2.49-2.35 (m, 4H), 2.22-1.84 (m, 12H), 1.69-1.46 (m, 7H), 1.32-1.15(m, 3H), 1.02 (t, *J* = 7.6 Hz, 3H).

### Example 2-20: Preparation of N-[2-({[4-({4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}carbonyl)cyclohexyl]methyl}oxy)ethyl]-6-[2-(methyldioxo-λ6-thio)pyrimidin-5-yl]hex-5-ynylamide (Compound D-L'-20)

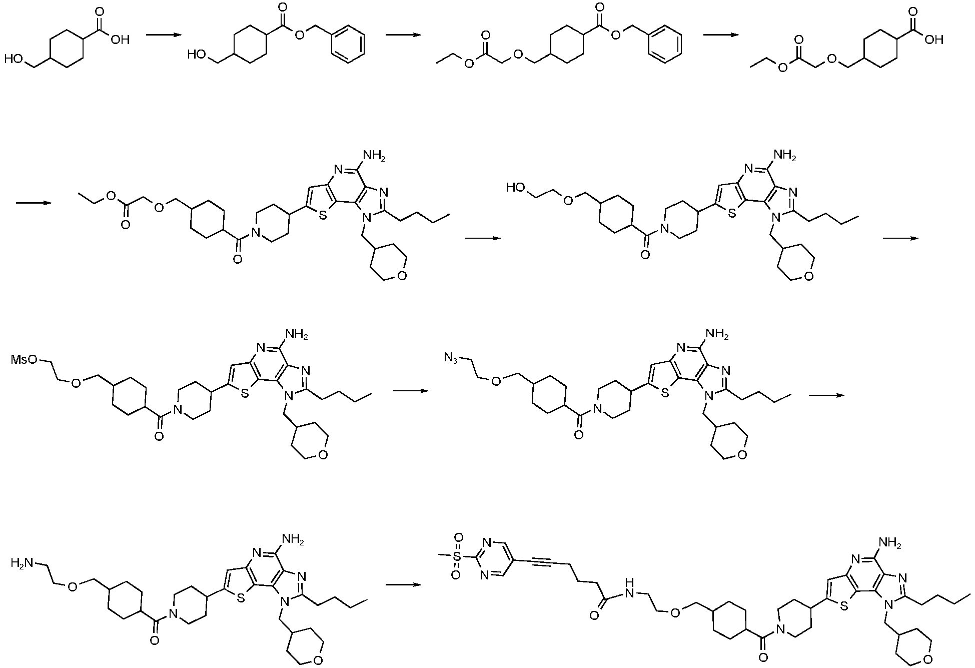

### Step 1: Preparation of benzyl 4-(hydroxymethyl)cyclohexane-1-carboxylate

4-(Hydroxymethyl)cyclohexane-1-carboxylic acid (3 g, 19.0 mmol), potassium carbonate (5.25 g, 38.0 mmol) and tetrabutylammonium iodide (70.0 mg, 0.19 mmol) were sequentially added to *N,N*-dimethylformamide (20 mL), cooled in an ice-water bath, bromotoluene (3.9 g, 22.8 mmol) was slowly added to the reaction solution at 0°C, slowly heated to room temperature and stirred for 24 hours. The reaction solution was poured into water (150 mL), extracted three times with ethyl acetate (75 mL), the organic phases were combined, washed three times with saturated aqueous sodium bicarbonate (30 mL), washed three times with saturated brine (30 mL), and the organic phase was dried over anhydrous sodium sulfate, and concentrated, and the resulting residue was purified by silica gel column chromatography (eluent: ethyl acetate:petroleum ether=5: 1, volume ratio) to obtain the title compound (4.3 g, yield: 91.7%).

MS m/z (ESI): 249.2 [M+H]⁺.

### Step 2: Preparation of benzyl 4-(4-oxo-2,5-dioxahept-1-yl)cyclohexane-1-carboxylate

Benzyl 4-(hydroxymethyl)cyclohexane-1-carboxylate (1 g, 4.03 mmol) and rhodium dipolyacetate (89.0 mg, 0.2 mmol) were sequentially added to dichloromethane (20 mL), cooled in an ice-water bath, ethyl diazoacetate (690.0 mg, 6.04 mmol) was slowly added dropwise to the reaction solution at 0°C, the reaction system was bubbled with nitrogen for 5 minutes, and reacted at room temperature for 18 hours. The reaction solution was poured into water (60 mL), extracted three times with ethyl acetate (30 mL), the organic phases were combined, washed twice with saturated brine (15 mL), the organic phase was dried over anhydrous sodium sulfate and concentrated, and the resulting residue was purified by silica gel column chromatography (eluent: ethyl acetate:petroleum ether=5:1, volume ratio) to obtain the title compound (475.7 mg, yield: 35.3%).

MS m/z (ESI): 335.2 [M+H]⁺.

### Step 3: Preparation of 4-(4-oxo-2,5-dioxahept-1-yl)cyclohexane-1-carboxylic acid

Benzyl 4-(4-oxo-2,5-dioxahept-1-yl)cyclohexane-1-carboxylate (300 mg, 0.90 mmol) was dissolved in methanol (3 mL), added with 10% palladium on carbon (15.0 mg, 0.09 mmol), and subjected to replacement with hydrogen three times, the reaction system was stirred at room temperature for 2 hours, and subjected to suction filtration, the filter cake was washed with methanol (3 mL), and the filtrate was concentrated to obtain the title compound (190.0 mg, yield: 86.3%).

MS m/z (ESI): 245.2 [M+H]⁺.

### Step 4: Preparation of ethyl ({[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}carbonyl)cyclohexyl]methyl}oxy)acetate

The synthetic route of Example 2-2 was applied, the raw material 1-(4-aminobutyl)- 2-butyl-7-methyl-1*H*-imidazo[4,5-*d*]thieno[3,2-*b*]pyridine-4-amine of step 1 was replaced with 2-butyl-7-(hexahydropyridin-4-yl)-1-(3,4,5,6-tetrahydro-2*H*-pyran-4-ylmethyl)thieno[3,2-*b*] imidazo[4,5-*d*]pyridine-4-amine, and 4-(hydroxymethyl)cyclohexyl-1-carboxylic acid was replaced with 4-(4-oxo-2,5-dioxahept-1-yl)cyclohexane-1-carboxylic acid to obtain the title compound (277 mg, yield: 98.3%).

MS m/z (ESI): 654.4 [M+H]⁺.

### Step 5: Preparation of {4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}(4-{[(2-hydroxyethyl)oxy]methyl}cyclohexyl)methanone

Ethyl ({[4-({4-[4-amino-2-butyl-1-(3,4,5,6-tetrahydro-2*H*-pyran-4-ylmethyl)thieno[3,2-*b*] imidazo[4,5-*d*]pyridin-7-yl]hexahydropyridin-1-yl}carbonyl)cyclohexyl]methyl}oxy)acetate (270 mg, 0.41 mmol) was dissolved in a mixed solvent of tetrahydrofuran (4 mL) and absolute ethanol (6 mL), calcium chloride (64.4 mg, 0.58 mmol) and sodium borohydride (65.4 mg, 1.73 mmol) were added at room temperature, heated to 60°C and stirred for 8 hours. The reaction solution was poured into water (60 mL), extracted three times with dichloromethane (45 mL), the organic phases were combined, washed three times with saturated brine (15 mL), the organic phase was dried over anhydrous sodium sulfate, concentrated, and the obtained residue was purified by silica gel column chromatography (eluent: dichloromethane:methanol=8:1, volume ratio) to obtain the title compound (150 mg, yield: 59.8%).

MS m/z (ESI): 612.4 [M+H]⁺.

### Step 6: Preparation of 2-({[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl} carbonyl)cyclohexyl]methyl} oxy)ethyl methanesulfonate

The synthetic route of Example 2-2 was applied, the raw material *N*-[4-(4-amino- 2-butyl-7-methylthieno[3,2-*b*]imidazo[4,5-*d*]pyridin-1-yl)butyl]-4-(hydroxymethyl)cyclohexyl carboxamide of step 2 was replaced with {4-[4-amino-2-butyl-1-(3,4,5,6-tetrahydro-2*H*-pyran- 4-ylmethyl)thieno[3,2-*b*]imidazo[4,5-*d*]pyridin-7-yl]hexahydropyridin-1-yl}(4-{[(2-hydroxyethyl)oxy]methyl}cyclohexyl)methanone, and p-toluenesulfonyl chloride was replaced with methanesulfonyl chloride to obtain the title compound (140 mg, yield: 82.8%).

MS m/z (ESI): 690.3 [M+H]⁺.

### Step 7: Preparation of {4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl}(4-{[(2-azidoethyl)oxy]methyl}cyclohexyl)methanone

The synthetic route of embodiment 2-2 was applied, the raw material [4-({ [4-(4-amino-2-butyl-7-methylthieno[3,2-*b*]imidazo[4,5-*d*]pyridine-1-yl)butyl]amino}carbonyl)cyclohexyl]methyl 4-methylbenzenesulfonate of step 3 was replaced with 2-({[4-({4- [4-amino-2-butyl-1-(3,4,5,6-tetrahydro-2*H*-pyran-4-ylmethyl)thieno[3,2-*b*]imidazo[4,5-*d*]pyridine-7-yl]hexahydropyridin-1-yl}carbonyl)cyclohexyl]methyl}oxy)ethyl methanesulfonate to obtain the title compound (119 mg, yield: 92.2%).

MS m/z (ESI): 637.4 [M+H]⁺.

### Step 8: Preparation of {4-[4-amino-2-butyl-1-(3,4,5,6-tetrahydro-2H-pyran-4-ylmethyl) thieno[3,2-b]imidazo[4,5-d]pyridine-7-yl]hexahydropyridin-1-yl}(4-{[(2-aminoethyl)oxyl]methyl}cyclohexyl)methanone

{4-[4-Amino-2-butyl-1-(3,4,5,6-tetrahydro-2*H*-pyran-4-ylmethyl)thieno[3,2-*b*]imidazo[4,5-*d*]pyridine-7-yl]hexahydropyridin-1-yl}(4-{[(2-azidoethyl)oxyl]methyl}cyclohexyl)methanone (119 mg, 0.19 mmol) was dissolved in a mixed solvent of tetrahydrofuran (3 mL) and ammonia in water (14 wt.% in H₂O, 2 mL), triphenylphosphine (62.9 mg, 0.24 mmol) was added at room temperature, heated to 50°C and stirred for 6 hours. After being cooled to room temperature, the reaction system was concentrated to remove the solvent, and the obtained residue was purified by silica gel column chromatography (eluent: dichloromethane:methanol=9:1, volume ratio) to obtain the title compound (101.8 mg, yield: 87.7%).

MS m/z (ESI): 611.4 [M+H]⁺.

### Step 9: Preparation of N-[2-({[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}carbonyl)cyclohexyl]methyl}oxy)ethyl]-6-[2-(methyldioxo-λ6-thio)pyrimidin-5-yl]hex-5-ynylamide

The synthetic route of Example 2-3 was applied, the reaction material 2-{[23-(4-{2-butyl- 4-(tert-butylamino)-1*H*-thieno[3,2-*b*]imidazo[4,5-*d*]pyridin-7-yl}hexahydropyridin-1-yl)-3,6,9, 12,15,18,21-heptaoxatricosan-1-yl]oxy}ethyl-1-amine of step 6 was replaced with {4-[4-amino-2-butyl-1-(3,4,5,6-tetrahydro-2*H*-pyran-4-ylmethyl)thieno[3,2-*b*]imidazo[4,5-*d*]pyridin-7-yl]hexahydropyridin-1-yl}(4-{[(2-aminoethyl)oxy]methyl}cyclohexyl)methanone to obtain the title compound (20 mg, yield: 44.9%).

MS m/z (ESI): 861.4 [M+H]⁺.

¹H NMR (400 MHz, DMSO-*d₆*): δ 9.11 (s, 2H), 7.95-7.93 (m, 1H), 7.03 (s, 1H), 6.07 (s, 2H), 4.50 (s, 1H), 4.13 (d, *J* = 7.6 Hz, 2H), 3.99 (s, 1H), 3.84-3.82 (m, 2H), 3.41 (s, 3H), 3.40-3.38 (m, 2H), 3.24-3.15 (m, 5H), 2.88-2.82 (m, 2H), 2.69-2.65 (m, 3H), 2.56 (d, *J* = 7.2 Hz, 2H), 2.33 (s, 1H), 2.26 (t, *J* = 7.2 Hz, 2H), 2.05 (s, 4*H*), 1.84-1.74 (m, 4H), 1.63-1.38 (m, 16*H*), 1.23 (s, 1H), 0.94 (t, *J* = 7.2 Hz, 3H).

### Example 2-21: Preparation of N-{1-[4-({4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl } carbonyl)cyclohexyl]-2, 5, 8,11,14,17,20,23,26-nonaoxaoctacosan-28-yl }-6-[2-(methyldioxo-λ6-thio)pyrimidin-5-yl]hex-5-ynylamide (Compound D-L'-21)

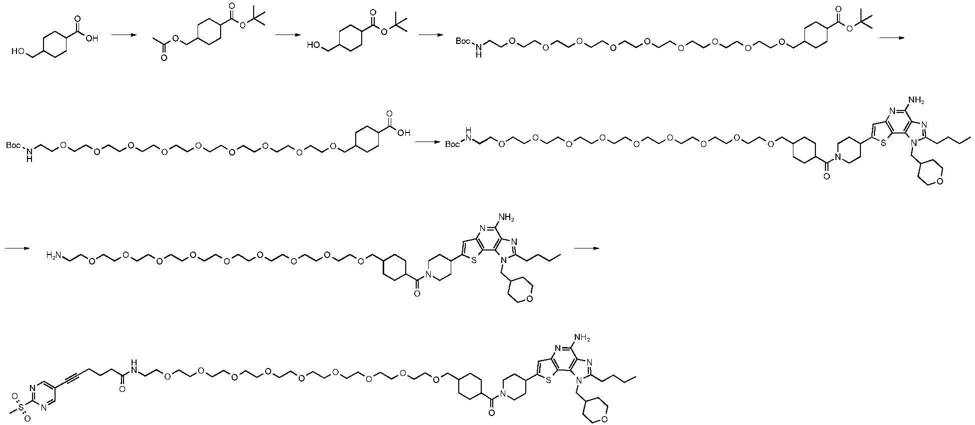

### Step 1: Preparation of tert-butyl 4-(acetoxymethyl)cyclohexane-1-carboxylate

4-(Hydroxymethyl)cyclohexane-1-carboxylic acid (2 g, 12.6 mmol) was added to tert-butyl acetate (30 mL), cooled in an ice-water bath, and perchloric acid (0.13 g, 1.26 mmol) was slowly added to the reaction solution at 0°C, slowly warmed to room temperature and stirred for 12 hours. The reaction solution was poured into water (100 mL), extracted three times with dichloromethane (60 mL), the organic phases were combined, washed twice with saturated aqueous sodium bicarbonate (30 mL), and washed three times with saturated brine (45 mL), and the organic phase was dried over anhydrous sodium sulfate and concentrated to obtain the title compound (3 g, yield: 92.9%).

MS m/z (ESI): 257.2 [M+H]⁺.

### Step 2: Preparation of tert-butyl 4-(hydroxymethyl)cyclohexane-1-carboxylate

Tert-butyl 4-(acetoxymethyl)cyclohexane-1-carboxylate (3 g, 11.7 mmol) was dissolved in a mixed solvent of methanol (30 mL) and water (6 mL), cooled in an ice-water bath, lithium hydroxide (1.4 g, 58.5 mmol) was slowly added to the reaction solution at 0°C, slowly warmed to room temperature and stirred for 3 hours. The reaction solution was poured into water (100 mL), extracted three times with ethyl acetate (75 mL), the organic phases were combined, washed three times with saturated brine (30 mL), the organic phase was dried over anhydrous sodium sulfate and concentrated, the resulting residue was purified by silica gel column chromatography (eluent: ethyl acetate:petroleum ether=1:5, volume ratio) to obtain the title compound (1.2 g, yield: 47.9%).

MS m/z (ESI): 215.2 [M+H]⁺.

### Step 3: Preparation of tert-butyl 4-(32,32-dimethyl-30-oxo-29-aza-2,5,8,11,14,17,20,23, 26,31-decaoxatritriacontan-1-yl)cyclohexane-1-carboxylate

Tert-butyl 4-(hydroxymethyl)cyclohexane-1-carboxylate (0.22 g, 1.03 mmol) was dissolved in tetrahydrofuran (6 mL), cooled in an ice-water bath, and potassium tert-butoxide (0.12 g, 1.03 mmol) was added to the reaction solution at 0°C; after being stirred for 20 minutes, the reaction solution was slowly added dropwise with a solution of tert-butyl [(26-bromo-3,6,9,12,15,18,21,24-octaoxahexacosan-1-yl)amino]carboxylate (0.59 g, 1.03 mmol) in 2 mL of tetrahydrofuran, warmed slowly to room temperature and stirred for 12 hours. The reaction solution was poured into saturated aqueous ammonium chloride solution (50 mL), extracted three times with dichloromethane (45 mL), the organic phases were combined, washed three times with saturated brine (30 mL), the organic phase was dried over anhydrous sodium sulfate and concentrated to obtain the title compound (0.5 g, yield: 68.6%).

MS m/z (ESI): 710.5 [M+H]⁺.

### Step 4: Preparation of 4-(32,32-dimethyl-30-oxo-29-aza-2,5,8,11,14,17,20,23,26,31-decaoxatritriacontan-1-yl)cyclohexane-1-carboxylic acid

Tert-butyl 4-(32,32-dimethyl-30-oxo-29-aza-2,5,8, 11, 14, 17,20,23,26,31-decaoxa tritriacontan-1-yl)cyclohexane-1-carboxylate (0.18 g, 0.25 mmol) was dissolved in a mixed solvent of tetrahydrofuran (2 mL) and methanol (1 mL), cooled in an ice-water bath, sodium hydroxide (0.6 g, 15.2 mmol) in aqueous solution (3.8 mL) was slowly added to the reaction solution at 0°C, slowly heated to 60°C and stirred for 12 hours. The reaction solution was poured into water (30 mL), extracted three times with dichloromethane (15 mL), the remaining aqueous phase was slowly adjusted to pH = 7 with dilute hydrochloric acid (2N, 7.5 mL), and extracted three times with dichloromethane (30 mL), the organic phases were combined, washed three times with saturated brine (15 mL), the organic phase was dried over anhydrous sodium sulfate, concentrated, and the resulting residue was purified by high performance liquid chromatography to obtain the title compound (0.1 g, yield: 61.2%).

MS m/z (ESI): 654.4 [M+H]⁺.

### Step 5: Preparation of tert-butyl ({1-[4-({4-[4-amino-2-butyl-1-(3,4,5,6-tetrahydro-2H-pyran-4-ylmethyl)thieno[3,2-b]imidazo[4,5-d]pyridin-7-yl]hexahydropyridin-1-yl}carbonyl)cyclohexyl]-2,5,8,11,14,17,20,23,26-nonaoxaoctacosan-28-yl}amino)carboxylate

The synthetic route of Example 2-2 was applied, the raw material 1-(4-aminobutyl)- 2-butyl-7-methyl-1*H*-imidazo[4,5-*d*]thieno[3,2-*b*]pyridine-4-amine of step 1 was replaced with 2-butyl-7-(hexahydropyridin-4-yl)-1-(3,4,5,6-tetrahydro-2*H*-pyran-4-ylmethyl)thieno[3,2-*b*] imidazo[4,5-*d*]pyridine-4-amine, and 4-(hydroxymethyl)cyclohexyl-1-carboxylic acid was replaced with 4-(32,32-dimethyl-30-oxo-29-aza-2,5,8,11,14,17,20,23,26,31-decaoxa tritriacontan-1-yl)cyclohexane-1-carboxylic acid to obtain the title compound (37 mg, yield: 61.5%).

MS m/z (ESI): 1063.6 [M+H]⁺.

### Step 6: Preparation of {4-[4-amino-2-butyl-1-(3,4,5,6-tetrahydro-2H-pyran-4-ylmethyl) thieno[3,2-b]imidazo[4,5-d]pyridin-7-yl]hexahydropyridin-1-yl} [4-(28-amino-2,5,8,11,14,17,20,23,26-nonaoxaoctacosan-1-yl)cyclohexyl]methanone

The synthetic route of Example 1 was applied, the raw material tert-butyl (4-(4-amino-2-butyl-7-methyl-1*H*-imidazo[4,5-*d*]thieno[3,2-*b*]pyridin-1-yl)butyl)carbamate of step 9 was replaced with tert-butyl ({1-[4-({4-[4-amino-2-butyl-1-(3,4,5,6-tetrahydro-2*H*-pyran-4-ylmethyl)thieno[3,2-*b*]imidazo[4,5-*d*]pyridin-7-yl]hexahydropyridin-1-yl}carbonyl)cyclohexyl]-2,5,8,11,14,17,20,23,26-nonaoxaoctacosan-28-yl}amino)carboxylate to obtain the title compound (32 mg, yield: 95.2%).

MS m/z (ESI): 963.6 [M+H]⁺.

### Step 7: Preparation of N-{-[4-({4-[4-amino-2-butyl-1-(3,4,5,6-tetrahydro-2H-pyran-4-yl methyl)thieno[3,2-b]imidazo[4,5-d]pyridin-7-yl]hexahydropyridin-1-yl}carbonyl)cyclohexyl]-2,5,8,11,14,17,20,23,26-nonaoxaoctacosan-28-yl}-6-[2-(methyldioxo-λ6-thio)pyrimidin-5-yl]hex-5-ynylamide

The synthetic route of Example 2-3 was applied, the raw material 2-{[23-(4-{2-butyl-4-(tert-butylamino)-1*H*-thieno[3,2-*b*]imidazo[4,5-*d*]pyridin-7-yl}hexahydropyridin-1-yl)-3,6,9,12,15,18,21-heptaoxatricosan-1-yl]oxy}ethyl-1-amine of step 6 was replaced with {4-[4-amino-2-butyl-1-(3,4,5,6-tetrahydro-2*H*-pyran-4-ylmethyl)thieno[3,2-*b*]imidazo[4,5-*d*]pyridin-7-yl]hexahydropyridin-1-yl}[4-(28-amino-2,5, 8,11,14,17,20,23,26-nonaoxaoctacosan-1-yl)cyclohexyl]methanone to obtain the title compound (23 mg, yield: 55.3%).

MS m/z (ESI): 1213.6 [M+H]⁺.

¹H NMR (400 MHz, CD₃OD): δ 8.97 (s, 2H), 7.11 (s, 1H), 4.69-4.67 (m, 1H), 4.25-4.10 (m, 3H), 3.94 (d, *J* = 11.2 Hz, 3H), 3.73-3.43 (m, 36H), 3.42-3.33 (m, 6H), 3.26-3.21 (m, 2H), 3.01-2.90 (m, 2H), 2.75-2.70 (m, 2H), 2.58 (t, *J* = 7.2 Hz, 2H), 2.40 (t, *J* = 7.2 Hz, 2H), 2.26-2.11 (m, 3H), 2.00-1.40 (m, 20H), 1.32-1.22 (m 1H), 1.02 (t, *J* = 7.2 Hz, 3H).

### III. Preparation of immune-stimulating antibody conjugates (ISACs)

### Example 3-1: ISAC-1

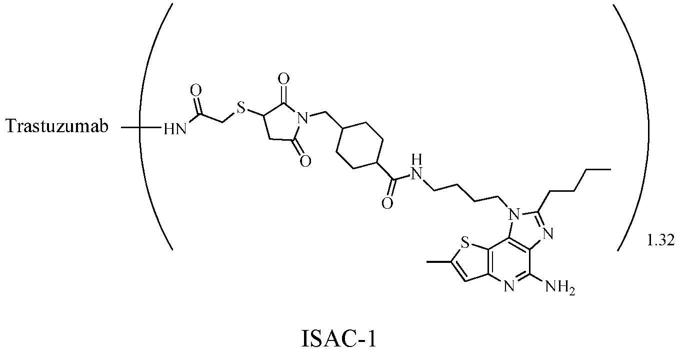

Trastuzumab monoclonal antibody stock solution was adjusted to pH = 7.24 with 1 M disodium hydrogen phosphate solution, and then diluted to 10 mg/mL with pH = 7.2-7.4 phosphate buffered saline (PBS) to obtain Trastuzumab monoclonal antibody buffer solution. DMSO was used as solvent, 70 mM SATA cross-linking agent solution was prepared. The SATA cross-linking agent solution in an amount of 10-fold molar of Trastuzumab monoclonal antibody was added to the Trastuzumab monoclonal antibody buffer solution at room temperature, shaken to mix well and reacted at room temperature for 1 hour, then purified with NAP-5 gel column, and washed with pH = 7.2-7.4 phosphate buffered saline (PBS), and the filtrate was collected as a buffer solution of Trastuzumab monoclonal antibody modified with SATA cross-linking agent.

0.05M Hydroxylamine and 2.5mM EDTA were added to the obtained a buffer solution of Trastuzumab monoclonal antibody modified with SATA cross-linking agent, shaken to mix well and reacted at room temperature for 2 hours, then purified with NAP-5 gel column, at the same time, washed with pH = 7.2-7.4 phosphate buffered saline (PBS) containing SmM EDTA, and the filtrate was collected as a buffer solution of purified deacetylated SATA-modified Trastuzumab monoclonal antibody.

To the buffer solution of purified deacetylated SATA-modified Trastuzumab monoclonal antibody, the target compound of Example 2-1 in DMSO solution (10 mM) in an amount of 10-fold molar of Trastuzumab monoclonal antibody was added, shaken to mix well and reacted at room temperature for 3 hours, then purified with NAP-5 gel column, and washed with pH = 7.2-7.4 phosphate buffered saline (PBS), and the filtrate was collected to obtain ISAC-1 in PBS buffer (3.4 mg/mL, 3.2 mL), which was stored at 4°C.

### Example 3-2

Trastuzumab monoclonal antibody stock solution was adjusted with 1M disodium hydrogen phosphate solution to pH = 7.6~7.7, and then diluted to 10 mg/mL with pH = 7.6-7.7 phosphate buffered saline (PBS) to obtain Trastuzumab monoclonal antibody buffer solution. To the trastuzumab monoclonal antibody buffer solution, 0.1M EDTA and TCEP (5.5 eq, 10 mM) were sequentially added, shaken to mix well and reacted at room temperature for 2 hours, the conjugate intermediate in DMSO solution (10 mM) in amount of 10-fold molar of Trastuzumab monoclonal antibody was added to the reaction system at room temperature, shaken to mix well and reacted at room temperature for 2 hours, then purified with NAP-5 gel column, and washed with pH = 6.0-6.1 histidine hydrochloride buffer solution, and the filtrate was collected to obtain ISAC-2, ISAC-3, ISAC-4, ISAC-5, ISAC-6 and ISAC-7 in PBS buffer, which were stored at 4°C.

### IV. Drug/antibody ratio of immune-stimulating antibody conjugate (ISAC): Determination of DAR value

Molecular weight of ISAC was determined by LC-MS, and drug/antibody ratio DAR value was calculated.

Chromatographic determination conditions:
Liquid chromatography column: Thermo MAbPac RP 3.0*100mm;
Mobile phase A: 0.1%FA/H₂O; Mobile phase B: 0.1%FA/ACN;
Flow rate: 0.25 ml/min; Sample chamber temperature: 8°C; Column temperature: 60°C; Injection volume: 2 µl;

| Time (minutes) | 2 | 20 | 22 | 25 | 26 | 30 |
|---|---|---|---|---|---|---|
| Mobile phase A (volume %) | 75 | 60 | 5 | 5 | 75 | 75 |
| Mobile phase B (volume %) | 25 | 40 | 95 | 95 | 25 | 25 |

### Mass Spectrometry Conditions:

Mass Spec Model: AB Sciex Triple TOF 5600+;
GS1 35; GS2 35; CUR 30; TEM 350; ISVF 5500; DP 250; CE 10; Accumulation time 0.5 s;
m/z 600-4000; Time bins-sum 40.

CE-SDS was used to calculate average DAR value of immune-stimulating antibody conjugate (ISAC), and the results were shown in Table 5:

**Table 5. Test results of average DAR values of immune-stimulating antibody conjugates (ISACs)**

| Immune-stimulating antibody conjugate (ISAC) | Linker-Payload (L-P) | Average DAR value |
|---|---|---|
| ISAC-1 | Example 2-1 | 1.32 |
| ISAC-2 | Example 2-3 | 8.15 |
| ISAC-3 | Example 2-4 | 7.77 |
| ISAC-4 | Example 2-5 | 7.86 |
| ISAC-5 | Example 2-6 | 8.00 |
| ISAC-6 | Example 2-7 | 7.91 |
| ISAC-7 | Example 2-8 | 7.04 |

### Biological tests

### Experimental Example 1: Determination of agonistic activity of compounds on HEK-Blue hTLR7 cells

### Experimental steps:

1. HEK-Blue hTLR7 cells (Invivogen) were cultured in DMEM medium (Hyclone) containing 10% FBS heat-inactivated fetal bovine serum (Corning). On the day of detection, the cell state was observed under a microscope, the cells were collected and resuspended, and the cell concentration was adjusted after counting, 50 µL per well, the total amount of cells was 2×10⁴, and the cells were inoculated in a 96-well plate.
2. After the cells adhered to the wall overnight, 50 µL of the test compound at different concentrations was added into the well plate, so that the final concentrations were 100 µM, 33.3 µM, 11.11 µM, 3.70 µM, 1.23 µM, 0.41 µM, 0.14 µM, 0.05 µM, 0 µM, and the final concentration of DMSO was 1%. The compound was incubated with the cells in a 37°C, 5% CO₂ incubator for 20 h.
3. After the incubation, observation under microscope was performed to determine the maximum signal well, the states of the cells with the highest and lowest concentrations of the compound, and whether the compound will crystalize at high concentration. 10 µL of cell culture supernatant was taken and transferred to a new 96-well transparent plate, 90 µL of QUANTI-Blue (Invivogen) detection reagent was added to each well, and incubated at 37°C for 3 h, and OD₆₂₀ was read with a multifunctional microplate reader (BMG LABTECH).
4. EC₅₀ was calculated by fitting with Graphpad Prism software log(agonist) vs. response - Variable slope, and Emax was the OD₆₂₀ at which the activation effect of the test compound reached the maximum.

### Experimental results:

The activation activity of the compounds on HEK-Blue hTLR7 cells was determined according to the above method, and the activity of the compounds was expressed by EC₅₀ and Emax, and the results were shown in Table 1:

**Table 1. Experimental results of the activation effect of compounds on HEK-Blue hTLR7 cells**

| Compound | EC₅₀ (µM) | Emax (OD₆₂₀) |
|---|---|---|
| 1 | 0.22 | 1.36 |
| 2 | 0.28 | 1.36 |
| 3 | 0.47 | 1.43 |
| 4 | 2.41 | 1.74 |
| 5 | 3.25 | 1.46 |
| 6 | 0.44 | 1.08 |
| 8 | 2.5 | 0.56 |
| 15 | 1.42 | 2.40 |
| 16 | 1.69 | 2.18 |
| 17 | 1.16 | 2.23 |
| 25 | 0.14 | 2.08 |
| 26 | 0.05 | 1.78 |
| 27 | 0.27 | 1.59 |
| 28 | 0.2 | 1.39 |
| 32 | 1.26 | 0.91 |
| 33 | 0.03 | 1.46 |
| 34 | 0.083 | 1.21 |
| 38 | 1.30 | 0.49 |
| 40 | 0.58 | 1.83 |
| 41 | 5.05 | 0.84 |
| 42 | 1.43 | 0.65 |
| 55 | 1.48 | 1.44 |
| 56 | 0.46 | 1.41 |
| 59 | 0.04 | 1.45 |

The results showed that Compounds **1, 2, 3, 4, 5, 6, 8, 15, 16, 17, 25, 26, 27, 28, 32, 33, 34, 38, 40, 41, 42, 55, 56** and **59** had strong activation effect on human TLR7.

Experimental Example 2: Determination of agonistic activity of compounds on HEK-Blue hTLR8 cells

### Experimental steps:

1. HEK-Blue hTLR8 cells (Invivogen) were cultured in DMEM medium (Hyclone) containing 10% FBS heat-inactivated fetal bovine serum (Corning). On the day of detection, the cell state was observed under a microscope, the cells were collected and resuspended, and the cell concentration was adjusted after counting, 50 µL per well, the total amount of cells was 2×10⁴, and the cells were inoculated in a 96-well plate.
2. After the cells adhered to the wall overnight, 50 µL of the test compound at different concentrations was added to the well plate, so that the final concentrations were 100 µM, 25 µM, 6.25 µM, 1.56 µM, 0.39 µM, 0.10 µM, 0.02 µM, 0.006 µM, 0 µM, or 100 µM, 33.3 µM, 11.11 µM, 3.70 µM, 1.23 µM, 0.41 µM, 0.14 µM, 0.05 µM, 0 µM, and the final concentration of DMSO was 1%. The compound was incubated with the cells in a 37°C, 5% CO₂ incubator for 20 hours.
3. After the incubation, observation was performed under microscope to determine the maximum signal well, the state of the cells with the highest and lowest concentrations of the compound, and whether the compound will crystalize at high concentration. 10 µL of cell culture supernatant was transferred to a new 96-well transparent plate, added with 90 µL of QUANTI-Blue (Invivogen) detection reagent to each well, incubated at 37°C for 3 hours, and OD₆₂₀ was read with a multifunctional microplate reader (BMG LABTECH).
4. EC₅₀ was calculated by fitting with Graphpad Prism software log(agonist) vs. Response - Variable slope, and Emax was the OD₆₂₀ at which the activation effect of the test compound reached the maximum.

### Experimental results:

The activation activity of the compounds on HEK-Blue hTLR8 cells was determined according to the above method, and the activity of the compounds was expressed as EC₅₀ and Emax, and the results were shown in Table 2:

**Table 2. Experimental results of the activation effect of compounds on HEK-Blue hTLR8 cells**

| Compound | EC₅₀ (µM) | Emax (OD₆₂₀) |
|---|---|---|
| 1 | 5.83 | 1.43 |
| 2 | 8.83 | 1.37 |
| 3 | 19.43 | 1.86 |
| 6 | 15.3 | 1.11 |
| 27 | 11.91 | 0.84 |
| 33 | 18.9 | 0.67 |

The results showed that Compounds **1, 2, 3, 6, 27** and **33** had a strong activation effect on human TLR8.

### Experimental Example 3: Determination of ability to stimulate peripheral blood mononuclear cells (PBMCs) to secrete TNF-α in the presence of tumor cells of immune-stimulating antibody conjugate (ISAC)

### Experimental steps:

1. HCC1954 tumor cells (Kangnuotai) in the logarithmic growth phase expressing HER2 were collected, washed twice with PBS, and the cells were resuspended with RPMI1640+10% heat-inactivated FBS complete medium and adjusted to have a cell density of 2×10⁵/ mL, and the HCC1954 tumor cells were inoculated in a 96-well plate (Corning), 1×10⁴ cells per well.
2. PBMCs (Sai Li Biology) were resuscitated, resuspended with RPMI1640+10% heat-inactivated FBS complete medium, adjusted to have a density of 6×10⁵ cells/mL, added to the 96-well plate inoculated with tumor cells, 3×10⁴ PBMCs per well.
3. 4. 68.4nM of Trastuzumab monoclonal antibody and immune-stimulating antibody conjugate ISAC-1, as well as 55.6 nM of immune-stimulating antibody conjugates ISAC-2, ISAC-3, ISAC-4, ISAC-5, ISAC-6 and ISAC-7, prepared by dilution with 100 µL of RPMI1640+10% heat-inactivated FBS complete medium, were added to the wells, respectively, and the blank wells were added with 100 µL RPMI1640+10% heat-inactivated FBS complete medium.
5. After being cultured in a 5% CO₂, 37°C incubator for 48 hours, 50 µL of supernatant was taken from each well for detection. Primary antibody and HRP-labeled secondary antibody were incubated, and subjected to color development according to the steps of human TNF-α ELISA detection kit (Invitrogen), and absorbance at 450 nm wavelength was read. The concentration of TNF-α was calculated according to the standard curve using Graphpad Prism software.

### Experimental results:

The ability to stimulate peripheral blood mononuclear cells (PBMC) to secrete TNF-α of Trastuzumab monoclonal antibody and each immune-stimulating antibody conjugate (ISAC) in the presence of HCC1954 tumor cells was determined according to the above method, and the results were shown in Table 3:

**Table 3. The results of the secretion of TNF-α stimulated by test substances in the co-incubation system of HCC1954 tumor cells and PBMC in vitro**

| Drug | TNF-α (pg/mL) |
|---|---|
| Blank control | 0 |
| Trastuzumab 68.4 nM | 17.39 |
| ISAC-1 68.4 nM | 35.17 |
| ISAC-2 55.6nM | 574.45 |
| ISAC-3 55.6nM | 792.85 |
| ISAC-4 55.6nM | 543.80 |
| ISAC-5 55.6nM | 838.30 |
| ISAC-6 55.6nM | 478.7 |
| ISAC-7 55.6nM | 910.1 |

The results showed that the immune-stimulating antibody conjugates ISAC-1, ISAC-2, ISAC-3, ISAC-4, ISAC-5, ISAC-6 and ISAC-7 stimulate more secretion of TNF-α under the co-incubation system of HCC1954 tumor cells and PBMCs in vitro than that of Trastuzumab monoclonal antibody at comparable concentration.

### Experimental Example 4: Drug efficacy evaluation of ISAC in SCID-beige mouse xenograft tumor model

### Experimental steps:

1. 5×10⁶ HCC1954 cells (mixed with Matrigel at 1:1 in equal volume, suspended in 0.1 mL PBS) were subcutaneously inoculated in the right underarm of each SCID-beige mouse (Weitong Lihua).
2. When the tumor grew to have an average volume of about 150 mm³, the mice with irregular tumor volume and too small or too large tumor volume were excluded, and the remaining mice were randomly divided into groups according to the tumor volume and animal weight, and administrated with a single intraperitoneal injection (i.p.) of physiological saline (vehicle control), Trastuzumab monoclonal antibody (9.95 mg/kg), immune-stimulating antibody conjugate ISAC-1 (10 mg/kg, same molar concentration as Trastuzumab monoclonal antibody), respectively, and the efficacy of the test compounds on the tumor-bearing mouse model and the animal tolerance of the test compounds were observed.
3. During the experiment, the mice were weighed twice a week and the tumor volume was measured, and the data were recorded.
4. Data statistics, tumor volume (V) calculation formula: V=1/2×a×b², wherein a and b represent length and width, respectively. The antineoplastic drug efficacy was evaluated with tumor growth inhibition rate TGI (%), calculation formula: TGI(%)_{(tumor volume)} = [1 - (Tvt - T_{V0})/(C_{Vt} - C_{V0})] × 100%, wherein T_{V0} represents the average tumor volume of the test compound group at the time of grouping and administration, Tvt represents the average tumor volume of the test compound group on day t after administration; C_{V0} represents the average tumor volume of the vehicle group at the time of grouping and administration; Cvt represents the average tumor volume of the vehicle group on day t after administration. When the tumor regressed, TGI(%)_{(tumor volume)} = 100% - (T_{Vt} - T_{V0})/T_{V0} × 100%. If the tumor shrunk from the initial volume, that was, when Vₜ < V₀, it was defined as partial tumor regression (PR); if the tumor completely disappeared, it was defined as complete tumor regression (CR).

### Experimental results:

Figure 1 shows the curve of tumor volume changing with time in each group in Experimental Example 4, showing the in vivo efficacy of each test substance on SCID-beige mouse xenograft tumor model subcutaneously inoculated with HCC1954 human breast cancer cells. After 21 days of administration, the immune-stimulating antibody conjugate ISAC-1 could significantly inhibit tumor growth, compared with the vehicle control group, the tumor volume TGI was 180.25%, P<0.001, while Trastuzumab monoclonal antibody had no obvious effect (Figure 1, Table 4). The results showed that in SCID-beige mouse xenograft tumor model subcutaneously inoculated with HCC1954 human breast cancer cells, a single intraperitoneal injection (i.p.) of the immune-stimulating antibody conjugate ISAC-1 showed a very significant anti-tumor effect, apparently better than Trastuzumab monoclonal antibody.

**Table 4. Drug efficacy of immune-stimulating antibody conjugate ISAC on SCID-beige mouse xenograft tumor**

| Drug | Inhibition rate, TGI% | | | Tumor regression |
|---|---|---|---|---|
| | Day 7 | Day 14 | Day 21 | |
| Trastuzumab 9.95 mg/kg | 17.64% | 1.82% | 5.13% | 0/6 |
| ISAC-1 10 mg/kg | >100% | > 100% | > 100% | 5/6(PR),1/6(CR) |

PR represents partial regression; CR represents complete regression.

The above examples do not limit the solution of the present application in any way. Various modifications of the present invention, in addition to those described herein, will be apparent to those skilled in the art from the foregoing description. Such modifications are also intended to fall within the scope of the appended claims. Each reference cited in the present application, including all patents, patent applications, journal articles, books, and any other publications, is hereby incorporated by reference in its entirety.

## Claims

1. A compound of Formula (I) or a pharmaceutically acceptable salt, ester, stereoisomer, tautomer, polymorph, solvate, *N*-oxide, isotope-labeled compound, metabolite or prodrug thereof: 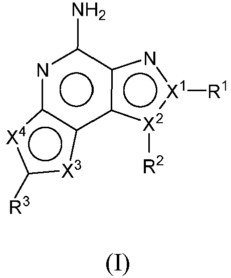 wherein,
X¹ is selected from the group consisting of N and C;
X² is selected from the group consisting of N and C;
and at least one of X¹ and X² is selected from N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is selected hydrogen, or has the formula -L¹-L²-L³-L⁴;
L¹ is selected from the group consisting of a covalent bond and C₁₋₆ alkylene;
L² is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L³ is selected from the group consisting of a covalent bond, -O-, -S-, -C(O)-, -O-C(O)-, - C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ₘ-NR⁵-, -NR⁵-S(O)ₘ- and -NR⁵-S(O)ₘ-NR⁵-;
L⁴ is selected from the group consisting of hydrogen, C₁₋₆ alkyl, -(O-CH₂CH₂)ₙ-O-C₁₋₆ alkyl and -C₁₋₆ alkylene-(O-CH₂CH₂)ₙ-O-C₁₋₆ alkyl, optionally, each alkyl is independently substituted with one or more groups independently selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R³ is hydrogen, or has the formula of -L⁵-L⁶-L⁷-L⁸;
L⁵ is selected from the group consisting of a covalent bond and C₁₋₆ alkylene;
L⁶ is selected from the group consisting of a covalent bond, C₃₋₁₀ cycloalkyl, 3- to 12-membered heterocyclyl, C₆₋₁₀ aryl and 5- to 10-membered heteroaryl, optionally, the cycloalkyl, heterocyclyl, aryl and heteroaryl are each independently substituted with one or more groups independently selected from the group consisting of hydrogen, halogen, cyano, C₁₋₆ alkyl, C₁₋₆ alkoxy and -C₁₋₆ alkylene-NH₂;
L⁷ is selected from the group consisting of a covalent bond, -O-, -S-, -C(O)-, -O-C(O)-, - C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L⁸ is selected from the group consisting of hydrogen, C₁₋₆ alkyl, -(O-CH₂CH₂)_{q}-O-C₁₋₆ alkyl and -C₁₋₆ alkylene-(O-CH₂CH₂)_{q}-O-C₁₋₆ alkyl, optionally, each alkyl is independently substituted with one or more groups independently selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently selected from the group consisting of hydrogen and C₁₋₆ alkyl;
each R⁶ is independently selected from the group consisting of hydrogen and C₁₋₆ alkyl;
each m is independently 1 or 2;
each n is independently selected from the group consisting of integers 1 to 25;
each p is independently 1 or 2;
each q is independently selected from the group consisting of integers 1 to 25.

2. The compound or pharmaceutically acceptable salt, ester, stereoisomer, tautomer, polymorph, solvate, *N*-oxide, isotope-labeled compound, metabolite or prodrug thereof according to claim 1, wherein:
R¹ is selected from the group consisting of C₁₋₄ alkyl and -C₁₋₄ alkylene-O-C₁₋₄ alkyl;
preferably, R¹ is selected from the group consisting of C₁₋₄ alkyl and -C₁₋₂ alkylene-O-C₁₋₂ alkyl;
preferably, R¹ is selected from the group consisting of n-butyl, 2-methoxyethyl and ethoxymethyl;
preferably, R¹ is n-butyl.

3. The compound or pharmaceutically acceptable salt, ester, stereoisomer, tautomer, polymorph, solvate, *N*-oxide, isotope-labeled compound, metabolite or prodrug thereof according to claim 1 or 2, wherein, R² is hydrogen, or,
R² has the formula of -L¹-L²-L³-L⁴, wherein,
L¹ is selected from the group consisting of a covalent bond and C₁₋₆ alkylene, such as -CH₂-, -CH₂-CH₂-, -CH₂-CH₂-CH₂-, or -CH₂-CH₂-CH₂-CH₂-; preferably, L¹ is a covalent bond or -CH₂-;
L² is selected from the group consisting of a covalent bond, C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl, such as piperidinyl; e.g., 3- to 6-membered saturated or unsaturated oxygen-containing monoheterocyclyl, such as tetrahydropyranyl), C₆₋₁₀ aryl (e.g., phenyl) and 5- to 10-membered heteroaryl (e.g., 5- to 6-membered nitrogen-containing heteroaryl, 5- to 6-membered oxygen-containing heteroaryl, 5- to 6-membered sulfur-containing heteroaryl), optionally, the cycloalkyl, heterocyclyl, aryl and heteroaryl are independently substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L² is selected from the group consisting of a covalent bond, 3- to 12-membered heterocyclyl (e.g., 3- to 6-membered saturated or unsaturated nitrogen-containing monoheterocyclyl, such as piperidinyl; e.g., 3- to 6-membered saturated or unsaturated oxygen-containing monoheterocyclyl, such as tetrahydropyranyl) and C₆₋₁₀ aryl (e.g., phenyl), optionally, the heterocyclyl and aryl are each independently substituted with one or more groups independently selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L² is selected from the group consisting of a covalent bond, piperidinyl and tetrahydropyranyl;
L³ is a covalent bond or -C(O)-;
L⁴ is selected from the group consisting of hydrogen, C₁₋₆ alkyl, -(O-CH₂CH₂)ₙ-O-C₁₋₆ alkyl (e.g., -(O-CH₂CH₂)ₙ-O-methyl, -(O-CH₂CH₂)ₙ-O-ethyl) and -C₁₋₆ alkylene-(O-CH₂CH₂)n-O-C₁₋₆ alkyl (e.g., -CH₂-(O-CH₂CH₂)ₙ-O-methyl, -CH₂-CH₂-(O-CH₂CH₂)ₙ-O-methyl, -CH₂-(O-CH₂CH₂)ₙ-O-ethyl, -CH₂-CH₂-(O-CH₂CH₂)ₙ-O-ethyl), optionally, each alkyl (e.g., methyl or ethyl) is independently substituted with one or more groups independently selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl and -C(O)-O-C₁₋₆ alkyl (e.g., -C(O)-O-methyl), each n is independently selected from the group consisting of 1, 2, 3, 4, 5, 6, 7; preferably, L⁴ is selected from the group consisting of hydrogen, C₁₋₆ alkyl and -C₁₋₆ alkylene-(O-CH₂CH₂)ₙ-O-C₁₋₆ alkyl, optionally, each alkyl is independently substituted with one or more groups independently selected from the group consisting of hydroxyl, -NH₂, C₁₋₆ alkoxy, carboxyl and - C(O)-O-C₁₋₆ alkyl, each n is selected from the group consisting of 1, 2, 3, 4, 5, 6 and 7; preferably, L⁴ is selected from the group consisting of hydrogen, C₁₋₄ alkyl and -C₁₋₄ alkylene-(O-CH₂CH₂)ₙ-O-C₁₋₄ alkyl, optionally, each alkyl is independently substituted with one or more groups independently selected from the group consisting of hydroxyl, -NH₂, C₁₋₄ alkoxy, carboxyl and - C(O)-O-C₁₋₄ alkyl, n is selected from the group consisting of 1, 2, 3, 4, 5, 6 and 7.

4. The compound or pharmaceutically acceptable salt, ester, stereoisomer, tautomer, polymorph, solvate, *N*-oxide, isotope-labeled compound, metabolite or prodrug thereof according to any one of claims 1-3, wherein R³ is hydrogen, or
R³ has the formula of -L⁵-L⁶-L⁷-L⁸, wherein
L⁵ is selected from the group consisting of a covalent bond and C₁₋₆ alkylene, such as -CH₂-, -CH₂-CH₂-, -CH₂-CH₂-CH₂- or -CH₂-CH₂-CH₂-CH₂-; preferably, L⁵ is a covalent bond;
L⁶ is selected from the group consisting of a covalent bond, C₃₋₁₀ cycloalkyl (e.g., C₃₋₆ cycloalkyl or C₅₋₆ cycloalkyl), 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)), C₆₋₁₀ aryl (e.g., phenyl) and 5- to 10-membered heteroaryl (e.g., 5- to 6-membered nitrogen-containing heteroaryl, 5- to 6-membered oxygen-containing heteroaryl, 5- to 6-membered sulfur-containing heteroaryl), optionally, the cycloalkyl, heterocyclyl, aryl and heteroaryl are each independently substituted with one or more groups selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L⁶ is selected from the group consisting of a covalent bond and 3- to 12-membered heterocyclyl (e.g., 3- to 6-membered saturated or unsaturated nitrogen-containing monoheterocyclyl (e.g., piperidinyl, tetrahydropyridyl (e.g., 1,2,3,6-tetrahydropyridyl), piperazinyl) or 3- to 6-membered saturated or unsaturated oxygen-containing monoheterocyclyl (e.g., tetrahydropyranyl)), the heterocyclyl is optionally substituted with one or more groups independently selected from the group consisting of hydrogen, halogen, cyano, C₁₋₆ alkyl (e.g., methyl, ethyl, n-propyl, isopropyl, n-butyl, isobutyl, sec-butyl, tert-butyl, n-pentyl, n-hexyl), C₁₋₆ alkoxy (e.g., methoxy, ethoxy) and -C₁₋₆ alkylene-NH₂ (e.g., -CH₂-NH₂); preferably, L⁶ is selected from the group consisting of a covalent bond, 1,2,3,6-tetrahydropyridyl, piperazinyl and tetrahydropyranyl;
L⁷ is a covalent bond or -C(O)-; preferably, L⁷ is a covalent bond;
L⁸ is selected from the group consisting of hydrogen, C₁₋₆ alkyl, -(O-CH₂CH₂)_{q}-O-C₁₋₆ alkyl (e.g., -(O-CH₂CH₂)_{q}-O-methyl, -(O-CH₂CH₂)_{q}-O-ethyl) and -C₁₋₆ alkylene-(O-CH₂CH₂)_{q}-O-C₁₋₆ alkyl (e.g., -CH₂-(O-CH₂CH₂)_{q}-O-methyl, -CH₂-CH₂-(O-CH₂CH₂)_{q}-O-methyl, -CH₂-(O-CH₂CH₂)_{q}-O-ethyl, -CH₂-CH₂-(O-CH₂CH₂)_{q}-O-ethyl), optionally, each alkyl (e.g., methyl or ethyl) is independently substituted with one or more groups independently selected from the group consisting of hydrogen, halogen, hydroxyl, -NH₂, -NH-C(O)-O-C₁₋₆ alkyl (e.g., -NH-C(O)-O-methyl), C₁₋₆ alkoxy (e.g., methoxy, ethoxy), carboxyl and -C(O)-O-C₁₋₆ alkyl (e.g., -C(O)-O-methyl), each q is independently selected from the group consisting of 1, 2, 3, 4, 5, 6, 7; preferably, L⁸ is selected from the group consisting of hydrogen, C₁₋₆ alkyl and -C₁₋₆ alkylene-(O-CH₂CH₂)_{q}-O-C₁₋₆ alkyl, optionally, each alkyl is substituted with one or more groups independently selected from the group consisting of hydroxyl, -NH₂, C₁₋₆ alkoxy, carboxyl and -C(O)-O-C₁₋₆ alkyl, q is selected from the group consisting of 1, 2, 3, 4, 5, 6 and 7; preferably, L⁸ is selected from the group consisting of hydrogen, C₁₋₄ alkyl and -C₁₋₄ alkylene-(O-CH₂CH₂)_{q}-O-C₁₋₄ alkyl, optionally, each alkyl is independently substituted with one or more C₁₋₄ alkoxy groups, and q is selected from the group consisting of 1, 2, 3, 4, 5, 6 and 7.

5. The compound or pharmaceutically acceptable salt, ester, stereoisomer, tautomer, polymorph, solvate, *N*-oxide, isotope-labeled compound, metabolite or prodrug thereof according to any one of claims 1-4, wherein the compound has a structure of Formula (II-A), Formula (II-B), Formula (II-C), Formula (II-D), Formula (II-E) or Formula (II-F): 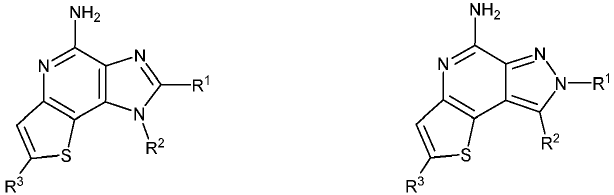 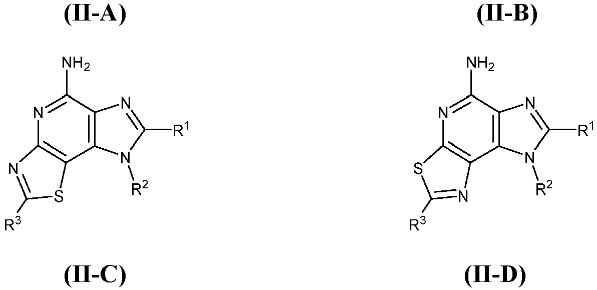 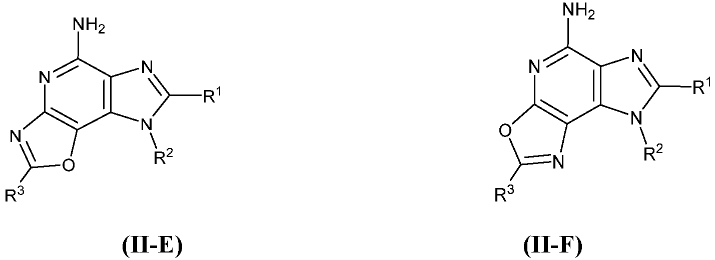 wherein, each of the groups R¹, R² and R³ is as defined in any one of claims 1-4.

6. The compound or pharmaceutically acceptable salt, ester, stereoisomer, tautomer, polymorph, solvate, *N*-oxide, isotope-labeled compound, metabolite or prodrug thereof according to any one of claims 1-5, wherein the compound has a structure of Formula (III-A) or Formula (III-B): 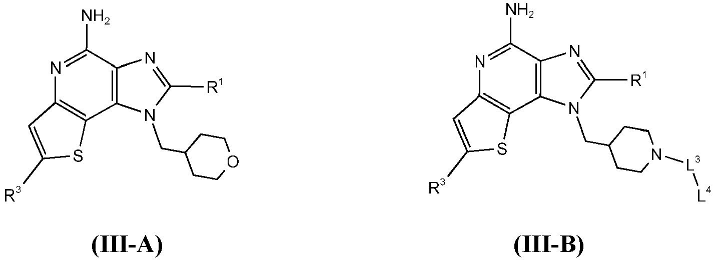 wherein, each of the groups R¹, R³, L³ and L⁴ is as defined in any one of claims 1-4.

7. The compound or pharmaceutically acceptable salt, ester, stereoisomer, tautomer, polymorph, solvate, *N*-oxide, isotope-labeled compound, metabolite or prodrug thereof according to any one of claims 1-6, wherein the compound has a structure of Formula (IV-A) or Formula (IV-B): 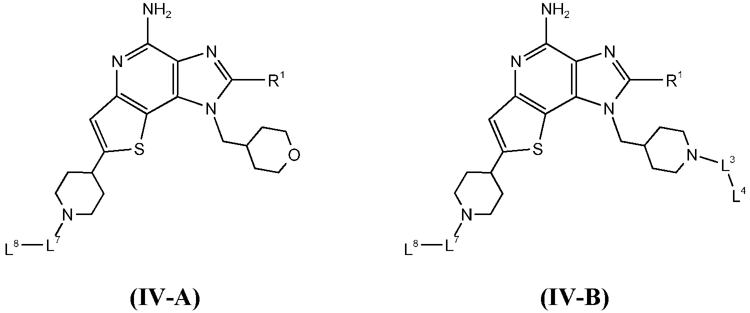 wherein, each of the groups R¹, L³, L⁴, L⁷ and L⁸ is as defined in any one of claims 1-4.

8. The compound or pharmaceutically acceptable salt, ester, stereoisomer, tautomer, polymorph, solvate, *N*-oxide, isotope-labeled compound, metabolite or prodrug thereof according to any one of claims 1-7, wherein the compound has a structure of Formula (V-A) or Formula (V-B): 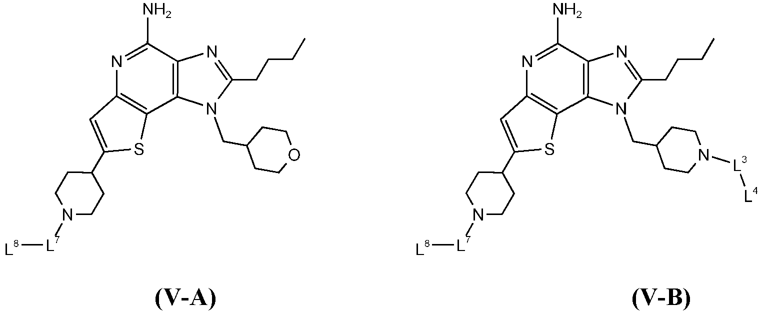 wherein, each of the groups L³, L⁴, L⁷ and L⁸ is as defined in any one of claims 1-4.

9. The compound or pharmaceutically acceptable salt, ester, stereoisomer, tautomer, polymorph, solvate, *N*-oxide, isotope-labeled compound, metabolite or prodrug thereof according to claim 1, wherein the compound has a structure as follows:

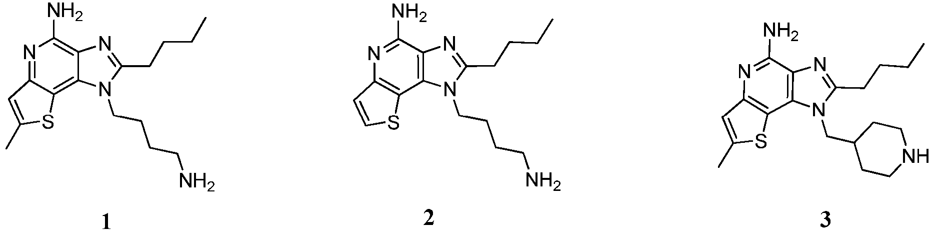

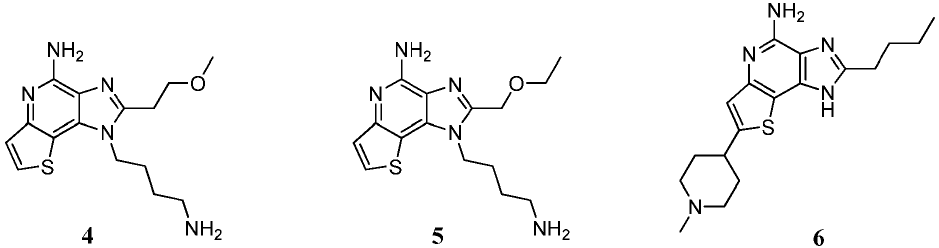

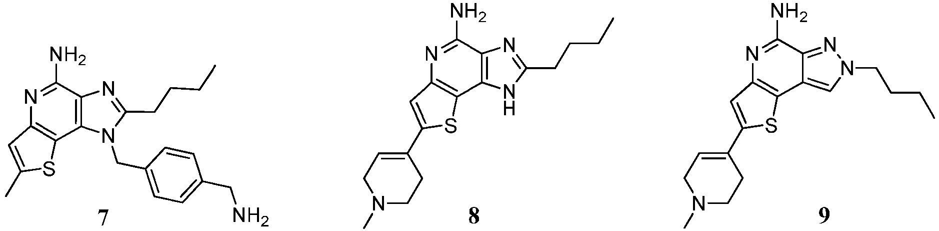

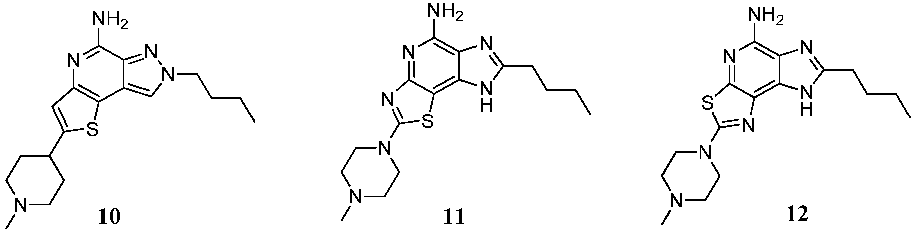

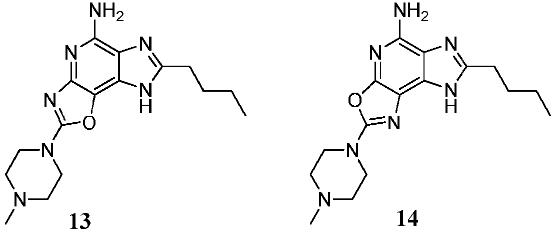

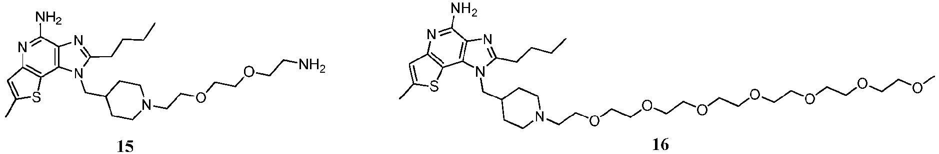

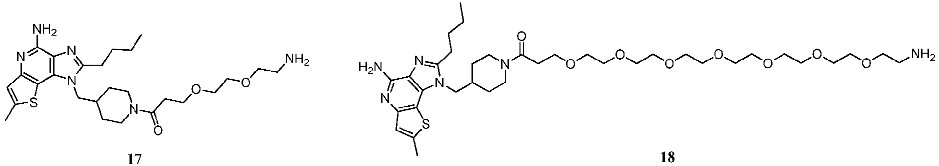

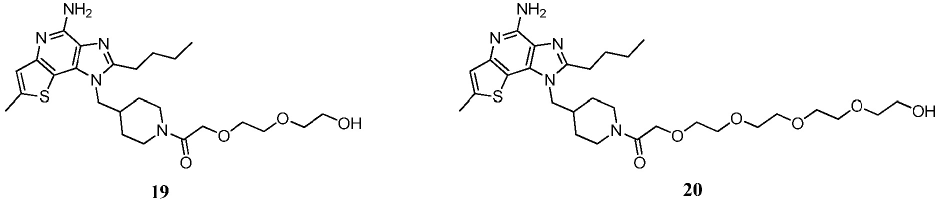

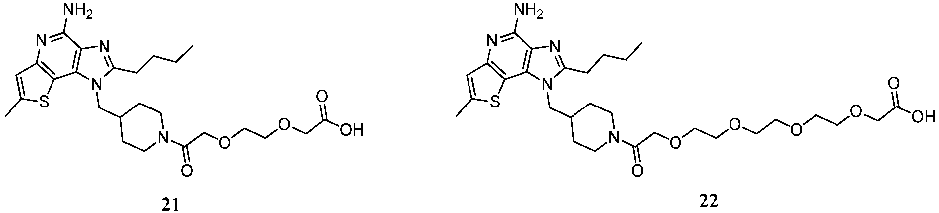

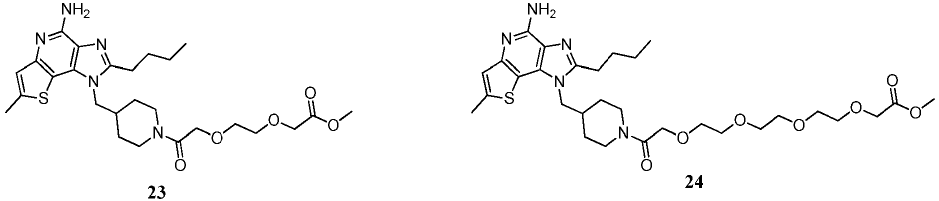

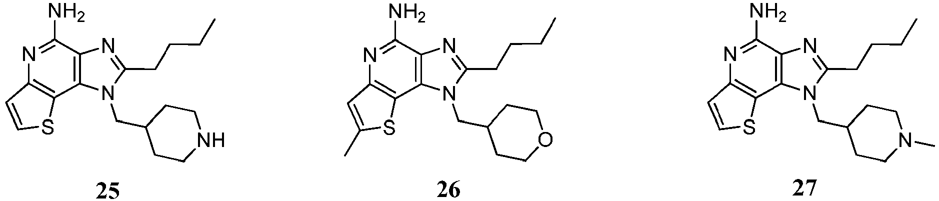

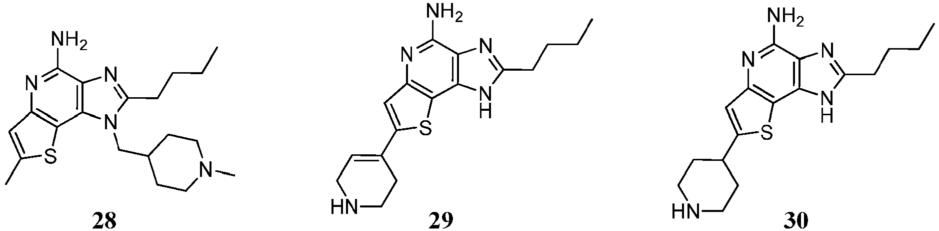

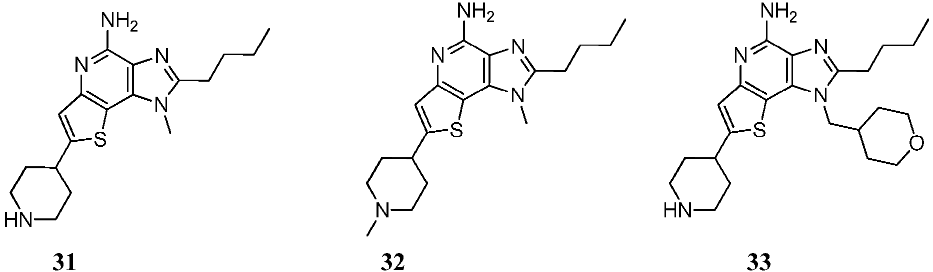

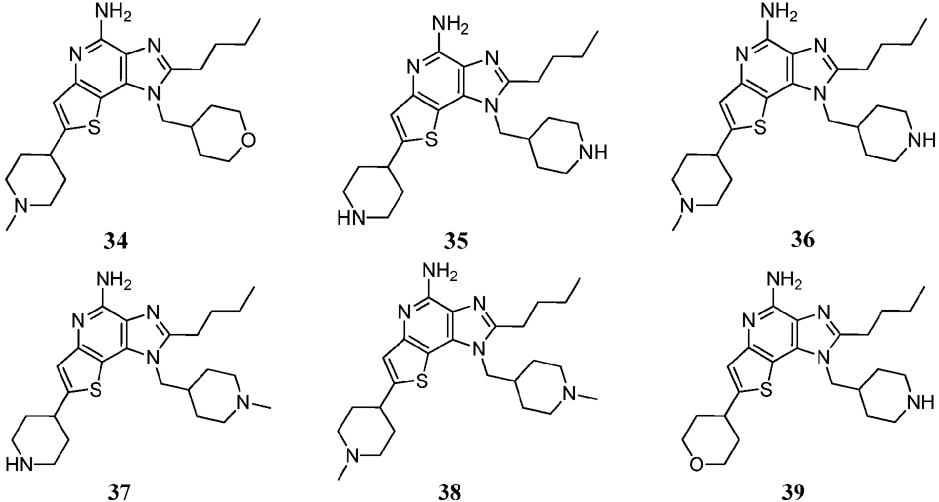

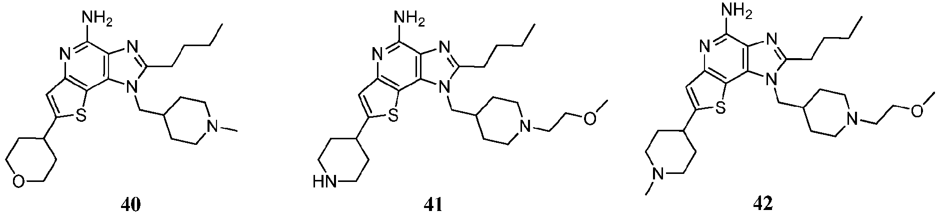

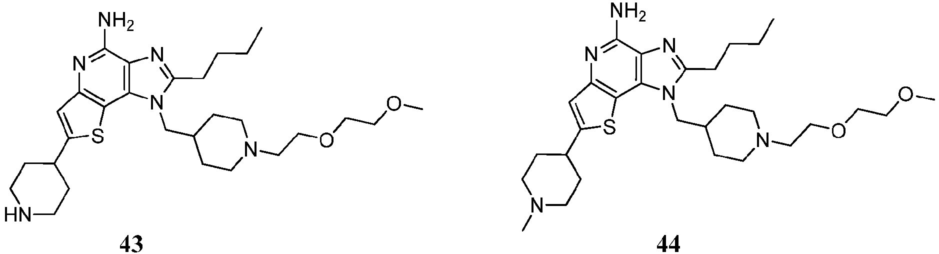

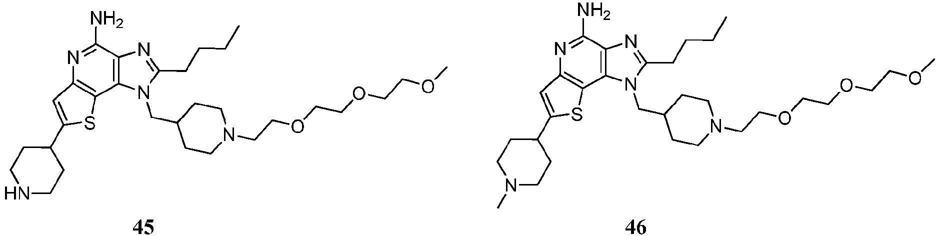

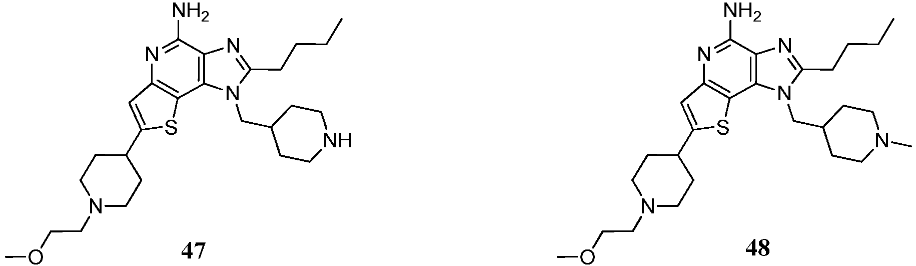

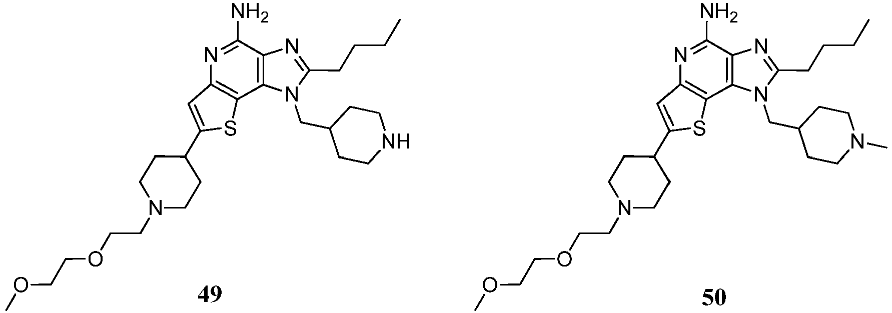

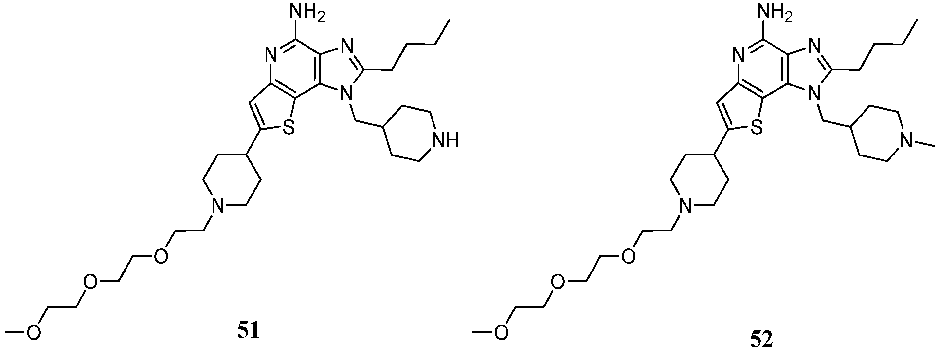

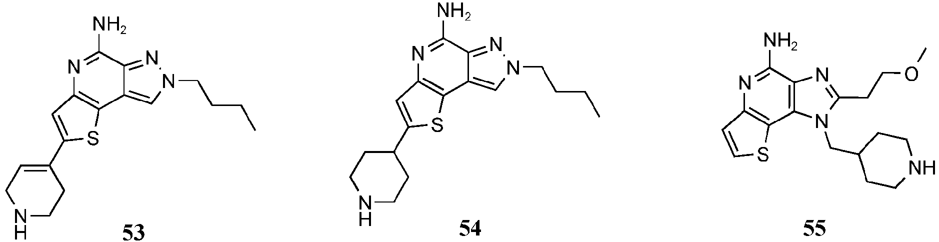

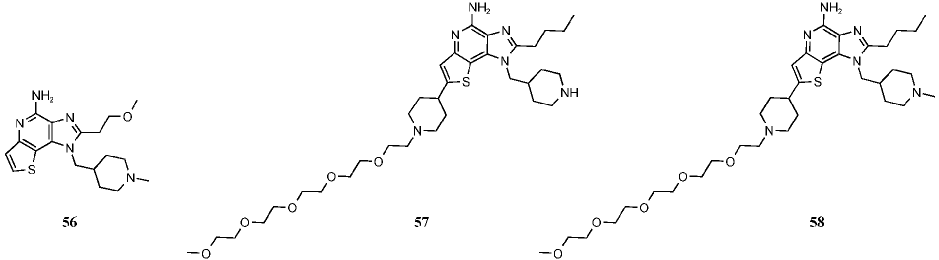

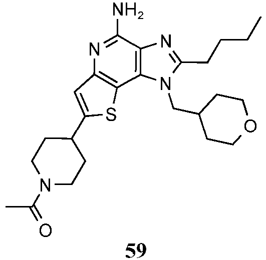

10. A drug-linker of the following formula or a pharmaceutically acceptable salt, ester, stereoisomer, tautomer, polymorph, solvate, *N*-oxide, isotope-labeled compound, metabolite or prodrug thereof: D-L'
wherein, D is a fragment of the compound according to any one of claims 1-9;
L' is a linker;
preferably, L' is selected from -L⁹-L¹⁰-L¹¹, wherein,
L⁹ is absent or selected from 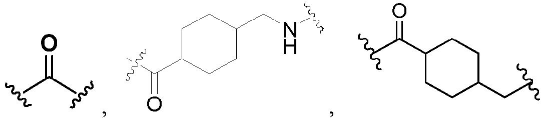 and 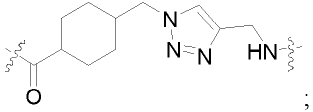
L¹⁰ is absent or selected from the group consisting of -(CH₂CH₂O)ₛCH₂CH₂NH-, - (OCH₂CH₂)ₛNH- and -C(O)-(CH₂CH₂O)ₛCH₂CH₂NH-; s is selected from the group consisting of integers 1 to 10;
L¹¹ is 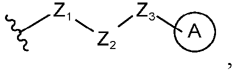 wherein,
Z₁ is selected from the group consisting of a chemical bond and 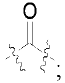
Z₂ is selected from the group consisting of a chemical bond and C₁₋₂₀ alkylene;
Z₃ is selected from the group consisting of a chemical bond, C₂₋₆ alkenylene and C₂₋₆ alkynylene;
A is selected from the group consisting of 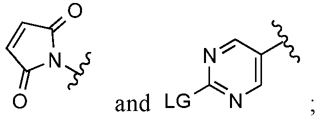
LG is a leaving group of nucleophilic substitution reaction, and selected from the group consisting of halogen, nitro, benzenesulfonate, p-toluenesulfonate, trifluoromethanesulfonate, - S(O)₂-R⁷ and -S(O)-R⁷;
R⁷ is selected from the group consisting of C₁₋₆ alkyl and C₁₋₆ haloalkyl;
preferably, L' is selected from 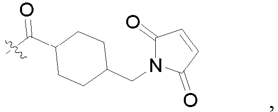 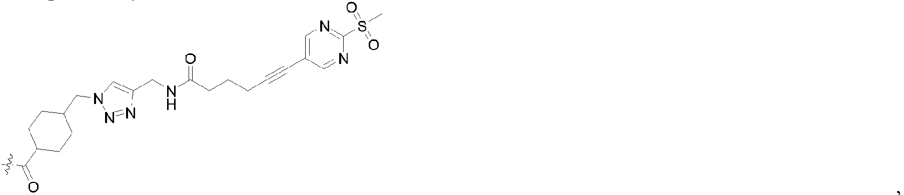 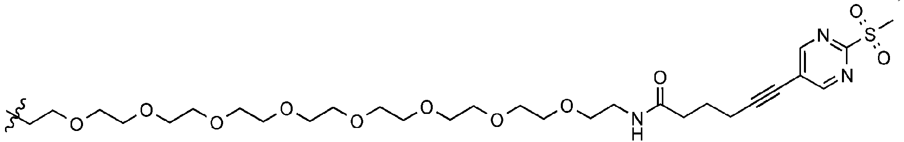 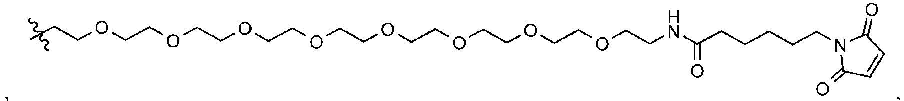 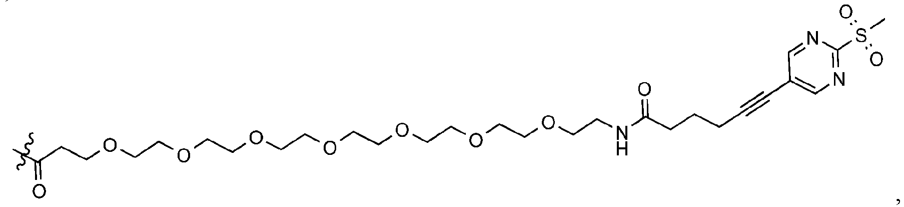 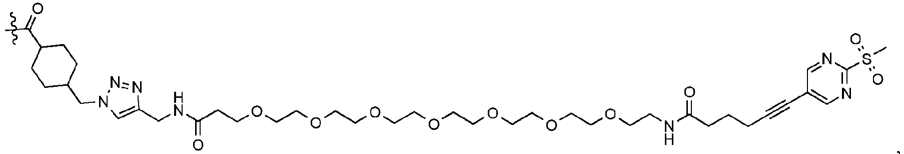 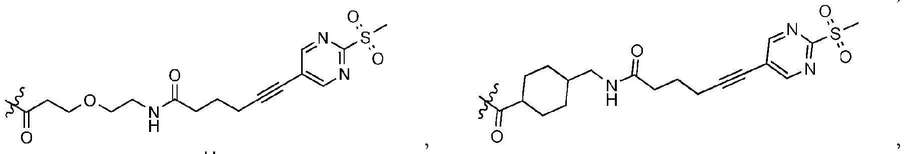 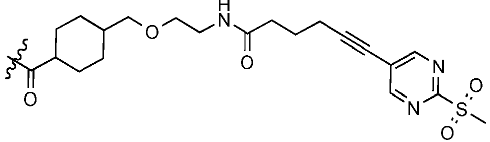
and

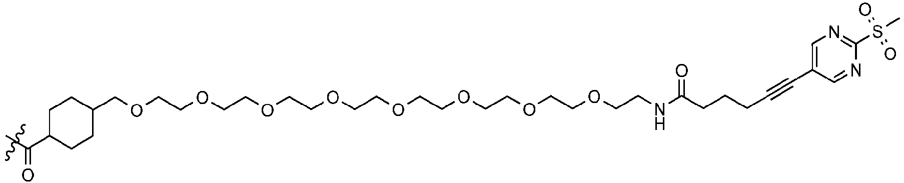

11. The drug-linker or pharmaceutically acceptable salt, ester, stereoisomer, tautomer, polymorph, solvate, *N*-oxide, isotope-labeled compound, metabolite or prodrug thereof according to claim 10, wherein the drug-linker is selected from the group consisting of the following structures:

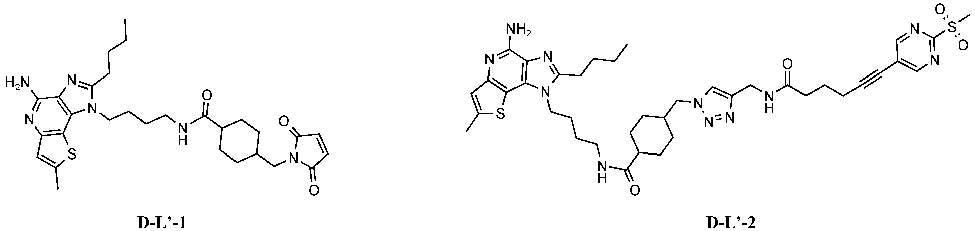

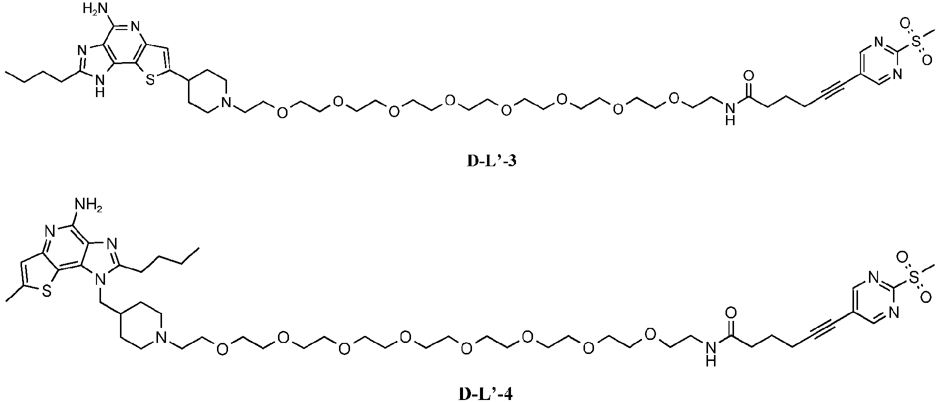

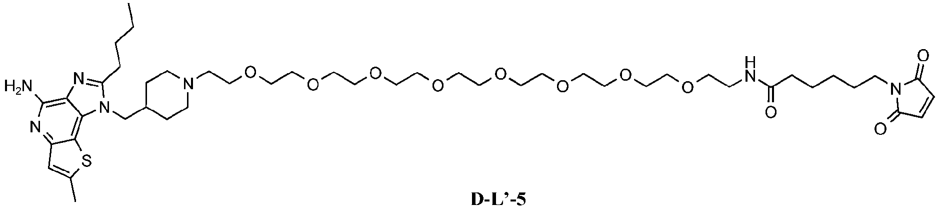

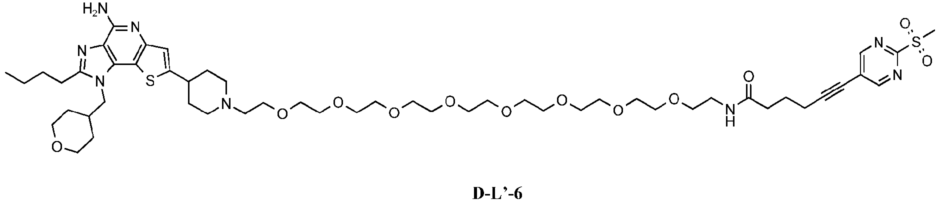

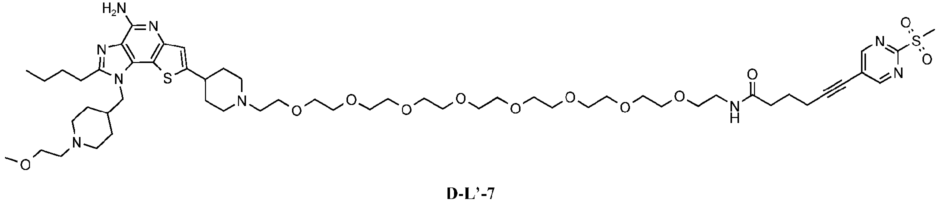

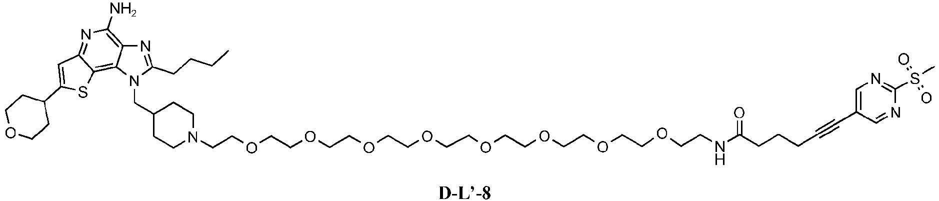

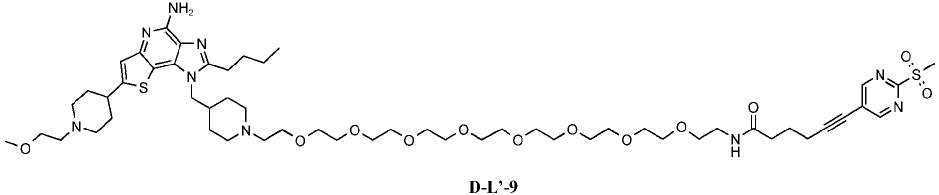

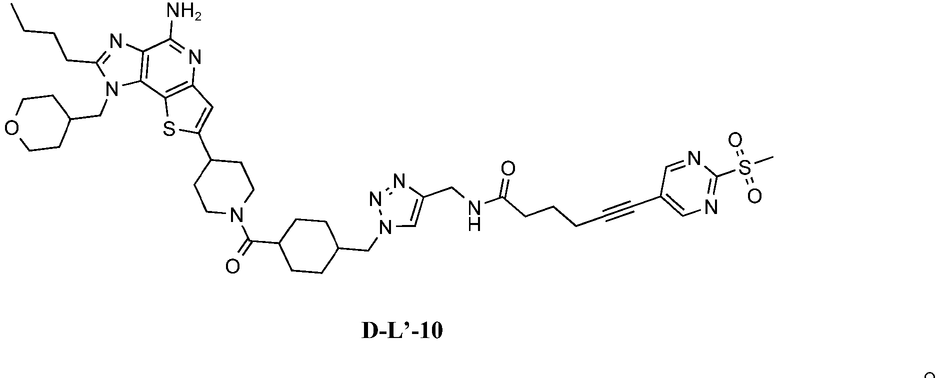

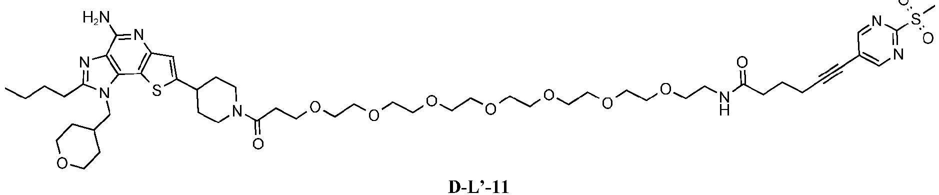

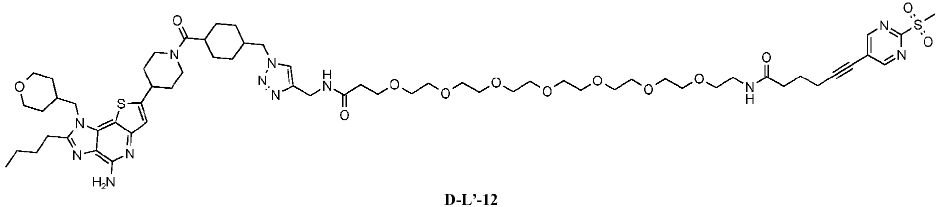

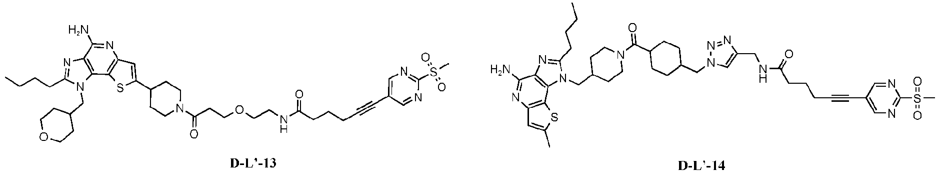

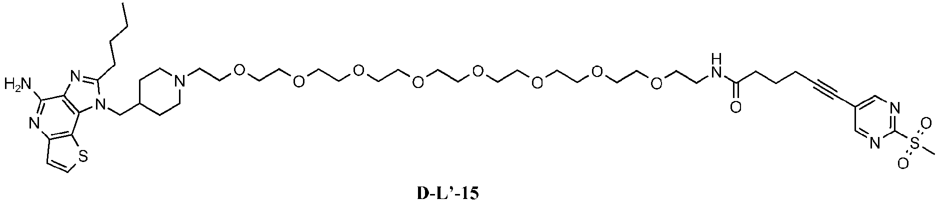

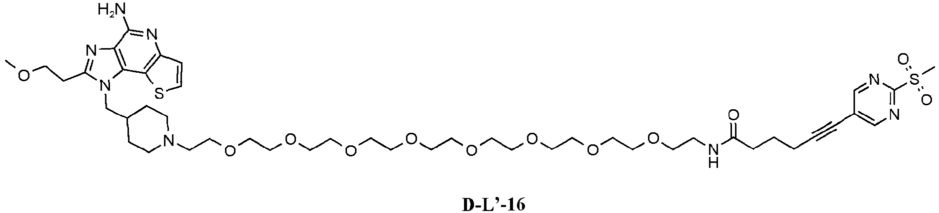

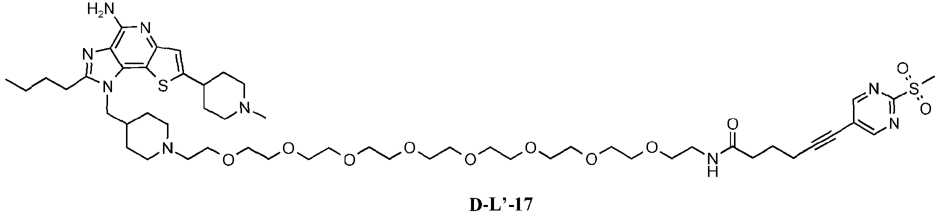

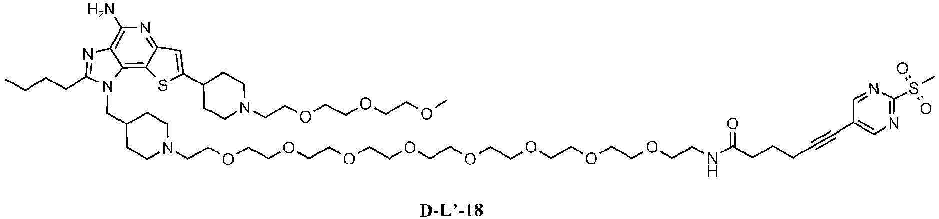

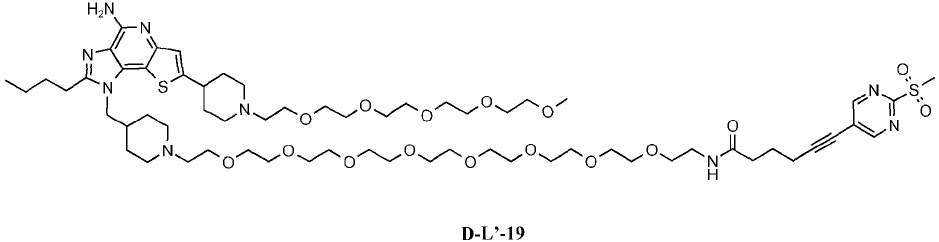

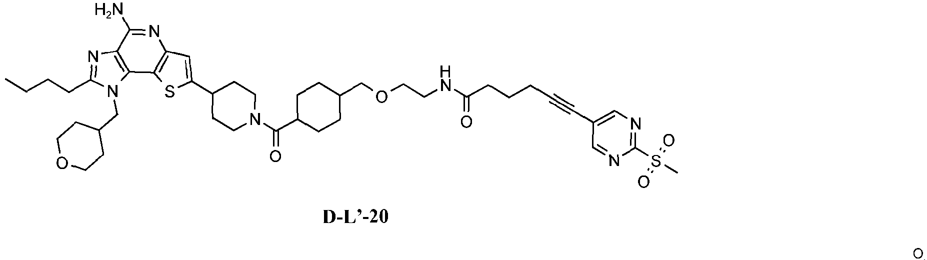

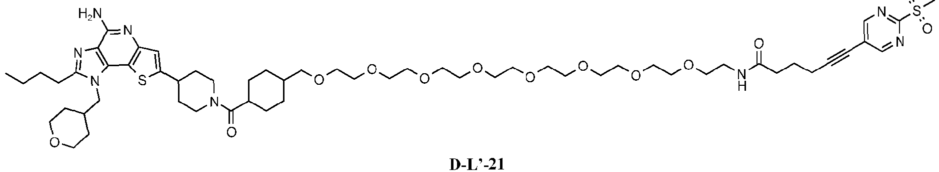

12. An immune-stimulating antibody conjugate of Formula (ISAC-I) or a pharmaceutically acceptable salt, ester, stereoisomer, tautomer, polymorph, solvate, *N*-oxide, isotope-labeled compound, metabolite or prodrug thereof: 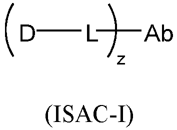 wherein,
D is a fragment of the compound according to any one of claims 1-9;
L is a fragment of the linker L', wherein L' is as defined in claim 10;
Ab is an antibody capable of targeting to a target antigen, optionally, the antibody is modified (e.g., modified by a cross-linking agent);
z is selected from the group consisting of 1 to 10;
preferably, the immune-stimulating antibody conjugate has a DAR value of 1 to 10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 1 to 8, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 1.0 to 5.5, 1.0 to 6.0, 1.0 to 6.5, 1.0 to 7.0, 1.0 to 7.5, 1.0 to 8.0, 1.5 to 2.0, 1.5 to 2.5, 1.5 to 3.0, 1.5 to 3.5, 1.5 to 4.0, 1.5 to 4.5, 1.5 to 5.0, 1.5 to 5.5, 1.5 to 6.0, 1.5 to 6.5, 1.5 to 7.0, 1.5 to 7.5, 1.5 to 8.0, 2.0 to 2.5, 2.0 to 3.0, 2.0 to 3.5, 2.0 to 4.0, 2.0 to 4.5, 2.0 to 5.0, 2.0 to 5.5, 2.0 to 6.0, 2.0 to 6.5, 2.0 to 7.0, 2.0 to 7.5, 2.0 to 8.0, 2.5 to 3.0, 2.5 to 3.5, 2.5 to 4.0, 2.5 to 4.5, 2.5 to 5.0, 2.5 to 5.5, 2.5 to 6.0, 2.5 to 6.5, 2.5 to 7.0, 2.5 to 7.5, 2.5 to 8.0, 3.0 to 3.5, 3.0 to 4.0, 3.0 to 4.5, 3.0 to 5.0, 3.0 to 5.5, 3.0 to 6.0, 3.0 to 6.5, 3.0 to 7.0, 3.0 to 7.5, 3.0 to 8.0, 3.5 to 4.0, 3.5 to 4.5, 3.5 to 5.0, 3.5 to 5.5, 3.5 to 6.0, 3.5 to 6.5, 3.5 to 7.0, 3.5 to 7.5, 3.5 to 8.0, 4.0 to 4.5, 4.0 to 5.0, 4.0 to 5.5, 4.0 to 6.0, 4.0 to 6.5, 4.0 to 7.0, 4.0 to 7.5, 4.0 to 8.0, 4.5 to 5.0, 4.5 to 5.5, 4.5 to 6.0, 4.5 to 6.5, 4.5 to 7.0, 4.5 to 7.5, 4.5 to 8.0, 5.0 to 5.5, 5.0 to 6.0, 5.0 to 6.5, 5.0 to 7.0, 5.0 to 7.5, 5.0 to 8.0, 5.5 to 6.0, 5.5 to 6.5, 5.5 to 7.0, 5.5 to 7.5, 5.5 to 8.0, 6.0 to 6.5, 6.0 to 7.0, 6.0 to 7.5, 6.0 to 8.0, 6.5 to 7.0, 6.5 to 7.5, 6.5 to 8.0, 7.0 to 7.5, 7.0 to 8.0 or 7.5 to 8.0.

13. Use of a compound in the manufacture of an immune-stimulating antibody conjugate, wherein the compound is selected from the compound according to any one of claims 1-9.

14. A method for preparing an immune-stimulating antibody conjugate, comprising a step of using the compound according to any one of claims 1-9.

15. A pharmaceutical composition, comprising a prophylactically or therapeutically effective amount of the compound or pharmaceutically acceptable salt, ester, stereoisomer, tautomer, polymorph, solvate, *N-*oxide, isotope-labeled compound, metabolite or prodrug thereof according to any one of claims 1-9, the drug-linker or pharmaceutically acceptable salt, ester, stereoisomer, tautomer, polymorph, solvate, *N-*oxide, isotope-labeled compound, metabolite or prodrug thereof according to of claim 10 or 11, or the immune-stimulating antibody conjugate according to claim 12, and one or more pharmaceutically acceptable carriers.

16. Use of the compound or pharmaceutically acceptable salt, ester, stereoisomer, tautomer, polymorph, solvate, *N-*oxide, isotope-labeled compound, metabolite or prodrug thereof according to any one of claims 1-9, the drug-linker or pharmaceutically acceptable salt, ester, stereoisomer, tautomer, polymorph, solvate, *N-*oxide, isotope-labeled compound, metabolite or prodrug thereof according to of claim 10 or 11, the immune-stimulating antibody conjugate according to claim 12, or the pharmaceutical composition according to claim 15 in the manufacture of a medicament for the prevention and/or treatment of a disease (e.g., a tumor) mediated by TLR7 and/or TLR8.

17. A method for preventing and/or treating a disease (e.g., a tumor) mediated by TLR7 and/or TLR8, comprising a step of administering to a subject in need thereof an effective amount of the compound or pharmaceutically acceptable salt, ester, stereoisomer, tautomer, polymorph, solvate, *N-*oxide, isotope-labeled compound, metabolite or prodrug thereof according to any one of claims 1-9, the drug-linker or pharmaceutically acceptable salt, ester, stereoisomer, tautomer, polymorph, solvate, *N-*oxide, isotope-labeled compound, metabolite or prodrug thereof according to of claim 10 or 11, the immune-stimulating antibody conjugate according to claim 12, or the pharmaceutical composition according to claim 15.

18. A kit, comprising the compound or pharmaceutically acceptable salt, ester, stereoisomer, tautomer, polymorph, solvate, *N-*oxide, isotope-labeled compound, metabolite or prodrug thereof according to any one of claims 1-9, the drug-linker or pharmaceutically acceptable salt, ester, stereoisomer, tautomer, polymorph, solvate, *N-*oxide, isotope-labeled compound, metabolite or prodrug thereof according to of claim 10 or 11, the immune-stimulating antibody conjugate according to claim 12, or the pharmaceutical composition according to claim 15.
